# EUROPÄISCHE PATENTSCHRIFT

(11) **EP 1 771 441 B1**
(45) Veröffentlichungstag und Bekanntmachung des Hinweises auf die Patenterteilung: **17.08.2011**
(21) Anmeldenummer: 05715433.8
(22) Anmeldetag: 22.02.2005
(51) Int. Cl.: C07D 403/12, C07D 235/14, C07D 401/12, C07D 413/12, C07D 417/12, C07D 413/14, C07D 471/04, A61K 31/4184, A61K 31/5377, A61K 31/541, A61P 7/02

(54) **NEUE CARBONSÄUREAMIDE ALS FAKTOR XA-INHIBITOREN**
NOVEL CARBOXAMIDES FOR USE AS XA INHIBITORS
NOUVEAUX CARBOXAMIDES COMME INHIBITEURS DU FACTEUR XA

(30) Priorität: 28.02.2004 DE 102004009835; 18.12.2004 DE 102004060984
(43) Veröffentlichungstag der Anmeldung: 11.04.2007
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: GERLACH, Kai, 88400 Biberach (DE); PFAU, Roland, 88400 Biberach (DE); PRIEPKE, Henning, 88447 Warthausen (DE); WIENEN, Wolfgang, 88400 Biberach (DE); SCHULER-METZ, Annette, Maria, 89081 Ulm (DE); DAHMANN, Georg, 88448 Attenweiler (DE); NAR, Herbert, 88416 Ochsenhausen (DE); HANDSCHUH, Sandra, Ruth, 88447 Warthausen (DE); HAUEL, Norbert, 88433 Schemmerhofen (DE); KAUFFMANN-HEFNER, Iris, 88448 Attenweiler (DE)
(74) Vertreter: Hammann, Heinz
(86) Internationale Anmeldenummer: PCT/EP2005/001796
(87) Internationale Veröffentlichungsnummer: WO 2005/082895

(56) Entgegenhaltungen:
- WO-A-01/47896
- WO-A-02/072558
- WO-A-2004/056784
- MEDERSKI, WERNER W. K. R. ET AL: "Halothiophene benzimidazoles as P1 surrogates of inhibitors of blood coagulation factor Xa" BIOORGANIC & MEDICINAL CHEMISTRY LETTERS , 14(14), 3763-3769 CODEN: BMCLE8; ISSN: 0960-894X, 25. Mai 2004 (2004-05-25), XP002330363

## Beschreibung

Gegenstand der vorliegenden Erfindung sind neue substituierte Carbonsäureamide der allgemeinen Formel 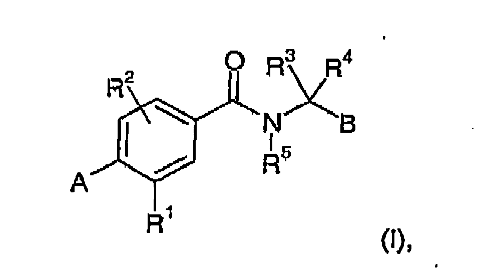 deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel I sowie deren Tautomere, deren Enantiomere, deren Diastereomere, deren Gemische und deren Salze, insbesondere deren physiologisch verträgliche Salze mit anorganischen oder organischen Säuren oder Basen, und deren Stereoisomere weisen wertvolle pharmakologische Eigenschaften auf, insbesondere eine antithrombotische Wirkung und eine Faktor Xa-inhibierende Wirkung.

WO 01/47896 beschreibt Benzimidazole, die wertvolle pharmakologische Eigenschaften aufweisen, insbesondere eine antithrombotische Wirkung.

WO 02/072558 beschreibt Carbonsäureamide, die wertvolle pharmakologische Eigenschaften aufweisen, insbesondere eine antithrombotische Wirkung und eine Faktor Xa-inhibierende Wirkung.

Mederski, Werner W. K. R. et al., Bioorganic & Medicinal Chemistry Letters, 2004, 14(14), Seiten 3763-3769 beschreibt Halothiophene Benzimidazole und deren Faktor Xa-inhibierende Wirkung.

WO 2004/056784 beschreibt Carbonsäureamide, die wertvolle pharmakologische Eigenschaften aufweisen, insbesondere eine antithrombotische Wirkung und eine Faktor Xa-inhibierende Wirkung.

Gegenstand der vorliegenden Anmeldung sind somit die neuen Verbindungen der obigen allgemeinen Formel I, deren Herstellung, die die pharmakologisch wirksamen Verbindungen enthaltenden Arzneimittel, deren Herstellung und Verwendung.

In der obigen allgemeinen Formel bedeuten in einer ersten Ausführungsform

A eine Gruppe der allgemeinen Formel 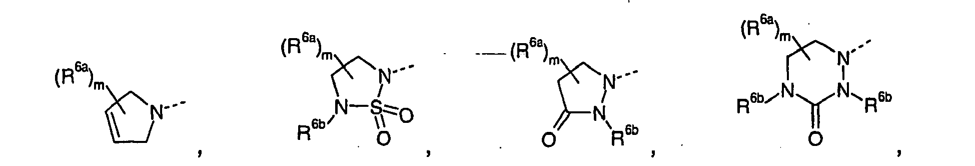 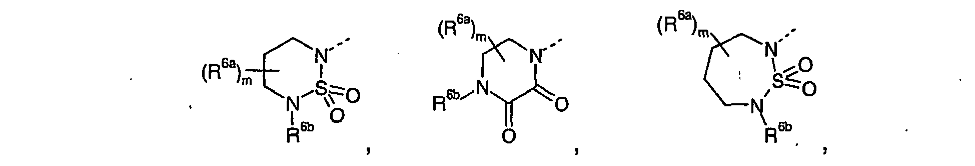 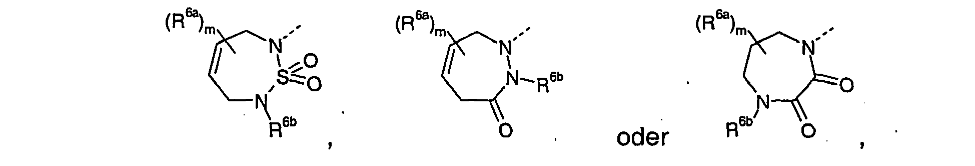 in der
m die Zahl 1 oder 2,
R^{6a} unabhängig voneinander ein Wasserstoff-, Fluor-, Chlor- oder Bromatom oder eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl- oder C₁₋₃-Alkylcarbonylaminogruppe darstellt und
R^{6b} unabhängig voneinander ein Wasserstoffatom, eine C₁₋₄-Alkyl-, C₁₋₄-Alkylcarbonyl-, C₁₋₄-Alkyloxycarbonyl- oder C₁₋₃-Alkylsulfonylgruppe sein können, mit der Maßgabe, dass
bei den voranstehend genannten substituierten 5- bis 7-gliedrigen Gruppen A die gegebenenfalls als Substituenten eingeführten Heteroatome nicht durch genau ein Kohlenstoffatom von einem anderen Heteroatom getrennt sind, oder
eine Gruppe der allgemeinen Formel 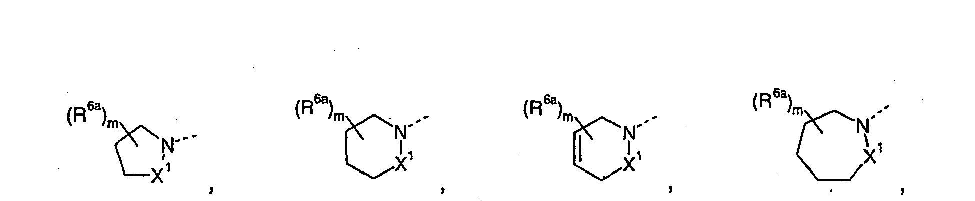 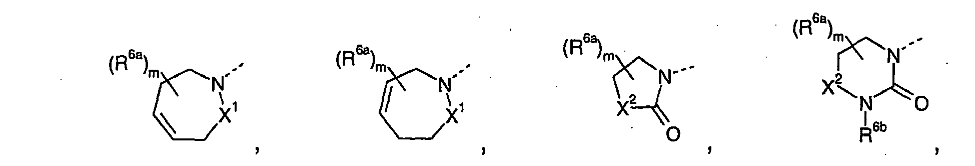 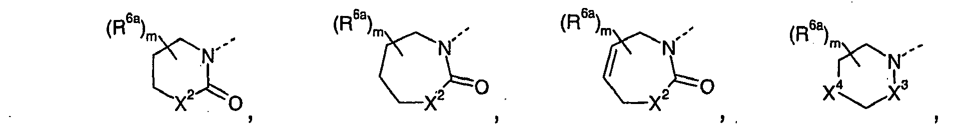 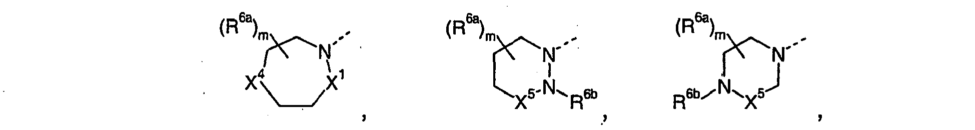 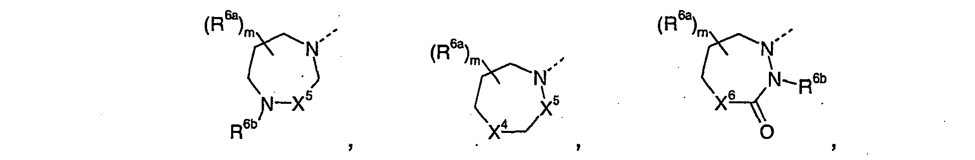 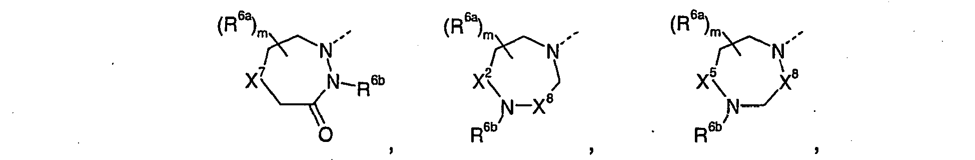 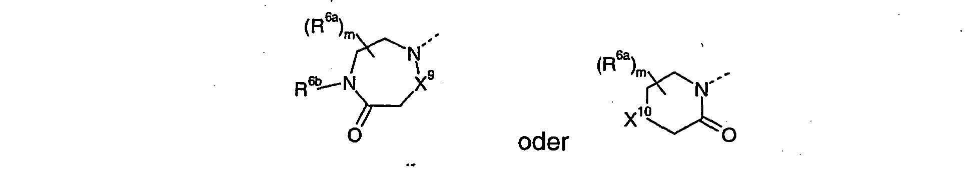 in der
m die Zahl 1 oder 2,
X¹ ein Sauerstoffatom oder eine Methylen-, -NR^{6b}-, Carbonyl- oder Sulfonylgruppe,
X² ein Sauerstoffatom oder eine -NR^{6b}-Gruppe,
X³ eine Methylen-, Carbonyl- oder Sulfonylgruppe,
X⁴ ein Sauerstoff- oder Schwefelatom, eine -NR^{6b}- oder Carbonylgruppe,
X⁵ eine Carbonyl- oder Sulfonylgruppe,
X⁶ ein Sauerstoffatom, eine -NR^{6b}- oder Methylengruppe,
X⁷ ein Sauerstoff- oder Schwefelatom oder eine -NR^{6b}-Gruppe,
X⁸ eine Methylen- oder Carbonylgruppe,
X⁹ eine -NR^{6b}- oder Carbonylgruppe,
X¹⁰ eine Sulfinyl- oder Sulfonylgruppe und
R^{6a} unabhängig voneinander ein Wasserstoff-, Fluor-, Chlor- oder Bromatom oder eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl- oder C₁₋₃-Alkylcarbonylaminogruppe darstellt und
R^{6b} unabhängig voneinander ein Wasserstoffatom, eine d₁₋₄-Alkyl-, C₁₋₄-Alkylcarbonyl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen Gruppen A die gegebenenfalls als Substituenten eingeführten Heteroatome nicht durch genau ein Kohlenstoffatom von einem anderen Heteroatom getrennt sind, bedeuten,
R¹ ein Wasserstoff-, Fluor-, Chlor- oder Bromatom, eine C₁₋₃-Alkylgruppe, in der die Wasserstoffatome ganz oder teilweise durch Fluoratome ersetzt sein können, eine C₂₋₃-Alkenyl-, C₂₋₃-Alkinyl-, Nitro-, Amino-, C₁₋₃-Alkoxy-, eine Mono-, Di- oder Trifluormethoxygruppe,
R² ein Wasserstoff-, Fluor-, Chlor- oder Bromatom oder eine C₁₋₃-Alkylgruppe,
R³ ein Wasserstoffatom, eine C₂₋₃-Alkenyl- oder C₂₋₃-Alkinylgruppe oder eine geradkettige oder verzweigte C₁₋₆-Alkylgruppe, in der die Wasserstoffatome ganz oder teilweise durch Fluoratome ersetzt sein können, und die gegebenenfalls durch eine Nitril-, Hydroxy-, eine C₁₋₅-Alkyloxygruppe, in der die Wasserstoffatome ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁-₃-alkyloxy-, C₁₋₅-Alkyloxycarbonyl-C₁₋₃-alkyloxy-, C₁₋₈-Alkyloxycarbonylamino-, Mercapto-, C₁₋₃-Alkylsulfanyl-, C₁₋₃-Alkylsulfinyl-, C₁₋₃-Alkylsulfonyl-, C₁₋₃-Alkylcarbonylamino-C₁₋₃-alkylsulfanyl-, C₁₋₃-Alkylcarbonylamino-C₁₋₃-alkylsulfinyl-, C₁₋₃-Alkylcarbonylamino-C₁₋₃-alkylsulfonyl-, Carboxy-, C₁₋₃-Alkyloxycarbonyl-, Allyloxycarbonyl-, Propargyloxycarbonyl-, Benzyloxycarbonyl-, Aminocarbonyl-, C₁₋₃-Alkylaminocarbonyl-, Di-(C₁₋₃-alkyl)-aminocarbonyl-, C₃₋₆-Cycloalkyleniminocarbonyl-, Aminosulfonyl-, C₁₋₃-Alkylaminosulfonyl-, Di-(C₁₋₃-alkyl)-aminosulfonyl-, C₃₋₆-Cycloalkyleniminosulfonyl-, Amino-, C₁₋₃-Alkylamino-, Di-(C₁₋₃-alkyl)-amino-, C₁₋₅-Alkylcarbonylamino-, C₁₋₃-Alkyisulfonylamino-, *N*-(C₁₋₃-Alkylsulfonyl)-C₁₋₃-alkylamino-, C₃₋₆-Cycloalkylcarbonylamino-, Aminocarbonylamino-, C₁₋₃-Alkylaminocarbonylamino-, Di-(C₁₋₃-alkyl)-aminocarbonylamino-, eine 4- bis 7-gliedrige Cycloalkyleniminocarbonylamino-, Benzyloxycarbonylamino-, Phenylcarbonylamino- oder Guanidinogruppe substituiert ist,
eine Carboxy-, Aminocarbonyl-, C₁₋₄-Alkylaminocarbonyl-, C₃₋₆-Cycloalkylamino-carbonyl-, Di-(C₁₋₃-alkyl)-aminocarbonyl-, C₁₋₄-Alkoxycarbonyl-, C₄₋₆-Cycloalkyleniminocarbonylgruppe,
eine Phenyl- oder Heteroaryl-, Phenylcarbonyl-C₁₋₃-alkyl-, Phenyl-C₁₋₃-alkyl-oder Heteroaryl-C₁₋₃-alkylgruppe, die im Phenyl- oder Heteroarylteil gegebenenfalls ein- oder mehrfach durch Fluor-, chlor- oder Bromatome, C₁₋₃-Alkyl-, Amino-, C₁₋₃-Alkylamino-, Di-(C₁₋₃-alkyl)-amino-, Hydroxy-, C₁₋₄-Alkyloxy-, Mono-, Di- oder Trifluormethoxy-, Benzyloxy-, Carboxy-C₁₋₃-alkyloxy-, C₁₋₃-Alkyloxycarbonyl-C₁₋₃-alkyloxy-, Aminocarbonyl-C₁₋₃-alkyloxy-, C₁₋₃-Alkylaminocarbonyl-C₁₋₃-alkyloxy-, Di-(C₁₋₃-alkyl)-aminocarbonyl-C₁₋₃-alkyloxy-, eine 4- bis 7-gliedrige Cycloalkyleniminocarbonyl-C₁₋₃-alkoxy-, Carboxy-, C₁₋₃-Alkyloxycarbonyl- oder C₁₋₃-Alkyloxy-carbonylaminogruppe substituiert ist,
eine 3- bis 7-gliedrige Cycloalkyl-, Cycloalkylenimino-, Cycloalkyl-C₁₋₃-alkyl-oder Cycloalkylenimino-C₁₋₃-alkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann und in der zusätzlich eine der einer -NH-, -N(C₁₋₃-Alkylcarbonyl)- oder -N(C₁₋₃-Alkyl)-Gruppe benachbarte Methylengruppe jeweils durch eine Carbonyl- oder Sulfonylgruppe ersetzt sein kann, mit der Maßgabe, dass eine wie oben definierte Cycloalkyleniminogruppe, in der zwei Stickstoffatome durch genau eine -CH₂-Gruppe voneinander getrennt sind, ausgeschlossen ist,
R⁴ ein Wasserstoffatom oder eine C₁₋₃-Alkylgruppe oder
R³ und R⁴ zusammen mit dem Kohlenstoffatom, an dem sie gebunden sind, eine C₃₋₇-Cycloalkylgruppe, wobei
eine der Methylengruppen der C₃₋₇-Cycloalkylgruppe durch eine Imino-, C₁₋₃-Alkylimino-, Acylimino- oder Sulfonyliminogruppe ersetzt sein kann,
R⁵ ein Wasserstoffatom oder eine C₁₋₃-Alkylgruppe,
B eine Gruppe der Formel 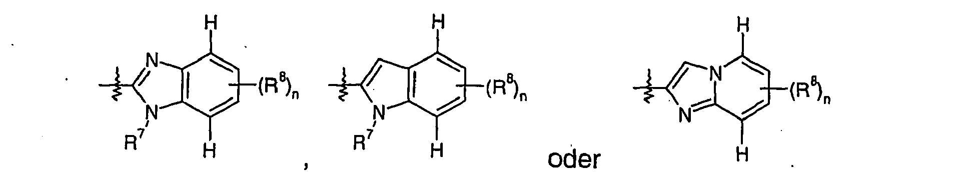 in der
n die Zahl 1 oder 2,
R⁷ ein Wasserstoffatom oder eine C₁₋₃-Alkyl-, Hydroxy-, C₁₋₅-Alkyloxycarbonyl-, Carboxy-C₁₋₃-alkyl-, C₁₋₃-Alkyloxycarbonyl-C₁₋₃-alkylAmino- oder C₁₋₃-Alkylaminogruppe und
R⁸ unabhängig voneinander ein Wasserstoff-, Fluor-, Chlor-, Brom- oder lodatom, eine C₁₋₃-Alkylgruppe, in der die Wasserstoffatome ganz oder teilweise durch Fluoratome ersetzt sein können, eine C₂₋₃-Alkenyl- oder C₂₋₃-Alkinyl-, eine Hydroxy-, C₁₋₃-Alkoxy-, Trifluormethoxy-, Amino-, Nitro-oder Nitrilgruppe darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine 4- bis 7-gliedrige Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Beispiele für monocyclische Heteroarylgruppen sind die Pyridyl-, *N*-Oxy-pyridyl-Pyrazolyl-, Pyridazinyl-, Pyrimidinyl-, Pyrazinyl-, [1,2,3]Triazinyl-, [1,3,5]Triazinyl-, [1,2,4]Triazinyl-, Pyrrolyl-, Imidazolyl-, [1,2,4]Triazolyl-, [1,2,3]Triazolyl-, Tetrazolyl-, Furanyl-, Isoxazolyl-, Oxazolyl-, [1,2,3]Oxadiazolyl-, [1,2,4]Oxadiazolyl-, Furazanyl-, Thiophenyl-, Thiazolyl-, Isothiazolyl-, [1,2,3]Thiadiazolyl-, [1,2,4]Thiadiazolyl- oder [1,2,5]Thiadiazolyl-Gruppe.

Beispiele für bicyclische Heteroarylgruppen sind die Benzimidazolyl, Benzofuranyl-, Benzo[*c*]furanyl-, Benzothiophenyl-, Benzo[*c*]thiophenyl-, Benzothiazolyl-, Benzo[*c*]isothiazolyl-, Benzo[*d*]isothiazolyl-, Benzooxazolyl-, Benzo[*c*]isoxazolyl-, Benzo[*d*]isoxazolyl-, Benzo[1,2,5]oxadiazolyl-, Benzo[1,2,5]thiadiazolyl-, Benzo[1,2,3]thiadiazolyl-, Benzo[*d*][1,2,3]triazinyl-, Benzo[1,2,4]triazinyl-, Benzotriazolyl-, Cinnolinyl-, Chinolinyl-, *N*-Oxy-chinolinyl-, Isochinolinyl-, Chinazolinyl-, *N*-Oxy-chinazolinyl-, Chinoxalinyl-, Phthalazinyl-, Indolyl-, Isoindolyl- oder 1-Oxa-2,3-diaza-indenyl-Gruppe.

Beispiele für die voranstehend in den Definitionen erwähnten C₁₋₈-Alkylgruppen sind die Methyl-, Ethyl-, 1-Propyl-, 2-Propyl-, *n-*Butyl-, *sec-*Butyl-, *tert-*Butyl-, 1-Pentyl-, 2-Pentyl-, 3-Pentyl-, *neo*-Pentyl-, 1-Hexyl-, 2-Hexyl-, 3-Hexyl-, 1-Heptyl-, 2-Heptyl-, 3-Heptyl-, 4-Heptyl-, 1-Octyl-, 2-Octyl-, 3-Octyl- oder 4-Octylgruppe.

Beispiele für die voranstehend in den Definitionen erwähnten C₁₋₈-Alkyloxygruppen sind die Methyloxy-, Ethyloxy-, 1-Propyloxy-, 2-Propyloxy-, *n-*Butyloxy-, *sec-*Butyloxy-, *tert-*Butyloxy-, 1-Pentyloxy-, 2-Pentyloxy-, 3-Pentyloxy-, *neo*-Pentyloxy-, 1-Hexyloxy-, 2-Hexyloxy-, 3-Hexyloxy-, 1-Heptyloxy-, 2-Heptyloxy-, 3-Heptyloxy-, 4-Heptyloxy-, 1-Octyloxy-, 2-Octyloxy-, 3-Octyloxy- oder 4-Octyloxygruppe.

Unter einer *in vivo* in eine Carboxygruppe überführbare Gruppe ist beispielsweise eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, ein C₅₋₇-Cycloalkenol, ein C₃₋₅-Alkeriol, ein Phenyl-C₃₋₅-alkenol, ein C₃₋₅-Alkinol oder Phenyl-C₃₋₅-alkinol mit der Maßgabe, dass keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol oder ein Alkohol der Formel

R⁹-CO-O-(R¹⁰CR¹¹)-OH,

in der
R⁹ eine C₁₋₈-Alkyl-, C₅₋₇-Cycloalkyl-, Phenyl- oder Phenyl-C₁₋₃-alkylgruppe,
R¹⁰ ein Wasserstoffatom, eine C₁₋₃-Alkyl-, C₅₋₇-Cycloalkyl- oder Phenylgruppe und
R¹¹ ein Wasserstoffatom oder eine C₁₋₃-Alkylgruppe darstellen,
zu verstehen.

Als bevorzugte von einer Carboxygruppe *in vivo* abspaltbare Reste kommen eine C₁₋₆-Alkoxygruppe wie die Methoxy-, Ethoxy-, *n-*Propyloxy-, Isopropyloxy-, *n-*Butyloxy-, *n-*Pentyloxy-, *n-*Hexyloxy- oder Cyclohexyloxygruppe oder eine Phenyl-C₁₋₃-alkoxygruppe wie die Benzyloxygruppe in Betracht.

Diejenigen Verbindungen der allgemeinen Formel I, in denen R³ eine *in vivo* in eine Carboxygruppe überführbare Gruppe enthält, stellen Prodrugs für diejenigen Verbindungen der allgemeinen Formel I dar, in denen R³ eine Carboxygruppe enthält.

Eine 2. Ausführungsform der vorliegenden Erfindung umfasst diejenigen Verbindungen der allgemeinen Formel I, in denen

A eine Gruppe der allgemeinen Formel 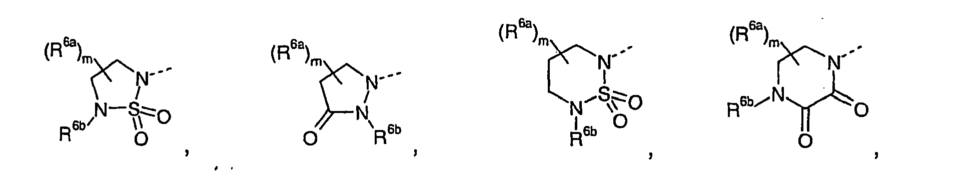 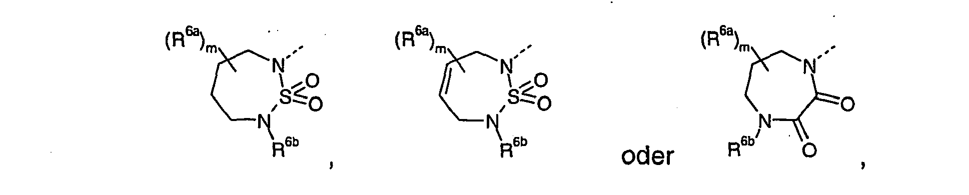 in der m die Zahl 1 oder 2,
R^{6a} unabhängig voneinander ein Wasserstoff- oder Fluoratom, eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl- oder C₁₋₃-Alkylcarbonylaminogruppe darstellt und
R^{6b} unabhängig voneinander ein Wasserstoffatom, eine C₁₋₄-Alkyl-, C₁₋₄-Alkylcarbonyl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen Gruppen A die gegebenenfalls als Substituenten eingeführten Heteroatome nicht durch genau ein Kohlenstoffatom von einem anderen Heteroatom getrennt sind, oder
eine Gruppe der allgemeinen Formel 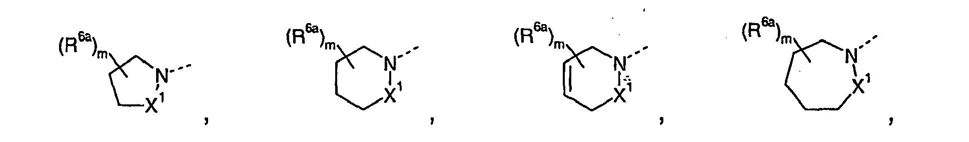 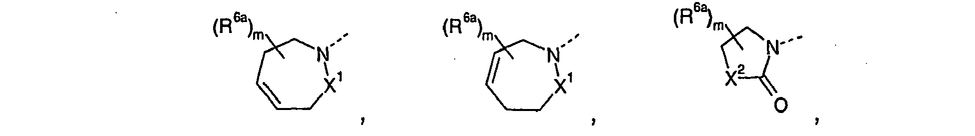 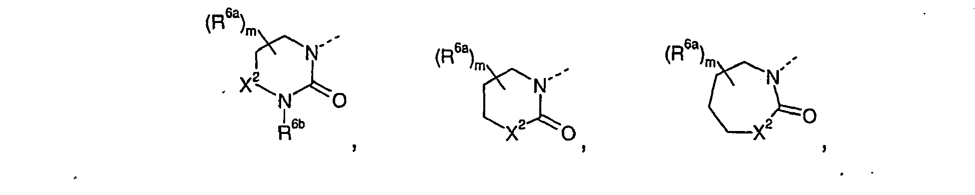 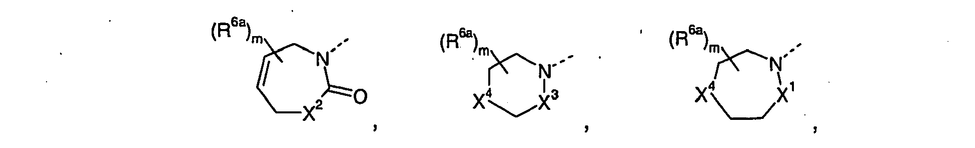 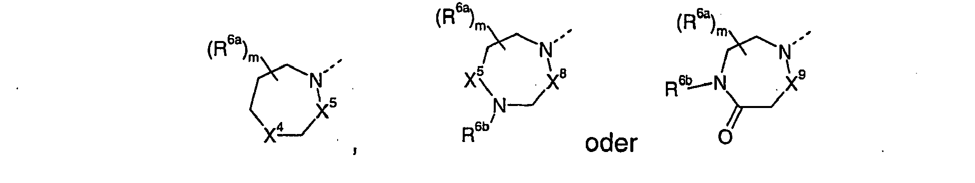 in der
m die Zahl 1 oder 2,
X¹ eine Methylen-, -NR^{6b}-, Carbonyl- oder Sulfonylgruppe,
X² ein Sauerstoffatom oder eine -NR^{6b}-Gruppe,
X³ eine Methylen-, Carbonyl- oder Sulfonylgruppe,
X⁴ ein Sauerstoff- oder Schwefelatom, eine -NR^{6b}- oder Carbonylgruppe,
X⁵ eine Carbonyl- oder Sulfonylgruppe,
X⁸ eine Carbonylgruppe,
X⁹ eine Carbonylgruppe,
R^{6a} unabhängig voneinander ein Wasserstoff- oder Fluoratom, eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl-oder C₁₋₃-Alkylcarbonylaminogruppe darstellt und
R^{6b} unabhängig voneinander ein Wasserstoffatom, eine C₁₋₄-Alkyl-, C₁₋₄-Alkylcarbonyl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen cyclischen Gruppen A die als Substituenten eingeführten Heteroatome nicht durch genau ein Kohlenstoffatom von einem anderen Heteroatom getrennt sind, bedeuten,
R¹ ein Wasserstoff-, Fluor-, Chlor- oder Bromatom, eine C₁₋₃-Alkylgruppe, in der die Wasserstoffatome ganz oder teilweise durch Fluoratome ersetzt sein können, eine C₂₋₃-Alkenyl-, C₂₋₃-Alkinyl-, Nitro-, Amino-, C₁₋₃-Alkoxy-, eine Mono-, Di- oder Trifluormethoxygruppe,
R² ein Wasserstoff-, Fluor-, Chlor- oder Bromatom oder eine C₁₋₃-Alkylgruppe,
R³ eine C₂₋₃-Alkenyl- oder C₂₋₃-Alkinylgruppe oder eine geradkettige oder verzweigte C₁₋₆-Alkylgruppe, in der die Wasserstoffatome ganz oder teilweise durch Fluoratome ersetzt sein können, und die gegebenenfalls durch eine Nitril-, Hydroxy-, eine C₁₋₅-Alkyloxygruppe, in der die Wasserstoffatome ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₃-alkyloxy-, C₁₋₅-Alkyloxycarbonyl-C₁₋₃-alkyloxy-, C₁₋₈-Alkyloxycarbonylamino-, Mercapto-, C₁₋₃-Alkylsulfanyl-, C₁₋₃-Alkylsulfinyl-, C₁₋₃-Alkylsulfonyl-, C₁₋₃-Alkylcarbonylamino-C₁₋₃-alkylsulfanyl-, C₁₋₃-Alkylcarbonylamino-C₁₋₃-alkylsulfinyl-, C₁₋₃-Alkylcarbonylamino-C₁₋₃-alkylsulfonyl-, Carboxy-, C₁₋₃-Alkyloxycarbonyl-, Allyloxycarbonyl-, Propargyloxycarbonyl-, Benzyloxycarbonyl-, Aminocarbonyl-, C₁₋₃-Alkylaminocarbonyl-, Di-(C₁₋₃-alkyl)-aminocarbonyl-, C₃₋₆-Cycloalkyleniminocarbonyl-, Aminosulfonyl-, C₁₋₃-Alkylaminosulfonyl-, Di-(C₁₋₃-alkyl)-aminosulfonyl-, C₃₋₆-Cycloalkyleniminosulfonyl-, Amino-, C₁₋₃-Alkylamino-, Di-(C₁₋₃-alkyl)-amino-, C₁₋₅-Alkylcarbonylamino-, C₁₋₃-Alkylsulfonylamino-, *N*-(C₁₋₃-Alkylsulfonyl)-C₁₋₃-alkylamino-, C₃₋₆-Cycloalkylcarbonylamino-, Aminocarbonylamino-, C₁₋₃-Alkylaminocarbonylamino-, Di-(C₁₋₃-alkyl)-aminocarbonylamino-, eine 4- bis 7-gliedrige Cycloalkyleniminocarbonylamino-, Benzyloxycarbonylamino-, Phenylcarbonylamino- oder Guanidinogruppe substituiert ist,
eine Carboxy-, Aminocarbonyl-, C₁₋₄-Alkylaminocarbonyl-, C₃₋₆-Cycloalkylamino-carbonyl-, Di-(C₁₋₃-alkyl)-aminocarbonyl-, C₁₋₄-Alkoxycarbonyl-, C₄₋₆-Cycloalkyleniminocarbonylgruppe,
eine Phenyl- oder Heteroaryl-, Phenylcarbonyl-C₁₋₃-alkyl-, Phenyl-C₁₋₃-alkyl-oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, Amino-, C₁₋₃-Alkylamino-, Di-(C₁₋₃-alkyl)-amino-, Hydroxy-, C₁₋₄-Alkyloxy-, Mono-, Di- oder Trifluormethoxy-, Benzyloxy-, Carboxy-C₁₋₃-alkyloxy-, C₁₋₃-Alkyloxycarbonyl-C₁₋₃-alkyloxy-, Aminocarbonyl-C₁₋₃-alkyloxy-, C₁₋₃-Alkylaminocarbonyl-C₁₋₃-alkyloxy-, Di-(C₁₋₃-alkyl)-aminocarbonyl-C₁₋₃-alkyloxy-, eine 4- bis 7-gliedrige Cycloalkyleniminocarbonyl-C₁₋₃-alkoxy-, Carboxy-, C₁₋₃-Alkyloxycarbonyl- oder C₁₋₃-Alkyloxy-carbonylaminogruppe substituiert ist,
eine 3- bis 7-gliedrige Cycloalkyl-, Cycloalkylenimino-, Cycloalkyl-C₁₋₃-alkyl-oder Cycloalkylenimino-C₁₋₃-alkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann und in der zusätzlich eine der einer -NH-, -N(C₁₋₃-Alkylcarbonyl)- oder -N(C₁₋₃-Alkyl)-Gruppe benachbarte Methylengruppe jeweils durch eine Carbonyl- oder Sulfonylgruppe ersetzt sein kann, mit der Maßgabe, dass eine wie oben definierte Cycloalkyleniminogruppe, in der zwei Stickstoffatome durch genau eine -CH₂-Gruppe voneinander getrennt sind, ausgeschlossen ist,
R⁴ ein Wasserstoffatom oder eine C₁₋₃-Alkylgruppe,
R⁵ ein Wasserstoffatom oder eine C₁₋₃-Alkylgruppe,
B eine Gruppe der Formel 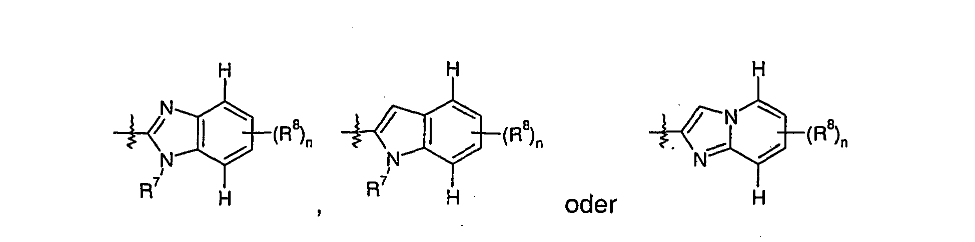 in der
n die Zahl 1 oder 2,
R⁷ ein Wasserstoffatom, eine C₁₋₃-Alkyl-, Hydroxy-, C₁₋₅-Alkyloxycarbonyl-, Carboxy-C₁₋₃-alkyl-, C₁₋₃-Alkyloxycarbonyl-C₁₋₃-alkyl-, Amino- oder C₁₋₃-Alkylaminogruppe und
R⁸ unabhängig voneinander ein Wasserstoff-, Fluor-, Chlor-, Brom- oder lodatom, eine C₁₋₃-Alkylgruppe, in der die Wasserstoffatome ganz oder teilweise durch Fluoratome ersetzt sein können, eine C₂₋₃-Alkenyl- oder C₂₋₃-Alkinyl-, eine Hydroxy-, C₁₋₃-Alkoxy-, Trifluormethoxy-, Amino-, Nitro-oder Nitrilgruppe darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine 4- bis 7-gliedrige Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 3. Ausführungsform der vorliegenden Erfindung umfasst diejenigen Verbindungen der allgemeinen Formel I, in denen

A eine Gruppe der allgemeinen Formel 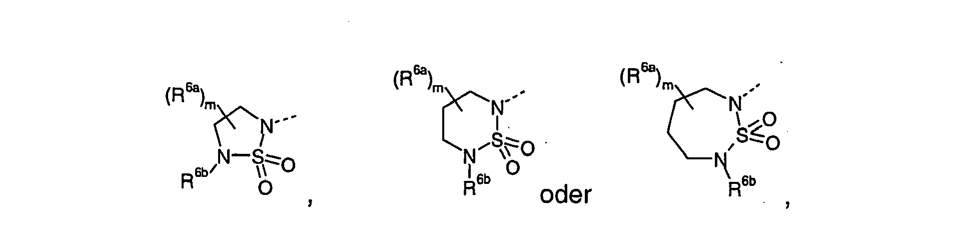 in der m die Zahl 1 oder 2,
R^{6a} unabhängig voneinander ein Wasserstoff- oder Fluoratom, eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl- oder C₁₋₃-Alkylcarbonylaminogruppe, darstellt und
R^{6b} unabhängig voneinander ein Wasserstoffatom, eine C₁₋₄-Alkyl-, C₁₋₄-Alkylcarbonyl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen Gruppen A die gegebenenfalls als Substituenten eingeführten Heteroatome nicht durch genau ein Kohlenstoffatom von einem anderen Heteroatom getrennt sind, oder
eine Gruppe der allgemeinen Formel 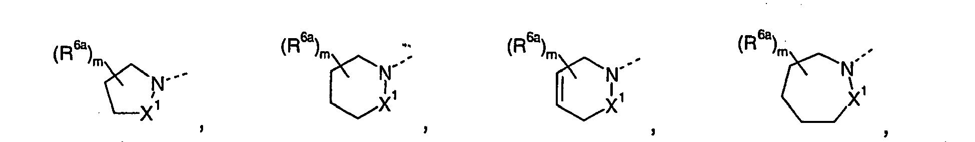 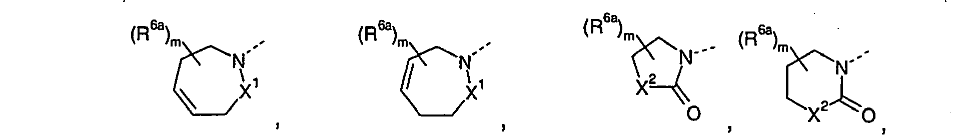 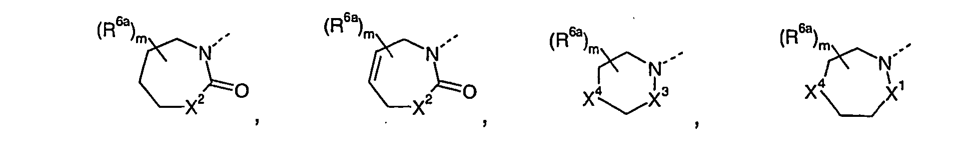 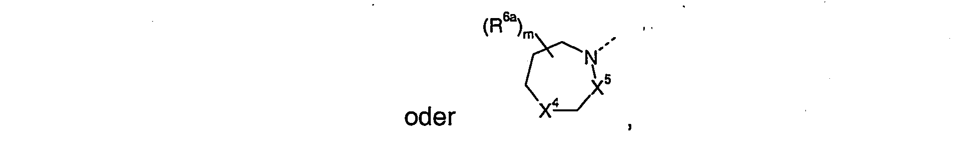 in der
m die Zahl 1 oder 2,
X¹ eine Methylen-, -NR^{6b}-, Carbonyl- oder Sulfonylgruppe,
X² ein Sauerstoffatom oder eine -N^{R6b}-Gruppe,
X³ eine Methylen-, Carbonyl- oder Sulfonylgruppe,
X⁴ ein Sauerstoff- oder Schwefelatom oder eine -NR^{6b}-Gruppe,
X⁵ eine Carbonyl- oder Sulfonylgruppe,
R^{6a} unabhängig voneinander ein Wasserstoff- oder Fluoratom, eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl-oder C₁₋₃-Alkylcarbonylaminogruppe darstellt und
R^{6b} unabhängig voneinander ein Wasserstoffatom, eine C₁₋₄-Alkyl-, C₁₋₄-Alkylcarbonl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen cyclischen Gruppen A die als Substituenten eingeführten Heteroatome nicht durch genau ein Kohlenstoffatom von einem anderen Heteroatom getrennt sind, bedeuten,
R¹ ein Fluor-, Chlor- oder Bromatom, eine C₁₋₃-Alkylgruppe, in der die Wasserstoffatome ganz oder teilweise durch Fluoratome ersetzt sein können, eine Nitro-, C₁₋₃-Alkoxy-, eine Mono-, Di- oder Trifluormethoxygruppe,
R² ein Wasserstoffatom,
R³ eine geradkettige oder verzweigte C₁₋₆-Alkylgruppe in der die Wasserstoffatome ganz oder teilweise durch Fluoratome ersetzt sein können, und die gegebenenfalls durch eine Nitril-, Hydroxy-, Benzyloxy-, eine C₁₋₅-Alkyloxygruppe, in der die Wasserstoffatome ganz oder teilweise durch Fluoratome ersetzt sein können, eine Allyloxy-, C₁₋₅-Alkylcarbonyloxy-, C₁₋₅-Alkyloxycarbonyloxy-, Carboxy-C₁₋₃-alkyloxy-, C₁₋₅-Alkyloxycarbonyl-C₁₋₃-alkyloxy-, C₁₋₈-Alkyloxycarbonylamino-, C₁₋₃-Alkylsulfanyl-, C₁₋₃-Alkylsulfonyl-, Carboxy-, C₁₋₃-Alkyloxycarbonyl-, C₁₋₃-Alkylaminocarbonyl-, Di-(C₁₋₃-alkyl)-aminocarbonyl-, C₃₋₆-Cycloalkyleniminocarbonyl-, Aminocarbonylamino-, C₁₋₃-Alkylaminocarbonylamino- oder Di-(C₁₋₃-alkyl)-aminocarbonylaminogruppe substituiert ist,
eine Aminocarbonyl-, C₁₋₄-Alkylaminocarbonyl-, C₃₋₆-Cycloalkylaminocarbonyl-oder Di-(C₁₋₃-alkyl)-aminocarbonylgruppe,
eine Phenyl- oder Heteroaryl-, Phenyl-C₁₋₃-alkyl- oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, Amino-, C₁₋₃-Alkylamino-, Di-(C₁₋₃-alkyl)-amino-, Hydroxy-, C₁₋₄-Alkyloxy-, Mono-, Di-oder Trifluormethoxy-, Carboxy-, oder C₁₋₃-Alkyloxycarbonylgruppe substituierte ist,
eine 3- bis 7-gliedrige Cycloalkylgruppe, in der im cyclischen Teil eine Methylengruppe durch eine gegebenenfalls durch eine C₁₋₃-Alkyl- oder d₁₋₃-Alkylcarbonylgruppe substituierte -NH-Gruppe oder ein Sauerstoffatom ersetzt sein kann,
R⁴ ein Wasserstoffatom.
R⁵ ein Wasserstoffatom,
B eine Gruppe der Formel 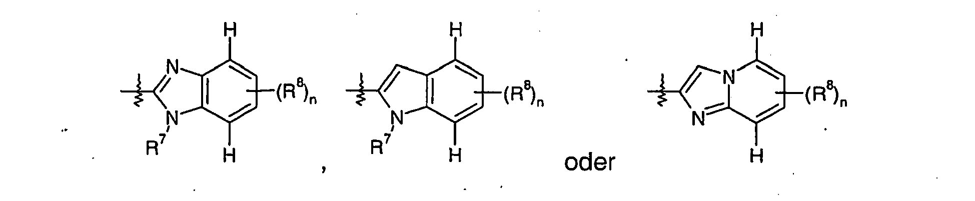 in der
n die Zahl 1,
R⁷ ein Wasserstoffatom und
R⁸ ein Wasserstoff-, Fluor-, Chlor-, Brom- oder lodatom, eine Methyl-, C₂₋₃-Alkinyl-, oder Methoxygruppe, in denen die Wasserstoffatome ganz oder teilweise durch Fluoratome ersetzt sein können, darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe substituierte Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder.
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 4. Ausführungsform der vorliegenden Erfindung umfasst diejenigen Verbindungen der allgemeinen Formel I, in denen
A, R¹, R², R⁴, R⁵ und B wie unter der 3. Ausführungsform beschrieben definiert sind und
R³ eine geradkettige oder verzweigte C₁₋₆-Alkylgruppe, in der die Wasserstoffatome ganz oder teilweise durch Fluoratome ersetzt sein können, und die gegebenenfalls durch eine Nitril-, Hydroxy-, Benzyloxy-, eine C₁₋₅-Alkyloxygruppe, in der die Wasserstoffatome ganz oder teilweise durch Fluoratome ersetzt sein können, eine Allyloxy-, C₁₋₅-Alkylcarbonyloxy-, C₁₋₅-Alkyloxycarbonyloxy-, Carboxy-C₁₋₃-alkyloxy-, C₁₋₅-Alkyloxycarbonyl-C₁₋₃-alkyloxy-, C₁₋₈-Alkyloxycarbonylamino-, C₁₋₃-Alkylsulfanyl-, C₁₋₃-Alkylsulfonyl-, Carboxy-, C₁₋₃-Alkyloxycarbonyl-, C₁₋₃-Alkylaminocarbonyl-, Di-(C₁₋₃-alkyl)-aminocarbonyl-, C₃₋₆-Cycloalkyleniminocarbonyl-, Aminocarbonylamino-, C₁₋₃-Alkylaminocarbonylamino- oder Di-(C₁₋₃-alkyl)-aminocarbonylaminogruppe substituiert ist,
eine Aminocarbonyl-, C₁₋₄-Alkylaminocarbonyl-, C₃₋₆-Cycloalkylaminocarbonyl-oder Di-(C₁₋₃-alkyl)-aminocarbonylgruppe darstellt,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 5. Ausführungsform der vorliegenden Erfindung umfasst diejenigen Verbindungen der allgemeinen Formel I, in denen
A, R¹, R², R⁴, R⁵ und B wie unter der 3. Ausführungsform beschrieben definiert sind und
R³ eine Phenyl- oder Heteroaryl-, Phenyl-C₁₋₃-alkyl- oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, Amino-, C₁₋₃-Alkylamino-, Di-(C₁₋₃-alkyl)-amino-, Hydroxy-, C₁₋₄-Alkyloxy-, Mono-, Di-oder Trifluormethoxy-, Carboxy-, oder C₁₋₃-Alkyloxycarbonylgruppe substituiert ist, eine 3- bis 7-gliedrige Cycloalkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann, darstellt,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 6. Ausführungsform der vorliegenden Erfindung umfasst diejenigen Verbindungen der allgemeinen Formel I, in denen

A eine Gruppe der allgemeinen Formel 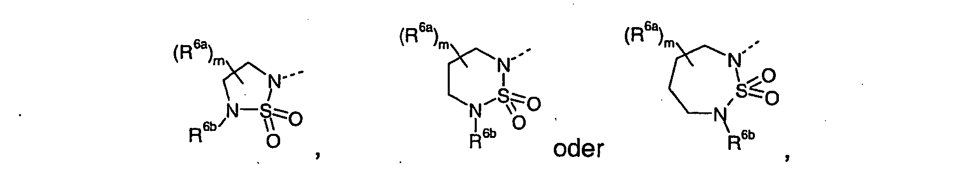 in der m die Zahl 1 oder 2,
R^{6a} unabhängig voneinander ein Wasserstoff- oder Fluoratom oder eine C₁₋₃-Alkylgruppe darstellt und
R^{6b} ein Wasserstoffatom oder eine C₁₋₃-Alkylgruppe sein kann, mit der Maßgabe, dass
bei den voranstehend genannten substituierten 5- bis 7-gliedrigen Gruppen A die gegebenenfalls als Substituenten eingeführten Heteroatome nicht durch genau ein Kohlenstoffatom von einem anderen Heteroatom getrennt sind, oder
eine Gruppe der allgemeinen Formel 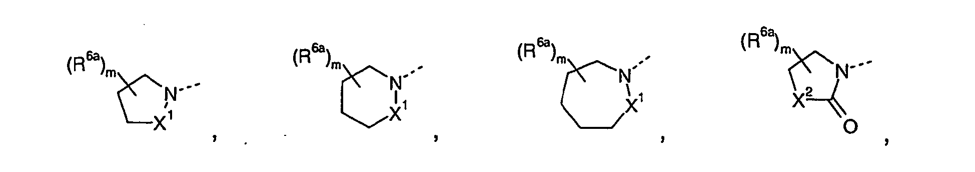 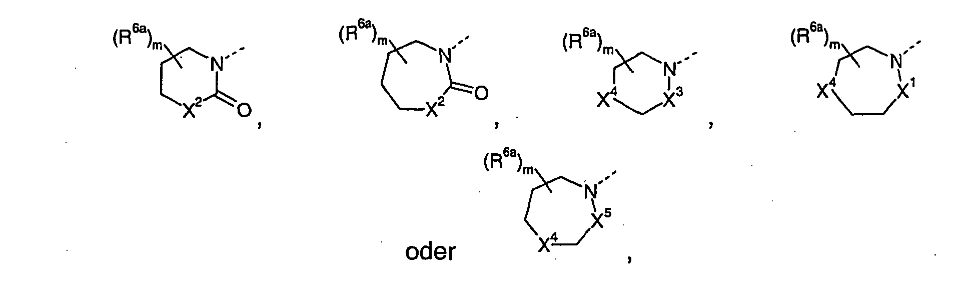 in der
m die Zahl 1 oder 2,
X¹ eine Methylen-, -NR^{6b}-, Carbonyl- oder Sulfonylgruppe,
X² ein Sauerstoffatom oder eine -NR^{6b}-Gruppe,
X³ eine Methylen-, Carbonyl- oder Sulfonylgruppe,
X⁴ ein Sauerstoff- oder Schwefelatom, oder eine -NR^{6b}-Gruppe,
X⁵ eine Carbonyl- oder Sulfonylgruppe,
R^{6a} unabhängig voneinander ein Wasserstoff- oder Fluoratom oder eine C₁₋₃-Alkylgruppe darstellt und
R^{6b} unabhängig voneinander ein Wasserstoffatom oder eine C₁₋₃-Alkylgruppe sein können, mit der Maßgabe, dass
bei den voranstehend genannten substituierten 5- bis 7-gliedrigen cyclischen Gruppen A die als Substituenten eingeführten Heteroatome nicht durch genau ein Kohlenstoffatom von einem anderen Heteroatom getrennt sind, bedeuten,
R¹ ein Chlor- oder Bromatom, eine Methyl- oder Methoxygruppe, in denen die Wasserstoffatome ganz oder teilweise durch Fluoratome ersetzt sein können, oder eine Nitrogruppe,
R² ein Wasserstoffatom,
R³ eine geradkettige oder verzweigte C₁₋₄-Alkylgruppe, in der die Wasserstoffatome ganz oder teilweise durch Fluoratome ersetzt sein können, und die gegebenenfalls durch eine Hydroxy-, eine C₁₋₄-Alkyloxygruppe, in der die Wasserstoffatome ganz oder teilweise durch Fluoratome ersetzt sein können, eine C₁₋₃-Alkylsulfanyl-, C₁₋₃-Alkylsulfonyl-, Carboxy- oder C₁₋₃-Alkyloxycarbonylgruppe substituiert ist,
eine Phenyl- oder Heteroaryl-, Phenyl-C₁₋₃-alkyl- oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, C₁₋₄-Alkyloxy-, Mono-, Di- oder Trifluormethoxy-, Carboxy-, oder C₁₋₃-Alkyloxycarbonylgruppe substituiert ist,
R⁴ ein Wasserstoffatom,
R⁵ ein Wasserstoffatom und
B eine Gruppe der Formel 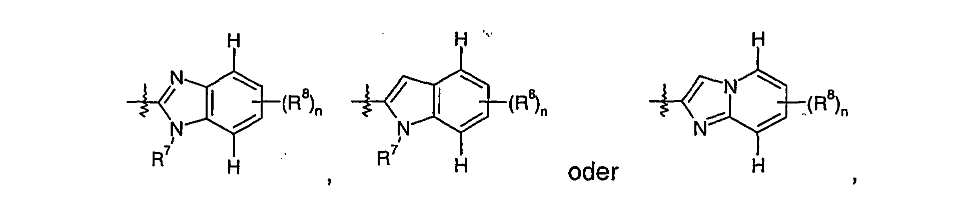 in der
n die Zahl 1,
R⁷ ein Wasserstoffatom und
R⁸ ein Chlor- oder Bromatom oder die Ethinylgruppe darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können, deren Tautomere, deren Enantiomere, deren Diastereomere, deren Gemische und deren Salze.

Eine 7. Ausführungsform der vorliegenden Erfindung umfasst diejenigen Verbindungen der allgemeinen Formel I, in denen
A, R¹, R², R⁴, R⁵ und B wie unter der 6. Ausführungsform beschrieben definiert sind und
R³ eine geradkettige oder verzweigte C₁₋₄-Alkylgruppe, in der die Wasserstoffatome ganz oder teilweise durch Fluoratome ersetzt sein können, und die gegebenenfalls durch eine Hydroxy-, eine C₁₋₄-Alkyloxygruppe, in der die Wasserstoffatome ganz oder teilweise durch Fluoratome ersetzt sein können, eine C₁₋₃-Alkylsulfanyl-, C₁₋₃-Alkylsulfonyl-, Carboxy- oder C₁₋₃-Alkyloxycarbonylgruppe substituiert ist,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 8. Ausführungsform der vorliegenden Erfindung umfasst diejenigen Verbindungen der allgemeinen Formel I, in denen
A, R¹, R², R⁴, R⁵ und B wie unter der 6. Ausführungsform beschrieben definiert sind und
R³ eine Phenyl- oder Heteroaryl-, Phenyl-C₁₋₃-alkyl- oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, C₁₋₄-Alkyloxy-, Mono-, Di- oder Trifluormethoxy-, Carboxy-, oder C₁₋₃-Alkyloxycarbonylgruppe substituiert ist,
eine 3- bis 7-gliedrige Cycloalkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 9. Ausführungsform der vorliegenden Erfindung umfasst diejenigen Verbindungen der allgemeinen Formel I, in denen

A eine Gruppe der Formel 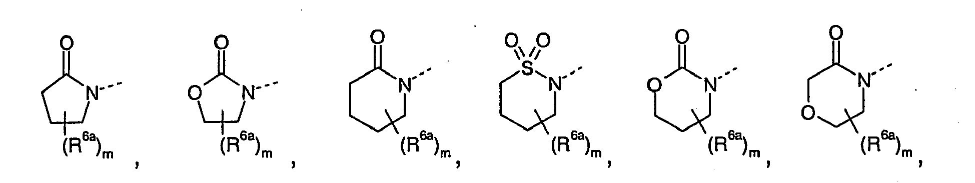 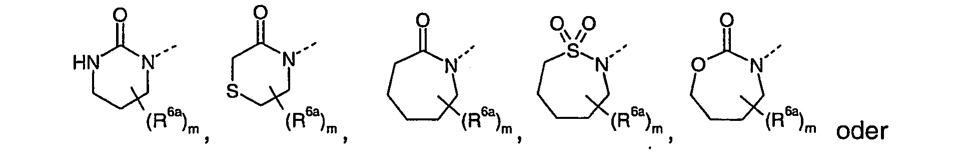 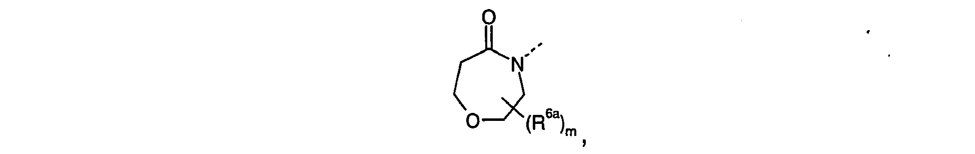 in der
m die Zahl 1 oder 2,
R^{6a} unabhängig voneinander ein Wasserstoff- oder Fluoratom oder eine C₁₋₃-Alkylgruppe darstellt, mit der Maßgabe, dass
bei den voranstehend genannten substituierten 5- bis 7-gliedrigen cyclischen Gruppen A die als Substituenten eingeführten Fluoratome nicht durch genau ein Kohlenstoffatom von einem anderen Heteroatom getrennt sind, bedeuten,
R¹ ein Chlor- oder Bromatom, eine Methyl- oder Methoxygruppe, in denen die Wasserstoffatome ganz oder teilweise durch Fluoratome ersetzt sein können, oder eine Nitrogruppe,
R² ein Wasserstoffatom,
R³ eine geradkettige oder verzweigte C₁₋₄-Alkylgruppe, in der die Wasserstoffatome ganz oder teilweise durch Fluoratome ersetzt sein können, und die gegebenenfalls durch eine Hydroxy-, eine C₁₋₄-Alkyloxygruppe, in der die Wasserstoffatome ganz oder teilweise durch Fluoratome ersetzt sein können, eine C₁₋₃-Alkylsulfanyl-, C₁₋₃-Alkylsulfonyl-, Carboxy- oder C₁₋₃-Alkyloxycarbonylgruppe substituiert ist,
eine Furanyl-, Thiophenyl-, Pyrrolyl-, Pyrazolyl-, Imidazolyl-, Oxazolyl-, Isoxazolyl-, Thiazolyl-, Pyridinyl-, Pyrimidinyl-, Pyrazinyl-, Pyridinyl-C₁₋₂-alkyl-oder Imidazolyl-C₁₋₂-alkylgruppe, die gegebenenfalls im Heteroaryl-Teil mit einer oder zwei C₁₋₃-Alkylgruppen, C₁₋₃-Alkyloxygruppen, Carboxy- oder C₁₋₃-Alkyloxycarbonylgruppen substituiert sein können, bedeutet und
R⁴ ein Wasserstoffatom,
R⁵ ein Wasserstoffatom und
B eine Gruppe der Formel 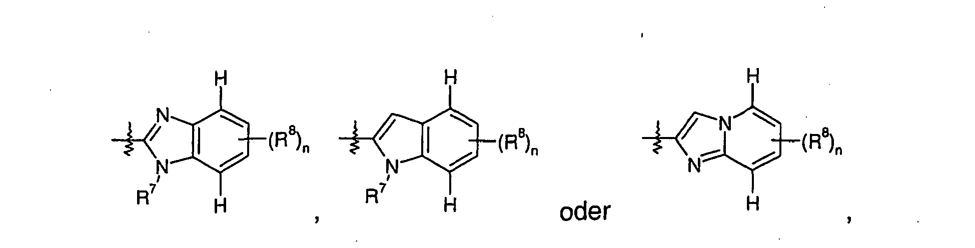 in der
n die Zahl 1,
R⁷ ein Wasserstoffatom und
R⁸ ein Chlor- oder Bromatom oder eine Ethinylgruppe darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 10. Ausführungsform der vorliegenden Erfindung umfasst diejenigen Verbindungen der allgemeinen Formel I, in denen
A, R¹, R², R⁴, R⁵ und B wie unter der 9. Ausführungsform beschrieben definiert sind und
R³ eine geradkettige oder verzweigte C₁₋₄-Alkylgruppe, in der die Wasserstoffatome ganz oder teilweise durch Fluoratome ersetzt sein können, und die gegebenenfalls durch eine Hydroxy-, eine C₁₋₄-Alkyloxygruppe, in der die Wasserstoffatome ganz oder teilweise durch Fluoratome ersetzt sein können, eine C₁₋₃-Alkylsulfanyl-, C₁₋₃-Alkylsulfonyl-, Carboxy- oder C₁₋₃-Alkyloxycarbonylgruppe substituiert ist,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 11. Ausführungsform der vorliegenden Erfindung umfasst diejenigen Verbindungen der allgemeinen Formel I, in denen
A, R¹, R², R⁴, R⁵ und B wie unter der 9. Ausführungsform beschrieben definiert sind und
eine Furanyl-, Thiophenyl-, Pyrrolyl-, Pyrazolyl-, Imidazolyl-, Oxazolyl-, Isoxazolyl-, Thiazolyl-, Pyridinyl-, Pyrimidinyl-, Pyrazinyl-, Pyridinyl-C₁₋₂-alkyl-oder Imidazolyl-C₁₋₂-alkylgruppe, die gegebenenfalls im Heteroaryl-Teil mit einer oder zwei C₁₋₃-Alkylgruppen, bei denen die Wasserstoffatome ganz oder teilweise durch Fluoratome ersetzt sein können, C₁₋₃-Alkyloxygruppen, bei denen die Wasserstoffatome ganz oder teilweise durch Fluoratome ersetzt sein können, Carboxy- oder C₁₋₃-Alkyloxycarbonylgruppen substituiert sein können, bedeutet und
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

Eine 12. Ausführungsform der vorliegenden Erfindung umfasst diejenigen Verbindun-gen der allgemeinen Formel I entsprechend den Ausführungsformen 1, 2, 3, 4, 5, 6, 7 und 8, in denen die Gruppe X¹ eine Methylengruppe darstellt.
Eine 13. Ausführungsform der vorliegenden Erfindung umfasst diejenigen Verbindun-gen der allgemeinen Formel I entsprechend den Ausführungsformen 1, 2, 3, 4, 5, 6, 7 und 8, in denen die Gruppe X¹ eine Carbonylgruppe darstellt.
Eine 14. Ausführungsform der vorliegenden Erfindung umfasst diejenigen Verbindun-gen der allgemeinen Formel I entsprechend den Ausführungsformen 1, 2, 3, 4, 5, 6, 7, 8, 12 und 13, in denen die Gruppe X³ eine Methylengruppe darstellt.

Eine 15. Ausführungsform der vorliegenden Erfindung umfasst diejenigen Verbindun-gen der allgemeinen Formel I entsprechend den Ausführungsformen 1, 2, 3, 4, 5, 6, 7, 8, 12 und 13 in denen die Gruppe X³ eine Carbonylgruppe darstellt.

Eine 16. Ausführungsform der vorliegenden Erfindung umfasst diejenigen Verbindun-gen der allgemeinen Formel I entsprechend den Ausführungsformen 1, 2, 3, 4, 5, 6, 7, 8, 12, 13, 14 und 15, in denen die Gruppe X⁴ ein Sauerstoffatom darstellt.

Eine 17. Ausführungsform der vorliegenden Erfindung umfasst diejenigen Verbindun-gen der allgemeinen Formel I entsprechend den Ausführungsformen 1, 2, 3, 4, 5, 6, 7, 8, 9, 10, 11, 12, 13, 14, 15 und 16, in denen der Rest B die Gruppe 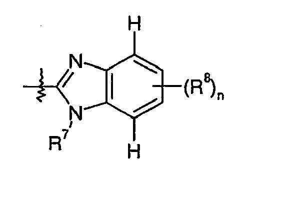 bedeutet.

Eine 18. Ausführungsform der vorliegenden Erfindung umfasst diejenigen Verbindun-gen der allgemeinen Formel I entsprechend den Ausführungsformen 1, 2, 3, 4, 5, 6, 7, 8, 9, 10, 11, 12, 13, 14, 15 und 16, in denen der Rest B die Gruppe 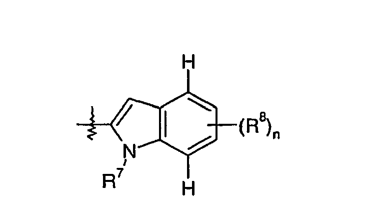 bedeutet.

Eine 19. Ausführungsform der vorliegenden Erfindung umfasst diejenigen Verbindun-gen der allgemeinen Formel I entsprechend den Ausführungsformen 1, 2, 3, 4, 5, 6, 7, 8, 9, 10, 11, 12, 13, 14, 15 und 16, in denen der Rest B die Gruppe 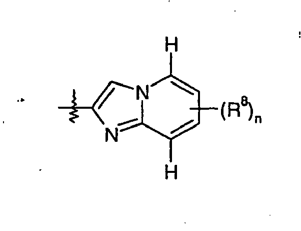 bedeutet.

Eine 20. Ausführungsform der vorliegenden Erfindung umfasst diejenigen Verbindun-gen der allgemeinen Formel I entsprechend den Ausführungsformen 1, 2, 3, 4, 5, 6, 7, 8, 9, 10, 11, 12, 13, 14, 15, 16, 17, 18 und 19, in denen der Rest R⁸ ein Chloratom darstellt.

Eine 21. Ausführungsform der vorliegenden Erfindung umfasst diejenigen Verbindun-gen der allgemeinen Formel I entsprechend den Ausführungsformen 1, 2, 3, 4, 5, 6, 7, 8, 9, 10, 11, 12, 13, 14, 15, 16, 17, 18 und 19, in denen der Rest R⁸ ein Bromatom darstellt.

Eine 22. Ausführungsform der vorliegenden Erfindung umfasst diejenigen Verbindun-gen der allgemeinen Formel I entsprechend den Ausführungsformen 1, 2, 3, 4, 5, 6, 7, 8, 9, 10, 11, 12, 13, 14, 15, 16, 17, 18 und 19, in denen der Rest R⁸ eine Ethinylgruppe darstellt.

Eine 23. Ausführungsform der vorliegenden Erfindung umfasst diejenigen Verbindun-gen der allgemeinen Formel I entsprechend den Ausführungsformen 1, 2, 3, 4, 5, 6, 7, 8, 9, 10, 11, 12, 13, 14, 15, 16, 17, 18, 19, 20, 21 und 22, die der allgemeinen Formel Ia 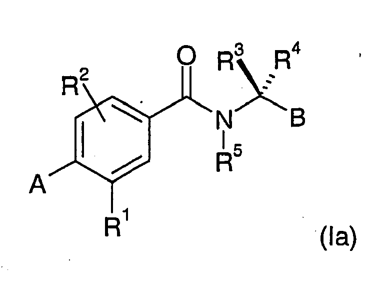 entsprechen.

Beispielsweise seien folgende bevorzugte Verbindungen der allgemeinen Formel I erwähnt:
- (1): 3-Chlor-*N*-[(1*S*)-1-(5-chlor-1*H*-benzimidazol-2-yl)-ethyl]-4-([1,4]diazepan- 1-yl)-benzamid,
- (2): 4-(4-*N*-Boc-piperazin-1-yl)-*N*-[(1*S*)-1-(5-chlor-1*H*-benzimidazol-2-yl)- ethyl]-3-trifluormethyl-benzamid,
- (3): *N*-[(1*S*)-1-(5-Chlor-1*H*-benzimidazol-2-yl)-ethyl]-4-(piperazin-1-yl)-3- trifluormethyl-benzamid,
- (4): *N*-[(1*R*)-1-(5-Chlor-1*H*-benzimidazol-2-yl)-2-methoxy-ethyl)-4-(piperazin- 1-yl)-3-trifluormethyl-benzamid,
- (5): 4-(4-*N*-Acetyl-piperazin-1-yl)-*N*-[(1*S*)-1-(5-chlor-1*H*-benzimidazol-2-yl)- ethyl]-3-trifluormethyl-benzamid,
- (6): 4-(Azepan-2-on-1-yl)-*N*-[(1*S*)-1-(5-chlor-1*H*-benzimidazol-2-yl)-ethyl]-3- methyl-benzamid,
- (7): 4-(Azepan-2-on-1-yl)-*N*-[(1*R*)-1-(5-chlor-1*H*-benzimidazol-2-yl)-2- methoxy-ethyl]-3-methyl-benzamid,
- (8): 4-(Azepan-2-on-1-yl)-*N*-[(1*R*)-1-(5-chlor-1*H*-benzimidazol-2-yl)-2-hydroxy-ethyl]-3-methyl-benzamid,
- (9): *N*-[(1*S*)-1-(5-Chlor-1*H*-benzimidazol-2-yl)-ethyl]-4-(pyrrolidin-2-on-1-yl)- benzamid,
- (10): *N*-[(1*S*)-1-(5-Chlor-1*H*-benzimidazol-2-yl)-ethyl]-3-methyl-4-(pyrrolidin-2- on-1-yl)-benzamid,
- (11): *N*-[(1*S*)-1-(5-Chlor-1*H*-benzimidazol-2-yl)-3-methylsulfanyl-propyl]-3- methyl-4-(pyrrolidin-2-on-1-yl)-benzamid,
- (12): 3-Chlor-*N*-[(1*S*)-1-(5-chlor-1*H*-benzimidazol-2-yl)-ethyl]-4-(4-*N*-methyl- [1,4]diazepan-1-yl)-benzamid,
- (13): 3-Chlor-*N*-[(1*S*)-1-(5-chlor-1*H*-benzimidazol-2-yl)-ethyl]-4-(2-methyl- pyrrolidin-1-yl)-benzamid,
- (14): *N*-[(1*S*)-1-(5-Chlor-1*H*-benzimidazol-2-yl)-ethyl]-3-chlor-4-(morpholin-1- yl)-benzamid,
- (15): *N*-[(1*S*)-1-(5-Chlor-1*H*-benzimidazol-2-yl)-ethyl]-3,5-difluor-4-(morpholin- 1-yl)-benzamid,
- (16): *N*-[(1*R*)-1-(5-Chlor-1*H*-benzimidazol-2-yl)-2-methoxy-ethyl]-3- trifluormethyl-4-(morpholin-1-yl)-benzamid,
- (17): 4-(Azepan-1-yl)-*N*-[(1*S*)-1-(5-chlor-1*H*-benzimidazol-2-yl)-ethyl]-3- trifluormethyl-benzamid,
- (18): 4-(Azepan-1-yl)-3-chlor-*N*-[(1*S*)-1-(5-chlor-1*H*-benzimidazol-2-yl)-ethyl]- benzamid, (19) 3-Chlor-*N*-[(1*S*)-1-(5-chlor-1*H*-benzimidazol-2-yl)-ethyl]-4-(piperidin-1-yl)- benzamid,
- (20): 3-Chlor-*N*-[(1*S*)-1-(5-chlor-1*H*-benzimidazol-2-yl)-ethyl]-4-([1,4]oxazepan- 4-yl)-benzamid,
- (21): *N*-[(1*R*)-1-(5-Chlor-1*H*-benzimidazol-2-yl)-2-methoxy-ethyl]-4-(morpholin- 3-on-4-yl)-3-trifluormethyl-benzamid,
- (22): *N*-[(1*R*)-1-(5-Chlor-1*H*-benzimidazol-2-yl)-2-methoxy-ethyl]-3-methyl-4- (morpholin-3-on-4-yl)-benzamid,
- (23): *N*-[(1*S*)-1-(5-Chlor-1*H*-benzimidazol-2-yl)-3-methylsulfanyl-propyl]-3- methyl-4-(morpholin-3-on-4-yl)-benzamid,
- (24): *N*-[(1*R*)-1-(5-Chlor-1*H*-benzimidazol-2-yl)-2-hydroxy-ethyl]-3-methyl-4- (morpholin-3-on-4-yl)-benzamid,
- (25): 4-(Azepan-2-on-1-yl)-3-chlor-*N*-[(1*R*)-1-(5-chlor-1*H*-benzimidazol-2-yl)-2- methoxy-ethyl]-benzamid,
- (26): 4-(Azepan-2-on-1-yl)-*N*-[(1*R*)-1-(5-chlor-1*H*-benzimidazol-2-yl)-2- methoxy-ethyl]-3-trifluormethyl-benzamid,
- (27): *N*-[(1*S*)-1-(5-Chlor-1*H*-benzimidazol-2-yl)-ethyl]-4-(morpholin-3-on-4-yl)- benzamid,
- (28): *N*-[(1*S*)-1-(5-Chlor-1*H*-benzimidazol-2-yl)-ethyl]-3-methyl-4-(morpholin-3- on-4-yl)-benzamid,
- (29): *N*-[(1*S*)-1-(5-Chlor-1*H*-benzimidazol-2-yl)-ethyl]-4-(morpholin-3-on-4-yl)- benzamid,
- (30): *N*-[(1*S*)-1-(5-Chlor-1*H*-benzimidazol-2-yl)-ethyl]-3-methyl-4-(piperazin-1- yl)-benzamid,
- (31): *N-*[(1*S*)-1-(5-Chlor-1*H-*benzimidazol-2-yl)-ethyl]-3-methyl-4-(piperazin-2- on-1-yl)-benzamid,
- (32): *N-*[(1*S*)-1-(5-Chlor-1*H-*benzimidazol-2-yl)-ethyl]-3-methyl-4-(piperidin-2- on-1-yl)-benzamid,
- (33): *N-*[(1*S*)-1-(5-Chlor-1*H*-benzimidazol-2-yl)-ethyl]-4-(*N-*methyl-piperazin-1- yl)-3-trifluormethyl-benzamid,
- (34): *N-*[(1*S*)-1-(5-Chlor-1*H-*benzimidazol-2-yl)-3-methylsulfonyl-propyl]-3- methyl-4-(morpholin-3-on-4-yl)-benzamid,
- (35): *N-*[(1*S*)-1-(5-Chlor-1*H-*benzimidazol-2-yl)-3-methylsulfanyl-propyl]-4- (pyrrolidin-2-on-1-yl)-benzamid,
- (36): *N-*[1-(5-Chlor-1*H-*benzimidazol-2-yl)-1-phenyl-methyl)-4-(pyrrolidin-2-on- 1-yl)-benzamid,
- (37): *N-*[(1*S*)-1-(5-Chlor-1*H-*benzimidazol-2-yl)-ethyl]-4-(3,5-dimethylpiperidin- 1-yl)-benzamid,
- (38): 3-Chlor-*N-*[(1*S*)-1-(5-chlor-1*H-*benzimidazol-2-yl)-ethyl]-4-(3,4-didehydro- piperidin-1-yl)-benzamid;
- (39): *N-*[(1*S*)-1-(5-Chlor-1*H-*benzimidazol-2-yl)-ethyl]-3-methyl-4- ([1,3]oxazepan-2-on-3-yl)-benzamid
- (40): *N-*[(1*S*)-1-(5-Chlor-1*H-*benzimidazol-2-yl)-3-methylsulfanyl-propyl)-4- (piperazin-1-yl)-3-trifluormethyl-benzamid,
- (41): *N-*[(1*S*)-1-(5-Chlor-1*H-*benzimidazol-2-yl)-3-methylsulfanyl-propyl]-4- (morpholin-3-on-4-yl)-3-nitro-benzamid,
- (42): 3-Chlor-*N-*[(1*S*)-1-(5-ch)or-1*H-*benzimidazol-2-yl)-ethyl]-4-(tetrahydro- pyrimidin-2-on-1-yl)-benzamid,
- (43): *N-*[(1*R*)-1-(5-Chlor-1*H-*benzimidazol-2-yl)-2-methoxy-ethyl]-3-methyl-4- ([1,3]oxazepan-2-on-3-yl)-benzamid,
- (44): 3-Chlor-*N-*[(1*R*)-1-(5-chlor-1*H-*benzimidazol-2-yl)-2-methoxy-ethyl]-4- ([1,4]oxazepan-5-on-4-yl)-benzamid,
- (45): 3-Chlor-*N-*[(1*R*)-1-(5-chlor-1*H-*benzimidazol-2-yl)-2-methoxy-ethyl]-4- ([1,4]oxazepan-3-on-4-yl)-benzamid,
- (46): 3-Chlor-*N-*[(1*R*)-1-(5-chlor-1*H-*benzimidazol-2-yl)-2-methoxy-ethyl]-4- (piperidin-2-on-1-yl)-benzamid,
- (47): *N-*[(1*R*)-1-(5-Brom-1*H-*benzimidazol-2-yl)-2-hydroxy-ethyl]-3-methyl-4- (morpholin-3-on-4-yl)-benzamid,
- (48): *N-*[(1*S*)-1-(5-Chlor-1*H-*benzimidazol-2-yl)-ethyl]-4-(1,1-dioxo- isothiazolidin-2-yl)-3-methyl-benzamid,
- (49): *N-*[1-(5-Chlor-1*H-*indol-2-yl)-ethyl]-3-methyl-4-(morpholin-3-on-4-yl)- benzamid,
- (50): 3-Chlor-*N-*[(1*S*)-1-(5-chlor-1*H-*benzimidazol-2-yl)-ethyl]-4-[2-(4-methyl- piperazin-1-yl-methyl)-piperidin-1-yl)-benzamid,
- (51): *N-*[(1*S*)-1-(5-Chlor-1*H-*benzimidazol-2-yl)-ethyl]-3-methyl-4-(oxazolidin-2- on-3-yl)-benzamid,
- (52): 3-Chlor-*N-*[(1*S*)-1-(5-chlor-1*H-*benzimidazol-2-yl)-ethyl]-4-(morpholin-3- on-4-yl)-benzamid,
- (53): *N-*[(1*R*,2*S*)-1-(5-Chlor-1*H-*benzimidazol-2-yl)-2-methoxy-propyl]-3- methyl-4-(morpholin-3-on-4-yl)-benzamid,
- (54): *N-*[(1*S*)-1-(5-Chlor-1*H-*benzimidazol-2-yl)-ethyl]-4-(3,5-dimethylpiperidin- 1-yl)-benzamid,
- (55): *N-*[(1*S*)-1-(5-Chlor-1*H-*benzimidazol-2-yl)-ethyl]-3-methyl-4- (thiomorpholin-3-on-4-yl)-benzamid,
- (56): *N-*[(1*R*)-1-(5-Chlor-1*H-*benzimidazol-2-yl)-2-methoxy-ethyl]-3-methyl-4- (piperidin-2-on-1-yl)-benzamid,
- (57): *N-*[(1*S*)-1-(5-Chlor-1*H-*benzimidazol-2-yl)-ethyl]-3-methyl-4-([1,3]- oxazinan-2-on-3-yl)-benzamid,
- (58): *N-*[(1*R*)-1-(5-Chlor-1*H-*benzimidazol-2-yl)-2-methoxy-ethyl]-3-methyl-4- ([1,3]-oxazinan-2-on-3-yl)-benzamid,
- (59): *N-*[(1*R*)-1-(5-Chlor-1*H-*benzimidazol-2-yl)-2-methoxy-ethyl]-4-(piperidin-2- on-1-yl)-3-trifluormethyl-benzamid,
- (60): *N-*[(1*R*)-1-(5-Chlor-1*H-*benzimidazol-2-yl)-2-methoxy-ethyl]-4-(1,1-dioxo- [1,2]thiazinan-2-yl)-3-methyl-benzamid,
- (61): *N-*[(1*S*)-1-(5-Chlor-1*H-*benzimidazol-2-yl)-ethyl]-3-methyl-4-(5,6- didehydro-azepan-2-on-1-yl)-benzamid,
- (62): 4-(Azepan-2-on-1-yl)-*N-*[(1*S*)-1-(5-chlor-6-fluor-1*H-*benzimidazol-2-yl)- ethyl]-3-methyl-benzamid,
- (63): *N-*[(1*S*)-1-(5-Chlor-1*H-*benzimidazol-2-yl)-ethyl]-4-(1,3-dioxo- thiomorpholin-4-yl)-3-methyl-benzamid,
- (64): *N-*[(1*S*)-1-(5-Chlor-1*H-*benzimidazol-2-yl)-ethyl]-3-methyl-4-(1,1,3-trioxo- thiomorpholin-4-yl)-benzamid,
- (65): *N-*[(1*R*)-1-(5-Chlor-1*H-*benzimidazol-2-yl)-2-methoxy-ethyl]-3-methyl-4- (piperazin-1-yl)-benzamid,
- (66): *N-*[(1*R*)-1-(5-Chlor-1*H-*benzimidazol-2-yl)-2-methoxy-ethyl]-3-methyl-4- (piperazin-2-on-1-yl)-benzamid,
- (67): *N-*[(1*S*)-1-(5-Chlor-1*H-*benzimidazol-2-yl)-butyl]-3-methyl-4-(morpholin-3- on-4-yl)-benzamid,
- (68): *N-*[(1*R*)-1-(5-Chlor-1*H-*benzimidazol-2-yl)-2-methoxy-ethyl]-4- ([1,3]oxazepan-2-on-3-yl)-3-trifluormethyl-benzamid,
- (69): *N-*[(1*S*)-1-(5-Chlor-1*H-*benzimidazol-2-yl)-ethyl]-3-methoxy-4-(piperidin-2- on-1-yl)-benzamid,
- (70): *N-*[(1*R*)-1-(5-Chlor-1*H-*benzimidazol-2-yl)-2-methoxy-ethyl]-3-methoxy-4- (piperidin-2-on-1-yl)-benzamid,
- (71): *N-*[(1*R*)-1-(5-Chlor-1*H-*benzimidazol-2-yl)-2-methoxy-ethyl]-4-(1,1-dioxo- [1,2,6]thiadiazinan-2-yl)-3-methyl-benzamid,
- (72): *N-*[(1*S*)-1-(5-Chlor-1*H-*benzimidazol-2-yl)-ethyl]-4-(1,1-dioxo- [1,2,6]thiadiazinan-2-yl)-3-methyl-benzamid,
- (73): *N*-[(1*S*)-1-(5-Chlor-1*H-*benzimidazol-2-yl)-ethyl]-4-(1,1-dioxo-6-methyl- [1,2,6]thiadiazinan-2-yl)-3-methyl-benzamid,
- (74): *N-*[(1*R*)-1-(5-Chlor-1*H-*benzimidazol-2-yl)-2-methoxy-ethyl]-4-(1,1-dioxo- 3-methyl-[1,2,6]thiadiazinan-2-yl)-3-methyl-benzamid,
- (75): *N-*[(1*R*)-1-(5-Brom-1*H-*benzimidazol-2-yl)-2-methoxy-ethyl]-3-methyl-4- (morpholin-3-on-4-yl)-benzamid,
- (76): *N-*[(1*S*)-1-(5-Chlor-1*H-*benzimidazol-2-yl)-3-(1*H-*tetrazol-5-yl)-propyl]-3- methyl-4-(morpholin-3-on-4-yl)-benzamid,
- (77): *N-*[(1*S*)-1-(5-Chlor-1*H-*benzimidazol-2-yl)-3-methoxy-propyl]-3-methyl-4- (morpholin-3-on-4-yl)-benzamid,
- (78): *N-*[1-(5-Chlor-1*H-*benzimidazol-2-yl)-thiophen-3-yl-methyl]-3-methyl-4- (morpholin-3-on-4-yl)-benzamid,
- (79): *N-*[(1*R*)-1-(5-Chlor-1*H-*benzimidazol-2-yl)-2-methylsulfanyl-ethyl]-3- methyl-4-(morpholin-3-on-4-yl)-benzamid,
- (80): *N-*[(1*R*)-1-(5-Chlor-1*H-*benzimidazol-2-yl)-2-methoxy-ethyl]-3-methyl-4- (thiomorpholin-3-on-4-yl)-benzamid,
- (81): 3-Chlor-*N-*[(1*R*)-1-(5-chlor-1*H-*benzimidazol-2-yl)-2-methoxy-ethyl]-4- (morpholin-3-on-4-yl)-benzamid,
- (82): 3-Chlor-*N-*[(1*S*)-1-(5-chlor-1*H-*benzimidazol-2-yl)-ethyl]-4-(3,6-dihydro- [1,2]oxazin-2-yl)-benzamid,
- (83): 3-Chlor-*N-*[(1*R*)-1-(5-chlor-1*H-*benzimidazol-2-yl)-2-methoxy-ethyl]-4- (1,1-dioxo-[1,2]thiazinan-2-yl)-benzamid,
- (84): *N-*[(1*R*)-1-(5-Chlor-1*H-*benzimidazol-2-yl)-2-methoxy-ethyl]-4-(1,1-dioxo- [1,2]thiazepan-2-yl)-3-methyl-benzamid,
- (85): *N-*[(1*S*)-1-(5-Chlor-1*H-*benzimidazol-2-yl)-ethyl]-4-(piperidin-2-on-1-yl)-3- trifluormethoxy-benzamid,
- (87): 3-Brom-*N-*[(1*R*)-1-(5-chlor-1*H-*benzimidazol-2-yl)-2-methoxy-ethyl]-4- (piperidin-2-on-1-yl)-benzamid,
- (88): *N-*[1-(5-Chlor-1*H-*benzimidazol-2-yl)-1-(furan-2-yl)-methyl]-3-methyl-4- (piperidin-2-on-1-yl)-benzamid,
- (89): 3-Chlor-*N-*[(1*S*)-1-(5-chlor-1*H-*benzimidazol-2-yl)-ethyl]-4-([1,2]oxazinan- 2-yl)-benzamid,
- (90): *N-*[(1*R*)-1-(5-Chlor-1*H-*benzimidazol-2-yl)-2-methoxy-ethyl]-3-methyl-4- (5-oxo-[1,4]oxazepan-4-yl)-benzamid,
- (91): *N-*[(1*S*)-1-(5-Chlor-1*H-*benzimidazo)-2-yl)-ethyl]-4-(4,4-dimethyl-2-oxo- imidazolidin-1-yl)-3-methyl-benzamid,
- (92): *N-*[(1*R*)-1-(5-Chlor-1*H-*benzimidazol-2-yl)-2-methoxy-ethyl]-4-(4,4- dimethyl-2-oxo-imidazolidin-1-yl)-3-methyl-benzamid,
- (93): 3-Chlor-*N-*[(1*S*)-1-(5-chlor-1*H-*benzimidazol-2-yl)-ethyl]-4-(4-methyl-2- oxo-oxazolidin-3-yl)-benzamid,
- (94): 3-Chlor-*N-*[(1*R*)-1-(5-chlor-1*H-*benzimidazol-2-yl)-2-methoxy-ethyl]-4-(4- methyl-2-oxo-oxazolidin-3-yl)-benzamid,
- (95): *N-*[1-(5-Chlor-1*H-*benzimidazol-2-yl)-1-phenyl-methyl]-3-methyl-4- (morpholin-3-on-4-yl)-benzamid,
- (96): *N-*[(15)-1-(5-Chlor-1*H-*benzimidazol-2-yl)-propyl]-3-methyl-4-(morpholin- 3-on-4-yl)-benzamid,
- (97): 3-Brom-*N-*[(1*R*)-1-(5-chlor-1*H-*benzimidazol-2-yl)-2-methoxy-ethyl]-4- (morpholin-3-on-4-yl)-benzamid,
- (98): *N-*[(1*S*)-1-(5-Chlor-1*H-*benzimidazol-2-yl)-ethyl]-3-methyl-4-(7-oxo- [1,4]diazepan-1-yl)-benzamid,
- (99): *N-*[(1*R*)-1-(5-Chlor-1*H-*benzimidazol-2-yl)-1-(thiophen-2-yl)-methyl]-3- methyl-4-(morpholin-3-on-4-yl)-benzamid,
- (100): *N-*[(1*R*)-1-(5-Brom-1*H-*benzimidazol-2-yl)-2-hydroxy-ethyl]-4-(1,1-dioxo- [1,2]thiazinan-2-yl)-3-methyl-benzamid,
- (101): *N-*[(1*R*)-1-(5-Brom-1*H-*benzimidazol-2-yl)-2-hydroxy-ethyl]-3-methyl-4- ([1,3]oxazepan-2-on-3-yl)-benzamid,
- (102): *N-*[(1*R*)-1-(5-Chlor-1*H-*benzimidazol-2-yl)-2-methoxy-ethyl]-3-methyl-4- (4-(4*S*)-methyl-2-oxo-oxazolidin-3-yl)-benzamid,
- (103): 3-Chlor-*N-*[(1*R*)-1-(5-chlor-1*H-*benzimidazol-2-yl)-2-methoxy-ethyl]-4- (4,4-dimethyl-2-oxo-oxazolidin-3-yl)-benzamid,
- (104): *N-*[(1*R*)-1-(5-Chlor-1*H-*benzimidazol-2-yl)-2-methoxy-ethyl]-3-methyl-4- (4-(4*R*)-methyl-2-oxo-oxazolidin-3-yl)-benzamid,
- (105): 3-Chlor-*N-*[(1*R*)-1-(5-chlor-1*H-*benzimidazol-2-yl)-2-methoxy-ethyl]-4-(4- (4*R*)-ethyl-2-oxo-oxazolidin-3-yl)-benzamid,
- (106): *N-*[(1*R*)-1-(5-Chlor-1*H-*benzimidazol-2-yl)-2-methoxy-ethyl]-3-fluor-4- (morpholin-3-on-4-yl)-benzamid,
- (107): *N*-[1-(5-chlor-1*H-*benzimidazol-2-yl)-1-(1*H-*pyrazol-3-yl)-methyl]-3-methyl- 4-(morpholin-3-on-4-yl)-benzamid,
- (108): *N-*[1-(1*S*)-(5-Chlor-1*H-*benzimidazol-2-yl)-2-cyano-ethyl]-3-methyl-4- (morpholin-3-on-4-yl)-benzamid,
- (109): *N-*[1-(5-Chlor-1*H-*benzimidazol-2-yl)-1-pyridin-3-yl-methyl]-3-methyl-4- (morpholin-3-on-4-yl)-benzamid,
- (110): *N-*[1-(5-Chlor-1*H-*benzimidazol-2-yl)-1-(1*H-*1-methyl-pyrazol-3-yl)- methyl]-3-methyl-4-(morpholin-3-on-4-yl)-benzamid,
- (111): 3-Chlor-*N-*[(1*R*)-1-(5-chlor-1*H-*benzimidazol-2-yl)-2-methoxy-ethyl]-4-(5- methyl-morpholin-3-on-4-yl)-benzamid,
- (112): 3-Chlor-*N-*[(1*S*)-1-(5-chlor-1*H-*benzimidazol-2-yl)-ethyl]-4-(3- dimethylamino-pyrrolidin-1-yl)-benzamid,
- (113): *N-*[(1*S*)-1-(5-Chlor-1*H-*benzimidazol-2-yl)-ethyl]-4-(pyrazolidin-3-on-1-yl)- 3-trifluormethyl-benzamid,
- (114): 3-Chlor-*N-*[(1*R*)-1-(5-chlor-1*H-*benzimidazol-2-yl)-2-methoxy-ethyl]-4- (tetrahydro-pyrimidin-2-on-1-yl)-benzamid,
- (115): *N*-[(1*R*)-1-(5-Chlor-1*H-*benzimidazol-2-yl)-2-methoxy-ethyl]-3-chlor-4- ([1,4]diazepan-1-yl)-benzamid,
- (116): 3-Chlor-*N*-[1-(5-chlor-1*H-*indol-2-yl)-2-methoxy-ethyl]-4-([1,4]diazepan-1- yl)-benzamid,
- (117): *N-*[1-(5-Chlor-1*H-*indol-2-yl)-2-methoxy-ethyl]-3-methyl-4-(morpholin-3- on-4-yl)-benzamid,
- (118): 3-Brom-*N-*[1-(5-chlor-1*H-*indol-2-yl)-1-(furan-2-yl)-methyl]-4- ([1,4]oxazepan-5-on-4-yl)-benzamid,
- (119): *N-*[1-(5-Brom-1*H-*indol-2-yl)-1-(1-methyl-1*H-*pyrazol-3-yl)-methyl]-3- methyl-4-([1,3]oxazepan-2-on-3-yl)-benzamid,
- (120): *N-*[1-(5-Chlor-1*H-*indol-2-yl)-3-(methyl-sulfonyl)-propyl]-3-methyl-4- (tetrahydropyrimidin-2-on-1-yl)-benzamid,
- (121): *N-*[1-(5-Chlor-1*H-*indol-2-yl)-2-hydroxy-ethyl]-3-methyl-4-(5-methyl- pyrrolidin-2-on-1-yl)-benzamid,
- (122): *N-*[1-(5-Chlor-1*H-*indol-2-yl)-1-phenyl-methyl]-4-(piperazin-1-yl)-3- trifluormethyl-benzamid,
- (123): 4-(Azepan-2-on-1-yl)-3-chlor-*N-*[1-(5-chlor-1*H-*indol-2-yl)-3-(1*H-*tetrazol- 5-yl)-propyl]-benzamid,
- (124): *N-*[1-(5-Brom-1*H-*indol-2-yl)-2-hydroxy-ethyl]-4-(1,1-dioxo-[1,2]thiazepan- 2-yl)-3-methyl-benzamid,
- (125): 3-Chlor-*N*-[1-(7-chlor-imidazo[1,2a]pyridin-2-yl)-2-methoxy-ethyl]-4- (diazepan-1-yl)-benzamid,
- (126): 3-Chlor-*N-*[1-(7-chlor-imidazo[1,2a]pyridin-2-yl)-ethyl]-4-([1,4]oxazepan- 5-on-4-yl)-benzamid,
- (127): *N-*[(1*S*)-1-(7-Chlor-imidazo[1,2a]pyridin-2-yl)-3-methyl-butyl]-3-methyl-4- (morpholin-3-on-4-yl)-benzamid,
- (128): *N-*[1-(7-Chlor-imidazo[1,2a]pyridin-2-yl)-2-methoxy-ethyl]-3-methyl-4- (morpholin-3-on-4-yl)-benzamid,
- (129): 3-Brom-*N-*[1-(7-chlor-imidazo[1,2a]pyridin-2-yl)-1-(1*H-*pyrazol-3-yl)- methyl]-4-([1,4]oxazepan-5-on-4-yl)-benzamid,
- (130): *N-*[1-(7-Brom-imidazo[1,2a]pyridin-2-yl)-2-hydroxy-ethyl]-3-chlor-4-(4- methyl-piperazin-1-yl)-benzamid,
- (131): *N-*[1-(7-Chlor-imidazo[1,2a]pyridin-2-yl)-2-hydroxy-ethyl]-3-methyl-4- (morpholin-3-on-4-yl)-benzamid,
- (132): *N-*[1-(7-Chlor-imidazo[1,2a]pyridin-2-yl)-1-(furan-2-yl)-methyl]-4- (diazepan-1-yl)-3-trifluormethyl-benzamid,
- (133): *N-*[1-(7-Chlor-imidazo[1,2a]pyridin-2-yl)-1-phenyl-methyl]-3-methyl-4- (1,1-dioxo-[1,2]thiazepan-2-yl)-benzamid,
- (134): 3-Chlor-*N-*[1-(7-chlor-imidazo[1,2a]pyridin-2-yl)-2-methoxy-ethyl]-4- (morpholin-3-on-4-yl)-benzamid,
- (135): 3-Brom-*N-*[1-(7-chlor-imidazo[1,2a]pyridin-2-yl)-3-(methyl-sulfanyl)- propyl]-4-([1,4]oxazepan-3-on-4-yl)-benzamid,
- (136): 3-Chlor-*N-*[1-(7-chlor-imidazo[1,2a]pyridin-2-yl)-2-hydroxy-ethyl]-4- (piperazin-2-on-1-yl)-benzamid,
- (137): *N-*[1-(7-Chlor-imidazo[1,2a]pyridin-2-yl)-2-methoxy-ethyl]-3-methyl-4- (piperidin-2-on-1-yl)-benzamid,
- (138): *N*-[1-(7-Brom-imidazo[1,2a]pyridin-2-yl)-3-methoxy-propyl]-3-chlor-4-(1,1- dioxo-[1,2]thiazinan-2-yl)-benzamid,
- (139): *N-*[1-(7-Chlor-imidazo[1,2a]pyridin-2-yl)-2-trifluormethoxy-ethyl]-3-methyl- 4-([1,4]oxazepan-5-on-4-yl)-benzamid,
- (140): *N-*[1-(5-Chlor-1*H-*benzimidazol-2-yl)-3-methyl-butyl]-3-methyl-4- (morpholin-3-on-4-yl)-benzamid,
- (141): *N-*[(1*R*)-1-(5-Chlor-1*H-*benzimidazol-2-yl)-2-benzyloxy-ethyl]-3-methyl-4- (morpholin-3-on-4-yl)-benzamid,
- (142): *N-*[1-(5-Chlor-1*H-*benzimidazol-2-yl)-3-benzyloxycarbonyl-propyl]-3- methyl-4-(morpholin-3-on-4-yl)-benzamid,
- (143): *N-*[1-(5-Chlor-1*H-*benzimidazol-2-yl)-3-hydroxycarbonyl-propyl]-3-methyl- 4-(morpholin-3-on-4-yl)-benzamid,
- (144): *N-*[1-(5-Chlor-1*H-*benzimidazol-2-yl)-1-(pyrazin-2-yl)-methyl]-3-methyl-4- (morpholin-3-on-4-yl)-benzamid,
- (145): *N-*[1-(5-Chlor-1*H-*benzimidazol-2-yl)-1-(oxazol-2-yl)-methyl]-3-methyl-4- (morpholin-3-on-4-yl)-benzamid,
- (146): *N-*[1-(5-Chlor-1*H-*benzimidazol-2-yl)-1-(1*H-*imidazol-4-yl)-methyl]-3- methyl-4-(morpholin-3-on-4-yl)-benzamid,
- (147): *N-*[1-(5-Brom-1*H-*benzimidazol-2-yl)-2-methoxy-ethyl]-3-difluormethoxy- 4-(morpholin-3-on-4-yl)-benzamid,
- (148): *N-*[1-(5-Chlor-1*H-*benzimidazol-2-yl)-1-methyl-ethyl]-3-methyl-4- (morpholin-3-on-4-yl)-benzamid,
- (149): *N-*[3-(5-Chlor-1*H-*benzimidazol-2-yl)-tetrahydrofuran-3-yl]-3-methyl-4- (morpholin-3-on-4-yl)-benzamid,
- (150): *N-*[1-(5-Chlor-1*H-*benzimidazol-2-yl)-3-(pyrrolidin-1-yl-carbonyl)-propyl]-3- methyl-4-(morpholin-3-on-4-yl)-benzamid,
- (151): *N-*[1-(5-Ethinyl-1*H-*benzimidazol-2-yl)-ethyl]-3-methyl-4-(morpholin-3-on- 4-yl)-benzamid,
- (152): *N-*[1-(5-Ethinyl-1*H-*benzimidazol-2-yl)-2-methoxy-ethyl]-3-methyl-4- (morpholin-3-on-4-yl)-benzamid,
- (153): *N-*[1-(5-Ethinyl-1*H-*benzimidazol-2-yl)-2-methoxy-ethyl]-3-methyl-4- (piperidin-2-on-1-yl)-benzamid,
- (154): 3-Brom-*N-*[1-(5-ethinyl-1*H-*benzimidazol-2-yl)-2-methoxy-ethyl]-4- (morpholin-3-on-4-yl)-benzamid,
- (155): *N-*[1-(5-Ethinyl-1*H-*benzimidazol-2-yl)-2-methoxy-ethyl]-3-methyl-4-(4- methyl-oxazolidin-2-on-3-yl)-benzamid,
- (156): *N-*[1-(5-Ethinyl-1*H-*benzimidazol-2-yl)-2-methoxy-ethyl]-3-methyl-4- ([1,4]oxazepan-5-on-4-yl)-benzamid,
- (157): *N-*[(1*S*)-1-(5-Chlor-1*H-*benzimidazol-2-yl)-2-methoxy-ethyl]-3-methyl-4-(2- methyl-tetrahydropyridazin-1-yl)-benzamid,
deren Tautomere, deren Enantiomere, deren Diastereomere, deren Gemische
- und: deren Salze, wobei die Verbindungen
- (1): 4-(Azepan-2-on-1-yl)-*N-*[(1*R*)-1-(5-chlor-1*H-*benzimidazol-2-yl)-2- methoxy-ethyl]-3-methyl-benzamid,
- (2): 4-(Azepan-2-on-1-yl)-*N-*[(1*R*)-1-(5-chlor-1*H-*benzimidazol-2-yl)-2- hydroxy-ethyl]-3-methyl-benzamid,
- (3): *N-*[(1*S*)-1-(5-Chlor-1*H-*benzimidazol-2-yl)-3-methylsulfanyl-propyl]-3- methyl-4-(pyrrolidin-2-on-1-yl)-benzamid,
- (4): *N-*[(1*R*)-1-(5-Chlor-1*H-*benzimidazol-2-yl)-2-methoxy-ethyl]-4-(morpholin- 3-on-4-yl)-3-trifluormethyl-benzamid,
- (5): *N-*[(1*R*)-1-(5-Chlor-1*H-*benzimidazol-2-yl)-2-methoxy-ethyl]-3-methyl-4- (morpholin-3-on-4-yl)-benzamid,
- (6): *N-*[(1*S*)-1-(5-Chlor-1*H-*benzimidazol-2-yl)-3-methylsulfanyl-propyl]-3- methyl-4-(morpholin-3-on-4-yl)-benzamid,
- (7): *N-*[(1*R*)-1-(5-Chlor-1*H-*benzimidazol-2-yl)-2-hydroxy-ethyl]-3-methyl-4- (morpholin-3-on-4-yl)-benzamid,
- (8): 4-(Azepan-2-on-1-yl)-3-chlor-*N-*[(1*R*)-1-(5-chlor-1*H-*benzimidazol-2-yl)-2- methoxy-ethyl]-benzamid;
- (9): 4-(Azepan-2-on-1-yl)-*N-*[(1*R*)-1-(5-chlor-1*H-*benzimidazol-2-yl)-2- methoxy-ethyl]-3-trifl uormethyl-benzamid,
- (10): *N-*[(1*S*)-1-(5-Chlor-1*H-*benzimidazol-2-yl)-ethyl]-3-methyl-4- ([1,3]oxazepan-2-on-3-yl)-benzamid,
- (11): *N-*[(1*S*)-1-(5-Chlor-1*H-*benzimidazol-2-yl)-3-methylsulfanyl-propyl]-4- (morpholin-3-on-4-yl)-3-nitro-benzamid,
- (12): *N-*[(1*R*)-1-(5-Chlor-1*H-*benzimidazol-2-yl)-2-methoxy-ethyl]-3-methyl-4- ([1,3]oxazepan-2-on-3-yl)-benzamid,
- (13): 3-Chlor-*N-*[(1*R*)-1-(5-chlor-1*H-*benzimidazol-2-yl)-2-methoxy-ethyl]-4- ([1,4]oxazepan-5-on-4-yl)-benzamid,
- (14): 3-Chlor-*N-*[(1*R*)-1-(5-chlor-1*H-*benzimidazol-2-yl)-2-methoxy-ethyl]-4- (piperidin-2-on-1-yl)-benzamid,
- (15): *N-*[(1*R*)-1-(5-Brom-1*H-*benzimidazol-2-yl)-2-hydroxy-ethyl]-3-methyl-4- (morpholin-3-on-4-yl)-benzamid,
- (16): *N-*[(1*R*,2*S*)-1-(5-Chlor-1*H-*benzimidazol-2-yl)-2-methoxy-propyl]-3- methyl-4-(morpholin-3-on-4-yl)-benzamid,
- (17): *N-*[(1*S*)-1-(5-chlor-1*H-*benzimidazol-2-yl)-ethyl]-3-methyl-4- (thiomorpholin-3-on-4-yl)-benzamid,
- (18): *N-*[(1*R*)-1-(5-Chlor-1*H-*benzimidazol-2-yl)-2-methoxy-ethyl]-3-methyl-4- (piperidin-2-on-1-yl)-benzamid,
- (19): *N-*[(1*R*)-1-(5-Chlor-1*H-*benzimidazol-2-yl)-2-methoxy-ethyl]-3-methyl-4- ([1,3]-oxazinan-2-on-3-yl)-benzamid,
- (20): *N-*[(1*R*)-1-(5-Chlor-1*H-*benzimidazol-2-yl)-2-methoxy-ethyl]-4-(piperidin-2- on-1-yl)-3-trifluormethyl-benzamid,
- (21): *N-*[(1*R*)-1-(5-Chlor-1*H-*benzimidazol-2-yl)-2-methoxy-ethyl]-4-(1,1-dioxo- [1,2]thiazinan-2-yl)-3-methyl-benzamid,
- (22): In-[(1*S*)-1-(5-Chlor-1*H-*benzimidazol-2-yl)-butyl]-3-methyl-4-(morpholin-3- on-4-yl)-benzamid,
- (23): *N-*[(1*R*)-1-(5-Chlor-1*H-*benzimidazol-2-yl)-2-methoxy-ethyl]-4- ([1,3]oxazepan-2-on-3-yl)-3-trifluormethyl-benzamid,
- (24): *N-*[(1*R*)-1-(5-Chlor-1*H-*benzimidazol-2-yl)-2-methoxy-ethyl]-4-(1,1-dioxo- [1,2,6]thiadiazinan-2-yl)-3-methyl-benzamid,
- (25): *N-*[(1*R*)-1-(5-Brom-1*H-*benzimidazol-2-yl)-2-methoxy-ethyl]-3-methyl-4- (morpholin-3-on-4-yl)-benzamid,
- (26): *N-*[(1*S*)-1-(5-Chlor-1*H-*benzimidazol-2-yl)-3-(1*H-*tetrazol-5-yl)-propyl]-3- methyl-4-(morpholin-3-on-4-yl)-benzamid,
- (27): *N-*[(1*S*)-1-(5-Chlor-1*H-*benzimidazol-2-yl)-3-methoxy-propyl]-3-methyl-4- (morpholin-3-on-4-yl)-benzamid,
- (28): *N-*[(1*R*)-1-(5-Chlor-1*H-*benzimidazol-2-yl)-2-methylsulfanyl-ethyl]-3- methyl-4-(morpholin-3-on-4-yl)-benzamid,
- (29): *N-*[(1*R*)-1-(5-Chlor-1*H-*benzimidazol-2-yl)-2-methoxy-ethyl]-3-methyl-4- (thiomorpholin-3-on-4-yl)-benzamid;
- (30): 3-Chlor-*N-*[(1*R*)-1-(5-chlor-1*H-*benzimidazol-2-yl)-2-methoxy-ethyl]-4- (morpholin-3-on-4-yl)-benzamid,
- (31): 3-Chlor-*N-*[(1*R*)-1-(5-chlor-1*H-*benzimidazol-2-yl)-2-methoxy-ethyl]-4- (1,1-dioxo-[1,2]thiazinan-2-yl)-benzamid,
- (32): *N-*[(1*R*)-1-(5-Chlor-1*H-*benzimidazol-2-yl)-2-methoxy-ethyl]-4-(1,1-dioxo- [1,2]thiazepan-2-yl)-3-methyl-benzamid,
- (33): 3-Brom-*N-*[(1*S*)-1-(5-chlor-1*H-*benzimidazol-2-yl)-ethyl]-4-(piperidin-2-on- 1-yl)-benzamid,
- (34): 3-Brom-*N-*[(1*R*)-1-(5-chlor-1*H-*benzimidazol-2-yl)-2-methoxy-ethyl]-4- (piperidin-2-on-1-yl)-benzamid"
- (35): *N-*[1-(5-Chlor-1*H-*benzimidazol-2-yl)-1-(furan-2-yl)-methyl]-3-methyl-4- (piperidin-2-on-1-yl)-benzamid,
- (36): *N-*[(1*R*)-1-(5-Chlor-1*H-*benzimidazol-2-yl)-2-methoxy-ethyl]-3-methyl-4- (5-oxo-[1,4]oxazepan-4-yl)-benzamid,
- (37): 3-Chlor-*N-*[(1*R*)-1-(5-chlor-1*H-*benzimidazol-2-yl)-2-methoxy-ethyl]-4-(4- methyl-2-oxo-oxazolidin-3-yl)-benzamid,
- (38): *N-*[1-(5-Chlor-1*H-*benzimidazol-2-yl)-1-phenyl-methyl]-3-methyl-4- (morpholin-3-on-4-yl)-benzamid,
- (39): 3-Brom-*N-*[(1*R*)-1-(5-chlor-1*H-*benzimidazol-2-yl)-2-methoxy-ethyl]-4- (morpholin-3-on-4-yl)-benzamid,
- (40): *N-*[(1*R*)-1-(5-Chlor-1*H-*benzimidazol-2-yl)-1-(thiophen-2-yl)-methyl]-3- methyl-4-(morpholin-3-on-4-yl)-benzamid,
- (41): *N-*[(1*R*)-1-(5-Brom-1*H-*benzimidazol-2-yl)-2-hydroxy-ethyl]-4-(1,1-dioxo- [1,2]thiazinan-2-yl)-3-methyl-benzamid,
- (42): *N-*[(1*R*)-1-(5-Brom-1*H-*benzimidazol-2-yl)-2-hydroxy-ethyl]-3-methyl-4- ([1,3]oxazepan-2-on-3-yl)-benzamid,
- (43): *N-*[(1*R*)-1-(5-Chlor-1*H-*benzimidazol-2-yl)-2-methoxy-ethyl]-3-methyl-4- (4-(4*S*)-methyl-2-oxo-oxazolidin-3-yl)-benzamid,
- (44): 3-Chlor-*N-*[(1*R*)-1-(5-chlor-1*H-*benzimidazol-2-yl)-2-methoxy-ethyl]-4- (4,4-dimethyl-2-oxo-oxazolidin-3-yl)-benzamid,
- (45): *N-*[(1*R*)-1-(5-Chlor-1*H-*benzimidazol-2-yl)-2-methoxy-ethyl]-3-methyl-4- (4-(4*R*)-methyl-2-oxo-oxazolidin-3-yl)-benzamid,
- (46): 3-Chlor-*N-*[(1*R*)-1-(5-chlor-1*H-*benzimidazol-2-yl)-2-methoxy-ethyl]-4-(4- (4*R*)-ethyl-2-oxo-oxazolidin-3-yl)-benzamid,
- (47): *N-*[(1*R*)*-*1-(5-Chlor-1*H-*benzimidazol-2-yl)-2-methoxy-ethyl]-3-fluor-4- (morpholin-3-on-4-yl)-benzamid,
- (48): *N-*[1-(5-chlor-1*H-*benzimidazot-2-yl)-1-(1*H-*pyrazo)-3-yl)-methyl]-3-methyl- 4-(morpholin-3-on-4-yl)-benzamid,
- (49): *N-*[1-(5-Chlor-1*H-*benzimida-zol-2-yl)-1-(1*H-*1-methyl-pyrazol-3-yl)- methyl]-3-methyl-4-(morpholin-3-on-4-yl)-benzamid,
- (50): 3-Chlor-*N-*[(1*R*)-1-(5-chlor-1*H-*benzimidazol-2-yl)-2-methoxy-ethyl]-4-(5- methyl-morpholin-3-on-4-yl)-benzamid,
- (51): 3-Chlor-*N-*[(1*R*)-1-(5-chlor-1*H-*benzimidazol-2-yl)-2-methoxy-ethyl]-4- (tetrahydro-pyrimidin-2-on-1-yl)-benzamid,
- (52): *N-*[1-(5-Chlor-1*H-*benzimidazol-2-yl)-thiophen-3-yl-methyl]-3-methyl-4- (morpholin-3-on-4-yl)-benzamid,
- (53): *N-*[(1*S*)-1-(5-Chlor-1*H-*benzimidazol-2-yl)-ethyl]-3-methyl-4- ([1,3]oxazepan-2-on-3-yl)-benzamid
- (54): *N-*[(1*S*)-1-(5-Chlor-1*H-*benzimidazol-2-yl)-3-methylsulfanyl-propyl)-4- (piperazin-1-yl)-3-trifluormethyl-benzamid,
- (55): *N-*[(1*S*)-1-(5-Chlor-1*H-*benzimidazol-2-yl)-3-methylsulfanyl-propyl]-4- (morpholin-3-on-4-yl)-3-nitro-benzamid,
- (56): *N-*[(1*R*)-1-(5-Chlor-1*H-*benzimidazol-2-yl)-2-methoxy-ethyl]-3-methyl-4- (piperazin-1-yl)-benzamid,
- (57): *N-*[(1*S*)-1-(7-Chlor-imidazo[1,2a]pyridin-2-yl)-3-methyl-butyl]-3-methyl-4- (morpholin-3-on-4-yl)-benzamid,
deren Tautomere, deren Enantiomere, deren Diastereomere, deren Gemische und deren Salze besonders bevorzugt sind.

Im Rahmen der vorliegenden Anmeldung ist, falls vorhanden, unter der Begriff "Isomere", "Stereoisomere", "Diastereomere", "Enantiomere", "chiral", "Racemat" oder "racemische Mischung" folgendes zu verstehen. Verbindungen der gleichen Summenformel, die sich in der Art oder Anordnung der Bindung ihrer Atome bzw. deren Konnektivität oder der räumlichen Anordnung der Atome im Molekül unterscheiden, werden "Isomere" genannt. Isomere, die sich bei gleicher Art und Weise der Konnektivität Ihrer Atome durch die räumliche Anordnung der Atome im Molekül unterscheiden und nicht deckungsgleich sind, werden "Stereoisomere" genannt. Stereoisomere, die sich nicht wie Bild und Spiegelbild zueinander verhalten, werden "Diastereomere" genannt, und Stereoisomere, die sich wie Bild und Spiegelbild zueinander verhalten, werden "Enantiomere" genannt. Bei Anwesenheit eines asymmetrischen Zentrums oder Atoms (auch Stereozentrum oder Chiralitätszentrum genannt), beispielsweise bei einem durch vier unterschiedliche Substituenten substituierten Kohlenstoffatom, hat das Molekül die Eigenschaft "chiral", und ein Paar von Enantiomeren ist möglich. Ein Enantiomer kann durch die absolute Konfiguration seines Stereozentrums charakterisiert werden. Die Beschreibung der absoluten Konfiguration geschieht mittels der Descriptoren (*R*) und (*S*), die durch die Anwendung der Sequenzregeln nach Cahn, Ingold und Prelog ermittelt werden, oder durch Beschreibung der Drehung der Ebene von polarisiertem Licht bei Wechselwirkung mit dem Molekül, die als rechtsdrehend oder linksdrehend (also entsprechend mit (+) oder (-) als Descriptor) bezeichnet wird. Eine chirale Verbindung kann sowohl als individuelles Enantiomer oder als Gemisch der entsprechenden Enantiomere vorliegen. Ein Gemisch, das gleiche Anteile beider Enantiomeren einer Verbindung enthält, wird "Racemat" oder "racemische Mischung" genannt.

Erfindungsgemäß erhält man die Verbindungen der allgemeinen Formel I nach an sich bekannten Verfahren, beispielsweise nach folgenden Verfahren:
(a) Zur Herstellung von Verbindungen der allgemeinen Formel 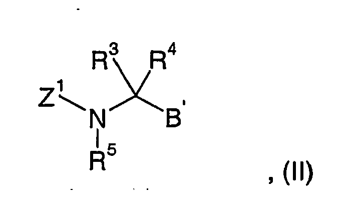 in der R³ bis R⁵ wie eingangs erwähnt definiert sind und Z¹ das Wasserstoffatom oder eine Schutzgruppe bedeuten und B' eine Gruppe der Formel 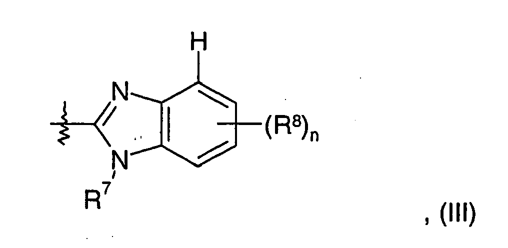 darstellt, in der R⁷ und R⁸ wie eingangs erwähnt definiert sind:
   Cyclisierung einer gegebenenfalls im Reaktionsgemisch gebildeten Verbindung der allgemeinen Formel 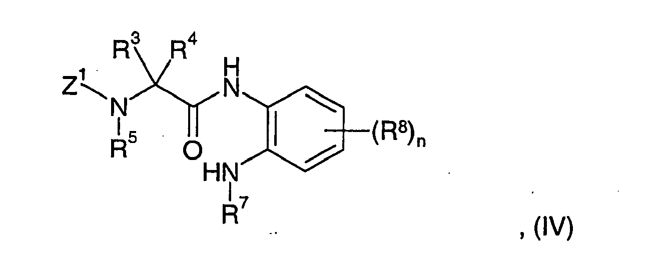 in der
   R³ bis R⁵, R⁷ und R⁸ wie eingangs erwähnt definiert sind und Z¹ das Wasserstoffatom oder eine Schutzgruppe bedeutet, wobei anschließend eine eventuell vorhandene Schutzgruppe abgespalten wird.
   Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glykol, Glykolmonomethylether, Diethylenglykoldimethylether, Sulfolan, Dimethylformamid oder Tetralin, Dimethylsulfoxid, Methylenchlorid, Chloroform, Tetrachlormethan, beispielsweise bei Temperaturen zwischen 0 und 250°C, vorzugsweise jedoch zwischen 20 und 100°C, gegebenenfalls in Gegenwart eines Kondensationsmittels wie Phosphoroxychlorid, Thionylchlorid, Sulfurylchlorid, Schwefelsäure, *p-*Toluolsulfonsäure, Methansulfonsäure, Salzsäure, Phosphorsäure, Polyphosphorsäure, Essigsäure, Essigsäureanhydrid, *N,N'*-Dicyclohexylcarbodiimid oder gegebenenfalls auch in Gegenwart einer Base wie Kaliumethylat oder Kalium-*tert*.-butylat durchgeführt. Die Cyclisierung kann jedoch auch ohne Lösungsmittel und/oder Kondensationsmittel durchgeführt werden.
   Verbindungen der allgemeinen Formel (IV) können durch Acylierung von Verbindungen der allgemeinen Formel 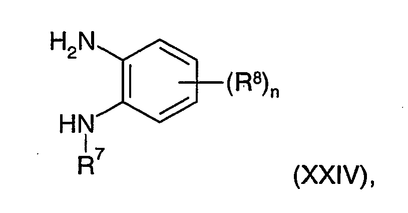 in der n, R⁷ und R³ wie eingangs erwähnt definiert sind, mit Verbindungen der allgemeinen Formel 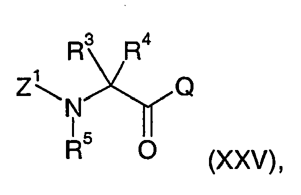 in der R³, R⁴ und R⁵ wie eingangs erwähnt definiert sind, Q ein Halogenatom oder eine Hydroxy-, C₁₋₄-Alkoxy- oder C₁₋₄-Acyloxygruppe und Z¹ eine Schutzgruppe bedeutet, nach unter (e) beschriebenen Verfahren hergestellt werden.
(b) Zur Herstellung einer Verbindung der allgemeinen Formel 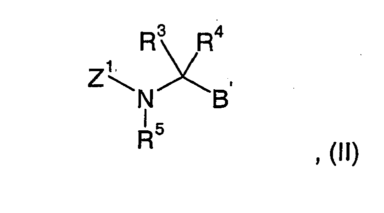 in der R³ bis R⁵ wie eingangs erwähnt definiert sind, Z¹ das Wasserstoffatom oder eine Schutzgruppe, beispielsweise eine C₁₋₅-Alkyloxycarbonyl- oder Benzyloxycarbonylgruppe, bedeutet und B' eine Gruppe der Formel 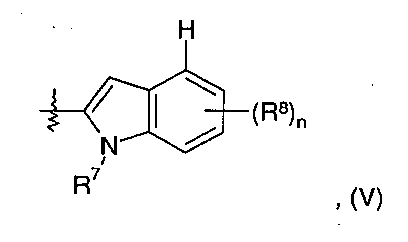 darstellt, in der R⁷ und R⁸ wie eingangs erwähnt definiert sind:
   i) Übergangsmetall-katalysierie Kupplung und Cyclisierung einer Verbindung der allgemeinen Formel 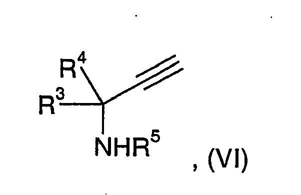 in der R³ eine Phenyl- oder Heteroarylgruppe und R⁴ ein Wasserstoffatom bedeuten und R⁵ wie eingangs erwähnt definiert ist, mit
      einer Verbindung der allgemeinen Formel 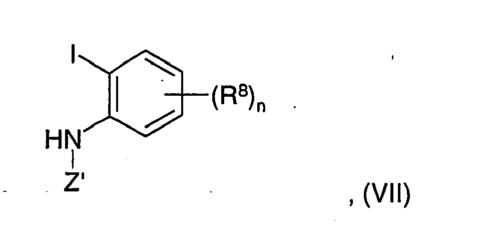 in der R⁸ wie eingangs erwähnt definiert ist und Z¹ eine Schutzgruppe, beispielsweise eine Äcetyl- oder Methylsulfonylgruppe, bedeutet, wobei diese Schutzgruppe anschließend abgespalten wird.
      Die Reaktionssequenz wird zweckmäßigerweise in einem Lösungsmittel oder Lösungsmittelgemisch wie Ethanol, Isopropanol, Eisessig, Benzol, Chlorbenzol, Toluol, Xylol, Glykol, Glykolmonomethylether, Diethylenglykoldimethylether, Sulfolan, Dimethylformamid, *N-*Methylpyrrolidinon, Tetralin, Dimethylsulfoxid, Methylenchlorid, Chloroform oder Tetrachlormethan, beispielsweise bei Temperaturen zwischen 0 und 250°C, vorzugsweise jedoch zwischen 20 und 120°C, zweckmäßigerweise in Gegenwart von Übergangsmetall-Katalysatoren wie Bis-(triphenylphoshin)-palladium(II)-chlorid, Bis-(tricyclohexylphosphin)-palladium(II)-chlorid, Bis-(triethylphosphin)-palladium(II)-chlorid oder Bis-(tri-o-tolylphosphin)-palladium(II)-chlorid und gegebenfalls in Gegenwart eines Übergangsmetall-Katalysators wie Kupfer(I)-iodid, Kupfer(I)-bromid oder Kupfer(I)-acetat und zweckmäßigerweise in Gegenwart einer Base wie Tetramethylguanidin, Tetramethylethylendiamin oder *N,N*'-Dimethylethylendiamin sowie gegebenenfalls Anwendung einer Inertgasatmosphäre (beispielsweise Stickstoff oder Argon) durchgeführt.
   ii) Alkylierung und anschließende reduktive Aminierung einer Verbindung der allgemeinen Formel 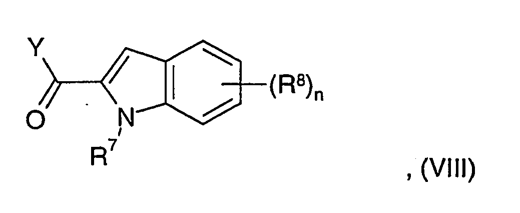 in der R⁷ und R⁸ wie eingangs erwähnt definiert sind und Y ein Halogenatom, eine C₁₋₄-Alkoxy-, C₁₋₄-Alkoxyamino- oder eine *N-*C₁₋₄-Alkoxy-*N-*C₁₋₄-alkyl-aminogruppe bedeutet, mit einer Verbindung der allgemeinen Formel 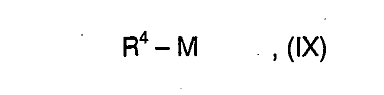 in der R⁴ wie eingangs erwähnt definiert ist und M ein Metall, wie beispielsweise Lithium, Natrium oder Kalium, oder ein Metall, wie beispielsweise Magnesium, Cadmium, Kupfer oder Zink, mit einem geeigneten Gegenion, wie beispielsweise Chlorid, Bromid oder Iodid, oder auch eine Kombination zweier Metalle, wie beispielsweise Magnesium und Kupfer, Lithium und Kupfer oder Zink und Kupfer, mit geeigneten Gegenionen, wie beispielsweise Cyanid, Chlorid, Bromid oder Iodid, sowie deren Kombinationen enthaltende Gruppierung bedeutet, und nachfolgende reduktive Aminierung der so erhaltenen Verbindungen.
      Die Alkylierung wird zweckmäßigerweise in einem Lösungsmittel oder Lösungsmittelgemisch wie Benzol, Chlorbenzol, Toluol, Xylol, Glykoldimethylether, Diethylenglykoldimethylether, Sulfolan, Dimethylformamid, *N-*Methylpyrrolidinon, Tetralin, Dimethylsulfoxid, Methylenchlorid, Chloroform, Tetrachlormethan, Diethylether, *tert*.-Butyl-methyl-ether oder Tetrahydrofuran beispielsweise bei Temperaturen zwischen -100 und +100°C, vorzugsweise jedoch zwischen -100 und 30°C, mit Alkylierungsreagentien wie Grignard-Reagentien, lithiumorganischen Reagentien, Gilman- oder Knochel-Cupraten, die nach literaturbekannten Verfahren erzeugt werden können, gegebenenfalls unter Anwendung einer Inertgasatmosphäre (Stickstoff oder Argon) durchgeführt. Die anschließende reduktive Aminierung der nach Alkylierung gebildeten Ketone erfolgt durch Umsetzung beispielsweise mit Ammoniak, Hydroxylamin, Alkoxylaminen, primären Aminen, Hydroxyl-alkylaminen oder Alkoxy-alkylaminen mit nachfolgender oder begleitet von Reduktion beispielsweise mit Hydrid-Donoren wie Natriumborhydrid, Lithiumaluminiumhydrid, Natriumcyanborhydrid, Natriumtriacetoxyborhydrid oder Diisobutylaluminiumhydrid in einem Lösungsmittel oder Lösungsmittelgemisch wie Ethanol, Isopropanol, Benzol, Toluol, Pyridin, Ethylenglykoldimethylether, Diethylenglykoldimethylether, *N-*Alkylmorpholin, Diethylether, *tert.*-Butyl-methylether, Tetrahydrofuran, Hexan oder Cyclohexan oder durch Hydrierung gegebenenfalls unter Druck und zweckmäßigerweise in Gegenwart eines Katalysators wie Raney-Nickel, Palladium, Palladiumkohle, Platin oder Platinoxid, in einem Lösungsmittel oder Lösungsmittelgemisch wie Ethylacetat, Ethanol, Isopropanol, Benzol, Toluol, Pyridin, Ethylenglykoldimethylether, Diethylenglykoldimethylether, *N-*C₁₋₅-Alkylmorpholin, Diethylether, *tert*.-Butylmethylether, Tetrahydrofuran, Hexan oder Cyclohexan.
(c) Zur Herstellung einer Verbindung der allgemeinen Formel 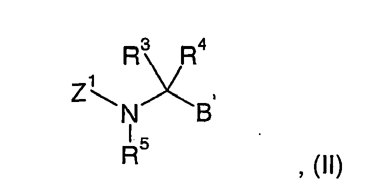 in der R³ bis R⁹ wie eingangs erwähnt definiert sind, Z¹ das Wasserstoffatom oder eine Schutzgruppe, beispielsweise eine C₁₋₅-Alkyloxycarbonyl- oder Benzyloxycarbonylgruppe, bedeutet und B' eine Gruppe der Formel 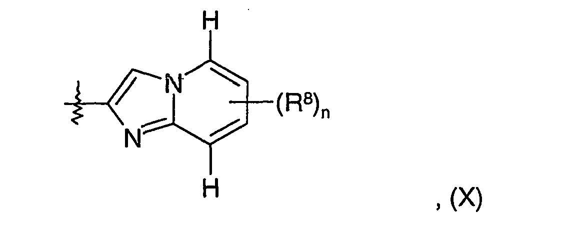 darstellt, in der R⁸ wie eingangs erwähnt definiert ist:
   Kupplung und nachfolgende Cyclisierung einer Verbindung der allgemeinen Formel 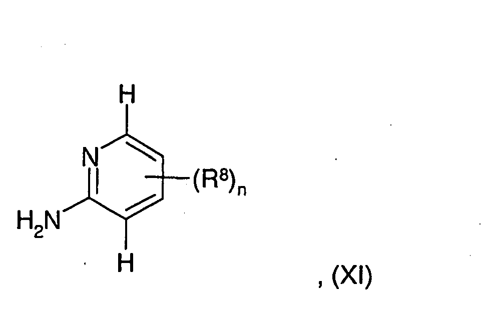 in der n und R⁸ wie eingangs erwähnt definiert sind, mit einer Verbindung der allgemeinen Formel 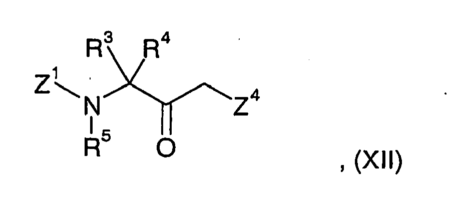 in der R³ bis R⁵ wie eingangs erwähnt definiert sind, Z¹ eine Schutzgruppe, beispielsweise eine C₁₋₅-Alkyloxycarbonyl- oder Benzyloxycarbonylgruppe, und Z⁴ eine nucleofuge Austrittsgruppe, beispielsweise ein Chlor-, Brom- oder lodatom, eine Tosylat-, Triflat- oder Mesylatgruppe, bedeuten, wobei die Schutzgruppe Z¹ anschließend nach literaturbekannten Verfahren abgespalten wird.
   Die Reaktionssequenz wird zweckmäßigerweise in einem Lösungsmittel oder Lösungsmittelgemisch wie Wasser, Ethanol, Isopropanol, Benzol, Chlorbenzol, Toluol, Xylol, Glykol, Glykoldimethylether, Diethylenglykoldimethylether, Dimethylformamid, *N-*Methylpyrrolidinon, Tetralin, Dimethylsulfoxid, Sulfolan, Methylenchlorid, Chloroform, Tetrachlormethan oder aber *N*-Ethyl-diisopropylamin, *N*-C₁₋₅-Alkylmorpholin, *N*-C₁₋₅-Alkylpiperidin, *N*-C₁₋₅-Alkylpyrrolidin, Triethylamin, Pyridin, beispielsweise bei Temperaturen zwischen -30 und 250°C, vorzugsweise jedoch zwischen 0 und 150°C, gegebenenfalls zweckmäßigerweise in Gegenwart von Basen wie Kaliumcarbonat, Natriumcarbonat, Natriumhydrogencarbonat, Kalium-*tert*.-butylat, Natrium-ethanolat, Kaliumhexamethyldisilazan, Natriumhydrid oder Lithiumdiisopropylamid durchgeführt.
(d) Zur Herstellung einer Verbindung der allgemeinen Formel 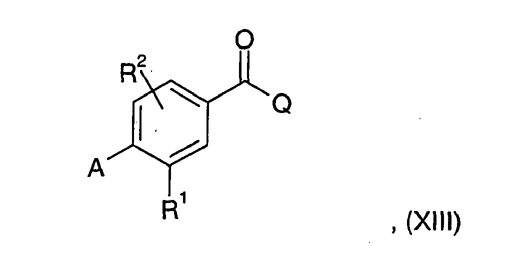 in der A, R¹ und R² wie eingangs erwähnt definiert sind und Q ein Halogenatom oder eine Hydroxy-, C₁₋₄-Alkoxy- oder C₁₋₄-Acyloxygruppe darstellt:
   i) Nucleophile Substitution einer Verbindung der allgemeinen Formel 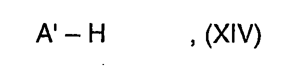 in der A' eine wie eingangs unter der Definition für A erwähnte 5- bis 7-gliedrige Cycloalkyleniminogruppe darstellt, am Aromaten der allgemeinen Formel 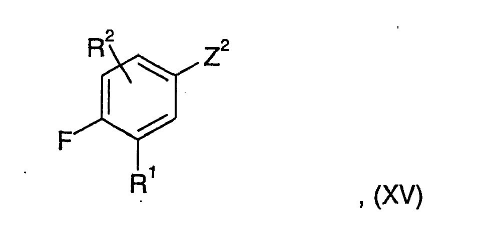 in der R¹ und R² wie eingangs erwähnt definiert sind und Z² die Nitrilgruppe oder eine C₁₋₅-Alkoxycarbonylgruppe darstellt, und anschließende Verseifung der Nitril- oder C₁₋₅-Alkoxycarbonylgruppe Z² und eventuelle weitere Umsetzung der daraus resultierenden Carboxylgruppe zu einem reaktiven Carbonsäurederivat der allgemeinen Formel XIII.
      Die nucleophile Substitution wird zweckmäßigerweise in einem Lösungsmittel oder Lösungsmittelgemisch wie Ethanol, Isopropanol, Benzol, Chlorbenzol, Toluol, Xylol, Glykol, Glykoldimethylether, Diethylenglykoldimethylether, Dimethylformamid, *N-*Methylpyrrolidinon, Tetralin, Dimethylsulfoxid, Sulfolan, Methylenchlorid, Chloroform, Tetrachlormethan oder aber *N-*Ethyl-diisopropylamin, *N*-C₁₋₅-Alkylmorpholin, *N*-C₁₋₅-Alkylpiperidin, *N*-C₁₋₅-Alkylpyrrolidin, Triethylamin, Pyridin, beispielsweise bei Temperaturen zwischen -30 und 250°C, vorzugsweise jedoch zwischen 0 und 150°C, gegebenenfalls zweckmäßigerweise in Gegenwart von Basen wie Kaliumcarbonat, Natriumcarbonat, Kalium-*tert*.-butylat, Natrium-ethanolat, Kaliumhexamethyldisilazan, Natriumhydrid oder Lithiumdiisopropylamid durchgeführt.
   ii) Übergangsmetall-katalysierte Kupplungsreaktion einer Verbindung der allgemeinen Formel 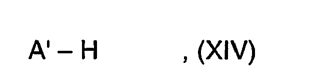 in der A' eine wie eingangs unter der Definition für A erwähnte 5- bis 7-gliedrige Cycloalkyleniminogruppe darstellt, am Aromaten der allgemeinen Formel 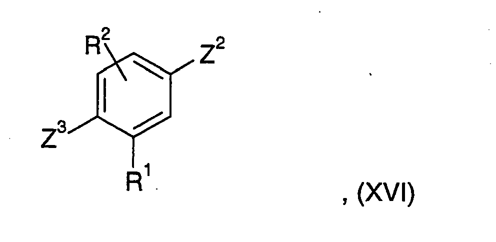 in der R¹ und R² wie eingangs erwähnt definiert sind und Z² die Nitrilgruppe oder eine C₁₋₅-Alkoxycarbonylgruppe und Z³ ein Chlor-, Brom- oder lodatom oder eine Triflatgruppe darstellen, und anschließende Verseifung der Nitril- oder C₁₋₅-Alkoxycarbonylgruppe Z² und eventuelle weitere Umsetzung der daraus resultierenden Carboxylgruppe zu einem reaktiven Carbonsäurederivat der allgemeinen Formel XIII.
      Die Umsetzung wird zweckmäßigerweise in einem Lösungsmittel oder Lösungsmittelgemisch wie Benzol, Toluol, Xylol, Tetrahydrofuran, Dioxan, Diethylether, *tert*.-Butyl-methyl-ether, Ethylenglykoldimethylether, Diethylenglykoldimethylether, Sulfolan, Dimethylformamid, *N-*Methylpyrrolidinon, Tetralin, Dimethylsulfoxid, Methylenchlorid, Chloroform oder Tetrachlormethan, beispielsweise bei Temperaturen zwischen -30 und 250°C, vorzugsweise jedoch zwischen 0 und 150°C, zweckmäßigerweise in Gegenwart von Übergangsmetall-Katalysatoren wie Nickel auf Aktivkohle, Palladiumkohle, Tetrakis-(triphenylphosphin)-palladium(0), Tris-(dibenzylidenaceton)-dipalladium(0), Palladium(II)acetat, Palladium(II)chlorid, Bis-(triphenylphoshin)-palladium(II)-chlorid, Bis-(tricyclohexylphosphin)-palladium(II)-chforid, Bis-(triethylphosphin)-palladium(II)-chlorid, Bis-(tri-o-tolylphosphin)-palladium(II)-chlorid, gegebenfalls in Gegenwart von Liganden wie Triphenylphosphin, Tri-*o-*tolylphosphin, Tri-*tert*.-butylphosphin, 1,3-Bis-(diphenylphosphino)-propan, 2,2'-Bis-(diphenylphosphino)-1,1'-dinaphthyl, 1,1'-Bis-(diphenylphosphino)-ferrocen, Xantphos, und zweckmäßigerweise in Gegenwart einer Base wie Natriummethanolat, Natriumethanolat, Natrium-*tert*.-butylat, Kalium-*tert*.-butylat, Natrium-*tert*.-butyldimethyl-silanoat, Kaliumhexamethyldisilazan, Lithiumdiisopropylamid, Kaliumcarbonat, Rubidiumcarbonat, Cäsiumcarbonat, Kaliumphosphat, Natriumhydrid, gegebenenfalls in Gegenwart eines Komplexbildners wie 18-Krone-6-Ether sowie zweckmäßigerweise unter Anwendung einer Inertgasatmosphäre (beispielsweise Stickstoff oder Argon) und gegebenenfalls unter Druck durchgeführt.
   iii) Selektive Oxidation einer Cycloalkyleniminogruppe in Verbindungen der allgemeinen Formel 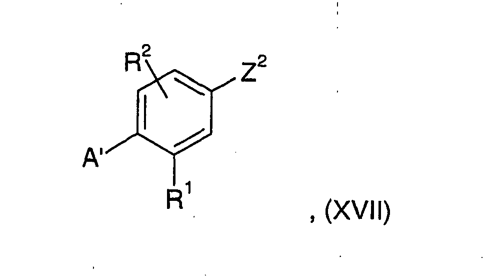 in der A' eine wie eingangs unter der Definition für A erwähnte 5- bis 7-gliedrige, gegebenenfalls auch substituierte 2-Oxocycloalkylenimingruppe darstellt, R¹ und R² wie eingangs erwähnt definiert sind und Z² die Carboxylgruppe darstellt, und eventuelle weitere Umsetzung zu einem reaktiven Carbonsäurederivat der allgemeinen Formel XIII.
      Die Umsetzung einer beispielsweise nach vorher beschriebenen Verfahren erhaltenen Verbindung der allgemeinen Formel XIII, in der A' eine Cycloalkyleniminogruppe und Q die Hydroxygruppe bedeuten, zum entsprechenden Lactam durch Oxidation einer dem Stickstoff benachbarten Methylengruppe wird beispielsweise mit Oxidationsmitteln wie Kaliumpermanganat, Kaliumchromat, Kaliumdichromat, Chrom(VI)oxid, Quecksilber(II)chlorid, Selen(IV)oxid, Blei(IV)oxid, Blei(IIIV)oxid, Kaliumperoxomonosulfat, Wasserstoffperoxid, Natriumhypochlorit, gegebenenfalls in Gegenwart eines geeigneten Katalysators wie Nickel(II)chlorid, Cobalt(II)chlorid, Ruthenium(III)chlorid, Osmium(VIII)oxid, Vanadium(IV)oxid und/oder in Gegenwart eines Kronenethers wie 18-Krone-6, in einem Lösungmittel oder Lösungsmittelgemisch wie Wasser, Ameisensäure, Essigsäure, Ethylacetat, Benzol, Pyridin, Dichlormethan, Chloroform, Tetrachlormethan, gegebenenfalls unter 2-Phasen-Bedingungen in Gegenwart eines geeigneten Phasentransfer-Katalysators wie beispielsweise Tetrabutylammoniumchlorid, Tetrabutylammoniumbromid, Benzyl-triethylammoniumchlorid oder Methyl-trioctyl-ammoniumchlorid, gegebenenfalls in Gegenwart einer Säure wie Essigsäure, Salzsäure, Bromwasserstoffsäure, Schwefelsäure, Phosphorsäure, Natriumhydrogensulfat, Natriumdihydrogenphosphat und/oder einer Base wie Natriumhydroxid, Kaliumhydroxid, Ammoniak, Pyridin, Kaliumphosphat, Dikaliumhydrogenphosphat oder Natriumacetat bei Temperaturen zwischen -30 und 250°C, vorzugsweise jedoch zwischen 0 und 150°C durchgeführt. Beispielsweise kann diese Umsetzung wie beschrieben bei J. H. Markgraf, C. A. Stickney, J. Heterocycl. Chem. 2000, 37(1), 109, durchgeführt werden.
   iv) Übergangsmetall-katalysierte Kupplungsreaktion einer Verbindung der allgemeinen Formel 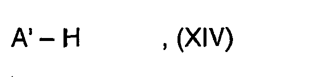 in der A' eine wie eingangs unter der Definition für A erwähnte 5- bis 7-gliedrige, gegebenenfalls substituierte 2-Oxo-cycloalkyleniminogruppe darstellt, am Aromaten der allgemeinen Formel 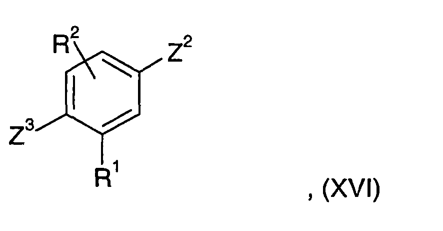 in der R¹ und R² wie eingangs erwähnt definiert sind und Z² die Nitrilgruppe oder eine C₁₋₅-Alkoxycarbonylgruppe und Z³ ein Chlor-, Brom- oder lodatom oder eine Triflatgruppe darstellen, und anschließende Verseifung der Nitril- oder C₁₋₅-Alkoxycarbonylgruppe Z² und eventuelle weitere Umsetzung der daraus resultierenden Carboxylgruppe zu einem reaktiven Carbonsäurederivat der allgemeinen Formel XII.
      Die Umsetzung wird zweckmäßigerweise in einem Lösungsmittel oder Lösungsmittelgemisch wie Benzol, Toluol, Xylol, Tetrahydrofuran, Dioxan, Diethylether, *tert*.-Butyl-methyl-ether, Ethylenglykoldimethylether, Diethylenglykoldimethylether, Sulfolan, Dimethylformamid, *N-*Methylpyrrolidinon, Tetralin, Dimethylsulfoxid, Methylenchlorid, Chloroform oder Tetrachlormethan, beispielsweise bei Temperaturen zwischen -30 und 250°C, vorzugsweise jedoch zwischen 0 und 200°C, zweckmäßigerweise in Gegenwart von Übergangsmetall-Katalysatoren wie Tetrakis-(triphenylphosphin)-palladium(0), Tris-(dibenzylidenaceton)-dipalladium(0), Palladium(II)acetat, Palladium(II)chlorid, Bis-(triphenylphoshin)-palladium(II)-chlorid, Bis-(tricyclohexylphosphin)-palladium(II)-chlorid, Bis-(triethylphosphin)-palladium(II)-chlorid, Bis-(tri-o-tolylphosphin)-palladium(II)-chlorid, gegebenenfalls in Gegenwart von Liganden wie Triphenylphosphin, Tri-*o*-tolylphosphin, Tri-*tert*.-butylphosphin, 1,3-Bis-(diphenylphosphino)-propan, 2,2'-Bis-(diphenylphosphino)-1,1'-dinaphthyl, 1,1'-Bis-(diphenylphosphino)-ferrocen, Xantphos, oder beispielsweise in Gegenwart eines Übergangsmetall-Katalysators wie Kupfer(I)-iodid, Kupfer(I)-bromid oder Kupfer(I)-acetat und zweckmäßigerweise in Gegenwart einer Base wie Tetramethylguanidin, Tetramethylethylendiamin oder *N,N*'-Dimethylethylendiamin und zweckmäßigerweise in Gegenwart einer Base wie Natriummethanolat, Natriumethanolat, Natrium-*tert*.-butylat, Kalium-*tert*.-butylat, Natrium-*tert*.-butyldimethyl-silanoat, Kaliumhexamethyldisilazan, Lithiumdiisopropylamid, Kaliumcarbonat, Rubidiumcarbonat, Cäsiumcarbonat, Kaliumphosphat, Natriumhydrid, gegebenenfalls in Gegenwart eines Komplexbildners wie 18-Krone-6-Ether sowie zweckmäßigerweise unter Anwendung einer Inertgasatmosphäre (beispielsweise Stickstoff oder Argon) und gegebenenfalls unter Druck durchgeführt.
   v) Acylierung/Sulfonylierung und Alkylierung einer Verbindung der allgemeinen Formel 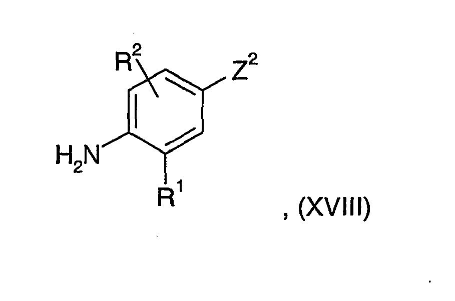 in der R¹ und R² wie eingangs erwähnt definiert sind und Z² die Nitrilgruppe oder eine C₁₋₅-Alkoxycarbonylgruppe darstellen, mit einer Verbindung der allgemeinen Formel 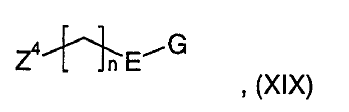 in der E eine Carbonyl-, Oxycarbonyl-, Sulfonyl- oder eine gegebenenfalls am Stickstoffatom einer wie eingangs erwähnten substituierten Sulfamoylgruppe, G ein Chlor-, Brom- oder lodatom oder eine Anhydrid-, C₁₋₅-Alkoxy- oder Benzotriazoloxygruppe oder E und G zusammen eine Isocyanogruppe und Z⁴ eine nucleofuge Austrittsgruppe, beispielsweise ein Chlor-, Brom- oder lodatom, eine Tosylat-, Triflat- oder Mesylatgruppe, darstellen und n eine Zahl zwischen 3 und 5 ist, wobei einzelne Methylengruppen gemäß der eingangs erwähnten Beschreibung zusätzlich substituiert oder durch Heteroatome ausgetauscht sein können, und anschließende intramolekulare Cyclisierung durch Alkylierung des anilidischen Stickstoffs unter Abspaltung der nucleofugen Austrittsgruppe Z⁴, gefolgt von Verseifung der Nitril- oder C₁₋₅-Alkoxycarbonylgruppe Z² und eventuelle weitere Umsetzung der daraus resultierenden Carboxylgruppe zu einem reaktiven Carbonsäurederivat der allgemeinen Formel XIII.
      Die Acylierung/Sulfonylierung wird zweckmäßigerweise in einem Lösungsmittel oder Lösungsmittelgemisch wie Benzol, Chlorbenzol, Toluol, Xylol, Glykoldimethylether, Diethylenglykoldimethylether, Dimethylformamid, *N-*Methylpyrrolidinon, Tetralin, Dimethylsulfoxid, Sulfolan, Methylenchlorid, Chloroform, Tetrachlormethan, *N-*Ethyl-diisopropylamin, *N-*C₁₋₅-Alkylmorpholin, *N-*C₁₋₅-Alkylpiperidin, *N-*C₁₋₅-Alkylpyrrolidin, Triethylamin, Pyridin, beispielsweise bei Temperaturen zwischen -30 und 250°C, vorzugsweise jedoch zwischen 0 und 150°C, zweckmäßigerweise in Gegenwart von Basen wie Pyridin, Triethylamin, *p-*Dimethylaminopyridin, Kaliumcarbonat, Natriumcarbonat, Kalium-*tert*.-butylat, Natriummethanolat, Natriumethanolat oder basischem Ionenaustauscher durchgeführt.
      Die nachfolgende intramolekulare Alkylierung wird zweckmäßigerweise in einem Lösungsmittel oder Lösungsmittelgemisch wie Benzol, Chlorbenzol, Toluol, Xylol, Glykoldimethylether, Diethylenglykoldimethylether, Dimethylformamid, Dimethylsulfoxid, Sulfolan, Methylenchlorid, Tetrachlormethan, *N-*Ethyl-diisopropylamin, *N*-C₁₋₅-Alkylmorpholin, *N-*C₁₋₅-Alkylpiperidin, *N-*C₁₋₅-Alkylpyrrolidin, Triethylamin, Pyridin beispielsweise bei Temperaturen zwischen -30 und 250°C, vorzugsweise jedoch zwischen 0 und 150°C, zweckmäßigerweise in Gegenwart von Basen wie Pyridin, Triethylamin, Kaliumcarbonat, Natriumcarbonat, Kalium-*tert*.-butylat, Natriummethanolat, Natriumethanolat, Natriumhydrid, Kaliumhexamethyldisilazan oder Lithiumdiisopropylamid durchgeführt.
   vi) Carbamoylierung / Harnstoffbildung mit einer Verbindung der allgemeinen Formel 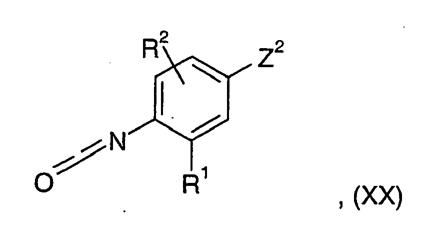 in der R¹ und R² wie eingangs erwähnt definiert sind und Z² die Nitrilgruppe oder eine C₁₋₅-Alkoxycarbonylgruppe darstellt, und die durch literaturbekannte Verfahren aus Verbindungen der allgemeinen Formel XVIII, beispielsweise durch Umsetzen mit Phosgen in Toluol, erhalten werden kann, mit einer Verbindung der allgemeinen Formel 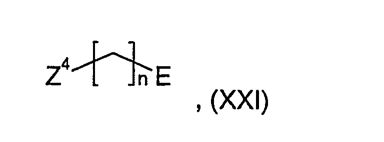 in der Z⁴ eine nucleofuge Austrittsgruppe, beispielsweise ein Chlor-, brom- oder lodatom, eine Tosylat-, Triflat- oder Mesylatgruppe, und E eine Hydroxyl-, Amino- oder C₁₋₃-Alkylaminofunktion darstellt und n eine Zahl zwischen 2 und 4 ist, wobei einzelne Methylengruppen zusätzlich gemäß der eingangs erwähnten Beschreibung substituiert sein können, und anschließende intramolekulare Cyclisierung durch Alkylierung des anilidischen Stickstoffs unter Abspaltung der nucleofugen Austrittsgruppe Z⁴, gefolgt von Verseifung der Nitril- oder C₁₋₅-Alkoxycarbonylgruppe Z² und eventuelle weitere Umsetzung der daraus resultierenden Carboxylgruppe zu einem reaktiven Carbonsäurederivat der allgemeinen Formel XIII.
      Die Carbamoylierung wird zweckmäßigerweise in einem Lösungsmittel oder Lösungsmittelgemisch wie Benzol, Chlorbenzol, Toluol, Xylol, Glykoldimethylether, Diethylenglykoldimethylether, Dimethylformamid, *N-*Methylpyrrolidinon, Tetralin, Dimethylsulfoxid, Sulfolan, Methylenchlorid, Chloroform, Tetrachlormethan, *N-*Ethyl-diisopropylamin, *N-*C₁₋₅-Alkylmorpholin, *N-*C₁₋₅-Alkylpiperidin, *N-*C₁₋₅-Alkylpyrrolidin, Triethylamin, Pyridin, beispielsweise bei Temperaturen zwischen -30 und 250°C, vorzugsweise jedoch zwischen 0 und 150°C, durchgeführt.
      Die nachfolgende intramolekulare Alkylierung wird beispielsweise analog wie unter v) beschrieben durchgeführt.
   vii) Ringschlussmetathese einer Verbindung der allgemeinen Formel 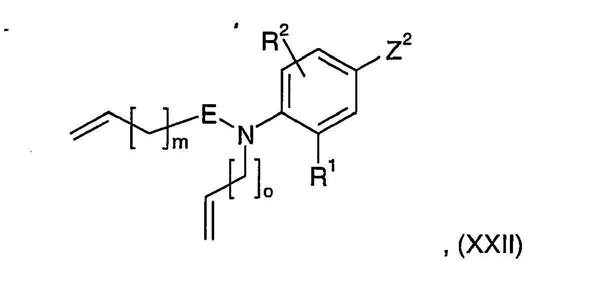 in der R¹ und R² wie eingangs beschrieben definiert sind, Z² eine Nitril-, C₁₋₅-Alkoxycarbonyl- oder Carboxylgruppe, E eine gegebenenfalls der eingangs erwähnten Beschreibung entsprechend substituierte Aminocarbonyl-, Aminosulfonyl- oder Aminogruppe oder eine Carbonyl-oder Sulfonylgruppe oder ein Sauerstoff- oder Schwefelatom oder eine Bindung darstellt, wobei m und o unabhängig voneinander gleiche oder verschiedene Zahlen zwischen 1 und 3 darstellen, die durch eine Sequenz aus Alkylierung und Acylierung / Sulfonylierung / Carbamoylierung / Sulfamoylierung mit entsprechenden Reagentien nach den hier schon beschriebenen oder literaturbekannten Verfahren erhalten werden können, gefolgt von Verseifung der Nitril- oder C₁₋₅-Alkoxycarbonylgruppe Z² und eventuelle weitere Umsetzung der daraus resultierenden Carboxylgruppe zu einem reaktiven Carbonsäurederivat der allgemeinen Formel XIII.
      Der Ringschluß durch Metathesereaktion wird zweckmäßigerweise in einem Lösungsmittel oder Lösungsmittelgemisch wie Benzol, Chlorbenzol, Toluol, Xylol, Methanol, Ethanol, Propanol, Diethylether, *tert*.-Butyl-methyl-ether, Tetrahydrofuran, Dioxan, Glykoldimethylether, Diethylenglykoldimethylether, Dimethylformamid, *N-*Methylpyrrolidinon, Tetralin, Dimethylsulfoxid, Sulfolan, Methylenchlorid, Chloroform, Tetrachlormethan, Pyridin, in Gegenwart eines Katalysators wie Benzyliden-bis-(tricyclohexylphosphin)-dichloro-ruthenium (Grubbs-Katalysator 1. Generation) oder Benzyliden-[1,3-bis(2,4,6-trimethylphenyl)-2-imidazolidinyliden]-dichloro-(tricyclohexylphosphin)-ruthenium (Grubbs-Katalysator 2. Generation) beispielsweise bei Temperaturen zwischen -30 und 250°C, vorzugsweise jedoch zwischen 0 und 150°C, zweckmäßigerweise unter einer Inertgas-Atmosphäre, beispielsweise Argon, durchgeführt.
(e) Zur Herstellung einer Verbindung der allgemeinen Formel 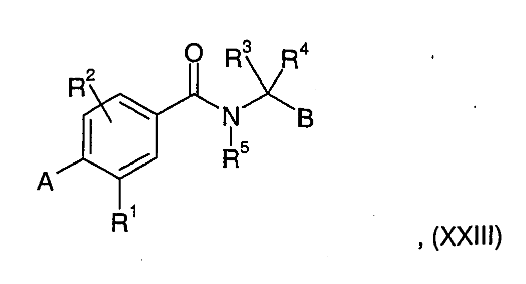 in der A, B und R¹ bis R⁵ wie eingangs erwähnt definiert sind:
   Acylierung einer Verbindung der allgemeinen Formel 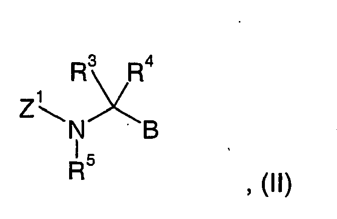 in der B und R³ bis R⁵ wie eingangs erwähnt definiert sind und Z¹ das Wasserstoffatom bedeuten,
   mit einer Carbonsäure oder einem reaktiven Carbonsäurederivat der allgemeinen Formel 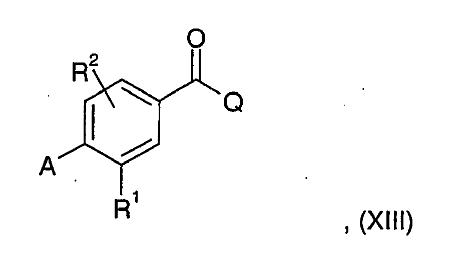 in der A, R¹ und R² wie eingangs erwähnt definiert sind und Q eine Hydroxy-oder C₁-₄-Alkoxygruppe, ein Halogenatom oder eine Acyloxygruppe darstellt.
   Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril, Dimethylformamid, Natronlauge oder Sulfolan gegebenenfalls in Gegenwart einer anorganischen oder organischen Base bei Temperaturen zwischen -20 und 200°C, vorzugsweise jedoch bei Temperaturen zwischen -10 und 160°C, durchgeführt.
   Die Acylierung kann jedoch auch mit der freien Säure gegebenenfalls in Gegenwart eines die Säure aktivierenden Mittels oder eines wasserentziehenden Mittels, z.B. in Gegenwart von Chlorameisensäureisobutylester, Thionylchlorid, Trimethylchlorsilan, Chlorwasserstoff, Schwefelsäure, Methansulfonsäure, *p*-Toluolsulfonsäure, Phosphortrichlorid, Phosphorpentoxid, *N,N*'-Dicyclohexylcarbodiimid, *N,N*'-Dicyclohexylcarbodiimid/*N*-Hydroxysuccinimid oder 1-Hydroxy-benztriazol, *N,N*'-Carbonyldiimidazol, *O*-(Benzotriazol-1-yl)-*N,N,N,N*'-tetramethyl-uroniumtetrafluorborat/*N*-Methylmorpholin, *O-*(Benzotriazol-1-yl)-*N,N,N',N*'-tetramethyl-uroniumtetrafluorborat/*N-*Ethyldiisopropylamin, *O-*Pentafluorophenyl-*N,N,N',N*'-tetramethyluronium-hexafluorophosphat/Triethylamin, *N,N*'-Thionyldiimidazol oder Triphenylphosphin/Tetrachlorkohlenstoff, bei Temperaturen zwischen -20 und 200°C, vorzugsweise jedoch bei Temperaturen zwischen -10 und 160°C, durchgeführt werden.
   Weitere Verfahren zur Amidkupplung sind beispielsweise in P.D. Bailey, I.D. Collier, K.M. Morgan in "Comprehensive Functional Group Interconversions", Vol. 5, Seite 257ff., Pergamon 1995 beschrieben.
(f) Zur Herstellung einer Verbindung der allgemeinen Formel (II), (XXIV), (VII), (VIII), (XI) oder (XXIII), in der A, B und R¹ bis R⁷ wie eingangs erwähnt definiert sind und R⁸ eine C₂₋₃-Alkinylgruppe bedeutet, die über das Kohlenstoffatom mit dem Aromaten verknüpft ist, das gleichzeitig die Dreifachbindung trägt, aus einer korrespondierenden Verbindung, bei der R⁸ ein Brom- oder lodatom oder die Triflat-, Boronsäure- oder Boronsäureester-Gruppe bedeutet:
   Übergangsmetall-katalysierte Kupplungsreaktion einer Verbindung der allgemeinen Formel 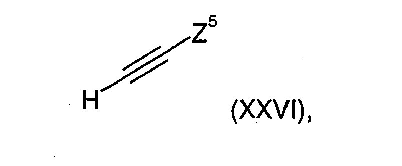 in der Z⁵ das Wasserstoffatom, die Methylgruppe oder eine Schutzgruppe wie beispielsweise eine Trimethylsilyl-, *tert.*-Butyl-dimethylsilyl-, *tert*.-Butyl-diphenylsilyl- oder Triisopropylgruppe bedeutet, die anschließend abgespalten werden kann,
   mit einer Verbindung der allgemeinen Formel (II), (XXIV), (VII), (VIII), (XI) oder (XXIII), in der A, B und R¹ bis R⁷ wie eingangs, erwähnt definiert sind und R⁸ ein Brom- oder lodatom oder die Triflat-, Boronsäure- oder Boronsäureester-Gruppe bedeutet.
   Die Umsetzung wird vorzugsweise in einem Lösungsmittel oder Lösungsmittelgemisch wie Acetonitril, Diethylether, Tetrahydrofuran, Dioxan, Wasser oder Dimethylformamid oder einem Lösungsmittelgemisch in Gegenwart eines Palladium-Katalysators wie beispielsweise Bis(triphenylphosphin)-palladium(II)chorid, Palladium(II)-[1,1'-bis-(diphenylphosphino)ferrocen]-chlorid oder Tetrakis-(triphenylphosphin)-palladium(0) in Gegenwart einer Base wie Triethylamin, *N*-Isopropyldiethylamin, *N,N*-Diisopropyl-ethylamin, Kalium-*tert*.-butylat, Natriumcarbonat oder Cäsiumcarbonat, gegebenenfalls in Gegenwart von Liganden wie Triphenylphosphin, Tri-o-tolylphosphin, Tri-*tert*.-butylphosphin, 1,3-Bis-(diphenylphosphino)-propan, 2,2'-Bis-(diphenylphosphino)-1,1'-dinaphthyl, 1,1'-Bis-(diphenylphosphino)-ferrocen, Xantphos und gegebenenfalls in Gegenwart einer Übergangsmetallverbindung wie einem Kupferhalogenid wie beispielsweise Kupfer(I)iodid und bei Temperaturen zwischen 20 und 120°C, vorzugsweise bei Temperaturen zwischen 20 und 90°C unter Argon- oder Stickstoffatmosphäre durchgeführt (siehe auch K. Sonogashira, Comprehensive Organic Synthesis, Vol. 3, Seite 52ff., Pergamon Press, Oxford 1991).

Die Abspaltung einer etwaig vorhandenen Silyl-Schutzgruppe wie beispielsweise Trimethylsilyl erfolgt vorzugsweise in einem Lösungsmittel oder Lösungsmittelgemisch wie Wasser, Methanol, Ethanol, Isopropanol, Aceton, Dioxan, Tetrahydrofuran oder Dimethylformamid in Gegenwart einer Base wie Lithiumhydroxid, Natriumhydroxid, Kaliumcarbonat oder Natriummethylat. Zur Abspaltung in organischen Lösungsmitteln wie beispielsweise Diethylether, Tetrahydrofuran, Dimethylformamid oder Dichlormethan eignen sich auch Fluoridreagenzien, wie beispielsweise Tetrabutylammoniumfluorid, Lithiumfluorid oder Kaliumfluorid, gegebenenfalls unter Zusatz eines Komplexbildners wie 18-Krone-6-Ether.

Bei den vorstehend beschriebenen Umsetzungen können gegebenenfalls vorhandene reaktive Gruppen wie Hydroxy-, Carboxy-, Amino-, Alkylamino-oder Iminogruppen während der Umsetzung durch übliche Schutzgruppen geschützt werden, welche nach der Umsetzung wieder abgespalten werden. Beispielsweise kommt als Schutzrest für eine Hydroxygruppe die Methoxy-, Benzyloxy-, Trimethylsilyl-, Acetyl-, Benzoyl-, *tert*.-Butyl-, Trityl-, Benzyl- oder Tetrahydropyranylgruppe,
als Schutzreste für eine Carboxylgruppe die Trimethylsilyl-, Methyl-, Ethyl-, *tert.-*Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, *tert*.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.
Weitere Schutzgruppen und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in

Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxygruppe erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines *tert*.-Butyl- oder *tert*.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder *n-*Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(1)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Die Herstellung von Verbindungen der allgemeinen Formeln (II), in der die Gruppe B' durch einen Rest der allgemeinen Formel (III) wiedergegeben wird, und (IV) kann beispielsweise analog K. Maekawa, J. Ohtani, Agr. Biol. Chem. 1976, 40, 791-799 erfolgen.

Ferner können die erhaltenen Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gegebenenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel I sowie deren Tautomere, deren Enantiomere, deren Diastereomere und deren physiologisch verträglichen Salze wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xahemmenden Wirkung, auf einer die aPTT-Zeit verlängernden Wirkung und/oder auf einer Hemmwirkung auf verwandte Serinproteasen wie z. B. Urokinase, Faktor VIIa, Faktor IXa, Faktor Xla und Faktor XIIa.

Die im Experimentellen Teil angeführten Verbindungen wurden auf ihre Wirkung auf die Hemmung des Faktors Xa wie folgt untersucht:

### Methodik:

Enzymkinetische Messung mit chromogenem Substrat. Die durch humanen Faktor Xa aus dem farblosen chromogenen Substrat freigesetzte Menge an *p-*Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die IC₅₀ berechnet als diejenige Konzentration, die den eingesetzten Faktor Xa um 50 % hemmt.

### Material:

Tris(hydroxymethyl)-aminomethan-Puffer (100 mMol) und Natriumchlorid (150 mmol), pH 8.0 plus 1 mg/ml Human Albumin Fraction V, Proteasefrei
Faktor Xa (Calbiochem), Spez. Aktivität: 217 IU/mg, Endkonzentration: 7 IU/ml pro Reaktionsansatz
Substrat S 2765 (Chromogenix), Endkonzentration: 0.3 mM/l (1 KM) pro Reaktionsansatz
Testsubstanz: Endkonzentration 100, 30, 10, 3, 1, 0.3, 0.1, 0.03, 0.01, 0.003, 0.001 µMol/l

Durchführung: 10 µl einer 23.5-fach konzentrierteren Ausgangslösung der Testsubstanz bzw. Lösungsmittel (Kontrolle), 175 µl TRIS/HSA-Puffer und 25 µl Faktor Xa-Gebrauchslösung von 65.8 U/L werden 10 Minuten bei 37°C inkubiert. Nach Zugabe von 25 µl S 2765-Gebrauchslösung (2.82 mMol/L) wird die Probe im Photometer (SpectraMax 250) bei 405 nm für 600 Sekunden bei 37°C gemessen.

### Auswertung:

1. Ermittlung der maximalen Zunahme (deltaOD/Minuten) über 21 Messpunkte.
2. Ermittlung der %-Hemmung bezogen auf die Lösungsmittelkontrolle.
3. Erstellen einer Dosiswirkungskurve (%-Hemmung vs Substanzkonzentration).
4. Ermittlung der IC₅₀ durch Interpolation des X-Wertes (Substanzkonzentration) der Dosiswirkungskurve bei Y = 50 % Hemmung.

Alle getesteten Verbindungen zeigten IC₅₀-Werte, die kleiner als 100 µmol/L sind.

Die erfindungsgemäß hergestellten Verbindungen sind im allgemeinen gut verträglich.

Aufgrund ihrer pharmakologischen Eigenschaften eignen sich die neuen Verbindungen und deren physiologisch verträgliche Salze zur Vorbeugung und Behandlung venöser und arterieller thrombotischer Erkrankungen, wie zum Beispiel der Vorbeugung und Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulcerativen Colitis, der Prophylaxe und Behandlung der Koronarthrombose, der Prophylaxe des Schlaganfalls und der Verhinderung der Occlusion von Shunts. Zusätzlich sind die erfindungsgemäßen Verbindungen zur antithrombotischen Unterstützung bei einer thrombolytischen Behandlung, wie zum Beispiel mit Alteplase, Reteplase, Tenecteplase, Staphylokinase oder Streptokinase, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, z.B. bei der Behandlung der pulmonalen Fibrose, zur Prophylaxe und Behandlung der rheumatoiden Arthritis, zur Verhütung oder Verhinderung von Fibrin-abhängigen Gewebsadhäsionen und/oder Narbengewebebildung sowie zur Förderung von Wundheilungsprozessen geeignet. Die neuen Verbindungen und deren physiologisch verträgliche Salze können therapeutisch in Kombination mit Acetylsalicylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

Die zur Erzielung einer entsprechenden Wirkung erforderliche Dosierung beträgt zweckmäßigerweise bei intravenöser Gabe 0.01 bis 3 mg/kg, vorzugsweise 0.03 bis 1.0 mg/kg, und bei oraler Gabe 0.03 bis 30 mg/kg, vorzugsweise 0.1 bis 10 mg/kg, jeweils 1 bis 4 x täglich.

Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern, ohne diese jedoch in ihrem Umfang zu beschränken:

### Experimenteller Teil

Für die hergestellten Verbindungen liegen in der Regel Schmelzpunkte, IR-, UV-, ¹H-NMR und/oder Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die unter der Bezeichnung Reversed-Phase-8 ermittelten R_{f}-Werte wurden unter Verwendung von DC-Fertigplatten RP-8 F₂₅₄ₛ (E. Merck, Darmstadt, Artikel-Nr. 1.15684) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX^{™}, 35-70 µm) verwendet. Falls nähere Angaben zur Konfiguration fehlen, bleibt offen, ob es sich um reine Stereoisomere oder um Enantiomeren-/Diastereomerengemische handelt.

In den Versuchsbeschreibungen werden die folgenden Abkürzungen verwendet:
- Boc: *tert*.-Butoxycarbonyl
- DIPEA: *N*-Ethyl-diisopropylamin
- DMSO: Dimethylsulfoxid
- DMF: *N,N*-Dimethylformamid
- ges.: gesättigt
- h: Stunde(n)
- i. Vak.: im Vakuum
- konz.: Konzentriert
- NMM: *N*-Methyl-morpholin
- NMP: *N*-Methyl-pyrrolidin-2-on
- *o*: ortho
- PfTU: *O*-Pentafluorophenyl-*N,N,N',N*'-tetramethyluronium-hexafluorophosphat
- PPA: Propanphosphonsäurecycloanhydrid
- quant.: quantitativ
- R_{f}: Retentionsfaktor
- Rₜ: Retentionszeit
- *rac.*: Räcemisch
- TBTU: *O*-(Benzotriazol-1-yl)-*N,N,N',N*'-tetramethyluroniumtetrafluorborat
- TEA: Triethylamin
- TFA: Trifluoressigsäure
- THF: Tetrahydrofuran
- *tert.*: tertiär
- Σ: Ausbeute über alle analog durchgeführten beschriebenen Stufen

Die HPLC/MS-Daten für die Beispiele 35 bis 38 wurden unter den folgenden Bedingungen erzeugt:
(a) Waters ZMD, Alliance 2690 HPLC, Waters 2700 Autosampler, Waters 996 Diodenarraydetektor

Als mobile Phase wurde eingesetzt:
A: Wasser mit 0.1 % TFA
B: Acetonitril mit 0.1 % TFA

| Zeit in min | %A | %B | Flussrate in ml/min |
|---|---|---|---|
| 0.0 | 95 | 5 | 1.00 |
| 0.1 | 95 | 5 | 1.00 |
| 5.1 | 2 | 98 | 1.00 |
| 6.5 | 2 | 98 | 1.00 |
| 7.0 | 95 | 5 | 1.00 |

Als stationäre Phase diente eine Säule Waters X-Terra™ MS C₁₈ 3.5 µm, 4.6 mm x 50 mm (Säulentemperatur: konstant bei 25°C)

Die Diodenarraydetektion erfolgte im Wellenlängenbereich 210-500 nm Bereich der massenspektrometrischen Detektion: m/z 120 bis m/z 950
(b) Die HPLC/MS-Daten für die übrigen Beispiele wurden, soweit angegeben, unter den folgenden Bedingungen erzeugt:
   HP 1100 mit quarternärer Pumpe, Gilson G215 Autosampler, HP Diodenarraydedektor.

Als mobile Phase wurde eingesetzt:
A: Wasser mit 0.1 % TFA
B: Acetonitril mit 0.08% TFA

| Zeit in min | %A | %B | Flussrate in ml/min |
|---|---|---|---|
| 0.0 | 95 | 5 | 0.4 |
| 0.15 | 95 | 5 | 0.4 |
| 4.65 | 2 | 98 | 0.4 |
| 6.0 | 2 | 98 | 0.4 |
| 6.5 | 95 | 5 | 0.4 |

Als stationäre Phase diente eine Säule Waters X-Terra^{™} MS C 18 2.5um, 2.1 mm x 50mm (Säulentemperatur konstant bei 25°C)

Die Diodenarraydetektion erfolgte in Wellenlängenbereich 210-550 nm Bereich der massenspektrometrischen Detektion:m/z 120 bis m/z 1000.

### Beispiele 1

3-Chlor-*N*-[(1*S*)-1-(5-chlor-1*H*-benzimidazol-2-yl)-ethyl]-4-([1,4]diazepan-1-yl)-benzamid

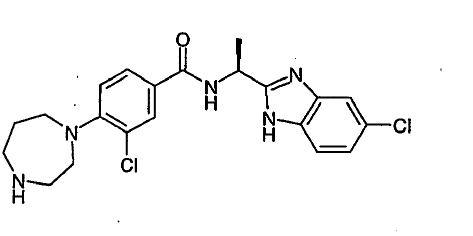

### (a) 4-(4-N-Acetyl-[1,4]diazepan-1-yl)-3-chlor-benzonitril

4.67 g (30 mmol) 3-Chlor-4-fluor-benzonitril und 4.27 g (30 mmol) *N*-1-Acetyl-[1,4]diazepan werden in 5.0 ml DIPEA für 6 Stunden bei 90°C gerührt. Anschließend wird i. Vak. eingeengt und der Rückstand in Dichlormethan gelöst. Die organische Phase wird 2 mal mit Wasser gewaschen, über Natriumsulfat getrocknet und i. Vak. eingeengt. Der Rückstand wird ohne weitere Reinigung weiter umgesetzt.
Ausbeute: 7.50 g (90 %)
R_{f}-Wert: 0.20 (Kieselgel; Dichlormethan/Ethanol = 50:1)

### (b) 3-Chlor-4-([1,4]diazepan-1-yl)-benzoesäure

Der in Beispiel 1a erhaltene Rückstand (7.50 g, 27.0 mmol) wird in 50 ml 25%-iger Kaliumhydroxid-Lösung für 8 h zum Rückfluß erhitzt. Anschließend wird mit konz. Salzsäure auf pH 5 eingestellt. Der ausfallende Feststoff wird abfiltriert, getrocknet und ohne weitere Reinigung weiter umgesetzt.
Ausbeute: 5.60 g (81 %)
R_{f}-Wert: 0.0 (Kieselgel; Dichlormethan/Ethanol = 9:1)

### (c) 3-Chlor-4-[1,4]diazepan-1-yl)-benzoesäure-methylester

Der in Beispiel 1b erhaltene Rückstand (3.00 g, 11.8 mmol) wird in 100 ml Methanol suspendiert und über 1 Stunde unter Rühren und Erhitzen zum Rückfluß Chlorwasserstoff eingeleitet. Anschließend wird i. Vak. eingeengt, der Rückstand mit 5%-iger Natriumhydrogencarbonat-Lösung versetzt und 3 mal mit Dichlormethan extrahiert. Die vereinigten organischen Phasen werden über Natriumsulfat getrocknet. Der nach Einengen i. Vak. verbleibende Rückstand wird ohne weitere Reinigung weiter umgesetzt.
Ausbeute: 2.25 g (71 %)
R_{f}-Wert: 0.10 (Kieselgel; Dichlormethan/Ethanol = 4:1 + 2% Ammoniak) C₁₃H₁₇ClN₂O₂ (268.75)
Massenspektrum: (M+H)⁺ = 269/271 (Chlorisotope)

### (d) 4-(4-N-Boc-[1,4]diazepan-1-yl)-3-chlor-benzoesäure-methylester

Der in Beispiel 1c erhaltene Rückstand (1.00 g, 3.72 mmol) wird in 10 ml Dichlormethan mit 0.53 g (3.80 mmol) Kaliumcarbonat versetzt und anschließend 0.83 g (3.80 mmol) Pyrokohlensäure-di-*tert*.-butylester zugetropft. Danach wird 16 Stunden bei Raumtemperatur gerührt. Anschließend wird mit Dichlormethan verdünnt, zweimal mit Wasser gewaschen, über Natriumsulfat getrocknet und i. Vak. eingeengt. Anschließend erfolgt Reinigung durch Chromatographie an Kieselgel (Eluens: Methylenchlorid/Ethanol 99:1).
Ausbeute: 1.34 g (98%)
R_{f}-Wert: 0.50 (Kieselgel; Dichlormethan/Ethanol = 50:1)
C₁₈H₂₅ClN₂O₄ (368.86)
Massenspektrum: (M+H)⁺ = 369/371 (Chlorisotope)

### (e) 4-(4-N-Boc-[1,4]diazepan-1-yl)-3-chlor-benzoesäure

0.60 g (1.63 mmol) 4-(4-*N-*Boc-[1,4]diazepan-1-yl)-3-chlor-benzoesäure-methylester werden in 10 ml Methanol gelöst, mit 4 ml 2-molarer Kaliumhydroxid-Lösung versetzt und 3 Stunden bei 40°C gerührt. Anschließend wird i. Vak. eingeengt, der Rückstand mit destilliertem Wasser verdünnt, mit gesättigter Kaliumhydrogensulfat-Lösung angesäuert und 3 mal mit Ethylacetat extrahiert. Die vereinigten organischen Phasen werden über Natriumsulfat getrocknet und eingedampft. Der Rückstand wird ohne weitere Reinigung weiter umgesetzt.
Ausbeute: 0.50 g (87 %)
R_{f}-Wert: 0.05 (Kieselgel; Dichlormethan/Ethanol = 50:1)
C₁₇H₂₃ClN₂O₄ (354.84)

### (f) 4-(4-N-Boc-[1.4]diazepan-1-yl)-3-chlor-N-[(1S)-1-(5-chlor-1H-benzimidazol-2-yl)-ethyl-benzamid

532 mg (1.50 mmol) 4-(4-*N*-Boc-[1,4]diazepan-1-yl)-3-chlor-benzoesäure werden in 15 ml THF suspendiert und nach Zugabe von 546 mg (1.70 mmol) TBTU und 646 mg (5.0 mmol) DIPEA 30 Minuten bei Raumtemperatur gerührt. Anschließend werden 403 mg (1.50 mmol) (1*S*)-1-(5-Chlor-1*H-*benzimidazol-2-yl)-ethylamin-dihydrochlorid zugefügt und weitere 16 Stunden bei Raumtemperatur gerührt. Dann wird i. Vak. eingeengt, der Rückstand in Ethylacetat gelöst, die organische Phase mit 5%-iger Natriumhydrogencarbonat-Lösung und Wasser gewaschen und über Natriumsulfat getrocknet. Nach Einengen i. Vak. wird der verbleibende Rückstand durch Chromatographie an Kieselgel gereinigt (Eluens-Gradient: Petrolether/Ethylacetat: 90:10 -> 60:40).
Ausbeute: 630 mg (79 %)
R_{f}-Wert: 0.50 (Kieselgel; Dichlormethan/Ethanol = 19:1)
C₂₆H₃₁Cl₂N₅O₃ (532.48)
Massenspektrum: (M+H)⁺ = 532/534/536 (Chlorisotope)

### (g) 3-Chlor-N-[(1S)-1-(5-chlor-1H-benzimidazol-2-yl)-ethyl]-4-([1,4]diazepan 1-yl)-benzamid

Eine Lösung von 610 mg (1.15 mmol) 4-(4-*N-*Boc-[1,4]diazepan-1-yl)-3-chlor-*N-*[(1*S*)-1-(5-chlor-1*H-*benzimidazol-2-yl)-ethyl]-benzamid in 30 ml Dichlormethan wird mit 3 ml TFA versetzt und 3 Stunden bei Raumtemperatur gerührt. Anschließend wird i. Vak. eingeengt, der Rückstand in Ethylacetat aufgenommen, mit 5%-iger Natriumhydrogencarbonat-Lösung und Wasser gewaschen und über Natriumsulfat getrocknet. Nach Einengen i. Vak. wird der Rückstand durch Chromatographie an Kieselgel gereinigt (Eluens-Gradient: Petrolether/Ethylacetat 80:20 -> 50:50).
Ausbeute: 380 mg (77%)
R_{f}-Wert: 0.10 (Kieselgel; Dichlormethan/Ethanol = 9:1)
C₂₁H₂₃Cl₂N₅O (432.36)
Massenspektrum: (M+H)⁺ = 432/434/436 (Chlorisotope)

Analog wurde folgende Verbindung hergestellt:

| **Nr.** | **Strukturformel** | **Ausbeute** | **Massenpeak(s)** | **R_{f}**-Wert / **Rₜ**-Wert |
|---|---|---|---|---|
| | **Name** | | | |
| **115** | | Σ: 13% | (M+H)⁺ = 462/464/466 (Chlorisotope) | 0.13 (Kieselgel, Dichlormethan/ Methanol = 9:1) |
| | *N-*[(1*R*)-1-(5-Chlor-1*H-*benzimidazol-2-yl)-2-methoxy-ethyl]-3-chlor-4-([1,4]-diazepan-1-yl)-benzamid | | | |

### Beispiel 2

4-(4-*N-*Boc-piperazin-1-yl)-*N-*[(1*S*)-1-(5-chlor-1*H-*benzimidazol-2-yl)-ethyl]-3-trifluormethyl-benzamid

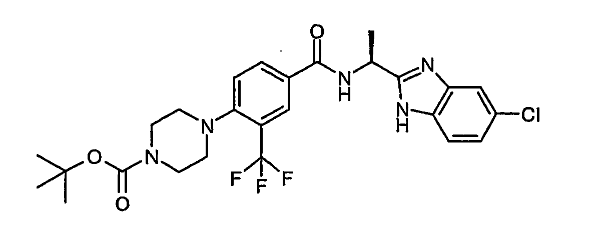

### (a) 4-(Piperazin-1-yl)-3-trifluormethyl-benzoesäure

37.9 g (195 mmol) Piperazin-Hexahydrat werden mit 12.4 g (66 mmol) 4-Fluor-3-trifluormethyl-benzonitril in 40 ml Ethanol suspendiert für 2 Stunden zum Rückfluß erhitzt. Dann werden 13.7 ml (261 mmol) 50%-ige Natronlauge sowie 13.7 ml Wasser zugegeben und weitere 3.5 Stunden zum Rückfluß erhitzt und weitere 15 Stunden bei Raumtemperatur gehalten. Anschließend werden 43.5 ml konz. Salzsäure zugegeben und unter Rühren 30 Minuten auf 10°C gekühlt. Der erhaltene Niederschlag wird abgesaugt, mit wenig Wasser gewaschen und 24 Stunden bei 40°C im Umlufttrockenschrank getrocknet.
Ausbeute: 20.8 g (quantitativ)

### (b) 4-(Piperazin-1-yl)-3-trifluormethyl-benzoesäure-methylester

5.00 g (18.2 mmol) 4-(Piperazin-1-yl)-3-trifluormethyl-benzoesäure werden in 50 ml methanolischer Salzsäure für 16 Stunden gerührt. Nach Einengen der Reaktionsmischung i. Vak. wird der Rückstand mit Isopropanol verrührt. Der Feststoff wird abfiltriert, mit Diethylether gewaschen und bei 60°C im Umlufttrockenschrank getrocknet.
Ausbeute: 5.00 g (76%)
C₁₃H₁₅F₃N₂O₂ * 2 HCl (288.27 / 361.19)
Massenspektrum: (M+H)⁺ = 289
R_{f}-Wert: 0.58 (Kieselgel; Cyclohexan/Dichlormethan/Methanol = 70:15:15 + 2% konz. Ammoniak-Lösung)

### (c) 4-(4-N-Boc-piperazin-1-yl)-3-trifluormethyl-benzoesäure-methylester

5.77 g (11.78 mmol) 4-(Piperazin-1-yl)-3-trifluormethyl-benzoesäure-methylester werden in 100 ml THF vorgelegt und mit 4.47 g (20.5 mmol) Pyrokohlensäure-di-*tert*.-butylester versetzt. Nach 40 Stunden Rühren bei Raumtemperatur unter Stickstoff-Atmosphäre wird die Reaktionsmischung i. Vak. eingeengt, der Rückstand mit Wasser und ges. Natriumchlorid-Lösung versetzt und mit Ethylacetat extrahiert. Die vereinigten organischen Phasen werden mit Wasser, halbgesättigter und ges. Natriumchlorid-Lösung gewaschen, über Magnesiumsulfat getrocknet und i. Vak. eingeengt. Der Rückstand wird durch Chromatographie an Kieselgel gereinigt (Eluens-Gradient: Petrolether/Ethylacetat 9:1 -> 8:1).
Ausbeute: 2.60 g (34%)
R_{f}-Wert: 0.52 (Kieselgel; Petrolether/Ethylacetat 7:3-+ 1 % konz. Ammoniak-Lösung)
C₁₈H₂₃F₃N₂O₄ (388.39)
Massenspektrum: (M+H)⁺ = 389

### (d) 4-(4-N-Boc-piperazin-1-yl)-3-trifluormethyl-benzoesäure

2.60 g (6.69 mmol) 4-(4-*N-tert*.-Butoxylcarbonyl-piperazin-1-yl)-3-trifluormethyl-benzoesäure-methylester werden in 10 ml Methanol gelöst und mit 12.3 ml (12.3 mmol) 1-molarer Natronlauge versetzt. Nach 15 Minuten werden weitere 10 ml Methanol zugegeben und das Reaktionsgemisch für 42 Stunden bei Raumtemperatur gerührt. Danach wird i. Vak. eingeengt, der Rückstand mit Eis versetzt und mit Essigsäure angesäuert. Der erhaltene Niederschlag wird abgesaugt, mit Wasser gewaschen und im Umlufttrockenschrank bei 50°C sowie in der Trockenpistole bei 40°C über KOH und SiO₂ getrocknet.
Ausbeute: 2.40 g (96%)
R_{f}-.Wert: 0.41 (Kieselgel; Petrolether/Ethylacetat = 1:1 + 1 % Essigsäure) C₁₇H₂₁F₃N₂O₄ (374.36)
Massenspektrum: (M-H)⁻ = 373

### (e) 4-(4-N-Boc-piperazin-1-yl)-N-[1S)-1-(5-chlor-1H-benzimidazol-2-yl)-ethyl]-3-trifluormethyl-benzamid

Hergestellt analog Beispiel 1f aus 4-(4-*N-*Boc-piperazin-1-yl)-3-trifluormethyl-benzoesäure, TBTU, NMM und (1*S*)-1-(5-Chlor-1*H-*benzimidazol-2-yl)ethylamin in NMP und anschließender Reinigung durch Chromatographie an Kieselgel (Eluens: Petrolether/Ethylacetat = 50:50).
Ausbeute: 57%
R_{f}-Wert: 0.20 (Kieselgel; Petrolether/Ethylacetat = 1:1)
C₂₆H₂₉ClF₃N₅O₃ (552.00)
Massenspektrum: (M+H)⁺ = 552/554 (Chlorisotope)

### Beispiel 3

*N*-[(1*S*)-1-(5-Chlor-1*H-*benzimidazol-2-yl)-ethyl]-4-(piperazin-1-yl)-3-trifluormethyl-benzamid

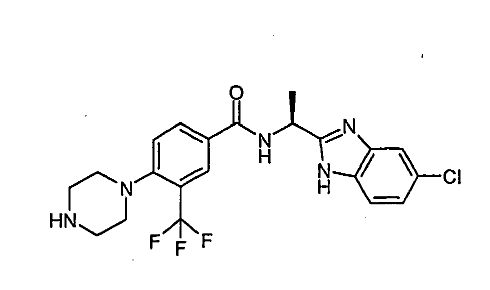

Hergestellt analog Beispiel 1g aus 4-(4-*N-*Boc-piperazin-1-yl)-*N-*[(1*S*)-1-(5-chlor-1*H-*benzimidazol-2-yl)-ethyl]-3-trifluormethyl-benzamid und TFA in Dichlormethan.
Ausbeute: 73%
R_{f}-Wert: 0.28 (Kieselgel; Dichlormethan/Methanol = 90:10 + Ammoniak-Lösung) C₂₁H₂₁ClF₃N₅O * 2 CF₃COOH (679.93 / 451.88)
Massenspektrum: (M+H)⁺ = 452/454 (Chlorisotope)

Analog der in Beispiel 2 und 3 beschriebenen Sequenz wurden hergestellt:

| **Nr.** | **Strukturformel** | **Ausbeute** | **Massenpeak(s)** | **R_{f}-Wert** |
|---|---|---|---|---|
| | **Name** | | | |
| **4** | 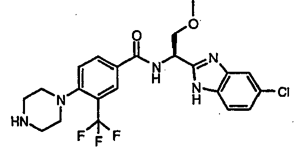 | Σ: 14% | (M+H)⁺ = 482/484 (Chlorisotope) | 0.15 (Kieselgel, CH₃COOC₂H₅ /C₂H₅OH 9:1 + NH₃) |
| | *N*-[(1*R)-1*-(5-Chlor-1*H*-benzimidazol-2-yl)-2-methoxy-ethyl)-4-(piperazin-1-yl)-3-trifluormethyl-benzamid | | | |
| **40** | 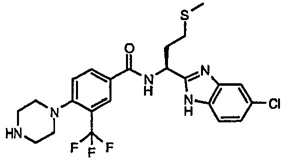 | Σ: 13% | (M+H)⁺ = 512/514 (Chlorisotope) | 0.20 (Kieselgel, CH₂Cl₂ /C₂H₅OH 9:1) |
| | *N*-[(1*S)*-1-(5-Chlor-1*H*-benzimidazol-2-yl)-3-methylsulfanyl-propyl)-4-(piperazin-1-yl)-3-trifluormethyl-benzamid | | | |

### Beispiel 5

4-(4-*N-*Acetyl-piperazin-1-yl)-*N-*[(1*S*)-1-(5-chlor-1*H*-benzimidazol-2-yl)-ethyl]-3-trifluormethyl-benzamid 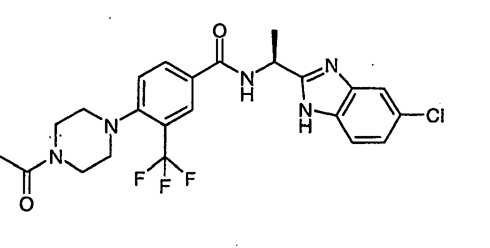 220 mg (0.32 mmol) *N-*[(1*S*)-1-(5-Chlor-1*H-*benzimidazol-2-yl)-ethyl]-4-(piperazin-1-yl)-3-trifluormethyl-benzamid-ditrifluoracetat werden in 5 ml THF mit 0.23 ml (1.65 mmol) TEA versetzt, unter Rühren und Kühlen im Eisbad 50 µl (0.70 mmol) Acetylchlorid zugetropft und 2 Stunden bei Raumtemperatur gerührt. Die Reaktionsmischung wird in Eiswasser eingegossen und anschließend mit Ethylacetat extrahiert. Die organische Phase wird mit Wasser und ges. Natriumchlorid-Lösung gewaschen, über Natriumsulfat getrocknet und i. Vak. eingeengt. Der Rückstand wird mit einem Lösungsmittelgemisch aus Ethylacetat und Diethylether verrieben, abfiltriert, mit Diethylether gewaschen und in einer Trockenpistole bei 50°C getrocknet.
Ausbeute: 0.13 g (81 %)
R_{f}-Wert: 0.55 (Kieselgel; Dichlormethan/Ethanol = 9:1 + Ammoniak-Lösung) C₂₃H₂₃ClF₃N₅O₂ (493.92)
Massenspektrum: (M+H)⁺ = 494/496 (Chlorisotope)

### Beispiel 6

4-(Azepan-2-on-1-yl)-*N-*[(1*S*)-1-(5-chlor-1*H-*benzimidazol-2-yl)-ethyl]-3-methyl-benzamid

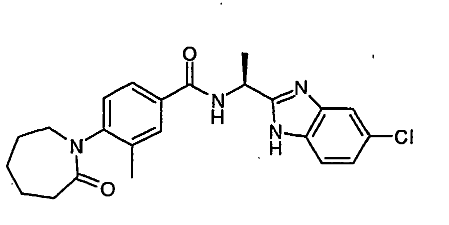

### (a) 4-(6-Brom-hexanol-amino)-3-methyl-benzoesäure-methylester

Zu 1.65 g (10 mmol) 4-Amino-3-methyl-benzoesäure-methylester in 50 ml THF mit 2 ml DIPEA wird unter Rühren bei Raumtemperatur langsam eine Lösung von 2.14 g (10 mmol) 6-Brom-hexanoylchlorid in 5.0 ml THF getropft. Nach 16 Stunden Rühren bei Raumtemperatur wird i. Vak. eingeengt, der Rückstand in Dichlormethan gelöst, 2 mal mit Wasser gewaschen und über Natriumsulfat getrocknet. Der nach Einengen i. Vak. verbleibende Rückstand wird ohne weitere Reinigung weiter umgesetzt.
Ausbeute: 3.30 g (96%)
R_{f}-Wert: 0.40 (Kieselgel; Petrolether/Ethylacetat = 4:1)
C₁₅H₂₀BrNO₃ (342.24)

### (b) 4-(Azepan-2-on-1-yl)-3-methyl-benzoesäure-methylester

3.20 g (9.35 mmol) des in Beispiel 5a erhaltenen Produkts wird in einer frisch hergestellten Lösung aus 1.00 g (43.5 mmol) Natrium in 80 ml Methanol 4 Stunden zum Rückfluß erhitzt. Anschließend wird i. Vak. eingeengt, der Rückstand mit 2-molarer Essigsäure angesäuert und mit Ethylacetat extrahiert. Die vereinigten organischen Phasen werden mit Wasser gewaschen und über Natriumsulfat getrocknet. Der nach Einengen i. Vak. erhaltene Rückstand wird ohne weitere Reinigung weiter umgesetzt.
Ausbeute: 1.90 g (verunreinigtes Produkt)
R_{f}-Wert: 0.30 (Kieselgel; Dichlormethan/Ethanol = 50:1)
C₁₅H₁₉NO₃ (261.32)
Massenspektrum: (M+H)⁺ = 262

### (c) 4-(Azepan-2-on-1-yl)-3-methyl-benzoesäure

1.90 g des in Beispiel 5b erhaltenen Produkts wird in 30 ml Methanol mit 10 ml 2-molarer Natronlauge versetzt und 16 Stunden bei Raumtemperatur gerührt. Nach Einengen i. Vak. wird der Rückstand mit Wasser versetzt und mit konz. Salzsäure angesäuert. Der auftretende Niederschlag wird abfiltriert und getrocknet. Nach Lösen in Methanol und Aufziehen auf Kieselgel erfolgt Reinigung durch Chromatographie an Kieselgel (Eluens-Gradient: Petrolether/Ethylacetat 70:30 -> 50:50).
Ausbeute: 0.23 g (10% über 2 Stufen)
R_{f}-Wert: 0.10 (Kieselgel; Petrolether/Ethylacetat = 1:2)
C₁₄H₁₇NO₃ (247.30)
Massenspektrum: (M+H)⁺ = 248

### (d) 4-(Azepan-2-on-1-yl)-N-[(1S)-1-(5-chlor-1H-benzimidazol-2-yl)-ethyl]-3-methyl-benzamid

Hergestellt analog Beispiel 1f aus 4-(Azepan-2-on-1-yl)-3-methyl-benzoesäure, TBTU, DIPEA und (1*S*)-1-(5-Chlor-1*H-*benzimidazol-2-yl)ethylamin in THF und anschließender Reinigung durch Chromatographie an Kieselgel (Eluens-Gradient: Ethylacetat/Ethanol 95:5 -> 90:10).
Ausbeute: 61%
R_{f}-Wert: 0.30 (Kieselgel; Ethylacetat)
C₂₃H₂₅ClN₄O₂ (424.93)
Massenspektrum: (M+H)⁺ = 425/427 (Chlorisotope)

Analog wurden folgende Verbindungen hergestellt:

| **Nr.** | **Strukturformel** | **Ausbeute** | **Massenpeak(s)** | **R_{f}-Wert** |
|---|---|---|---|---|
| | **Name** | | | |
| **7** | 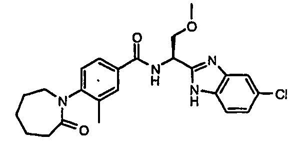 | Σ: 1.1 % | (M-H)⁻ = 453/455 (Chlorisotope) | 0.60 (Aluminiumoxid, CH₂Cl₂/C₂H₅OH 19:1) |
| | 4-(Azepan-2-on-1-yl)-*N-*[(1*R*)-1-(5-chlor-1*H-*benzimidazol-2-yl)-2-methoxy-ethyl]-3-methyl-benzamid | | | |
| **8** | 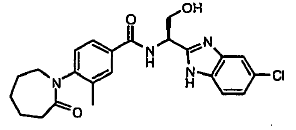 | Σ: 7.6% | (M+H)⁺ = 441/443 Chlorisotope) CH₂Cl₂/C₂H₅OH | 0.20 (Kieselgel, 19:1) |
| | 4-(Azepan-2-on-1-yl)-*N-*[(1*R*)-1-(5-chlor-1*H-*benzimidazol-2-yl)-2-hydroxy-ethyl]-3-methyl-benzamid | | | |
| **10** | 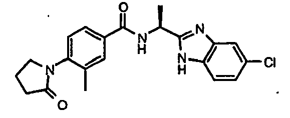 | Σ: 72% | (M+H)⁺ = 397/399 (Chlorisotope) | 0.50 (Kieselgel, CH₂Cl₂ /C₂H₅OH 9:1) |
| | *N-*[(1*S*)-1-(5-Chlor-1*H-*benzimidazol-2-yl)-ethyl]-3-methyl-4-(pyrrolidin-2-on-1-yl)-benzamid | | | |
| **11** | 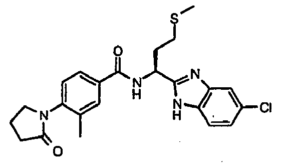 | Σ: 24% | (M+H)⁺ = 457/459 (Chlorisotope) | 0.63 (Kieselgel, CH₂Cl₂ /C₂H₅OH 9:1) |
| | *N-*[(1*S*)-1-(5-Chlor-1*H-*benzimidazol-2-yl)-3-methylsulfanyl-propyl]-3-methyl-4-(pyrrolidin-2-on-1-yl)-benzamid | | | |

### Beispiel 9

*N-*[(1*S*)-1-(5-chlor-1*H-*benzimidazol-2-yl)-ethyl]-4-(pyrrolidin-2-on-1-yl)-benzamid

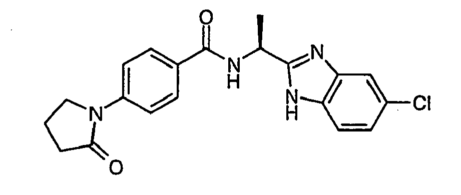

### (a) 4-(Pyrrolidin-2-on-1-yl)-benzoesäure

19.2 g (140 mmol) 4-Amino-benzoesäure werden zusammen mit 25.8 ml (180 mmol) 4-Brom-buttersäure-ethylester in 100 ml DMF für 20 Stunde zum Rückfluß erhitzt. Nach Einengen i. Vak. wird der Rückstand mit 100 ml Wasser und 50 ml Petrolether versetzt und 50 Minuten kräftig gerührt. Der Niederschlag wird abfiltriert, aus Ethanol umkristallisiert und bei 80°C getrocknet.
Ausbeute: 9.36 g (33%)
C₁₁H₁₁NO₃ (205.22)
Massenspektrum: (M+H)⁺ = 206

### (b) N-[(1S)-1-(5-Chlor-1H-benzimidazol-2-yl)-ethyl]-4-(pyrrolidin-2-on-1-yl)-benzamid

Hergestellt analog Beispiel 1f aus 4-(Pyrrolidin-2-on-1-yl)-benzoesäure, TBTU, DIPEA und (1*S*-1-(5-Chlor-1*H-*benzimidazol-2-yl)-ethylamin in THF und anschließender Reinigung durch Waschen der Lösung des Rückstands in Ethylacetat mit Wasser, verdünnter Natriumhydrogencarbonat-Lösung, Wasser und ges. Natriumchlorid-Lösung, Trocknen über Natriumsulfat und Entfernen des Lösungsmittels i. Vak..
Ausbeute: 61%
R_{f}-Wert: 0.50 (Kieselgel; Dichlormethan/Ethanol = 9:1)
C₂₀H₁₉ClN₄O₂ (382.85)
Massenspektrum: (M+H)⁺ = 383/385 (Chlorisotope)

### Beispiel 12

3-1Chlor-*N-*[(1*S*)-1-(5-chlor-1*H-*benzimidazol-2-yl)-ethyl]-4-(4-*N-*methyl-[1,4]diazepan-1-yl)-benzamid

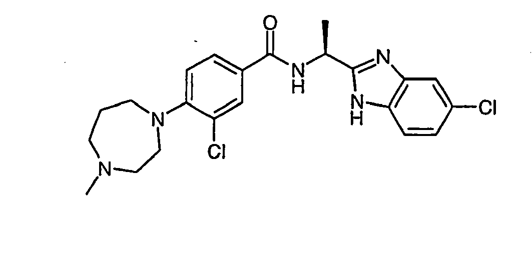

### (a) 3-Chlor-4-(4-N-methyl-[1,4]diazepan-1-yl)-benzonitril

Hergestellt analog Beispiel 1a aus 3-Chlor-4-fluor-benzonitril und 1-Methyl-[1,4]diazepan in DIPEA mit anschließender Reinigung durch Chromatographie an Kieselgel (Eluens-Gradient: Dichlormethan/Ethanol 98:2 -> 94:6). Ausbeute: 71%
R_{f}-Wert: 0.20 (Kieselgel; Methylenchlorid/Ethanol = 19:1)
C₁₃H₁₆ClN₃ (249.75)
Massenspektrum: (M+H)⁺ = 250/252 (Chlorisotope)

### (b) 3-Chlor-4-(4-N-methyl-[1,4]diazepan-1-yl)-benzoesäure

Hergestellt analog Beispiel 1b aus 3-Chlor-4-(4-methyl-[1,4]diazepan-1-yl)-benzonitril in 25%-iger wässriger Kaliumhydroxid-Lösung.
Ausbeute: 99%
C₁₃H₁₇ClN₂O₂ * HCl (268.74/305.21)
Massenspektrum: (M+H)⁺ = 269/271 (Chlorisotope)

### (c) 3-Chlor-N-[(1S)-1-(5-chlor-1H-benzimidazol-2-yl)-ethyl]-4-(4-N-methyl-[1,4]diazepan-1-yl)-benzamid

Hergestellt analog Beispiel 1f aus 3-Chlor-4-(4-methyl-[1,4]diazepan-1-yl)-benzoesäure, (*S*)-1-(5-Chlor-1*H-*benzimidazol-2-yl)-ethylamin, TBTU und DIPEA in THF mit anschließender Reinigung durch Chromatographie an Kieselgel (Eluens-Gradient: Petrolether/Ethylacetat 50:50 -> 20:80).
Ausbeute: 34%
R_{f}-Wert: 0.30 (Kieselgel; Dichlormethan/Ethanol = 9:1 + 1 % Ammoniak-Lösung) C₂₂H₂₅Cl₂N₅O (446.38)
Massenspektrum: (M+H)⁺ = 446/448/450 (Chlorisotope)

### Beispiel 13

3-Chlor-*N-*[(1*S*)-1-(5-chlor-1*H-*benzimidazol-2-yl)-ethyl]-4-(2-methyl-pyrrolidin-1-yl)-benzamid

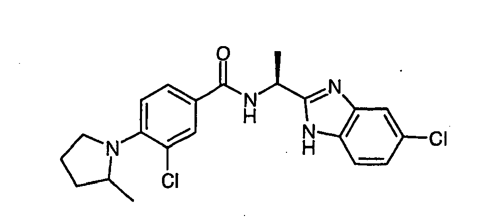

### (a) 3-Chlor-4-(2-methyl-pyrrolidin-1-yl)-benzonitril

5.90 g (37.9 mmol) 3-Chlor-4-fluor-benzonitril werden unter Stickstoff-Atmosphäre in 65 ml DMF gelöst und mit 5.45 g (39.5 mmol) Kaliumcarbonat und 4.2 ml (3.5 g, 39.5 mmol) 2-Methyl-pyrrolidin versetzt. Nach Rühren für 2.5 Tage bei 90°C wird das Reaktionsgemisch in 400 ml Wasser eingegossen und mit Ethylacetat extrahiert. Die vereinigten organischen Phasen werden mehrfach mit verdünnter und ges. Natriumchlorid-Lösung gewaschen, über Magnesiumsulfat getrocknet und i. Vak. eingeengt. Der verbleibende Rückstand wird ohne weitere Reinigung weiter umgesetzt.
Ausbeute: 7.90 g (94%)
R_{f}-Wert: 0.40 (Kieselgel; Petrolether/Ethylacetat = 9:1 + 0.5% Ammoniak-Lösung)
C₁₂H₁₃ClN₂ (220.70)
Massenspektrum: (M+H)⁺ = 221/223 (Chlorisotope)

### (b) 3-Chlor-4-(2-methyl-pyrrolidin-1-yl)-benzoesäure

8.0 g (40 mmol) des bei Beispiel 13a gewonnenen Produkts werden in einem Gemisch aus 65 ml 10-molarer Natronlauge und 65 ml Ethanol 2.75 Stunden bei 90°C gerührt. Danach wird das Reaktionsgemisch in Eiswasser eingegossen, mit konz. Salzsäure versetzt und flüchtige organische Bestandteile i. Vak. eingeengt. Die verbleibende wässrige Phase wird mit Dichlormethan extrahiert, mit Eis versetzt und mit halbkonzentrierter Salzsäure und 2-normaler Kaliumhydrogensulfat-Lösung auf pH 4.5 eingestellt. Der entstehende Niederschlag wird noch 10 Minuten gerührt, dann abfiltriert, mit Wasser gewaschen und bei 55°C getrocknet.
Ausbeute: 8.30 g (87%)
R_{f}-Wert: 0.55 (Kieselgel; Petrolether/Ethylacetat = 6:4+1% Essigsäure)
C₁₂H₁₄ClNO₂ (238.72)
Massenspektrum: (M+H)⁺ = 240/242 (Chlorisotope)

### (c) 3-Chlor-N-[(1S)-1-(5-chlor-1H-benzimidazol-2-yl)-ethyl)-4-(2-methyl-pyrrolidin-1-yl)-benzamid

Hergestellt analog Beispiel 1f aus 3-Chlor-4-(2-methyl-pyrrolidin-1-yl)-benzoesäure, (1*S*)-1-(5-Chlor-1*H-*benzimidazol-2-yl)-ethylamin, TBTU und TEA in DMF, dann Ausfällen durch Eingießen in verdünnte Natriumhydrogencarbonat-Lösung, Abfiltrieren und Waschen mit Wasser. Anschließend wird bei 55°C getrocknet.
Ausbeute: 92%
R_{f}-Wert: 0.66 (Kieselgel; Dichlormethan/Ethanol = 9:1)
C₂₁H₂₂Cl₂N₄O (417.34)
Massenspektrum: (M-H)⁻ = 415/417/419 (Chlorisotope)

Analog wurden folgende Verbindungen hergestellt:

| **Nr.** | **Strukturformel** | **Ausbeute** | **Massenpeak(s)** | **R_{f}-Wert / Rₜ-Wert** |
|---|---|---|---|---|
| | **Name** | | | |
| **14** | 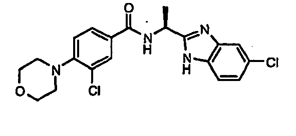 | Σ: 11% | (M+H)⁺ = 419/421/423 (Chlorisotope) | 0.55 (Kieselgel, Petrolether/CH₃COOC₂H₅ 19:1) |
| | *N-*[(1*S*)-1-(5-Chlor-1*H-*benzimidazol-2-yl)-ethyl]-3-chlor-4-(morpholin-4-yl)-benzamid | | | |
| **15** | 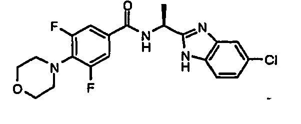 | Σ: 19% | (M+H)⁺ = 421/423 (Chlorisotope) | 0.30 (Kieselgel, Petrolether/CH₃COOC₂H₅ 1:1) |
| | *N-*[(1*S*)-1-(5-Chlor-1*H-*benzimidazol-2-yl)-ethyl]-3,5-difluor-4-(morpholin-4-yl)-benzamid | | | |
| **16** | 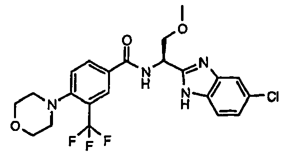 | Σ: 17% | (M+H)⁺ = 483/485 (Chlorisotope) | 3.46 min |
| | *N-*[(1*R*)-1-(5-Chlor4-*H-*benzimidazol-2-yl)-2-methoxy-ethyl]-3-trifluormethyl-4 (morpholin-4-yl)-benzamid | | | |
| **17** | 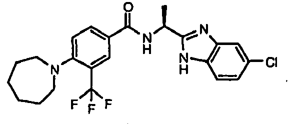 | Σ: 13% | (M+H)⁺ = 465/467 (Chlorisotope) | 3.21 min |
| | 4-(Azepan-1-yl)-*N-*[(1*S*)-1-(5-chlor-1*H-*benzimidazol-2-yl)-ethyl]-3-trifluormethyl-benzamid | | | |
| **18** | 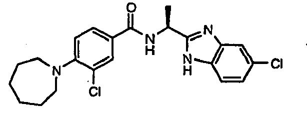 | Σ: 20% | (M+H)⁺ = 431/433/435 (Chlorisotope) | 3.02 min |
| | 4-(Azepan-1-yl)-3-chlor-*N-*[(1*S*)-1-(5-chlor-1*H-*benzimidazol-2-yl)-ethyl]-benzamid | | | |
| **19** | 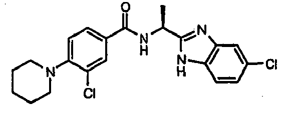 | Σ: 49% | (M+H)⁺ = 417/419/421 (Chlorisotope) | 0.47 (Kieselgel, CH₂Cl₂/Isopropanol 19:1) |
| | 3-Chlor-*N-*[(1*S*)-1-(5-chlor-1*H-*benzimidazol-2-yl)-ethyl]-4-(piperidin-1-yl)-benzamid | | | |
| **20** | 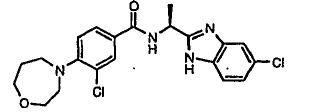 | Σ: 6.2% | (M+H)⁺= 433/435/437 (Chlorisotope) | 2.61 imin |
| | 3-Chlor-*N-*[(1*S*)-1-(5-chlor-1*H-*benzimidazol-2-yl)-ethyl]-4-([1,4]oxazepan-4-yl)-benzamid | | | |
| **37** | 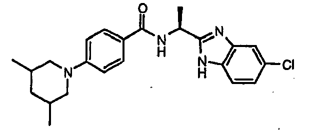 | Σ: 9.4% | (M+H)⁺ = 411/413 (Chlorisotope) | 0.53 (Kieselgel, CH₂Cl₂/CH₃OH 19:1) |
| | *N-*[(1*S*)-1-(5-Chlor-1*H-*benzimidazol-2-yl)-ethyl]-4-(3,5-dimethylpiperidin-1-yl)-benzamid | | | |
| **38** | 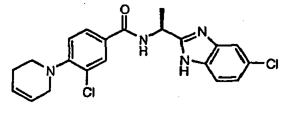 | Σ: 20% | (M-H)⁻ = 413/415/417 (Chlorisotope) | 2.84 min |
| | 3-Chlor-*N-*[(1*S*)-1-(5-chlor-1*H-*benzimidazol-2-yl)-ethyl]-4-(3,4-di-dehydro-piperidin-1-yl)-benzamid | | | |
| **50** | 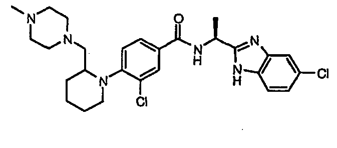 | Σ: 1.2% | (M+H)⁺ = 529/531/533 (Chlorisotope) | 0.29 (Kieselgel, CH₂Cl₂/CH₃OH 9:1) 2.38 min |
| | 3-Chlor-*N-*[(1*S*)-1-(5-chlor-1*H-*benzimidazol-2-yl)-ethyl]-4-[2-(4-methyl-piperazin-1-yl-methyl)-piperidin-1-yl)-benzamid | | | |

### Beispiel 21

*N-*[(1*R*)-1-(5-Chlor-1*H-*benzimidazol-2-yl)-2-methoxy-ethyl]-4-(morpholin-3-on-4-yl)-3-trifluormethyl-benzamid

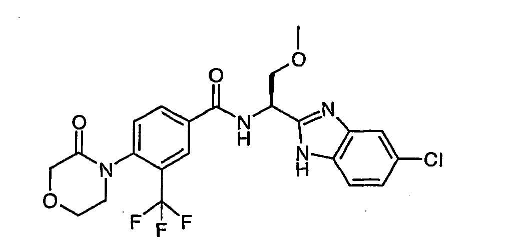

### (a) 4-(Morpholin-3-on-4-yl)-3-trifluormethyl-benzoesäure

1.00 g (3.63 mmol) 4-(Morpholin-4-yl)-3-trifluormethyl-benzoesäure (hergestellt durch Synthesesequenz analog Beispiel 13a und 13b) wird in 40 ml Wasser suspendiert und 150 mg (3.75 mmol) Natriumhydroxid zugegeben. Anschließend werden 1.73 g (10.92 mmol) Kaliumpermanganat zugefügt und 1.5 Stunden bei 45°C gerührt. Danach wird die Reaktionsmischung im Eisbad abgekühlt und bis zur vollständigen Entfärbung Natriumthiosulfat zugegeben. Nach 3-maliger Extraktion mit Ethylacetat werden die vereinigten organischen Phasen über Natriumsulfat getrocknet und i. Vak. eingeengt. Nach Aufziehen des Rückstandes auf Kieselgel wird dieser durch Chromatographie an Kieselgel gereinigt (Eluens: Dichlormethan/Methanol 95:5).
Ausbeute: 340 mg (32%)
C₁₂H₁₀F₃NO₄ (289.21)
Massenspektrum: (M+H)⁺ = 290

### (b) N'-(2-Amino-4-chlor-phenyl)-N-Boc-(S)-O-methyl-serinamid und N'-(2-Amino-5-chlor-phenyl)-N-Boc-(S)-O-methyl-serinamid

30.0 g (137 mmol) *N-*Boc-(*S*)-*O*-Methyl-serin werden zusammen mit 21.9 g (154 mmol) 4-Chlor-1,2-phenylendiamin in 658 ml THF gelöst und unter Rühren im Eisbad 43.9 ml (316 mmol) Triethylamin sowie 103 ml (173 mmol) einer 50 %-igen Lösung von PPA in Ethylacetat zugefügt. Nach 15 Minuten Rühren im Eisbad wird der Ansatz auf Raumtemperatur erwärmt, in Wasser eingegossen und die wässrige Phase mit Ethylacetat extrahiert. Die vereinigten organischen Phasen werden mit ges. Natriumcarbonat-Lösüng und Wasser gewaschen, über Natriumsulfat getrocknet und i. Vak. eingeengt. Der Rückstand wird durch Chromatographie an Kieselgel gereinigt (Eluens-Gradient: Dichlormethan/Methanol = 30:1 -> 9:1).
Ausbeute: 33.47 g (72 %) Gemisch beider Regioisomere
C₁₅H₂₂ClN₃O₄ (343.81)
Massenspektrum: (M-H)⁻ = 342/344 (Chlorisotope)
R_{f}-Wert: 0.80 (Kieselgel; Dichlormethan/Methanol = 9:1)

### (c) (1R)-N-Boc-1-(5-Chlor-1H-benzimidazol-2-yl)-2-methoxy-ethylamin

26.01 g (75.65 mmol) des bei Beispiel 21 b erhaltenen Gemisches werden in 1500 ml Toluol gelöst und 20.8 ml (364 mmol) Essigsäure sowie 10.0 g Molekularsieb, 4Ǻ, zugefügt. Das Reaktionsgemisch wird für 5 Stunden bei 60°C gerührt. Die Reaktionsmischung wird filtriert, mit Ethylacetat nachgewaschen und die organische Phase mit halbges. Natriumhydrogencarbonat-Lösung gewaschen, über Natriumsulfat getrocknet und i. Vak. eingeengt. Der Rückstand wird mit Diethylether verrührt und die entstehenden Kristalle abgesaugt. Das Filtrat wird i. Vak. eingeengt und der Rückstand durch dreimalige Chromatographie an Kieselgel gereinigt (Eluens-Gradient: Dichlormethan/Methanol 80:1 -> 50:1).
Ausbeute: 13.85 g (56 %)
C₁₅H₂₀ClN₃O₃ (325.79)
Massenspektrum: (M+H)⁺ = 326/328 (Chlorisotope)
R_{f}-Wert: 0.29 (Kieselgel; Dichlormethan/Methanol = 30:1)

### (d) (1R)-1-(5-Chlor-1H-benzimidazol-1-yl)-2-methoxy-ethylamin

0.50 g (1.54 mmol) (1R)-*N-*Boc-1-(5-Chlor-1*H-*benzimidazol-2-yl)-2-methoxyethylamin werden in 1.5 ml Dichlormethan mit 1.54 ml (20.0 mmol) TFA versetzt und bei Raumtemperatur für 2 h gerührt. Anschließend wird die Mischung in ges. Natriumhydrogencarbonat-Lösung eingegossen und nach gutem Durchmischen die wäßrige Phase mit Dichlormethan und Ethylacetat extrahiert. Die vereinigten organischen Phasen werden über Natriumsulfat getrocknet und durch Chromatographie an Kieselgel gereinigt (Eluens: Dichlormethan/Methanol = 9:1 + 1 % konz. Ammoniak-Lösung).
Ausbeute: 0.35 g (quant.)
C₁₀H₁₂CIN₃O (225.68)
Massenspektrum: (M-H)⁻ = 224/226 (Chlorisotope)
R_{f}-Wert: 0.40 (Kieselgel, Dichlormethan/Methanol 9:1 + 1% konz. Ammoniak-Lösung)

### (e) N-[(1R)-1-(5-Chlor-1H-benzimidazol-2-yl)-2-methoxy-ethyl]-4-(morpholin-3-on-4-yl)-3-trifluormethyl-benzamid

Hergestellt analog Beispiel 1f aus 4-(Morpholin-3-on-4-yl)-3-trifluormethyl-benzoesäure, (1*R*)-1-(5-Chlor-1*H-*benzimidazol-2-yl)-2-methoxy-ethylamin, TBTU und NMM in DMF, dann Ausfällen durch Eingießen in Wasser, Abfiltrieren und Trocknen i. Vak..
Ausbeute: 63%
R_{f}-Wert: 0.57 (Kieselgel; Dichlormethan/Methanol = 9:1)
C₂₂H₂₀ClF₃N₄O₄ (496.88)
Massenspektrum: (M+H)⁺ = 497/499 (Chlorisotope)

Analog wurden folgende Verbindungen hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **25** | 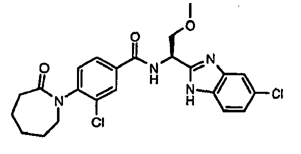 | ∑: 2.0% | (M+H)⁺ = 475/477/479 (Chlorisotope) | 2.57 min |
| | 4-(Azepan-2-on-1-yl)-3-chlor-*N-*[(1*R)*-1-(5-chlor-1*H-*benzimidazol-2-yl)-2-methoxy-ethyl]-benzamid | | | |
| **26** | 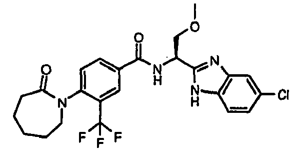 | ∑: 29% | (M+H)⁺ = 509/511 (Chlorisotope)-(Chlorisotope) | 2.65 min |
| | 4-(Azepan-2-on-1-yl)-*N-*[(1*R)*-1-(5-chlor-1*H-*benzimidazol-2-yl)-2-methoxy-ethyl]-3-trifluormethyl-benzamid | | | |
| **44** | 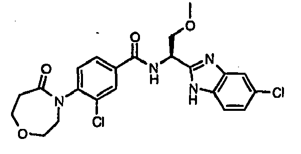 | ∑: 8.5% | (M+H)⁺ = 477/479/481 (Chlorisotope) | 0.47 (Kieselgel, CH₂Cl₂/CH₃OH CH₂Cl₂/CH₃OH 95:5) 2.37 min |
| | 3-Chlor-*N-*[(1*R)*-1-(5-chlor-1*H-*benzimidazol-2-yl)-2-methoxy-ethyl]-4-([1,4]oxazepan-5-on-4-yl)-benzamid | | | |
| **45** | 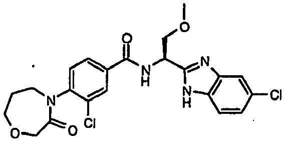 | ∑: 2.2% | (M+H)⁺ = 477/479/481 (Chlorisotope) | 0.45 (Kieselgel, CH₂Cl₂/CH₃OH 95:5) 2.41 min |
| | 3-Chlor-*N-*[(1*R)*-1-(5-chlor-1*H-*benzimidazol-2-yl)-2-methoxy-ethyl]-4-([1,4]oxazepan-3-on-4-yl)-benzamid | | | |
| **52** | 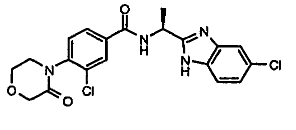 | ∑: 7.2% | (M-H)⁻ = 431/433/435 (Chlorisotope) | 0.70-(Kieselgel, CH₃COOC₂H₅ /CH₃OH9:1) |
| | 3-Chlor-*N-*[(1*S)*-1-(5-chlor-1*H-*benzimidazol-2-yl)-ethyl]-4-(morpholin-3-on-4-yl)-benzamid | | | |
| **81** | 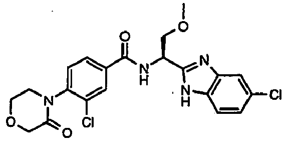 | ∑: 2.9% | (M+H)⁺ = 463/465/467 (Chlorisotope) | 0.44 (Kieselgel, CH₃COOC₂H₅ /CH3OH 95:5 + CH₃COOH) |
| | 3-Chlor-*N-*[(1*R)*-1-(5-chlor-1*H-*benzimidazol-2-yl)-2-methoxy-ethyl]-4-(morpholin-3-on-4-yl)-benzamid | | | |
| **97** | 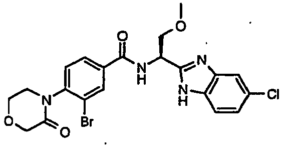 | ∑: 1.0% | (M-H)⁻ = 506/508/510 (Brom- und Chlorisotope) | 2.36 min |
| | 3-Brom-*N-*[(1*R)*-1-(5-chlor-1*H-*benzimidazol-2-yl)-2-methoxy-ethyl]-4-(morpholin-3-on-4-yl)-benzamid | | | |

### Beispiel 22

*N-*[(1*R)*-1-(5-Chlor-1*H-*benzimidazol-2-yl)-2-methoxy-ethyl]-3-methyl-4-(morpholin-3-on-4-yl)-benzamid

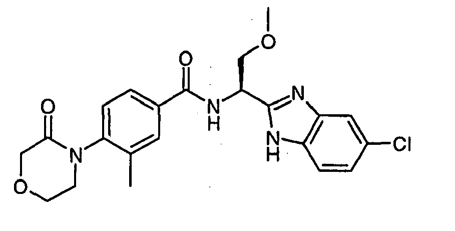

Hergestellt analog Beispiel 1f aus 3-Methyl-4-(morpholin-3-on-4-yl)-benzoesäure (hergestellt durch Synthesesequenz analog Beispiel 30a, 2d und 21a), (1*R)*-1-(5-Chlor-1*H-*benzimidazol-2-yl)-2-methoxy-ethylamin, TBTU und NMM in DMF, dann Eingießen in Wasser, Extrahieren mit Ethylacetat, Trocknen über Natriumsulfat, Einengen i. Vak. und Reinigen durch Chromatographie an Kieselgel (Eluens-Gradient: Ethylacetat/Isopropanol/Ethanol 9:1:0 -> 9:0:1).
Ausbeute: 99%
R_{f}-Wert: 0.13 (Kieselgel; Dichlormethan/Isopropanol = 19:1)
C₂₂H₂₃CIN₄O₄ (442.91)
Massenspektrum: (M+H)⁺ = 443/445 (Chlorisotope)

Analog wurden folgende Verbindungen hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **23** | 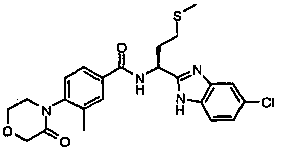 | 76% | (M+H)⁺ = 473/475 (Chlorisotope) | 0.50 (Kieselgel, CH₂Cl₂/C₂H₅OH 9:1) |
| | *N-*[(1*S)*-1-(5-Chlor-1*H-*benzimidazol-2-yl)-3-methylsulfanyl-propyl]-3-methyl-4-(morpholin-3-on-4-yl)-benzamid | | | |
| **24** | 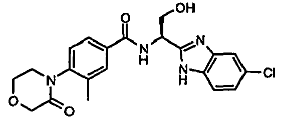 | 22% | (M+H)⁺ = 429/431 (Chlorisotope) | 0.22 (Kieselgel, CH₂Cl₂/C₂H₅OH 9:1) |
| | *N-*[(1*R)*-1-(5-Chlor-1*H-*benzimidazol-2-yl)-2-hydroxy-ethyl]-3-methyl-4-(morpholin-3-on-4-yl)-benzamid | | | |
| **49** | 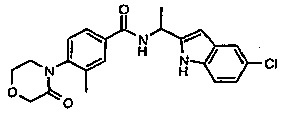 | quant. | (M+H)⁺ = 412/414 (Chlorisotope) | 0.60 (Kieselgel, CH₂Cl₂/C₂H₅OH 9:1) |
| | *N*-[1-(5-Chlor-1*H*-indol-2-yl)-ethyl]-3-methyl-4-(morpholin-3-on-4-yl)-benzamid | | | |

### Beispiel 27

*N-*[(1*S)*-1-(5-Chlor-1*H-*benzimidazol-2-yl)-ethyl]-4-(morpholin-3-on-4-yl)-benzamid

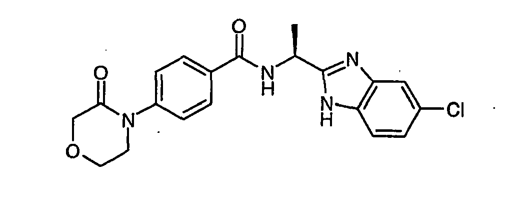

254 mg (1.15 mmol) 4-(Morpholin-3-on-4-yl)-benzoesäure (hergestellt durch Synthesesequenz analog Beispiel 30a, 2d und 21a) werden in 5 ml DMF vorgelegt und 428 mg (1.0 mmol) PfTU sowie 514 µl (3.0 mmol) DIPEA zugefügt. Nach Rühren bei Raumtemperatur für 10 Minuten werden 232 mg (1.0 mmol) (1*S)*-1-(5-Chlor-1*H-*benzimidazol-2-yl)-ethylamin-Hydrochlorid zugefügt und bei Raumtemperatur 16 Stunden gerührt. Danach wird das Reaktionsgemisch über basisches Aluminiumoxid filtriert und i. Vak. eingeengt. Der Rückstand wird durch Chromatographie an Kieselgel gereinigt (Eluens-Gradient: Dichlormethan/Methanol 100:0 -> 90:10), die entsprechenden Fraktionen i. Vak. eingeengt, der Rückstand in Acetonitril/Wasser gelöst und
lyophilisiert.
Ausbeute: 77%
C₂₀H₁₉ClN₄O₃ (398.85)
Massenspektrum: (M+H)⁺ = 399/401 (Chlorisotope)
Analog wurde die folgende Verbindung hergestellt:

| **Nr.** | **Strukturformel** | **Ausbaute** | **Massenpeak(s)** |
|---|---|---|---|
| | **Name** | | |
| **28** | 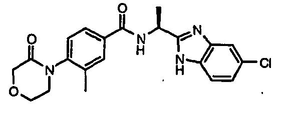 | 80% | (M+H)⁺ = 413/415 (Chlorisotope) |
| | *N-*[(1*S)*-1-(5-Chlor-1*H-*benzimidazol-2-yl)-ethyl]-3-methyl-4-(morpholin-3-on-4-yl)-benzamid | | |

### Beispiel 29

*N-*[(1*S)*-1-(5-Chlor-1*H-*benzimidazol-2-yl)-ethyl]-3-methyl-4-(4-*N-*methyl-[1,4]diazepan-1-yl)-benzamid

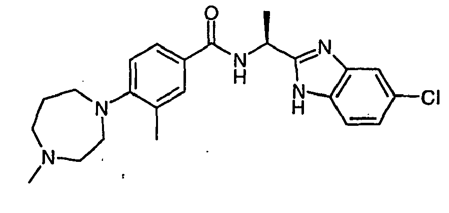

### (a) 4-Fluor-3-methyl-benzoesäurechlorid

14.00 g (90.8 mmol) 4-Fluor-3-methyl-benzoesäure werden zusammen mit 50 ml Thionylchlorid für 1 Stunde zum Rückfluß erhitzt und anschließend i. Vak. eingeengt. Der Rückstand wird ohne weitere Aufreinigung weiter umgesetzt.
Ausbeute: 15.70 g (quantitativ)
C₈H₆ClFO (172.59)

### (b) 4-Fluor-3-methyl-benzamid

15.70 g (91.0 mmol) 4-Fluor-3-methyl-benzoesäurechlorid werden in 30 ml THF gelöst zu 300 ml konz. Ammoniak-Lösung zugetropft und anschließend für 2 Stunden bei Raumtemperatur gerührt. Der entstandene Niederschlag wird abfiltriert, mit Wasser gewaschen und getrocknet.
Ausbeute: 10.00 g (72%)
C₈H₈FNO(153.16)
R_{f}-Wert: 0.31 (Aluminiumoxid; Dichlormethan/Methanol = 50:1)

### (c) 4-Fluor-3-methyl-benzonitril

10.00 g (65.29 mmol) 4-Fluor-3-methyl-benzoesäureamid werden zusammen mit 50 ml Phosphoroxychlorid für 4 Stunden bei 60°C gerührt und anschließend i. Vak. eingeengt. Der Rückstand wird in Eiswasser eingegossen, der entstehende Niederschlag abfiltriert und mit Wasser gewaschen. Nach Aufnehmen in Ethylacetat wird die organische Phase mit ges. Kaliumcarbonat-Lösung gewaschen, über Natriumsulfat getrocknet und i. Vak. vollständig eingeengt.
Ausbeute: 8.00 g (91 %)
C₈H₆FN (135.14)
R_{f}-Wert: 0.84 (Kieselgel; Dichlormethan)

### (d) 3-Methyl-4-(4-N-methyl-[1,4]diazepan-1-yl)-benzonitril

7.00 g (51.8 mmol) 4-Fluor-3-methyl-benzonitril werden zusammen mit 1-N Methyl-[1,4]diazepan für 1 Woche unter Rühren auf 110°C erhitzt. Nach Einengen i. Vak. wird der Rückstand an Aluminiumoxid getrennt (Eluens: Dichlormethan) und die entsprechenden Fraktionen nochmals an Kieselgel gereinigt (Eluens-Gradient: Dichlormethan/Methanol 100:1 -> 9:1).
Ausbeute: 1.70 g (14%)
C₁₄H₁₉N₃ (229.33)
Massenspektrum: (M+H)⁺ = 230
R_{f}-Wert: 0.25 (Kieselgel; Dichlormethan/Methanol = 9:1)

### (e) 3-Methyl-4-(4-N-methyl-[1,4]diazepan-1-yl)-benzoesäure

Hergestellt analog Beispiel 1b aus 3-Methyl-4-(4-*N-*methyl-[1,4]diazepan-1-yl)-benzonitril mit 25%-iger Kaliumhydroxid-Lösung durch Erhitzen zum Rückfluß für 36 Stunden. Nach Einengen i. Vak. wird der Rückstand an Aluminiumoxid getrennt (Eluens: Dichlormethan) und die entsprechenden Fraktionen nochmals an Kieselgel gereinigt (Eluens-Gradient: Dichlormethan/Methanol 100:1 -> 9:1).
Ausbeute: 14%
C₁₄H₁₉N₂O₂ (248.33)
Massenspektrum: (M+H)⁺ = 249
R_{f}-Wert: 0.30 (RP-18; Methanol/5%-ige wässrige Natriumchlorid-Lösung = 6:4)

### (f) 3-Methyl-4-(4-N-methyl-[1,4]diazepan-1-yl)-benzoesäurechlorid

Hergestellt analog Beispiel 29a aus 3-Methyl-4-(4-*N-*methyl-[1,4]diazepan-1-yl)-benzoesäure und Thionylchlorid.
Ausbeute: quantitativ
C₁₄H₁₉ClN₂O * HCl (266.77/303.23)

### (g) N-[(1S)-1-(5-Chlor-1H-benzimidazol-2-yl)-ethyl]-3-methyl-4-(4-N-methyl-[1,4]diazepan-1-yl)-benzamid

489 mg (1.61 mmol) 3-Methyl-4-(4-*N-*methyl-[1,4]diazepan-1-yl)-benzoesäurechlorid werden zusammen mit 400 mg (3.96 mmol) TEA in 10 ml THF bei Raumtemperatur vorgelegt und eine Lösung von 433 mg (1.61 mmol) (1*S)*-1-(5-Chlor-1*H-*benzimidazol-2-yl)-ethylamin unter Rühren zugetropft. Nach 16 Stunden Rühren bei Raumtemperatur wird die Mischung i. Vak. eingeengt, der Rückstand mit Wasser versetzt und mit Ethylacetat extrahiert. Die vereinigten organischen Phasen werden mit ges. Natriumchlorid-Lösung gewaschen, über Natriumsulfat getrocknet und i. Vak. eingeengt. Der Rückstand wird durch Chromatographie an Aluminiumoxid gereinigt (Eluens: Dichlormethan/Methanol 100:1). Die eingeengten Fraktionen werden mit etherischer Salzsäure behandelt, nach vollständigem Einengen 2 mal mit Ethylacetat und Diethylether abgedampft und bei 70°C i. Vak. getrocknet.
Ausbeute: 120 mg (16%)
C₂₃H₂₈ClN₅O * HCl (462.43/425.96)
Massenspektrum: (M+H)⁺ = 426/428 (Chlorisotope)
R_{f}-Wert: 0.47 (Aluminiumoxid; Dichlormethan/Methanol 19:1)

### Beispiel 30

*N-*[(1*S)*-1-(5-Chlor-1*H-*benzimidazol-2-yl)-ethyl]-3-methyl-4-(piperazin-1-yl)-benzamid

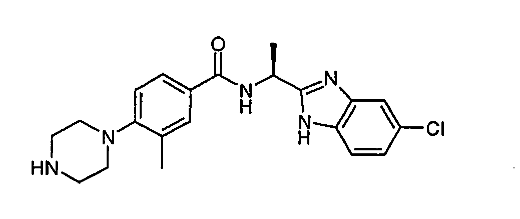

### (a) 4-(N-Boc-piperazin-1-yl)-3-methyl-benzoesäure-methylester

4.00 g (17.5 mmol) 4-Brom-3-methyl-benzoesäure-methylester werden zusammen mit 3.92 g (21.0 mmol) *N-*Boc-piperazin, 39.2 mg (175 µmol) Palladium(II)acetat, 50.7 mg (175 µmol) Tri-*tert*.-butyl-phosphonium-tetrafluoroborat und 11.12 g (52.4 mmol) Kaliumphosphat in 35 ml Toluol suspendiert und unter Argon-Atmosphäre in einem Mikrowellen-Ofen für 10 Minuten auf 150°C erhitzt. Anschließend wird die Reaktionsmischung in Wasser eingegossen, stark gerührt und 3 mal mit Ethylacetat extrahiert. Die vereinigten organischen Phasen werden über Natriumsulfat getrocknet, auf Kieselgel aufgezogen und durch Chromatographie an Kieselgel gereinigt (Eluens: Dichlormethan/Methanol 80:1).
Ausbeute: 1.42 g (24%)
C₁₈H₂₆N₂O₄ (334.42)
Massenspektrum: (M+H)⁺ = 335
R_{f}-Wert: 0.52 (Kieselgel; Dichlormethan/Methanol = 50:1)

### (b) 4-(N-Boc-piperazin-1-yl)-3-methyl-benzoesäure

1.42 g (4.24 mmol) 4-(*N-*Boc-piperazin-1-yl)-3-methyl-benzoesäure-methylester werden in 7 ml THF gelöst und 9.25 ml Wasser sowie 893 mg (21.3 mmol) Lithiumhydroxid-Monohydrat zugefügt. Nach Rühren für 16 Stunden bei Raumtemperatur wird für 2 Stunden auf 45°C erwärmt. Anschließend werden nochmals 893 mg (21.3 mmol) Lithiumhydroxid-Monohydrat zugegeben und bei Raumtemperatur für 4 Tage gerührt. Danach wird mit 1-molarer Salzsäure neutralisiert und die Reaktionsmischung mit Ethylacetat extrahiert. Die vereinigten organischen Phasen werden über Natriumsulfat getrocknet und vollständig i. Vak. eingeengt.
Ausbeute: 1.29 g (95%)
C₁₇H₂₄N₂O₄ (320.39)
Massenspektrum: (M+H)⁺ = 321
R_{f}-Wert: 0.29 (Kieselgel; Dichlormethan/Methanol = 15:1)

### (c) 4-(N-Boc-piperazin-1-yl)-N-[(1S)-1-(5-chlor-1H-benzimidazol-2-yl)-ethyl)-3-methyl-benzamid

Hergestellt analog Beispiel 1f aus 4-(*N-*Boc-piperazin-1-yl)-3-methyl-benzoesäure, (1*S*)-1-(5-Chlor-1*H-*benzimidazol-2-yl)-ethylamin, TBTU und NMM in DMF, dann Einrühren in konz. Natriumhydrogencarbonat-Lösung, Extraktion mit Ethylacetat, Trocknen über Natriumsulfat, Einengen i. Vak. und Reinigung des Rückstands durch Chromatographie an Kieselgel (Eluens-Gradient: Dichlormethan/Methanol 50:1 -> 15:1).
Ausbeute: 85%
R_{f}-Wert: 0.15 (Kieselgel; Dichlormethan/Methanol = 30:1)
C₂₆H₃₂ClN₅O₃ (498.03)
Massenspektrum: (M+H)⁺ = 498/500 (Chlorisotope)

### (d) N-[(1S)-1-5-Chlor-1H-benzimidazol-2-yl)-ethyl]-3-methyl-4-(piperazin-1-yl)-benzamid

Hergestellt analog Beispiel 1 g aus 4-(*N*-Boc-piperazin-1-yl)-*N-*[(1*S*)-1-(5-chlor-1*H-*benzimidazol-2-yl)-ethyl)-3-methyl-benzamid und TFA in Dichlormethan.
Ausbeute: quantitativ
C₂₁H₂₄ClN₅O (397.91)
Massenspektrum: (M+H)⁺ = 398/400 (Chlorisotope)
R_{f}-Wert: 0.14 (Kieselgel; Dichlormethan/Methanol = 9:1)

Analog wurde folgende Verbindung hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **65** | 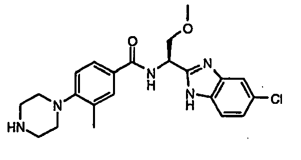 | ∑: 5.9% | (M+H)⁺ = 428/430 (Chlorisotope) | 0.11 (Kieselgel, CH₂Cl₂/CH₃OH 9:1) |
| | *N*-[(1*R)*-1-(5-Chlor-1*H*-benzimidazol-2-yl)-2-methoxy-ethyl]-3-methyl-4-(piperazin-1-yl)-benzamid | | | |

### Beispiel 31

*N-*[(1*S*)-1-(5-Chlor-1*H-*benzimidazol-2-yl)-ethyl]-3-methyl-4-(piperazin-2-on-1-yl)-benzamid

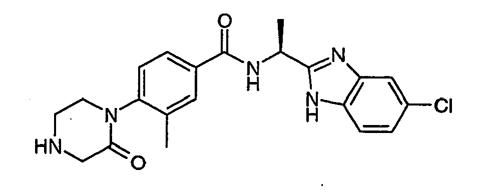

### (a) 4-(N-Boc-piperazin-2-on-1-yl)-3-methyl-benzoesäure-methylester

758 mg (3.31 mmol) 4-Brom-3-methyl-benzoesäure-methylester werden zusammen mit 796 mg (21.0 mmol) 4-*N-*Boc-piperazin-2-on, 31.8 mg (167 µmol) Kupfer(I)iodid, 35.1 µl (330 µmol) *N,N*'-Dimethyl-ethylendiamin und 0.92 g (6.62 mmol) Kaliumcarbonat in 6.6 ml Toluol suspendiert und unter Argon-Atmosphäre in einem Mikrowellen-Ofen unter Rühren für 1.5 Stunden auf 140°C erhitzt. Anschließend wird die Reaktionsmischung in Wasser eingegossen, stark gerührt und 3 mal mit Ethylacetat extrahiert. Die vereinigten organischen Phasen werden über Natriumsulfat getrocknet, auf Kieselgel aufgezogen und durch Chromatographie an Kieselgel gereinigt (Eluens:
Dichlormethan/Methanol 50:1).
Ausbeute: 679 mg (59%)
C₁₈H₂₄N₂O₅ (348.40)
Massenspektrum: (M+H)⁺ = 349
R_{f}-Wert: 0.25 (Kieselgel; Dichlormethan/Methanol = 50:1)

### (b) 4-(N-Boc-piperazin-2-on-1-yl)-3-methyl-benzoesäure

775 mg (2.22 mmol) 4-(*N-*Boc-piperazin-2-on-1-yl)-3-methyl-benzoesäure-methylester werden in 3.7 ml THF gelöst und 4.9 ml Wasser sowie 468 mg (11.2 mmol) Lithiumhydroxid-Monohydrat zugefügt. Nach Rühren für 16 Stunden bei Raumtemperatur wird mit 1-molarer Salzsäure neutralisiert und die Reaktionsmischung mit Ethylacetat extrahiert. Die vereinigten organischen Phasen werden über Natriumsulfat getrocknet und vollständig i. Vak. eingeengt.
Ausbeute: 664 mg (89%)
C₁₇H₂₂N₂O₅ (334.38)
Massenspektrum: (M+H)⁺ = 335
R_{f}-Wert: 0.31 (Kieselgel; Dichlormethan/Methanol = 15:1)

### (c) 4-(N-Boc-piperazin-2-on-1-yl)-N-[(1S)-1-(5-chlor-1H-benzimidazol-2-yl)-ethyl)-3-methyl-benzamid

Hergestellt analog Beispiel 1f aus 4-(*N-*Boc-piperazin-1-yl)-3-methyl-benzoesäure, (1*S)*-1-(5-Chlor-1*H*-benzimidazol-2-yl)-ethylamin, TBTU und NMM in DMF, dann Einrühren in konz. Natriumhydrogencarbonat-Lösung, Extraktion mit Ethylacetat, Trocknen über Natriumsulfat, Einengen i. Vak. und Reinigung des Rückstands durch Chromatographie an Kieselgel (Eluens: Dichlormethan/Methanol 30:1).
Ausbeute: 71 %
R_{f}-Wert: 0.30 (Kieselgel; Dichlormethan/Methanol = 15:1)
C₂₆H₃₀ClN₅O₄ (512.01)
Massenspektrum: (M+H)⁺ = 512/514 (Chlorisotope)

### (d) N-[(1S)-1-(5-Chlor-1H-benzimidazol-2-yl)-ethyl]-3-methyl-4-(piperazin-2-on-1-yl)-benzamid

Hergestellt analog Beispiel 1g aus 4-(*N*-Boc-piperazin-1-yl)-*N-*[(1*S)*-1-(5-chlor-1 *H-*benzimidazol-2-yl)-ethyl]-3-methyl-benzamid und TFA in Dichlormethan.
Ausbeute: 36%
C₂₁H₂₂ClN₅O₂ (411.90)
Massenspektrum: (M+H)⁺ = 412/414 (Chlorisotope) R_{f}-Wert: 0.42 (Kieselgel; Dichlormethan/Methanol = 9:1)

Analog wurden folgende Verbindungen hergestellt:

| **Nr.** | **Strukturformel** | **Ausbeute** | **Massenpeak(s)** | **R_{f}-Wert bzw. Rₜ** |
|---|---|---|---|---|
| | **Name** | | | |
| **66** | 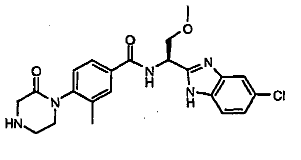 | ∑: 17% | (M+H)⁺ = 442/444 (Chlorisotope) | 0.18 (Kieselgel, CH₂Cl₂/CH₃OH 9:1) |
| | *N-*[(1*R)*-1-(5-Chlor-1*H-*benzimidazol-2-yl)-2-methoxy-ethyl]-3-methyl-4-(piperazin-2-on-1-yl)-benzamid | | | |
| **98** | 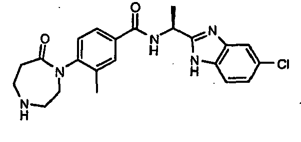 | ∑: 0.11% | (M+H)⁺ = 426/428 (Chlorisotope) | |
| | *N-*[(1*S*)-1-(5-Chlor-1*H-*benzimidazol-2-yl)-ethyl]-3-methyl-4-(7-oxo-[1,4]diazepan-1-yl)-benzamid | | | |

### Beispiel 32

*N-*[(1*S)*-1-(5-Chlor-1*H-*benzimidazol-2-yl)-ethyl]-3-methyl-4-(piperidin-2-on-1-yl)-benzamid

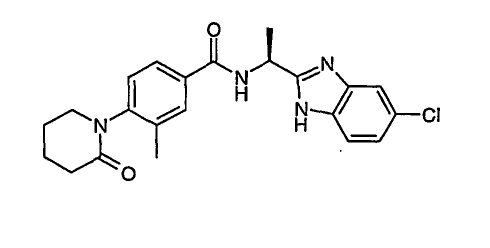

### (a) 3-Methyl-4-(piperidin-2-on-1-yl)-benzoesäure-methylester

Hergestellt analog Beispiel 31 a aus 4-Brom-3-methyl-benzoesäure-methylester und Piperidin-2-on in Gegenwart von Kupfer(I)iodid, *N,N*'-Dimethylethylendiamin und Kaliumcarbonat in Toluol und Dioxan unter Argon-Atmosphäre.
Ausbeute: 34%
C₁₄H₁₇NO₃ (247.30)
Massenspektrum: (M+H)⁺ = 248
R_{f}-Wert: 0.21 (Kieselgel; Petrolether/Ethylacetat =1:1)

### (b) 3-Methyl-4-(piperidin-2-on-1-yl)-benzoesäure

Hergestellt analog Beispiel 2d aus 3-Methyl-4-(piperidin-2-on-1-yl)-benzoesäure-methylester und 1-molarer Natronlauge in Methanol.
Ausbeute: 83%
C₁₃H₁₅NO₃ (233.27)
Massenspektrum: (M+H)⁺ = 234
R_{f}-Wert: 0.51 (Kieselgel; Ethylacetat/Ethanol = 9:1 + 1% konz. Ammoniak-Lösung)

### (c) N-[(1S)-1-(5-Chlor-1H-benzimidazol-2-yl)-ethyl)-3-methyl-4-(piperidin-2-on-1-yl)-benzamid

Hergestellt analog Beispiel 1f aus 3-Methyl-4-(piperidin-1-yl)-benzoesäure, (1*S*)-1-(5-Chlor-1*H-*benzimidazol-2-yl)-ethylamin, TBTU und NMM in DMF, dann Einrühren in Eiswasser, Versetzen mit Ammoniak-Lösung, Filtrieren, Aufnehmen in Dichlormethan, Einengen i. Vak. und Reinigung des Rückstands durch Chromatographie an Kieselgel (Eluens-Gradient: Ethylacetat/(Methanol/konz. Ammoniak-Lösung 19:1) 1:0 -> 9:1).
Ausbeute: 32%
R_{f}-Wert: 0.30 (Kieselgel; Ethylacetat/Ethanol = 9:1+1% Essigsäure)
C₂₂H₂₃ClN₄O₂ (410.91)
Massenspektrum: (M+H)⁺ = 411/413 (Chlorisotope)

Analog wurden die folgenden Verbindungen hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **55** | 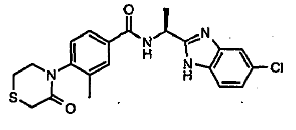 | ∑: 4.5% | (M+H)⁺ = 429/431 (Chlorisotope) | 0.40 (Kieselgel, CH₃COOC₂H₅/ C₂H₅OH 19:1 + 1% NH₃) |
| | *N-*[(1*S*)-1-(5-Chlor-1*H-*benzimidazol-2-yl)-ethyl]-3-methyl-4-(thiomorpholin-3-on-4-yl)-benzamid | | | |
| **56** | 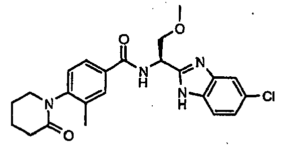 | ∑: 16% | (M+H)⁺ = 441/443 (Chlorisotope) | 0.30 (Kieselgel, CH₃COOC₂H₅/ C₂H₅OH 9:1 + 1% NH₃) |
| | *N-*[(1*R*)-1-(5-Chlor-1*H-*benzimidazol-2-yl)-2-methoxy-ethyl]-3-methyl-4-(piperidin-2-on-1-yl)-benzamid | | | |
| **80** | 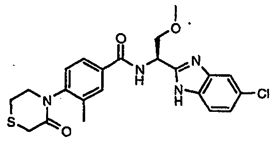 | ∑: 8.0% | (M+H)⁺ = 459/461 (Chlorisotope) | 0.60 (Kieselgel, CH₃COOC₂H₅/ C₂H₅OH 9:1 + 1% NH₃) |
| | *N-*[(1*R*)-1-(5-Chlor-1*H-*benzimidazol-2-yl)-2-methoxy-ethyl]-3-methyl-4-(thiomorpholin-3-on-4-yl)-benzamid | | | |

### Beispiel 33

*N-*[(1*S*)-1-(5-Chlor-1*H-*benzimidazol-2-yl)-ethyl]-4-(*N-*methyl-piperazin-1-yl)-3-trifluormethyl-benzamid 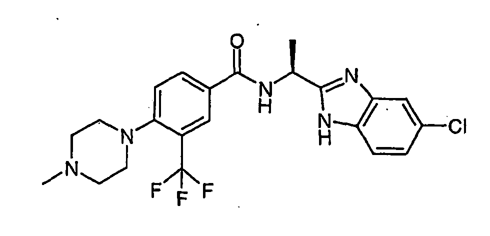 170 mg (0.24 mmol) *N-*[(1*S*)-1-(5-Chlor-1*H-*benzimidazol-2-yl)-ethyl]-4-(piperazin-1-yl)-3-trifluormethyl-benzamid werden in 2 ml 1,2-Dichlorethan suspendiert und unter Stickstoff-Atmosphäre mit 20 mg (0.67 mmol) Paraformaldehyd und 76 mg (0.36 mmol) Natriumtriacetoxyborhydrid versetzt. Nach Zugabe von 5 ml THF wird die Reaktionsmischung 6 Stunden bei Raumtemperatur gerührt, anschließend 100 mg (3.33 mmol) Paraformaldehyd und 100 mg (0.47 mmol) Natriumtriacetoxyborhydrid zugegeben und weitere 22 Stunden bei Raumtemperatur gerührt. Dann wird mit ges. Natriumhydrogencarbonat-Lösung versetzt und mit Ethylacetat extrahiert. Die vereinigten organischen Phasen werden über Magnesiumsulfat getrocknet, i. Vak. eingeengt und durch Chromatographie an Kieselgel gereinigt (Eluens-Gradient: Dichlormethan/(Methanol/konz. Ammoniak-Lösung 19:1) 100:0 -> 92:8).
Ausbeute: 70 mg (59%)
C₂₃H₂₅ClF₃N₅O₂ (495.94)
Massenspektrum: (M+H)⁺ = 496/498 (Chlorisotope)
R_{f}-Wert: 0.25 (Kieselgel; Dichlormethan/Methanol = 9:1 + 1 % konz. Ammoniak-Lösung)

### Beispiel 34

*N-*[(1*S*)-1-(5-Chlor-1*H-*benzimidazol-2-yl)-3-methylsulfonyl-propyl]-3-methyl-4-(morpholin-3-on-4-yl)-benzamid 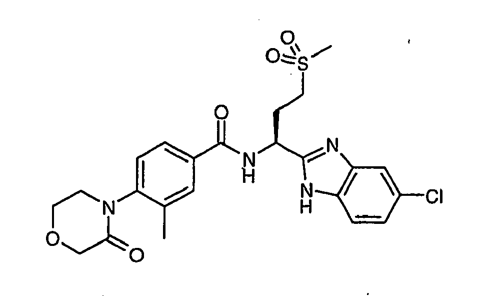 150 mg (0.32 mmol) *N-*[(1*S*)-1-(5-Chlor-1*H-*benzimidazol-2-yl)-3-methylsulfanyl-prop-1-yl]-3-methyl-4-(morpholin-3-on-4-yl)-benzamid werden in einem Gemisch aus 10 ml Dichlormethan und 1 ml Essigsäure bei -15°C gelöst vorgelegt und mit 204 mg (0.89 mmol) 3-Chlorperbenzoesäure versetzt. Danach wird die Mischung für 30 Minuten bei -15 - -10°C gerührt, auf Raumtemperatur aufgewärmt und weitere 16 Stunden gerührt. Anschließend wird das Reaktionsgemisch 2 mal mit 5%-iger Natriumhydrogencarbonat-Lösung gewaschen, über Natriumsulfat getrocknet, i. Vak. eingeengt und durch Chromatographie an Kieselgel gereinigt (Eluens: Dichlormethan/Ethanol 95:5).
Ausbeute: 80 mg (50%)
C₂₃H₂₅ClN₄O₅S (505.00)
Massenspektrum: (M+H)⁺ = 505/507 (Chlorisotope)
R_{f}-Wert: 0.45 (Kieselgel; Dichlormethan/Ethanol = 9:1)

### Beispiel 35

*N-*[(1*S*)-1-(5-Chlor-1*H-*benzimidazol-2-yl)-3-methylsulfanyl-propyl]-4-(pyrrolidin-2-on-1-yl)-benzamid

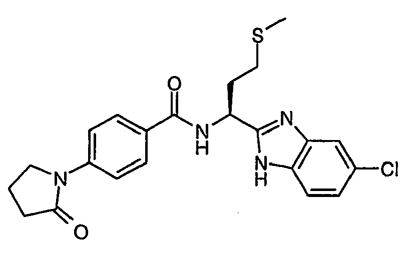

Hergestellt analog Beispiel 27 aus 4-(Pyrrolidin-2-on-1-yl)-benzoesäure, (1*S*)-1-(5-Chlor-1*H-*benzimidazol-2-yl)-3-methylsulfanyl-propylamin, PfTU und TEA in DMSO bei Raumtemperatur und anschließender Boc-Abspaltung mit TFA analog Beispiel 1 g.
HPLC-MS Ergebnisse:
Retentionszeit: 3.69 min
C₂₂H₂₃ClN₄O₂S (442.97)
Massenspektrum: (M+H)⁺ = 443/445 (Chlorisotope)

Analog wurde folgende Verbindung hergestellt:

| **Nr.** | **Strukturformel** | **Massenpeak(s)** | **Retentionszeit** |
|---|---|---|---|
| | **Name** | | |
| **36** | 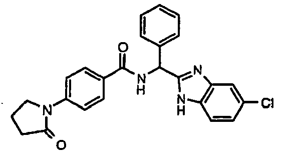 | (M+H)⁺ = 445/447 (Chlorisotope) | 3.80 min |
| | *N*-[1-(5-Chlor-1*H-*benzimidazol-2-yl)-1-phenyl-methyl]-4-(pyrrolidin-2-on-1-yl)-benzamid | | |

### Beispiel 39

*N-*[(1*S*)-1-(5-Chlor-1*H*-benzimidazol-2-yl)-ethyl]-3-methyl-4-([1,3]oxazepan-2-on-3-yl)-benzamid

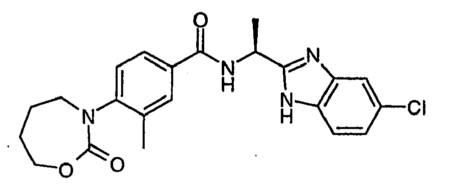

### (a) 4-Isocyanato-3-methyl-benzoesäure-methylester

1.50 g (9.08 mmol) 4-Amino-3-methyl-benzoesäure-methylester werden gelöst in 250 ml Dioxan mit 1.3 ml (10.7 mmol) Trichlormethyl-chlorformiat versetzt und unter Rühren für 5.5 Stunden zum Rückfluß erhitzt. Anschließend wird i. Vak. eingeengt und der Rückstand ohne weitere Reinigung weiter umgesetzt.
Ausbeute: 1.74 g (quantitativ)
C₁₀H₉NO₃ (191.19)

### (b) 4-(N-[4-Chlorbutoxycarbonyl]-amino)-3-methyl-benzoesäure-methylester

1.74 g (9.08 mmol) 4-Isocyanato-3-methyl-benzoesäure-methylester werden in 100 ml Toluol gelöst mit 1.07 ml (9.11 mmol) 85 %-igem 4-Chlor-butan-1-ol versetzt. Die Mischung wird für 17 Stunden unter Rühren zum Rückfluß erhitzt. Nach Einengen i. Vak. wird der Rückstand durch Chromatographie an Kieselgel gereinigt (Eluens-Gradient: Petrolether/Ethylacetat 17:3 -> 17:4).
Ausbeute: 1.19 g (44%)
C₁₄H₁₈ClNO₄ (299.76)
Massenspektrum: (M-H)⁻= 298/300 (Chlorisotope)
R_{f}-Wert: 0.45 (Kieselgel; Petrolether/Ethylacetat = 80:20)

### (c) 3-Methyl-4-([1,3]oxazepan-2-on-3-yl)-benzoesäure-methylester

300 mg (1.00 mmol) 4-(*N*-[4-Chlorbutoxycarbonyl]-amino)-3-methyl-benzoesäure-methylester werden gelöst in 10 ml DMF mit 168 mg (1.50 mmol) Kalium-*tert*.-butylat versetzt und für 3 Stunden bei 60°C gerührt. Der Reaktionsmischung wird Wasser zugefügt und 3 mal mit Ethylacetat extrahiert. Die vereinigten organischen Phasen werden über Natriumsulfat getrocknet und i. Vak. eingeengt. Anschließend erfolgt Reinigung des Rückstands durch Chromatographie an Kieselgel (Eluens: Petrolether/Ethylacetat 3:2). Ausbeute: 160 mg (61 %) R_{f}-Wert: 0.26 (Kieselgel; Petrolether/Ethylacetat 3:2)
C₁₄H₁₇NO₄ (263.30)
Massenspektrum: (M+H)⁺ = 264

### (d) 3-Methyl-4-([1,3]oxazepan-2-on-3-yl)-benzoesäure

150 mg (0.57 mmol) 3-Methyl-4-([1,3]oxazepan-2-on-3-yl)-benzoesäure-methylester werden in 1 ml Ethanol suspendiert und mit 0.26 ml (0.87 mmol) 8%-iger wässriger Lithiumhydroxid-Lösung versetzt. Es wird 3 Stunden bei Raumtemperatur gerührt und anschließend i. Vak. eingeengt. Der wässrige Rückstand wird 2 mal mit Ethylacetat extrahiert, dann sauer gestellt und nochmals 2 mal mit Ethylacetat extrahiert. Die vereinigten organischen Phasen werden über Natriumsulfat getrocknet und i. Vak. eingeengt.
Ausbeute: 122 mg (86%)
C₁₃H₁₅NO₄ (249.27)
Massenspektrum: (M+H)⁺ = 250
R_{f}-Wert: 0.14 (Kieselgel; Dichlormethan/Methanol = 95:5)

### (e) N-[(1S)-1-(5-Chlor-1H-benzimidazol-2-yl)-ethyl]-3-methyl-4-([1,3]oxazepan-2-on-3-yl)-benzamid

Hergestellt analog Beispiel 1f aus 3-Methyl-4-([1,3]oxazepan-2-on-3-yl)-benzoesäure, (1*S)*-1-(5-Chlor-1*H-*benzimidazol-2-yl)-ethylamin, TBTU und NMM in DMF, dann Ansäuern mit TFA und Reinigung des Rückstands durch Chromatographie (präparative HPLC).
Ausbeute: 54%
C₂₂H₂₃ClN₄O₃ * 2 CF₃COOH (654.96 / 426.90)
Massenspektrum: (M+H)⁺ = 427/429 (Chlorisotope)
Rₜ: 2.42 min

Analog wurden folgende Verbindungen hergestellt:

| **Nr.** | **Strukturformel** | **Ausbeute** | **Massenpeak(s)** | **Rₜ** |
|---|---|---|---|---|
| | **Name** | | | |
| **43** | 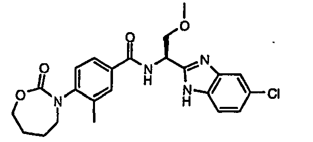 | ∑: 12% | (M-H)⁻ = 455/457 (Chlorisotope) | 2.43 min |
| | *N-*[(1*R*)-1-(5-Chlor-1*H-*benzimidazol-2-yl)-2-methoxy-ethyl]-3-methyl-4-([1,3]oxazepan-2-on-3-yl)-benzamid | | | |
| **51** | 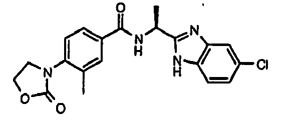 | ∑: 2.6% | (M-H)⁻ = 397/399 (Chlorisotope) | 3.70 min |
| | *N-*[(1*S*)-1-(5-Chlor-1*H-*benzimidazol-2-yl)-ethyl]-3-methyl-4-(oxazolidin-2-on-3-yl)-benzamid | | | |
| **57** | 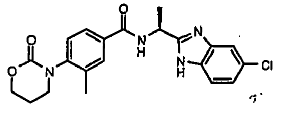 | ∑: 3.4% | (M+H)⁺ = 413/415 (Chlorisotope) | 3.63 min |
| | *N-*[(1*S*)-1-(5-Chlor-1*H-*benzimidazol-2-yl)-ethyl]-3-methyl-4-([1,3]-oxazinan-2-on-3-yl)-benzamid | | | |
| **58** | 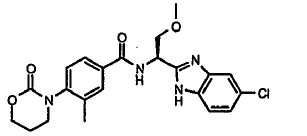 | ∑: 2.7% | (M+H)⁺ = 443/445 (Chlorisotope) | 3.66 min |
| | *N-*[(1*R*)-1-(5-Chlor-1*H-*benzimidazol-2-yl)-2-methoxy-ethyl]-3-methyl-4-([1,3]-oxazinan-2-on-3-yl)-benzamid | | | |
| **68** | 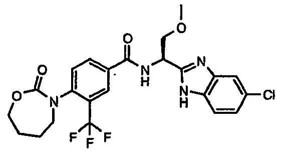 | ∑: 0.32% | (M+H)⁺ = 511/513 (Chlorisotope) | 4.21 min |
| | *N-*[(1*R*)-1-(5-Chlor-1*H-*benzimidazol-2-yl)-2-methoxy-ethyl]-4-([1,3]oxazepan-2-on-3-yl)-3-trifluormethyl-benzamid | | | |

### Beispiel 41

*N-*[(1*S*)-1-(5-Chlor-1*H-*benzimidazol-2-yl)-3-methylsulfanyl-propyl]-4-(morpholin-3-on-4-yl)-3-nitro-benzamid

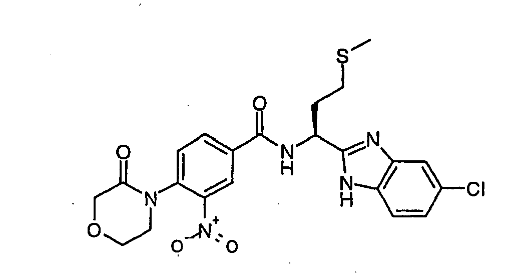

### (a) 4-(Morpholin-3-on-4-yl)-3-nitro-benzoesäure-methylester

1.00 g (3.85 mmol) 4-Brom-3-nitro-benzoesäure-methylester werden mit 389 mg (3.85 mmol) Morpholin-3-on unter Stickstoff-Atmosphäre in 6 ml Dioxan gelöst und 36.6 mg (40 µmol) Tris-(dibenzylidenaceton)-dipalladium(0), 67.1 mg (116 µmol) Xantphos und 1.75 g (5.38 mmol) Cäsiumcarbonat zugefügt. Unter Stickstoff-Atmosphäre und Rühren wird die Reaktionsmischung für 16 Stunden auf 95°C erhitzt. Anschließend wird filtriert, die Lösung i. Vak. eingeengt und mit Ether nachgedampft. Der Rückstand wird ohne weitere Reinigung weiter umgesetzt. Ausbeute: 1.31 g (quantitativ)
C₁₂H₁₂N₂O₆ (280.24)
Massenspektrum: (M+H)⁺ = 281
R_{f}-Wert: 0.47 (Reversed Phase 8; Methanol/5%-ige Natriumchlorid-Lösung = 6:4)

### (b) 4-(2-Carboxymethoxy-ethylamino)-3-nitro-benzoesäure

400 mg (1.43 mmol) 4-(Morpholin-3-on-4-yl)-3-nitro-benzoesäure-methylester werden in 15 ml Methanol gelöst mit 4.5 ml (4.5 mmol) 1-molarer Lithiumhydroxid-Lösung versetzt. Die Mischung wird für 2 Stunden bei Raumtemperatur gerührt. Dann wird i. Vak. eingeengt, der Rückstand mit Wasser verdünnt, im Eisbad gekühlt und mit 2-molarer Salzsäure angesäuert. Nach 10 Minuten Kühlen im Eisbad wird der gebildete Niederschlag abfiltriert, mit Wasser neutral gewaschen und im Trockenschrank bei 50°C getrocknet.
Ausbeute: 290 mg (72 %)
C₁₁H₁₂N₂O₇ (284.23)
Massenspektrum: M⁺ = 284
R_{f}-Wert: 0.59 (Reversed Phase 8; Methanol/5%-ige Natriumchlorid-Lösung = 6:4)

### (c) 4-(Morpholin-3-on-4-yl)-3-nitro-benzoesäure-chlorid

290 mg (1.02 mmol) 4-(2-Carboxymethoxy-ethylamino)-3-nitro-benzoesäure werden in 100 ml Dichlormethan mit 0.186 ml (2.55 mmol) Thionylchlorid und 2 Tropfen DMF versetzt und einen Tag zum Rückfluss erhitzt. Nach Einengen der Lösung i. Vak. wird mit Toluol nachgedampft und der Rückstand ohne weitere Reinigung weiter umgesetzt.
Ausbeute: 290 mg (quant.)
C₁₁H₉ClN₂O₅ (284.66)

### (d) N-[(1S)-1-(5-Chlor-1H-benzimidazol-2-yl)-3-methylsulfanyl-propyl]-4-(morpholin-3-on-4-yl)-3-nitro-benzamid

237 mg (0.93 mmol) (1*S*)-1-(5-Chlor-1*H-*benzimidazol-2-yl)-3-methylsulfanyl-propylamin werden mit 0.257 ml TEA in 10 ml THF gelöst und eine Lösung von 290 mg (1.02 mmol) 4-(Morpholin-3-on-4-yl)-3-nitro-benzoesäurechlorid in 10 ml THF zugetropft. Die Reaktionsmischung wird 16 Stunden bei Raumtemperatur gerührt und dann i. Vak. eingeengt. Der Rückstand wird durch Chromatographie an Kieselgel gereinigt (Eluens-Gradient: Dichlormethan/Ethanol 100:0 -> 95:5).
Ausbeute: 34%
C₂₂H₂₂ClN₅O₅S (503.97)
Massenspektrum: (M-H)⁻ = 502/504 (Chlorisotope)
R_{f}-Wert: 0.46 (Kieselgel; Dichlormethan/Ethanol = 9:1)

### Beispiel 42

3-Chlor-*N-*[(1*S*)-1-(5-chlor-1*H-*benzimidazol-2-yl)-ethyl]-4-(tetrahydro-pyrimidin-2-on-1-yl)-benzamid

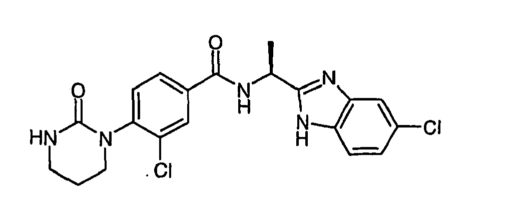

### (a) 4-(3-Boc-amino-propyl-amino)-3-chlor-benzonitril

3.49 g (20 mmol) 3-Boc-amino-propylamin werden in 5 ml DMF mit 2.75 ml (25 mmol) NMM versetzt. Nach Zugabe von 3.11 g (20 mmol) 3-Chlor-4-fluor-benzonitril wird 3.5 Stunden bei Raumtemperatur unter Stickstoff-Atmosphäre gerührt, für 20 Minuten auf 105°C erwärmt und mit Ethylacetat extrahiert. Die vereinigten organischen Phasen werden mit Wasser und ges. Natriumchlorid-Lösung gewaschen, über Magnesiumsulfat getrocknet und i. Vak. eingeengt. Der Rückstand wird ohne weitere Reinigung weiter umgesetzt.
Ausbeute: 5.50 g (89 %)
C₁₅H₂₀N₃O₂ (309.80)
Massenspektrum: (M+H)⁺ = 310/312 (Chlorisotope)
R_{f}-Wert: 0.40 (Kieselgel; Petrolether/Ethylacetat = 2:1)

### (b) 4-(3-Amino-propylamino)-3-chlor-benzonitril

4.50 g (14.5 mmol) 4-(3-Boc-amino-propylamino)-3-chlor-benzonitril werden in 50 ml Dioxan gelöst mit 200 ml 6-molarer Salzsäure versetzt. Die Mischung wird für 2 Stunden bei Raumtemperatur gerührt, dann mit Ether gewaschen und die wässrige Phase in 125 ml eisgekühlte konz. Ammoniak-Lösung eingegossen. Anschließend wird mit Ethylacetat extrahiert, die vereinigten organischen Phasen mit Wasser und ges. Natriumchlorid-Lösung gewaschen, über Magnesiumsulfat getrocknet und i. Vak. eingeengt. Der Rückstand wird ohne weitere Reinigung weiter umgesetzt.
Ausbeute: 1.40 g (46%)
C₁₀H₁₂ClN₃ (209.68)
Massenspektrum: (M+H)⁺ = 210/212 (Chlorisotope)
R_{f}-Wert: 0.30 (Kieselgel; Dichlormethan/Methanol = 9:1 + 1 % konz. Ammoniak-Lösung)

### (c) 3-Chlor-4-tetrahydro-pyrimidin-2-on-1-yl)-benzonitril

Eine Lösung von 349 mg (2.15 mmol) *N,N*'-Carbonyl-diimidazol in 3 ml NMP wird unter Rühren bei Raumtemperatur mit 450 mg (2.15 mmol) 4-(3-Amino-propylamino)-3-chlor-benzonitril versetzt und nach vollständiger Auflösung für eine Stunde auf 145°C und für 1.5 Stunden auf 155°C erhitzt. Die Reaktionsmischung wird mit Wasser gewaschen und mit Ethylacetat extrahiert. Die vereinigten organischen Phasen werden mit Wasser und ges. Natriumchlorid-Lösung gewaschen, über Magnesiumsulfat getrocknet und i. Vak. eingeengt. Anschließend erfolgt Reinigung des Rückstands durch Chromatographie an Kieselgel (Eluens-Gradient: Ethylacetat/(Methanol/konz. Ammoniak-Lösung 19:1) = 100:0 -> 95:5).
Ausbeute: 260 mg (51%)
R_{f}-Wert: 0.40 (Kieselgel; Ethylacetat/Ethanol 9:1 + 1% konz. Ammoniak-Lösung)
C₁₁H₁₀ClN₃O (235.68)
Massenspektrum: (M+H)⁺ = 236/238 (Chlorisotope)

### (d) 3-Chlor-4-(tetrahydro-pyrimidin-2-on-1-yl)-benzoesäure

350 mg (1.49 mmol) 3-Chlor-4-(tetrahydro-pyrimidin-2-on-1-yl)-benzonitril werden in 5 ml Ethanol suspendiert und mit 2.0 ml 10-molarer wässriger Natriumhydroxid-Lösung versetzt. Es wird eine Stunde bei 100°C gerührt und anschließend i. Vak. eingeengt. Der wässrige Rückstand wird mit Eis versetzt, mit Essigsäure angesäuert und mit Ethylacetat extrahiert. Die vereinigten organischen Phasen werden mit Wasser und ges. Natriumchlorid-Lösung gewaschen, über Magnesiumsulfat getrocknet und i. Vak. eingeengt. Die wässrige Phase wird mit 6-molarer Salzsäure angesäuert, mit Ethylacetat 5 mal extrahiert, die vereinigten organischen Phasen mit ges. Natriumchlorid-Lösung gewaschen, über Magnesiumsulfat getrocknet und i. Vak. eingeengt.
Ausbeute: 340 mg (90%)
C₁₁H₁₁ClN₂O₃ (254.68)
Massenspektrum: (M+H)⁺ = 255/257 (Chlorisotope)
R_{f}-Wert: 0.35 (Kieselgel; Dichlormethan/Methanol = 9:1 + 1% Essigsäure)

### (e) 3-Chlor-N-[(1S)-1-(5-chlor-1H-benzimidazol-2-yl)-ethyl]-4-(tetrahydro-pyrimidin-2-on-1-yl)-benzamid

Hergestellt analog Beispiel 1f aus 3-Chlor-4-(tetrahydro-pyrimidin-2-on-1-yl)-benzoesäure, (1*S*)-1-(5-Chlor-1*H-*benzimidazol-2-yl)-ethylamin, TBTU und NMM in DMF, dann Eingießen in Eiswasser, Zugabe von konz. Ammoniak-Lösung, Abfiltrieren, Waschen mit Wasser und Reinigung durch Chromatographie an Kieselgel (Eluens-Gradient Ethylacetat/(Methanol/konz. Ammoniak-Lösung 19:1) = 98:2 -> 90:10) mit anschließender Filtration über Aktivkohle, Verreiben mit Ether und Trocknen in der Trockenpistole bei 70°C.
Ausbeute: 32%
C₂₀H₁₉Cl₂N₅O₂ (432.31)
Massenspektrum: (M+H)⁺ = 432/434/436 (Chlorisotope)
R_{f}-Wert: 0.40 (Kieselgel; Dichlormethan/Methanol = 9:1 +1% Essigsäure)

Analog wurde folgende Verbindung hergestellt:

| **Nr.** | **Strukturformel** | **Ausbeute** | **Massenpeak(s)** | **R_{f}-Wert bzw. Rₜ** |
|---|---|---|---|---|
| | **Name** | | | |
| **114** | 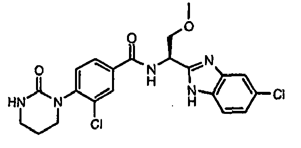 | Σ: 5.4% | (M+H)⁺ = 462/464/466 (Chlorisotope) | 0.52 (Ethylacetat / Ethanol = 8:2) |
| | 3-Chlor-*N-*[(1*R*)-1-(5-chlor-1*H-*benzimidazol-2-yl)-2-methoxy-ethyl]-4-(tetrahydro-pyrimidin-2-on-1-yl)-benzamid | | | |

### Beispiel 46:

3-Chlor-*N-*[(1*R*)-1-(5-chlor-1*H-*benzimidazol-2-yl)-2-methoxy-ethyl]-4-(piperidin-2-on-1-yl)-benzamid

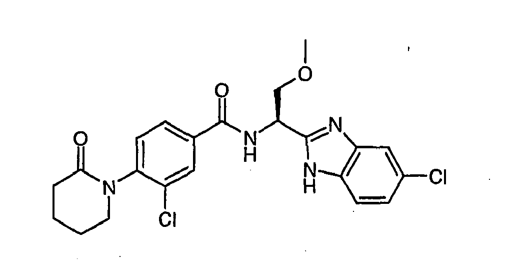

### (a) 3-Chlor-4-(5-chlor-Pentanoyl-amino)-benzoesäure-methylester

Zu 1.00 g (5.39 mmol) 4-Amino-3-chlor-benzoesäure-methylester in 20 ml THF mit 1 ml TEA wird unter Rühren im Eisbad langsam eine Lösung von 696 µl (0.84 g, 5.39 mmol) 5-Chlor-pentanoylchlorid in 10 ml THF getropft. Nach 16 Stunden Rühren bei Raumtemperatur, 3 Stunden bei 50°C und 3 Stunden bei Rückfluss wird in Wasser eingegossen und mit Ethylacetat extrahiert. Nach Trocknen der organischen Phasen über Natriumsulfat wird i. Vak. eingeengt und der verbleibende Rückstand durch Chromatographie an Kieselgel gereinigt (Eluens: Petrolether/Ethylacetat 85:15).
Ausbeute: 300 mg (14.6%) 80%iges Produkt
C₁₃H₁₅Cl₂NO₃ (304.18)
Massenspektrum: (M+H)⁺ =304/306/308 (Chlorisotope)
Rₜ-Wert: 3.29 min

### (b) 3-Chlor-4-(piperidin-2-on-1-yl)-benzoesäure-methylester

300 mg (0.79 mmol) des in Beispiel 46a erhaltenen Produkts wird in 10 ml DMF gelöst mit 180 mg (1.60 mmol) Kalium-*tert*.-butylat versetzt und für 3 Stunden auf 60°C erhitzt. Anschließend wird die Reaktionsmischung in Wasser eingegossen und mit Ethylacetat extrahiert. Die vereinigten organischen Phasen werden über Natriumsulfat getrocknet, i. Vak. eingeengt und der erhaltene Rückstand durch Chromatographie an Kieselgel gereinigt (Eluens:
Dichlormethan/Isopropanol 98:2).
Ausbeute: 159 mg (75 %)
C₁₃H₁₄ClNO₃ (267.71)
Massenspektrum: (M+H)⁺ = 268/270 (Chlorisotope)
R_{f}-Wert: 0.18 (Kieselgel; Dichlormethan/Isopropanol =49:1)

### (c) 3-Chlor-4-(piperidin-2-on-1-yl)-benzoesäure

Hergestellt analog Beispiel 39d aus 3-Chlor-4-(piperidin-2-on-1-yl)-benzoesäure-methylester und 8%-iger Lithiumhydroxid-Lösung in Ethanol.
Ausbeute: 36%
C₁₂H₁₂ClNO₃ (247.30)
Massenspektrum: (M+H)⁺ = 252/254 (Chlorisotope)
R_{f}-Wert: 0.27 (Kieselgel; Dichlormethan/Ethanol = 9:1)

### (d) 3-Chlor-N-[(1R)-1-(5-chlor-1H-benzimidazol-2-yl)-2-methoxy-ethy]-4-(piperidin-2-on-1-yl)-benzamid

Hergestellt analog Beispiel 1f aus 3-Chlor-4-(piperidin-2-on-1-yl)-benzoesäure, TBTU, NMM und (1*R*)-1-(5-Chlor-1*H-*benzimidazol-2-yl)-2-methoxy-ethylamin in DMF und anschließender Reinigung durch Chromatographie an Kieselgel (Eluens-Gradient: Ethylacetat/Ethanol 95:5 -> 90:10).
Ausbeute: 70%
C₂₂H₂₂Cl₂N₄O₃ (461.35)
Massenspektrum: (M-H)⁻ = 459/461/463 (Chlorisotope)
R_{f}-Wert: 0.19 (Kieselgel; Dichlormethan/Ethanol = 19: 1)

Analog wurde folgende Verbindung hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **59** | 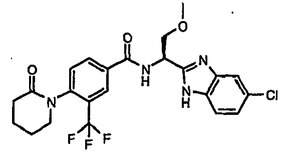 | Σ: 1.3% | (M+H)+ = 495/497 (Chlorisotope) | 2.51 min |
| | *N-*[(1*R*)-1-(5-Chlor-1*H-*benzimidazol-2-yl)-2-methoxy-ethyl]-4-(piperidin-2-on-1-yl)-3-trifluormethyl-benzamid | | | |

### Beispiel 47

*N-*[(1*R*)-1-(5-Brom-1*H-*benzimidazol-2-yl)-2-hydroxy-ethyl]-3-methyl-4-(morpholin-3-on-4-yl)-benzamid

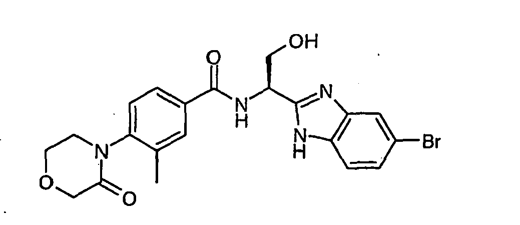

### (a) N'-(2-Amino-4-brom-phenyl)-N-Boc-(S)-serinamid und N'-(2-Amino-5-brom-phenyl)-N-Boc-(S)-serinamid

5.49 g (26.7 mmol) (*S*)-*N-*Boc-Serin werden mit 5.00 g (26.7 mmol) 4-Brom-1,2-phenylendiamin in 125 ml THF gelöst und eine Lösung von 5.52 g (26.7 mmol) *N,N*'-Dicyclohexylcarbodiimid in 20 ml THF wird unter Eiskühlung zugetropft. Die Reaktionsmischung wird für 16 Stunden bei Raumtemperatur gerührt. Nach Einengen i. Vak. wird der Rückstand durch Chromatographie an Kieselgel gereinigt (Eluens: Dichlormethan/Methanol 98:2).
Ausbeute: 4.76 g (48 %) Gemisch beider Regioisomere
C₁₄H₂₀BrN₃O₄ (374.24)
Massenspektrum: (M+H)⁺ =374/376 (Bromisotope)

### (b) (1R)-N-Boc-1-(5-Brom-1H-benzimidazol-2-yl)-2-hydroxy-ethylamin

3.00 g (8.02 mmol) des bei 47a erhaltenen Gemisches werden in 30 ml Essigsäure gelöst und für 2 Stunden bei 50°C gerührt. Die Reaktionsmischung wird in eine 10%-ige Natriumhydroxid-Lösung eingetropft und 3 mal mit Ethylacetat extrahiert. Die vereinigten organischen Phasen werden über Magnesiumsulfat getrocknet und i. Vak. eingeengt und der Rückstand aus Methanol umkristallisiert.
Ausbeute: 1.96 g (69 %)
C₁₄H₁₈BrN₃O₃ (356.22)
R_{f}-Wert: 0.59 (Kieselgel; Petrolether/Ethylacetat = 1:1)

### (c) (1R)-1-(5-Brom-1H-benzimidazol-2-yl)-2-hydroxy-ethylamin

2.00 g (5.62 mmol) (1*R*)-*N-*Boc-1-(5-Brom-1*H-*benzimidazol-2-yl)-2-hydroxyethylamin werden in 40 ml Ethylacetat unter Kühlen im Eisbad mit 8.0 ml 4-molarer Salzsäure in Dioxan versetzt und 16 Stunden bei Raumtemperatur gerührt. Die Reaktionsmischung wird eingeengt und der entstehende Niederschlag abfiltriert.
Ausbeute: 1.11 g (67 %)
C₉H₁₀BrN₃O *HCl (292.57 / 256.10)
Massenspektrum: (M+H)⁺ =256/258 (Bromisotope)

### (d) N-[(1R)-1-(5-Brom-1H-benzimidazol-2-yl)-2-hydroxy-ethyl]-3-methyl-4-(morpholin-3-on-4-yl)-benzamid

Hergestellt analog Beispiel 1f aus 3-Methyl-4-(morpholin-3-on-4-yl)-benzoesäure, (1*R*)-1-(5-Brom-1*H-*benzimidazol-2-yl)-2-hydroxy-ethylamin, TBTU und NMM in DMF mit anschließender Reinigung mittels präparativer
HPLC.
Ausbeute: 52%
C₂₁H₂₁BrN₄O₄ * CF₃COOH (587.35 / 473.32)
Massenspektrum: (M+H)⁺ = 473/475 (Bromisotope)
R_{f}-Wert: 0.33 (Kieselgel; Dichlormethan/Methanol = 19:1)

Analog wurden folgende Verbindungen hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **53** | 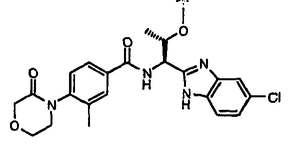 | Σ: 6.6% | (M+H)⁺= 457/459 (Chlorisotope) | 0.48 (Kieselgel, CH₂Cl₂/C₂H₅OH 9:1) |
| | *N-*[(1*R*,2*S*)-1-(5-Chlor-1*H-*benzimidazol-2-yl)-2-methoxy-propyl]-3-methyl-4-(morpholin-3-on-4-yl)-benzamid | | | |
| **62** | 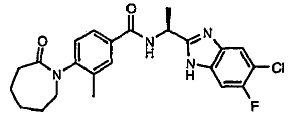 | Σ: 34% | (M-H)⁻ = 441/443 (Chlorisotope) | 0.40 (Aluminiumoxid, CH₂Cl₂/C₂H₅OH 19:1) |
| | 4-(Azepan-2-on-1-yl)-*N-*[(1*S*)-1-(5-chlor-6-fluor-1*H-*benzimidazol-2-yl)-ethyl]-3-methyl-benzamid | | | |
| **67** | 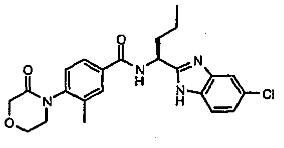 | Σ: 38% | (M+H)⁺= 441 /443 (Chlorisotope) | 0.50 (Kieselgel, CH₂Cl₂/C₂H₅OH 9:1) |
| | N-[(1*S*)-1-(5-Chlor-1*H-*benzimidazol-2-yl)-butyl]-3-methyl-4-(morpholin-3-on-4-yl)-benzamid | | | |
| **78** | 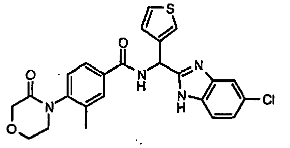 | Σ: 38% | (M+H)⁺= 481/483 (Chlorisotope) | 0.51 (Kieselgel, CH₂Cl₂/C₂H₅OH 9:1) |
| | *N-*[1-(5-Chlor-1*H-*benzimidazol-2-yl)-1-(thiophen-3-yl)-methyl]-3-methyl-4-(morpholin-3-on-4-yl)-benzamid | | | |
| **79** | 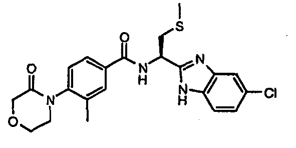 | Σ: 10.6% | (M+H)⁺= 459/461 (Chlorisotope) | 0.50 (Kieselgel, CH₂Cl₂/C₂H₅OH 9:1) |
| | *N-*[(1*R*)-1-(5-Chlor-1*H-*benzimidazol-2-yl)-2-methylsulfanyl-ethyl]-3-methyl-4-(morpholin-3-on-4-yl)-benzamid | | | |
| **99** | 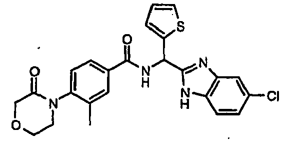 | Σ: 18% | Σ: (M+H)⁺ = 481/483 (Chlorisotope) | 2.43 min |
| | *N-*[1-(5-Chlor-1*H-*benzimidazol-2-yl)-1-(thiophen-2-yl)-methyl]-3-methyl-4-(morpholin-3-on-4-yl)-benzamid | | | |
| **101** | 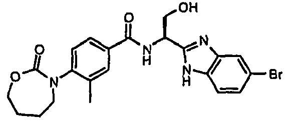 | Σ: 2.9% | (M+H)⁺ = (M+H)⁺ = 487/489 (Bromisotope) | 2.31 min |
| | *N-*[(1*R*)-1-(5-Brom-1*H-*benzimidazol-2-yl)-2-hydroxy-ethyl]-3-methyl-4-([1,3]oxazepan-2-on-3-yl)-benzamid | | | |

### Beispiel 48

N[(1*S*)-1-(5-Chlor-1*H-*benzimidazol-2-yl)-ethyl]-4-(1,1-dioxo-isothiazolidin-2-yl)-3-methyl-benzamid

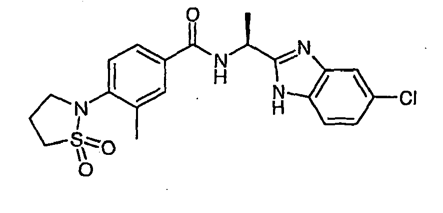

### (a) 4-(3-Chlor-propyl-sulfonyl-amino)-3-methyl-benzoesäure-methylester

100 mg (0.61 mmol) 4-Amino-3-methyl-benzoesäure-methylester werden in 3 ml Pyridin gelöst mit 82 µl (0.67 mmol) 3-Chlorpropan-sulfonsäurechlorid versetzt und für 16 Stunden bei Raumtemperatur gerührt. Die Reaktionslösung wird mit Wasser und Ethylacetat versetzt und anschließend die wässrige Phase nochmals mit Ethylacetat extrahiert. Die vereinigten organischen Phasen werden über Natriumsulfat getrocknet und i. Vak. eingeengt. Der Rückstand wird ohne weitere Reinigung weiter umgesetzt.
Ausbeute: 170 mg (92 %)
C₁₂H₁₆ClNO₄S (305.78)
Massenspektrum: (M+H)⁺ = 306/308 (Chlorisotope)
R_{f}-Wert: 0.10 (Kieseigel; Petrolether/Ethylacetat = 8:2)

### (b) 4-(1,1-Dioxo-isothiazolidin-2-yl)-3-methyl-benzoesäure-methylester

370 mg (0.85 mmol) 70%-iger 4-(3-Chlorpropan-sulfonylamino)-3-methyl-benzoesäure-methylester werden gelöst in 26 ml DMF mit 275 mg (2.45 mmol) Kalium-*tert*.-butylat versetzt und 16 Stunden bei Raumtemperatur gerührt. Die Reaktionsmischung wird mit Wasser versetzt und mit Ethylacetat extrahiert. Die vereinigten organischen Phasen werden über Natriumsulfat getrocknet, i. Vak. eingeengt und der Rückstand durch Chromatographie an Kieselgel gereinigt (Eluens: Dichlormethan/Isopropanol 98:2).
Ausbeute: 177 mg (47 %)
C₁₂H₁₅NO₄S (269.32)
Massenspektrum: (M+H)⁺ = 270
R_{f}-Wert: 0.10 (Kieselgel; Petrolether/Ethylacetat = 7:3)

### (c) 4-(1,1-Dioxo-isothiazolidin-2-yl)-3-methyl-benzoesäure

170 mg (0.63 mmol) 4-(1,1-Dioxo-isothiazolidin-2yl)-3-methyl-benzoesäure-methylester werden in 2 ml Ethanol bei Raumtemperatur für 3 Stunden mit 0.6 ml 2-molarer Natronlauge gerührt. Anschließend wird die Reaktionslösung i. Vak. eingeengt, mit Wasser und 0.6 ml 2-molarer Salzsäure versetzt und mit Ethylacetat extrahiert. Die vereinigten organischen Phasen werden über Natriumsulfat getrocknet, i. Vak. eingeengt und der Rückstand ohne weitere Reinigung weiter umgesetzt.
Ausbeute: 148 mg (92 %)
C₁₁H₁₃NO₄S (255.30)
Massenspektrum: (M-H)⁻= 254
Retentionszeit: 2.23 min

### (d) N-[(1S)-1-(5-Chlor-1H-benzimidazol-2-yl)-ethyl]-4-(1,1-dioxo-isothiazolidin-2-yl)-3-methyl-benzamid

Hergestellt analog Beispiel 1f aus 4-(1,1-Dioxo-isothiazolidin-2yl)-3-methyl-benzoesäure, (1*S*)-1-(5-Chlor-1*H-*benzimidazol-2-yl)-ethylamin, TBTU und NMM in DMF mit anschließender Extraktion mit Ethylacetat, Trocknen über Magnesiumsulfat, Aktivkohle und Kieselgel mit nachfolgender Reinigung an Kieselgel (Eluens: Dichlormethan/Isopropanol 95:5).
Ausbeute: 37 %
C₂₀H₂₁ClN₄O₃S (432.93)
Massenspektrum: (M+H)⁺ = 433/435 (Chlorisotope)
R_{f}-Wert: 0.32 (Kieselgel; Dichlormethan/Isopropanol = 19:1)

Analog wurden folgende Verbindungen hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **60** | 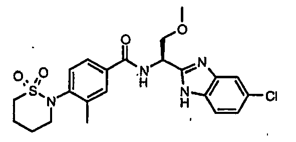 | Σ: 18% | (M+H)+ = 477/479 (Chlorisotope) | 2.46 min |
| | *N-*[(1*R*)-1-(5-Chlor-1*H-*benzimidazol-2-yl)-2-methoxy-ethyl]-4-(1,1-dioxo-[1,2]thiazinan-2-yl)-3-methyl-benzamid | | | |
| **71** | 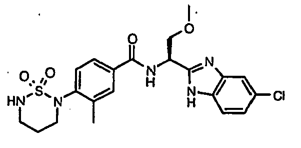 | Σ: 20% | (M+H)⁺= 478/480 (Chlorisotope) | 2.58 min |
| | *N-*[(1*R*)-1-(5-Chlor-1*H-*benzimidazol-2-yl)-2-methoxy-ethyl]-4-(1,1-dioxo-[1,2,6]thiadiazinan-2-yl)-3-methyl-benzamid | | | |
| **72** | 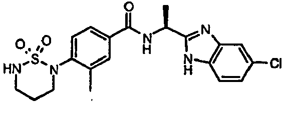 | Σ: 19% | (M+H)⁺= 448/450 (Chlorisotope) | 2.41 min |
| | *N-*[(1*S*)-1-(5-Chlor-1*H-*benzimidazol-2-yl)-ethyl]-4-(1,1-dioxo-[1,2,6]thiadiazinan-2-yl)-3-methyl-benzamid | | | |
| **100** | 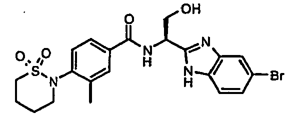 | Σ: 2.5% | (M+H)⁺ = (M+H)+ = 507/509 (Bromisotope) | 2.38 min |
| | *N-*[(1*R*)-1-(5-Brom-1*H-*benzimidazol-2-yl)-2-hydroxy-ethyl]-4-(1,1-dioxo-[1,2]thiazinan-2-yl)-3-methyl-benzamid | | | |

### Beispiel 54

3-Amino-*N-*[(1*S*)-1-(5-chlor-1*H-*benzimidazol-2-yl)-3-methylsulfanyl-propyl]-4-(morpholin-3-on-4-yl)-benzamid 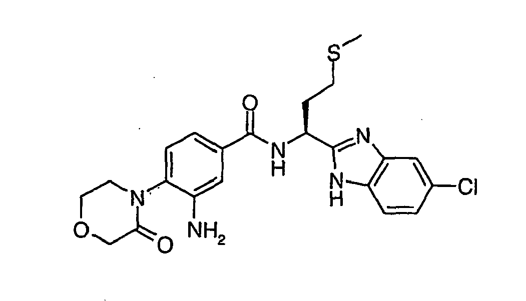 65 mg (0.13 mmol) *N-*[(1*S*)-1-(5-Chlor-1*H-*benzimidazol-2-yl)-3-methylsulfanyl-propyl]-4-(morpholin-3-on-4-yl)-3-nitro-benzamid werden in 2 ml Ethylacetat gelöst mit 143 mg (0.63 mmol) Zinn(II)chlorid-dihydrat und 130 mg (1.55 mmol) Natriumhydrogencarbonat versetzt und für 2 Stunden zum Rückfluss erhitzt. Die Reaktionslösung wird mit Eiswasser versetzt, 10 Minuten gerührt und anschließend der gebildete Niederschlag abfiltriert. Nach Trocknen für 3 Tage wird der Rückstand durch Chromatographie an Kieselgel gereinigt (Eluens-Gradient: Dichlormethan/Ethanol 100:0 -> 91:9).
Ausbeute: 5 mg (8.2 %)
C₂₂H₂₄ClN₅O₃S (473.99)
Massenspektrum: (M+H)⁺ = 474/476 (Chlorisotope)
R_{f}-Wert: 0.48 (Kieselgel; Dichlormethan/Ethanol = 9:1)

### Beispiel 61

*N-*[(1*S*)-1-(5-Chlor-1*H-*benzimidazol-2-yl)-ethyl]-3-methyl-4-(5,6-didehydro-azepan-2-on-1-yl)-benzamid

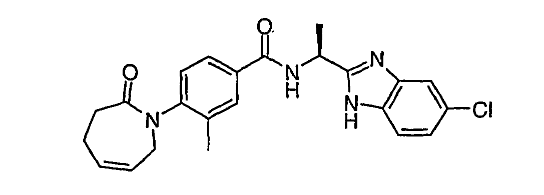

### (a) 3-Methyl-4-(pent-4-en-1-oyl-amino)-benzoesäure-methylester

Hergestellt analog Beispiel 46a aus 4-Amino-3-methyl-benzoesäure-methylester und 4-Penten-1-säure-chlorid in THF mit TEA.
Ausbeute: 46%
C₁₄H₁₇NO₃ (247.30)
Massenspektrum: (M+H)⁺ = 248
Rₜ-Wert: 2.88 min

### (b) 4-(Allyl-pent-4-en-1-oyl-amino)-3-methyl-benzoesäure-methylester

1.00 g (4.04 mmol) 3-Methyl-4-(pent-4-en-1-oyl-amino)-benzoesäure-methylester werden gelöst in 5 ml DMF mit 500 mg (4.37 mmol) Kalium-*tert*.-butylat versetzt und unter Rühren bei 40°C langsam 350 µl (489 mg, 4.04 mmol) Allylbromid zugegeben. Anschließend wird 3 Stunden auf 70°C erhitzt. Danach wird die Reaktionsmischung in Wasser eingegossen und mit Ethylacetat extrahiert. Die vereinigten organischen Phasen werden über Natriumsulfat getrocknet, i. Vak. eingeengt, der Rückstand auf Kieselgel aufgezogen und durch Chromatographie an Kieselgel gereinigt (Elunes: Petrolether/Ethylacetat 9:1).
Ausbeute: 58%
C₁₇H₂₁NO₃ (287.36)
Massenspektrum: (M+H)⁺ = 288
R_{f}-Wert: 0.46 (Petrolether/Ethylacetat = 9:1)

### (c) 4-(4,5-Didehydro-azepan-2-on-1-yl)-3-methyl-benzoesäure-methylester

150 mg (0.52 mmol) 4-(Allyl-pent-4-en-1-oyl-amino)-3-methyl-benzoesäure-methylester werden in 110 ml entgastem Dichlormethan gelöst 30 Minuten mit Argon gespült. Anschließend werden 88 mg (104 µmol) Benzyliden-[1,3-bis(2,4,6-trimethylphenyl)-2-imidazolidinyliden]-dichloro-(tricyclohexylphosphin)-ruthenium (2nd Generation Grubbs Catalyst) zugegeben und 4.5 Stunden zum Rückfluss erhitzt. Dann wird i. Vak. eingeengt, der Rückstand auf Kieselgel aufgezogen und durch Chromatographie an Kieselgel gereinigt (Eluens: Petrolether/Ethylacetat 3:2)
Ausbeute: 83 mg (61 %)
R_{f}-Wert: 0.22 (Kieselgel, Petrolether/Ethylacetat 3:2)
C₁₅H₁₇NO₃ (259.31)
Massenspektrum: (M+H)⁺ = 260

### (d) 4-(4,5-Didehydro-azepan-2-on-1-yl)-3-methyl-benzoesäure

Hergestellt analog Beispiel 39d aus 4-(4,5-Didehydro-azepan-2-on-1-yl)-3-methyl-benzoesäure-methylester und 8%-iger Lithiumhydroxid-Lösung in Ethanol. '
Ausbeute: 51%
R_{f}-Wert: 0.05 (Kieselgel; Dichlormethan/Methanol =19:1)
C₁₄H₁₅NO₃ (245.28)
Massenspektrum: (M+H)⁺ = 246

### (e) N-[(1S)-1-(5-Chlor-1H-benzimidazol-2-yl)-ethyl]-4-(4,5-didehydro-azepan-2-on-1-yl)-3-methyl-benzamid

Hergestellt analog Beispiel 1 f aus 4-(4,5-Didehydro-azepan-2-on-1-yl)-3-methyl-benzoesäure, TBTU, NMM und (1*S*)-1-(5-Chlor-1*H-*benzimidazol-2-yl)-ethylamin in DMF und anschließender Reinigung durch präparative HPLC.
Ausbeute: 32%
R_{f}-Wert: 0.44 (Kieselgel; Dichlormethan/Methanol = 19:1)
C₂₃H₂₃ClN₄O₂ * CF₃COOH (536.94/422.91)
Massenspektrum: (M+H)⁺ = 423/425 (Chlorisotope)

### Beispiel 63

*N-*[(1*S*)-1-(5-Chlor-1*H-*benzimidazol-2-yl)-ethyl]-4-(1,3-dioxo-thiomorpholin-4-yl)-3-methyl-benzamid 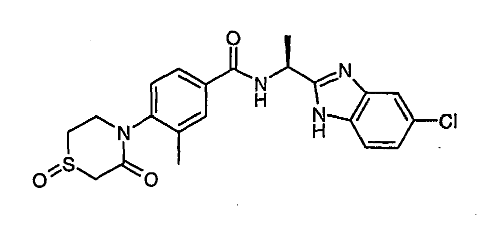 204 mg (0.48 mmol) *N-*[(1*S*)-1-(5-Chlor-1*H-*benzimidazol-2-yl)-ethyl]-3-methyl-4-(thiomorpholin-3-on-4-yl)-benzamid werden in einem Gemisch aus 12 ml Dichlormethan und 1.2 ml Essigsäure bei -15°C gelöst vorgelegt und mit 110 mg (0.48 mmol) 3-Chlorperbenzoesäure versetzt. Danach wird die Mischung für eine Stunde bei -15 bis -10°C gerührt, auf Raumtemperatur aufgewärmt und weitere 3 Stunden gerührt. Anschließend wird das Reaktionsgemisch mit halbkonzentrierter Natriumhydrogencarbonat-Lösung versetzt und mit einem Lösungsmittelgemisch Dichlormethan/Methanol 19:1 extrahiert. Die vereinigten organischen Phasen werden mit Wasser gewaschen, über Magnesiumsulfat getrocknet, i. Vak. eingeengt und durch Chromatographie an Kieselgel gereinigt (Eluens-Gradient: Ethylacetat/(Ethanol/konz. Ammoniak-Lösung 19:1) = 1:0 -> 4:1).
Ausbeute: 100 mg (47%)
C₂₁H₂₁ClN₄O₃S (444.94)
Massenspektrum: (M+H)⁺ = 445/447 (Chlorisotope)
R_{f}-Wert: 0.15 (Kieselgel; Ethylacetat/Ethanol = 4:1 + 1 % konz. Ammoniak-Lösung)

Analog wurde folgende Verbindung hergestellt:

| **Nr.** | **Strukturformel** | **Ausbeute** | **Massenpeak(s)** | **R_{f}**-Wert |
|---|---|---|---|---|
| | **Name** | | | |
| **64** | 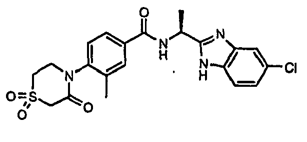 | Σ: 14% | (M+H)⁺ = 461/463 (Chlorisotope) | 0.70 (Kieselgel, CH₃COOC₂H₅ /C₂H₅OH 4:1 + 1% konz. Ammoniak-Lösung) |
| | *N-*[(1*S*)-1-(5-Chlor-1*H-*benzimidazol-2-yl)-ethyl]-3-methyl-4-(1,1,3-trioxo-thiomorpholin-4-yl)-benzamid | | | |

### Beispiel 69

*N-*[(1*S*)-1-(5-Chlor-1*H-*benzimidazol-2-yl)-ethyl]-3-methoxy-4-(piperidin-2-on-1-yl)-benzamid

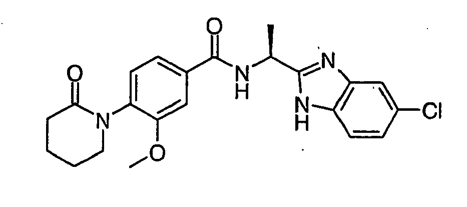

### (a) 4-(5-Chlor-pentanoyl-amino)-3-methoxy-benzoesäure-methylester

Hergestellt analog Beispiel 6a aus 4-Amino-3-methoxy-benzoesäure-methylester und 5-Chlor-pentansäure-chlorid in THF mit TEA.
Ausbeute: 99%
C₁₄H₁₈ClNO₄ (299.76)
Massenspektrum: (M+H)⁺ = 300/302 (Chlorisotope)
Rₜ-Wert: 3.14 min

### (b) 3-Methoxy-4-(piperidin-2-on-1-yl)-benzoesäure-methylester

2.25 g (7.51 mmol) 4-(5-Chlor-pentanoyl-amino)-3-methoxy-benzoesäure-methylester werden in 60 ml DMF gelöst mit 1.26 g (11.2 mmol) Kalium-*tert*.-butylat versetzt und 2.5 Stunden bei 60°C gerührt. Anschließend wird i. Vak. eingeengt, der Rückstand mit Wasser versetzt und mit Ethylacetat extrahiert. Die vereinigten organischen Phasen werden über Natriumsulfat getrocknet, i. Vak. eingeengt und der erhaltene Rückstand durch Chromatographie an Kieselgel gereinigt (Eluens-Gradient: Petrolether/Ethylacetat 3:2 -> 0:1).
Ausbeute: 0.99 g (50%)
Rₜ-Wert: 2.56 min
C₁₄H₁₇NO₄ (263.30)
Massenspektrum: (M+H)⁺ = 264

### (c) 3-Methoxy-4-(piperidin-2-on-1-yl)-benzoesäure

Hergestellt analog Beispiel 39d aus 3-Methoxy-4-(piperidin-2-on-1-yl)-benzoesäure-methylester und Lithiumhydroxid in Ethanol.
Ausbeute: 95%
R_{f}-Wert: 0.10 (Kieselgel; Petrolether/Ethylacetat = 1:2)
C₁₃H₁₅NO₄ (249.27)
Massenspektrum: (M+H)⁺ = 250

### (d) N-[(1S)-1-(5-chlor-1H-benzimidazol-2-yl)-ethyl]-3-methoxy-4-(piperidin-2-on-1-yl)-benzamid

Hergestellt analog Beispiel 1f aus 3-Methoxy-4-(piperidin-2-on-1-yl)-benzoesäure, TBTU, NMM und (1*S*)-1-(5-Chlor-1*H-*benzimidazol-2-yl)ethylamin in DMF und anschließender Reinigung durch präparative HPLC.
Ausbeute: 75%
Rₜ-Wert: 2.35 min
C₂₂H₂₃ClN₄O₃ * CF₃COOH (540.93 / 426.90)
Massenspektrum: (M+H)⁺ = 427/429 (Chlorisotope)

Analog wurden folgende Verbindungen hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **70** | 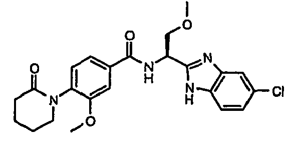 | Σ: 31% | (M+H)⁺= 457/459 (Chlorisotope) | 2.31 min |
| | *N-*[(1*R*)-1-(5-Chlor-1*H-*benzimidazol-2-yl)-2-methoxy-ethyl]-3-methoxy-4-(piperidin-2-on-1-yl)-benzamid | | | |
| **85** | 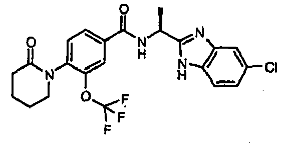 | Σ:0.8% | (M+H)⁺ = 481/483 (Chlorisotope) | 2.56 min |
| | *N-*[(1*S*)-1-(5-Chlor-1*H-*benzimidazol-2-yl)-ethyl]-4-(piperidin-2-on-1-yl)-3-trifluormethoxy-benzamid | | | |
| **86** | 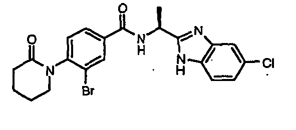 | 6.2% | (M+H)⁺ = 475/477M79 (Brom- und Chlorisotope) | 2.57 min |
| | 3-Brom-*N-*[(-1*S*)-1-(5-chlor-1*H-*benzimidazol-2-yl)-ethyl]-4-(piperidin-2-on-1-yl)-benzamid | | | |
| **87** | 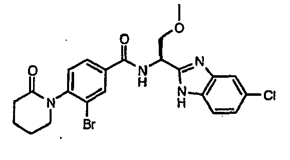 | Σ: 6.2% | (M+H)⁺ = 505/507/509 (Brom- und Chlorisotope) | 2.57 min |
| | 3-Brom-*N-*[(1*R*)-1-(5-chlor-1*H-*benzimidazol-2-yl)-2-methoxy-ethyl]-4-(piperidin-2-on-1-yl)-benzamid | | | |

### Beispiel 73

*N-*[(1*S*)-1-(5-Chlor-1*H-*benzimidazol-2-yl)-ethyl]-4-(1,1-dioxo-6-methyl-[1,2,6]thiadiazinan-2-yl)-3-methyl-benzamid

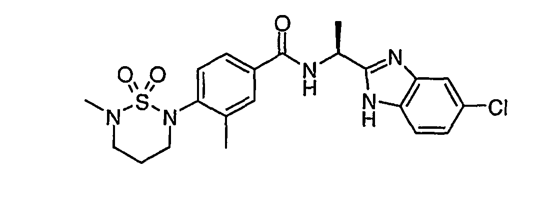

### (a) 4(1,1-Dioxo-6-methyl-[1,2,6]-thiadiazinan-2-yl)-3-methyl-benzoesäure-methylester

400 mg (1.41 mmol) 4-(1,1-Dioxo-[1,2,6]-thiadiazinan-2-yl)-3-methyl-benzoesäure-methylester werden in 4 ml DMF gelöst bei 40°C mit 240 mg (2.14 mmol) Kalium-*tert*.-butylat und 96 µl (1.54 mmol) Methyliodid versetzt und für 5 Stunden bei 40°C gerührt. Die Reaktionslösung wird i. Vak. eingeengt, mit Wasser versetzt und die wässrige Phase mit Ethylacetat extrahiert. Die vereinigten organischen Phasen werden über Natriumsulfat getrocknet und i. Vak. eingeengt.
Ausbeute: 220 mg (52 %)
C₁₃H₁₈N₂O₄S (298.36)
Massenspektrum: (M+H)⁺ = 299
R_{f}-Wert: 0.44 (Kieselgel; Petrolether/Ethylacetat = 3:2)

### (b) 4-(1,1-Dioxo-6-methyl-[1,2,6]thiadiazinan-2-yl)-3-methyl-benzoesäure

Hergestellt analog Beispiel 39d aus 4-(1,1-Dioxo-3-methyl-[1,2,6]thiadiazinan-2-yl)-3-methyl-benzoesäure-methylester und Lithiumhydroxid in Ethanol.
Ausbeute: (81%)
C₁₂H₁₆N₂O₄S (284.34)
Massenspektrum: (M+H)⁺ = 285
R_{f}-Wert: 0.07 (Kieselgel; Dichlormethan/Methanol =19:1)

### (c) N-[(1S)-1-(5-Chlor-1H-benzimidazol-2-yl)-ethyl]-4-(1,1-dioxo-6-methyl-[1,2,6]thiadiazinan-2-yl)-3-methyl-benzamid

Hergestellt analog Beispiel 1f aus 4-(1,1-Dioxo-3-methyl-[1,2,6]thiadiazinan-2-yl)-3-methyl-benzoesäure, (1*S*)-1-(5-Chlor-1*H-*benzimidazol-2-yl)-ethylamin, TBTU und NMM in DMF mit anschließender Reinigung durch präparative
HPLC.
Ausbeute: 55 %
C₂₁H₂₄ClN₅O₃S * CF₃COOH (576.00/461.97)
Massenspektrum: (M+H)⁺ = 462/464 (Chlorisotope)
Rₜ-Wert: 2.50 min

Analog wurde folgende Verbindung hergestellt:

| **Nr.** | **Strukturformel** | **Ausbeute** | **Massenpeak(s)** | **Rₜ**-Wert |
|---|---|---|---|---|
| | **Name** | | | |
| **74** | 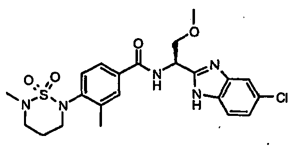 | Σ: 20% | (M+H)⁺ = 492/494 (Chlorisotope) | 2.56min |
| | *N-*[(1*R*)-1-(5-Chlor-1*H-*benzimidazol-2-yl)-2-methoxy-ethyl]-4-(1,1-dioxo-6-methyl-[1,2,6]thiadiazinan-2-yl)-3-methyl-benzamid | | | |

### Beispiel 75

*N-*[(1*R*)-1-(5-Brom-1*H-*benzimidazol-2-yl)-2-methoxy-ethyl]-3-methyl-4-(morpholin-3-on-4-yl)-benzamid

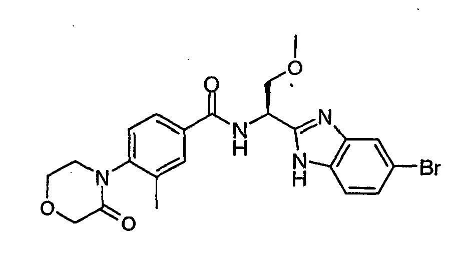

### (a) N'-(2-Amino-4-brom-phenyl)-N-Boc-(S)-O-methyl-serinamid und N'-(2-Amino-5-brom-phenyl)-N-Boc-(S)-O-methyl-serinamid

2.50 g (6.24 mmol) des Dicyclohexylammoniumsalzes von *N-*Boc-(*S*)-O-Methyl-serin_werden in 20 ml 5 %-iger Citronensäure gelöst, die wässrige Phase 2x mit je 20 ml Ethylacetat extrahiert, die vereinigten organischen Phasen über Natriumsulfat getrocknet und i. Vak. vom Lösemittel befreit. Der Rückstand wird zusammen mit 1.23 g (6.55 mmol) 4-Brom-1,2-phenylendiamin in 30 ml THF gelöst und unter Rühren im Eisbad 1.42 ml (14.0 mmol) Triethylamin sowie 4.97 ml (7.80 mmol) einer 50 %igen Lösung von PPA in Ethylacetat zugefügt. Nach 5 Minuten Rühren im Eisbad wird der Ansatz auf Raumtemperatur erwärmt und 23 Stunden bei Raumtemperatur gerührt. Das Reaktionsgemisch wird in 100 ml Wasser eingegossen und die wässrige Phase mit Ethylacetat extrahiert. Die vereinigten organischen Phasen werden mit ges. Natriumcarbonat-Lösung und Wasser ausgeschüttelt, über Natriumsulfat getrocknet und i. Vak. eingeengt.
Ausbeute: 2.38 g (98 %) Gemisch beider Regioisomere
C₁₅H₂₂BrN₃O₄ (388.26)
Massenspektrum: (M+H)⁺ = 388/390 (Bromisotope)
R_{f}-Wert: 0.63/0.68 (Kieselgel; Dichlormethan/Ethanol = 9:1)

### (b) (1R)-N-Boc-1(5-Brom-1 H-benzimidazol-2-yl)-2-methoxy-ethylamin

2.38 g (6.13 mmol) des bei 75a erhaltenen Gemisches werden in 150 ml Toluol gelöst und 1.84 ml (30.7 mmol) Essigsäure sowie 4.00 g Molekularsieb, 3Ǻ, zugefügt. Das Reaktionsgemisch wird für 3 Stunden bei 55°C gerührt und anschließend 15 Minuten im Eisbad abgekühlt. Die Reaktionsmischung wird filtriert und in ein Gemisch aus je 500 ml Wasser und Ethylacetat eingegossen. Nach heftigem Durchmischen wird die organische Phase abgetrennt, mit ges. Natriumchlorid-Lösung gewaschen, über Natriumsulfat getrocknet und i. Vak. eingeengt. Der Rückstand wird durch Chromatographie an Kieselgel gereinigt (Eluens-Gradient: Dichlormethan/Ethanol 100:0 -> 97:3).
Ausbeute: 1.40 g (62 %)
C₁₅H₂₀BrN₃O₃ (370.24)
Massenspektrum: (M+H)⁺ = 370/372 (Bromisotope)
Rf-Wert:0.81 (Kieselgel; Dichlormethan/Ethanol = 9:1)

### (c) (1R)-1-(5-Brom-1H-benzimidazol-1-yl)-2-methoxy-ethylamin

Hergestellt analog Beispiel 1g aus (1*R*)-*N-*Boc-1-(5-Brom-1*H-*benzimidazol-2-yl)-2-methoxy-ethylamin und TFA in Dichlormethan.
Ausbeute: 51 %
C₁₀H₁₂BrN₃O (270.13)
Massenspektrum: (M+H)⁺ = 270/272 (Bromisotope)
R_{f}-Wert: 0.20 (Kieselgel, Dichlormethan/Ethanol 9:1)

### (d) N-[(1R)-1-(5-Brom-1H-benzimidazol-2-yl)-2-methoxy-ethyl]-3-methyl-4-(morpholin-3-on-4-yl)-benzamid

Hergestellt analog Beispiel 1f aus 3-Methyl-4-(morpholin-3-on-4-yl)-benzoesäure, (1*R*)-1-(5-Brom-1*H-*benzimidazol-2-yl)-2-methoxy-ethylamin, TBTU und DIPEA in THF mit anschließender Reinigung durch Chromatographie an Kieselgel.
Ausbeute: quant.
C₂₂H₂₃BrN₄O₄ (487.35)
Massenspektrum: (M+H)⁺ = 487/489 (Bromisotope)
R_{f}-Wert: 0.56 (Kieselgel; Dichlormethan/Ethanol = 9:1)

### Beispiel 76

*N-*[(1*S*)-1-(5-Chlor-1*H-*benzimidazol-2-yl)-3-(1*H-*tetrazol-5-yl)-propyl]-3-methyl-4-(morpholin-3-on-4-yl)-benzamid

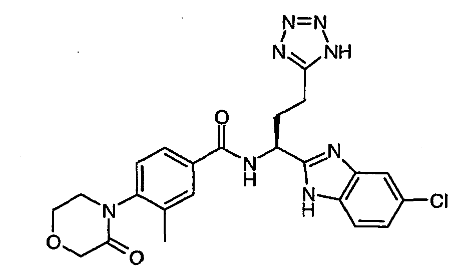

### (a) (2S)-2-(Benzyloxycarbonyl-amino)-4-cyano-buttersäure-ethylester

5.00 g (11.3 mmol) Dicyclohexylammonium-(2*S*)-2-(benzyloxycarbonyl-amino)-4-cyano-butanoat werden in 100 ml 5%-iger Citronensäurelösung gerührt und anschließend mit Ethylacetat extrahiert. Die vereinigten organischen Phasen werden über Natriumsulfat getrocknet und das Lösemittel i. Vak. abdestilliert. Der Rückstand wird in 70 ml THF gelöst, 4.35 g (13.5 mmol) TBTU und 6.35 ml (33.8 mmol) DIPEA zugesetzt und 10 Minuten bei Raumtemperatur gerührt. Dann werden 50 ml Ethanol zugefügt und das Gemisch für 16 Stunden zum Rückfluss erhitzt. Anschließend wird das Reaktionsgemisch i. Vak. eingeengt, in Ethylacetat aufgenommen, mit halbgesättigter Natriumhydrogencharbonat-Lösung und Wasser gewaschen, über Natriumsulfat getrocknet und i. Vak. eingeengt.
Ausbeute: 3.20 g (98 %)
C₁₅H₁₈N₂O₄ (290.32)
Massenspektrum: (M+NH₄)⁺ =308
R_{f}-Wert: 0.80 (Kieselgel; Dichlormethan/Ethanol =9:1)

### (b) (2S)-2-(Benzyloxycarbonyl-amino)-4-(1H-tetrazol-5-yl)-buttersäure-ethylester

3.20 g (11.0 mmol) (2*S*)-2-(Benzyloxycarbonyl-amino)-4-cyano-buttersäure-ethylester werden in 40 ml Toluol gelöst und 1.08 g (16.5 mmol) Natriumazid sowie 2.28 g (16.5 mmol) Triethylamin-hydrochlorid zugefügt. Die Reaktionsmischung wird bei 85°C für 24 Stunden erhitzt, auf Raumtemperatur abgekühlt und mit Wasser extrahiert. Die vereinigten wässrigen Phasen werden mit halbkonz. Salzsäure auf pH 2 angesäuert, der entstehende Niederschlag abgesaugt, mit Wasser gewaschen und bei 50°C getrocknet.
Ausbeute: 2.90 g (79 %)
C₁₅H₁₉N₅O₄ (333.34)
Massenspektrum: (M+H)⁺ = 334
R_{f}-Wert: 0.50 (Kieselgel; Dichlormethan/Ethanol = 9:1)

### (c) (2S)-2-(Benzyloxycarbonyl-amino)-4-(1H-tetrazol-5-yl)-buttersäure

Hergestellt analog Beispiel 30b aus (2*S*)-2-(Benzyloxycarbonyl-amino)-4-(1 *H-*tetrazol-5-yl)-buttersäure-ethylester und Lithiumhydroxid in einem Lösungsmittelgemisch aus Wasser und THF.
Ausbeute: quant.
C₁₃H₁₅N₅O₄ (305.29)
Massenspektrum: (M+H)⁺ = 306
R_{f}-Wert: 0.30 (Kieselgel; Dichlormethan/Ethanol = 4:1)

### (d) N'-(2-Amino-4-chlor-phenyl)-(2S)-2-(benzyloxycarbonyl-amino)-4-(1H tetrazol-5-yl)-buttersäureamid und N'-(2-Amino-5-chlor-phenyl)-(2S)-2-benzyloxycarbonyl-amino)-4-(1H-tetrazol-5-yl)-buttersäureamid

Hergestellt analog Beispiel 47a aus (2*S*)-2-(Benzyloxycarbonyl-amino)-4-(1*H-*tetrazol-5-yl)-buttersäure und 4-Chlor-1,2-phenylendiamin mit DCC in THF. Ausbeute: quant., Gemisch beider Regioisomere, leicht verunreinigt C₁₉H₂₀ClN₇O₃ (429.86)
R_{f}-Wert: 0.20 (Kieselgel; Dichlormethan/Ethanol = 9:1)

### (e) (1S)-N-(Benzyloxycarbonyl)-1-(5-chlor-1H-benzimidazol-2-yl)-3-(1H-tetrazol-5-yl)-propylamin

Hergestellt analog Beispiel 47b aus dem bei Beispiel 76d erhaltenen Produkt und Essigsäure.
Ausbeute: quant.
C₁₉H₁₉ClN₇O₂ * CH₃COOH (471.90 / 411.85)
R_{f}-Wert: 0.25 (Kieselgel; Dichlormethan/Ethanol/konz. Ammoniak = 4:1:0.1)

### (f) (1S)-1-(5-Chlor-1H-benzimidazol-2-yl)-3-(1H-tetrazol-5-yl)-propylamin

2.10 g (4.45 mmol) (1*S*)-*N-*(Benzyloxycarbonyl)-1-(5-chlor-1*H-*benzimidazol-2-yl)-3-(1*H-*tetrazol-5-yl)-propylamin werden in 30 ml Dichlormethan mit 1.9 ml (13.4 mmol) lod-trimethylsilan versetzt und für 16 Stunden bei Raumtemperatur gerührt. Dann werden 20 ml Methanol zugefügt, weitere 30 Minuten bei Raumtemperatur gerührt und die Reaktionsmischung i. Vak. vollständig eingeengt. Der Rückstand wird durch Chromatographie an Kieselgel gereinigt (Eluens-Gradient: Dichlormethan / (Ethanol / konz. Ammoniak 95:5) = 70/30 -> 60:40).
Ausbeute: 690 mg (56 %)
C₁₁H₁₂ClN₇ (277.71)
Massenspektrum: (M+H)⁺ = 278/280 (Chlorisotope)
R_{f}-Wert: 0.15 (Kieselgel; Dichlormethan/Ethanol = 3:2 + 1 % konz. Ammoniak)

### (g) N-[(1S)-1-(5-Chlor-1H-benzimidazol-2-yl)-3-(1H-tetrazol-5-yl)-propyl]-3-methyl-4-(morpholin-3-on-4-yl)-benzamid

Hergestellt analog Beispiel 1f aus 3-Methyl-4-(morpholin-3-on-4-yl)-benzoesäure, (1*S*)-1-(5-Chlor-1*H*-benzimidazol-2-yl)-3-(1*H*-tetrazol-5-yl)-propylamin, TBTU und DIPEA in THF mit anschließender Reinigung durch Chromatographie an Kieselgel.
Ausbeute: 33 %
C₂₃H₂₃ClN₈O₃ (494.93)
Massenspektrum: (M+H)⁺ = 495/497 (Chlorisotope)
R_{f}-Wert: 0.20 (Kieselgel; Dichlormethan/Ethanol = 4:1)

### Beispiel 77

*N*-[(1*S*)-1-(5-Chlor-1*H*-benzimidazol-2-yl)-3-methoxy-propyl]-3-methyl-4-(morpholin-3-on-4-yl)-benzamid

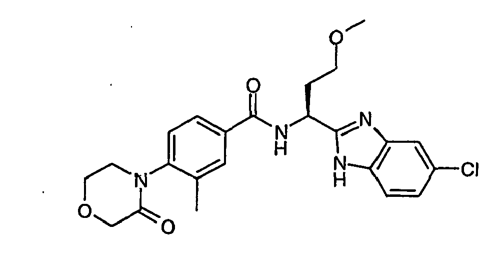

### (a) N'-(2-Amino-4-chlor-phenyl)-(2S)-2-(Boc-amino)-3-methoxy-propionsäureamid und N'-(2-Amino-5-chlor-phenyl)(2S)-2-(Boc-amino)-3-methoxy-propionsäureamid

4.90 g (21.0 mmol) (2*S*)-2-(Boc-Amino)-3-methoxy-propionsäure werden gelöst in 20 ml THF mit 13.57 g (42.0 mmol) TBTU und 5.76 ml (52.5 mmol) Triethylamin versetzt und 30 Minuten bei Raumtemperatur gerührt. Dann werden 3.00 g (21.0 mmol) 4-Chlor-1,2-phenylendiamin in 20 ml THF zugefügt und das Gemisch für 16 Stunden bei Raumtemperatur gerührt. Anschließend wird das Reaktionsgemisch i. Vak. eingeengt, in Wasser eingegossen und mit Ethylacetat extrahiert. Die vereinigten organischen Phasen werden mit ges. Natriumhydrogencharbonat-Lösung gewaschen, über Natriumsulfat getrocknet und i. Vak. eingeengt. Der Rückstand wird durch Chromatographie an Kieselgel gereinigt (Eluens: Dichlormethan/Methanol 99:1).
Ausbeute: 5.60 g (75%) Gemisch beider Regioisomere
C₁₆H₂₄ClN₃O₃ (357.83)
Massenspektrum: (M+H)⁺ = 358/360 (Chlorisotope)
R_{f}-Wert: 0.26 (Kieselgel; Dichlormethan/Methanol = 99:1)

### (b) (1S)-N-Boc-1-(5-Chlor-1H-benzimidazol-2-yl)-3-methoxy-propylamin

hergestellt analog Beispiel 47b aus dem bei Beispiel 77a erhaltenen Produkt und Essigsäure.
Ausbeute: 96%
C₁₆H₂₂ClN₃O₃ (339.82)
Massenspektrum: (M+H)⁺ = 340/342 (Chlorisotope)
R_{f}-Wert: 0.80 (Kieselgel; Dichlormethan/Methanol = 19:1)

### (c) (1S)-1-(5-Chlor-1H-benzimidazol-2-yl)-3-methoxy-propylamin

Hergestellt analog Beispiel 1g aus (1*S*)-*N*-Boc-1-(5-Chlor-1*H*-benzimidazol-2-yl)-3-methoxy-propylamin und TFA in Dichlormethan.
Ausbeute: 31 %
C₁₁H₁₄CIN₃O (239.70)
Massenspektrum: (M+H)⁺ = 240/242 (Chlorisotope)
R_{f}-Wert: 0.10 (Kieselgel; Dichlormethan/Ethanol = 9:1)

### (d) N-[(1S)-1-(5-Chlor-1H-benzimidazol-2-yl)-3-methoxy-propyl]-3-methvl-4-(morpholin-3-on-4-yl)-benzamid

Hergestellt analog Beispiel 1f aus 3-Methyl-4-(morpholin-3-on-4-yl)-benzoesäure, (1*S*)-1-(5-Chlor-1*H*-benzimidazol-2-yl)-3-methoxy-propylamin, TBTU und DIPEA in THF mit anschließender Reinigung durch Chromatographie an Kieselgel.
Ausbeute: 59 %
C₂₃H₂₅ClN₄O₄ (456.92)
Massenspektrum: (M+H)⁺ = 457/459 (Chlorisotope)
R_{f}-Wert: 0.51 (Kieselgel; Dichlormethan/Ethanol =9:1)

Analog wurde folgende Verbindung hergestellt:

| **Nr_{.}** | **Strukturformel** | **Ausbeute** | **Massenpeak(s)** | **R_{f}-Wert bzw. Rₜ** |
|---|---|---|---|---|
| | **Name** | | | |
| **148** | 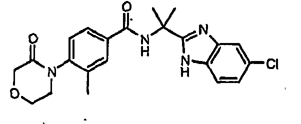 | Σ: 57% | (M+H)+ = 427/429 (Chlorisotope) | 0.5 (Kieselgel; Dichlormethan/ Ethanol = 9:1) |
| | *N*-[1-(5-Chlor-1*H*-benzimidazol-2-yl)-1-methyl-ethyl]-3-methyl-4-(morpholin-3-on-4-yl)-benzamid | | | |
| **88** | 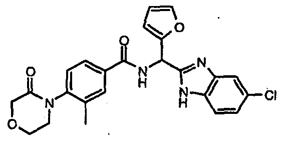 | Σ: 3.6% | (M+H)⁺ = 465/467 (Chlorisotope) | 2.38 min |
| | *N*-[1-(5-Chlor-1*H*-benzimidazol-2-yl)-1-(furan-2-yl)-methyl]-3-methyl-4-(morpholin-3-on-4-yl)-benzamid | | | |

### Beispiel 82

3-Chlor-*N*-[(1*S*)-1-(5-chlor-1*H*-benzimidazol-2-yl)-ethyl]-4-(3,6-dihydro-[1,2]oxazin-2-yl)-benzamid

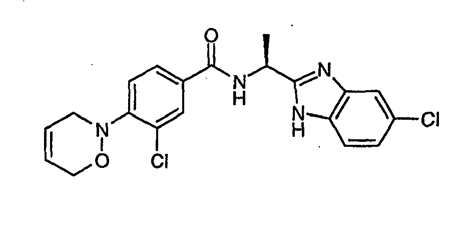

### (a) 3-Chlor-4-nitroso-benzoesäure-methylester

8.00 g (29.6 mmol) Kaliumperoxodisulfat werden unter Rühren in 6.0 ml konz. Schwefelsäure eingetragen, 30 Minuten bei Raumtemperatur unter Stickstoff-Atmosphäre gerührt, die Mischung in 50 g Eis eingerührt und mit 14 g Natriumcarbonat neutralisiert. Die erhaltene Lösung wird mit einer Suspension von 2.78 g (15.0 mmol) 4-Amino-3-chlor-benzoesäure-methylester in 300 ml Wasser versetzt und 16 Stunden bei Raumtemperatur gerührt. Das Reaktionsgemisch wird abgesaugt, der Filterkuchen mit Wasser gewaschen, an der Luft getrocknet und durch Chromatographie an Kieselgel gereinigt (Eluens: Petrolether/Ethylacetat 9:1).
Ausbeute: 1.00 g (33 %)
C₈H₆ClNO₃ (199.59)
Massenspektrum: (M+H)⁺ = 199/201 (Chlorisotope)
R_{f}-Wert: 0.55 (Kieselgel; Petrolether/Ethylacetat = 4:1)

### (b) 3-Chlor-4-(3,6-dihydro-[1,2]oxazin-2-yl)-benzoesäure-methylester

1.00 g (5.01 mmol) 3-Chlor-4-nitroso-benzoesäure-methylester werden in 10 ml Chloroform vorgelegt und bei 0°C unter Rühren eine frisch hergestellte Lösung von 1.10 g (20.3 mmol) Butadien in 6 ml Chloroform zugetropft. Die Reaktionsmischung wird für 30 Minuten bei 0-10°C und für 16 Stunden bei Raumtemperatur gerührt, i. Vak. eingeengt und durch Chromatographie an Kieselgel gereinigt (Eluens: Petrolether/Ethylacetat = 19:1).
Ausbeute: 1.00 g (79 %)
C₁₂H₁₂ClNO₃ (253.68)
Massenspektrum: (M+H)⁺ = 254/256 (Chlorisotope)
R_{f}-Wert: 0.50 (Kieselgel; Petrolether/Ethylacetat =4:1)

### (c) 3-Chlor-4-(3,6-dihydro-[1,2]oxazin-2-yl)-benzoesäure

Hergestellt analog Beispiel 2d aus 3-Chlor-4-(3,6-dihydro-[1,2]oxazin-2-yl)-benzoesäure-methylester und Natriumhydroxid in einem Lösungsmittelgemisch aus Wasser und Ethanol.
Ausbeute: 90%
C₁₁H₁₀CINO₃ (239.66)
Massenspektrum: (M-H)⁻= 238/240
R_{f}-Wert: 0.20 (Kieselgel; Petrolether/Ethylacetat =4:1)

### (d) 3-Chlor-N-[(1S)-1-(5-chlor-1H-benzimidazol-2-yl)-ethyl]-4-(3,6-dihydro-[1,2]oxazin-2-yl)-benzamid

Hergestellt analog Beispiel 1f aus 3-Chlor-4-(3,6-dihydro-[1,2]oxazin-2-yl)-benzoesäure, (1*S*)-1-(5-Chlor-1*H*-benzimidazol-2-yl)-ethylamin, TBTU und NMM in DMF.
Ausbeute: 88 %
C₂₀H₁₈Cl₂N₄O₂ (417.29)
Massenspektrum: (M+H)⁺ = 417/419/421 (Chlorisotope)
R_{f}-Wert: 0.35 (Kieselgel; Petrolether/Ethylacetat =1:1)

### Beispiel 83

3-Chlor-*N*-[(1*R*)-1-(5-chlor-1*H*-benzimidazol-2-yl)-2-methoxy-ethyl]-4-(1,1-dioxo-[1,2]thiazinan-2-yl)-benzamid

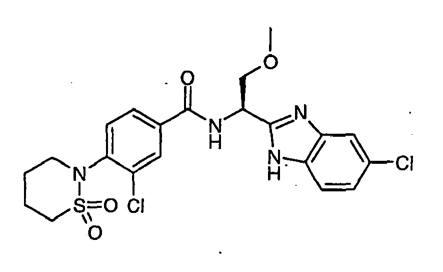

### (a) 2-Chlor-N-(4-chlorbutyl-sulfonyl)-4-methyl-anilin

1.30 ml (10.7 mmol) 2-Chlor-4-methyl-anilin werden in 30 ml Pyridin vorgelegt mit 2.67 g (10.5 mmol) 75%igem 4-Chlorbutyl-sulfonsäurechlorid versetzt 16 Stunden bei Raumtemperatur gerührt. Das Reaktionsgemisch wird in Wasser eingegossen und mit Ethylacetat extrahiert. Die vereinigten organischen Phasen werden mit 6-molarer Salzsäure gewaschen, über Natriumsulfat getrocknet und i. Vak. eingeengt. Der Rückstand wird durch Chromatographie an Kieselgel gereinigt (Eluens-Gradient: Petrolether/Ethylacetat =9:1 -> 7:3).
Ausbeute: 1.27 g (40 %)
C₁₁H₁₅Cl₂NO₂S (296.21)
Massenspektrum: (M+H)⁺ = 296/298/300 (Chlorisotope)
R*_{f}*-Wert: 0.42 (Kieselgel; Petrolether/Ethylacetat = 4:1)

### (b) 3-Chlor-4-(1,1-dioxo-[1,2]thiazinan-2-yl)-toluol

1.27 g (4.29 mmol) 2-Chlor-*N*-(4-chlorbutyl-sulfonyl)-4-methyl-anilin werden zusammen mit 722 mg (6.43 mmol) Kalium-tert.-butylat in 50 ml DMF für 16 Stunden bei 60°C gerührt. Dann wird die Reaktionsmischung in Wasser eingegossen und mit Ethylacetat extrahiert. Die vereinigten organischen Phasen werden über Natriumsulfat getrocknet und i. Vak. eingeengt. Der Rückstand wird durch Chromatographie an Kieselgel gereinigt (Eluens: Petrolether/Ethylacetat = 3:2).

Ausbeute: 850 mg (76 %)
C₁₁H₁₄CINO₂S (259.75)
Massenspektrum: (M+H)⁺ = 260/262 (Chlorisotope)
R_{f}-Wert: 0.27 (Kieselgel; Petrolether/Ethylacetat = 4:1)

### (c) 3-Chlor-4-(1,1-dioxo-[1,2]thiazinan-2-yl)-benzoesäure

250 mg (0.96 mmol) 3-Chlor-4-(1,1-dioxo-[1,2]thiazinan-2-yl)-toluol werden in 10 ml Wasser suspendiert mit 456 mg (2.89 mmol) Kaliumpermanganat und 39 mg (0.98 mmol) Natriumhydroxid versetzt. Die Reaktionsmischung wird für 4 Stunden zum Rückfluss erhitzt. Nach Abkühlen auf Raumtemperatur wird bis zur Entfärbung Natriumthiosulfat zugesetzt und dann mit Ethylacetat extrahiert. Die vereinigten organischen Phasen werden über Natriumsulfat getrocknet und i. Vak. eingeengt. Der Rückstand wird durch Chromatographie an Kieselgel gereinigt (Eluens: Dichlormethan/Methanol = 19:1).
Ausbeute: 50 mg (18 %)
C₁₁H₁₂CINO₄S (289.74)
Massenspektrum: (M+H)⁺ = 290/292
Rₜ-Wert: 2.50 min

### (d) 3-Chlor-N-[(1R)-1-(5-chlor-1H-benzimidazol-2-yl)-2-methoxy-ethyl]-4-(1,1-dioxo-[1,2]thiazinan-2-yl)-benzamid

Hergestellt analog Beispiel 1f aus 3-Chlor-4-(1,2-dioxo-[1,2]thiazinan-2-yl)-benzoesäure, (1*R*)-1-(5-Chlor-1*H*-benzimidazol-2-yl)-2-methoxy-ethylamin, TBTU und NMM in DMF mit anschließender Reinigung durch präparative
HPLC.
Ausbeute: 34 %
C₂₁H₂₂Cl₂N₄O₄S (497.40)
Massenspektrum: (M+H)⁺ = 497/499/501 (Chlorisotope) Rₜ-Wert: 2.53 min

### Beispiel 84

*N*-[(1*R*)-1-(5-Chlor-1*H*-benzimidazol-2-yl)-2-methoxy-ethyl]-4-(1,1-dioxo-[1,2]thiazepan-2-yl)-3-methyl-benzamid

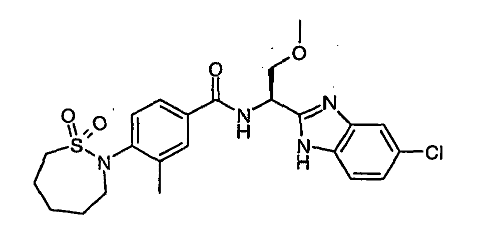

### (a) 4-(5-Chlorpentyl-sulfonyl-amino)-3-methyl-benzoesäure-methylester

Hergestellt analog Beispiel 83a aus 4-Amino-3-methyl-benzoesäure-methylester und 5-Chlorpentyl-sulfonsäurechlorid in Pyridin.
Ausbeute: 43 %
C₁₄H₂₀ClNO₄S (333.83)
Massenspektrum: (M+H)⁺ = 334/336 (Chlorisotope)
R_{f}-Wert: 0.72 (Kieselgel; Petrolether/Ethylacetat = 7:3)

### (b) 4-(1,1-Dioxo-[1,2]thiazepan-2-yl)-3-methyl-benzoesäure-methlyester

Hergestellt analog Beispiel 83b aus 4-(5-Chlorpentyl-sulfonyl-amino)-3-methyl-benzoesäure-methylester und Kalium-*tert*.-butylat in DMF.

Ausbeute: 19 %

C₁₄H₁₉NO₄S (297.37)
Massenspektrum: (M+H)⁺ = 298
Rₜ-Wert: 0.30 (Kieselgel; Petrolether/Ethylacetat = 4:1)

### (c) 4-(1,1-Dioxo-[1,2]thiazepan-2-yl)-3-methyl-benzoesäure

Hergestellt analog Beispiel 39d aus 4-(1,1-Dioxo-[1,2]thiazepan-2-yl)-3-methyl-benzoesäure-methylester und Lithiumhydroxid in einen Lösungsmittel-Gemisch aus Wasser und Ethanol.
Ausbeute: 60 %
C₁₃H₁₇NO₄S (283.34)
Massenspektrum: (M+H)⁺ =284
Rₜ-Wert: 2.60 min

### (d) N-[(1R)-1-5-Chlor-1H-benzimidazol-2-yl)-2-methoxy-ethyl]-4-dioxo-[1,2]thiazepan-2-yl)-3-methyl-benzamid

Hergestellt analog Beispiel 1f aus 4-(1,2-Dioxo-[1,2]thiazepan-2-yl)-3-methyl-benzoesäure, (1*R*)-1-(5-Chlor-1*H*-benzimidazol-2-yl)-2-methoxy-ethylamin, TBTU und NMM in DMF mit anschließender Reinigung durch Chromatographie an Kieselgel.
Ausbeute: 75 %
C₂₃H₂₇ClN₄O₄S (491.00)
Massenspektrum: (M+H)⁺ = 491/493 (Chlorisotope)
Rₜ-Wert: 2.60 min

### Beispiel 89

3-Chlor-*N*-[(1*S*)-1-(5-chlor-1*H*-benzimidazol-2-yl)-ethyl]-4-([1,2]oxazinan-2-yl)-benzamid 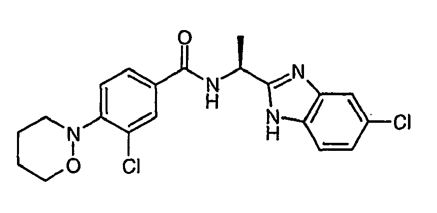 209 mg (0.50 mmol) 3-Chlor-*N*-[(1*S*)-1-(5-chlor-1*H*-benzimidazol-2-yl)-ethyl]-4-(3,6-dihydro-[1,2]oxazin-2-yl)-benzamid zusammen mit 100 mg 10%iger Palladiumkohle in 5 ml Ethylacetat für 7 Minuten bei Raumtemperatur unter einer Wasserstoff-Atmosphäre bei 5 bar hydriert. Anschließend wird abgesaugt, das Filtrat i. Vak. eingeengt und mit Ether nachgedampft.
Ausbeute: 200 mg (95 %)
C₂₀H₂₀Cl₂N₄O₂ (419.30)
Massenspektrum: (M+H)⁺ = 419/421/423 (Chlorisotope)
R_{f}-Wert: 0.40 (Kieselgel; Petrolether/Ethylacetat = 1:1)

### Beispiel 90

*N*-[(1*R*)-1-(5-Chlor-1*H*-benzimidazol-2-yl)-2-methoxy-ethyl]-3-methyl-4-(5-oxo-[1,4]oxazepan-4-yl)-benzamid

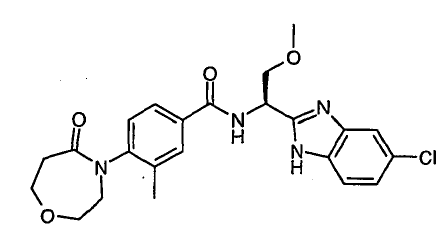

### (a) 3-(2-Benzyloxy-ethoxy)-propionsäure-ethylester

8.53 ml (60.0 mmol) Benzyloxy-ethanol werden in 40 ml THF mit 13 mg (0.57 mmol) Natrium versetzt, nach dessen Auflösung unter Argon-Atmosphäre 5.95 ml (54.7 mmol) Acrylsäureethylester zugesetzt und 20 Stunden bei Raumtemperatur gerührt. Nach Neutralisieren mit 0.6 ml 1-molarer Salzsäure wird das Reaktionsgemisch i. Vak. eingeengt, der Rückstand in ges. Natriumchlorid-Lösung aufgenommen und mit Ethylacetat extrahiert. Die vereinigten organischen Phasen werden mit ges. Natriumchlorid-Lösung gewaschen, über Natriumsulfat getrocknet und i. Vak. eingeengt. Der Rückstand wird durch Chromatographie an Kieselgel gereinigt (Eluens: Petrolether/Ethylacetat = 4:1).
Ausbeute: 3.73 g (25 %)
C₁₄H₂₀O₄ (252.31)
Massenspektrum: (M+H)⁺ = 253
R_{f}-Wert: 0.48 (Kieselgel; Petrolether/Ethylacetat = 4:1)

### (b) 3-(2-Hydroxy-ethoxy)-propionsäure-ethylester

3.73 g (14.8 mmol) 3-(2-Benzyloxy-ethoxy)-propionsäureethylester werden zusammen mit 665 mg 10%iger Palladiumkohle in 70 ml Ethanol für 44 Minuten bei Raumtemperatur unter einer Wasserstoff-Atmosphäre bei 3 bar hydriert. Anschließend wird abgesaugt und das Filtrat i. Vak. eingeengt.
Ausbeute: 2.26 g (94 %)
C₇H₁₄O₄(162.18)
Massenspektrum: (M+H)⁺ =163

### (c) 3-(2-Chlor-ethoxy)-propionsäure-ethylester

2.26 g (13.9 mmol) 3-(2-Hydroxy-ethoxy)-propionsäureethylester werden in 5 ml (68.5 mmol) Thionylchlorid suspendiert und 20 µl (0.27 mmol) DMF zugefügt. Die Reaktionsmischung wird für 4 Stunden zum Rückfluss erhitzt und anschließend i. Vak. eingeengt. Das Produkt wird ohne weitere Reinigung weiter umgesetzt.
Ausbeute: quant.
C₇H₁₃O₃ (180.63)
Massenspektrum: (M+H)⁺ = 181/183 (Chlorisotope)

### (d) 3-(2-Chlor-ethoxy)-propionsäure

2.00 g (11.1 mmol) 3-(2-Chlor-ethoxy)-propionsäure-ethylester werden in 8 ml Ethanol suspendiert und 4.96 ml (16.6 mmol) 8%-ige Lithiumhydroxid-Lösung zugefügt. Die Mischung wird für 4 Stunden bei Raumtemperatur gerührt, anschließend i. Vak. eingeengt, mit 2-molarer Salzsäure angesäuert, mit Diethylether versetzt und über Natriumsulfat getrocknet. Anschließend wird abfiltriert und i. Vak. eingeengt.
Ausbeute: 1.51 g (89 %)
C₅H₉ClO₃ (152.58)
Massenspektrum: (M-H)⁻ = 151/153 (Chlorisotope)

### (e) 3-(2-Chlor-ethoxy)-propionsäure-chlorid

Hergestellt analog Beispiel 90c aus 3-(2-Chlor-ethoxy)-propionsäure und Thionylchlorid mit DMF.
Ausbeute: 91 %
C₅H₈Cl₂O₂ (171.02)

### (f) 4-[3-(2-Chlor-ethoxyl)-propionyl-amino]-3-methyl-benzoesäure-methylester

1.70 g (10.3 mmol) 4-Amino-3-methyl-benzoesäure-methylester werden in 10 ml THF mit 2.84 ml (20.6 mmol) Triethylamin versetzt und für 20 Minuten bei Raumtemperatur gerührt. Dann wird eine Lösung von 1.78 g (10.4 mmol) 3-(2-Chlor-ethoxy)-propionsäure-chlorid in 25 ml THF zugetropft und weitere 2.5 Stunden bei Raumtemperatur gerührt. Anschließend wird Wasser zugesetzt und mit Ethylacetat extrahiert. Die vereinigten organischen Phasen werden über Natriumsulfat getrocknet und i. Vak. vollständig eingeengt. Der Rückstand wird durch Chromatographie an Kieselgel gereinigt (Eluens-Gradient: Petrolether/Ethylacetat = 7:3 -> 6:4).
Ausbeute: 1.25 g (41 %)
C₁₄H₁₈ClNO₄ (299.75)
Massenspektrum: (M+H)⁺ = 300/302 (Chlorisotope)
R_{f}-Wert: 0.15 (Kieselgel; Petrolether/Ethylacetat = 7:3)

### (g) 3-Methyl-4-(5-oxo-[1,4]oxazepan-4-yl)-benzoesäure-methylester

900 mg (3.00 mmol) 4-[3-(2-Chlor-ethoxy)-propionyl-amino]-3-methyl-benzoesäure-methylester werden in 40 ml DMF zusammen mit 520 mg (4.63 mmol) kalium-*tert*.-butylat und 12 mg (80 µmol) Natriumiodid für 3 Stunden bei 60°C gerührt. Nach Einengen i. Vak. wird der Rückstand mit Wasser versetzt, mit Ethylacetat extrahiert und die vereinigten organischen Phasen über Natriumsulfat getrocknet und i. Vak. eingeengt. Der Rückstand wird durch Chromatographie an Kieselgel gereinigt (Eluens: Petrolether/Ethylacetat = 11:9).
Ausbeute: 310 mg (39 %)
C₁₄H₁₇NO₄ (263.29)
Massenspektrum: (M+H)⁺ = 264

### (h) 3-Methyl-4-(5-oxo-[1,4]oxazepan-4-yl)-benzoesäure

Hergestellt analog Beispiel 39d aus 3-Methyl-4-(5-oxo-[1,4]oxazepan-4-yl)-benzoesäure-methylester und Lithiumhydroxid in einen Lösungsmittel-Gemisch aus Wasser und Ethanol.
Ausbeute: 69 %
C₁₃H₁₅NO₄ (249.26)
Massenspektrum: (M+H)⁺ = 250
Rₜ-Wert: 2.06 min

### (i) N-[(1R)-1-(5-Chlor-1H-benzimidazol-2-yl)-2-methoxy-ethyl]-3-methyl-4-(5-oxo-[1,4]oxazepan-4-yl)-benzamid

Hergestellt analog Beispiel 1f aus 3-Methyl-4-(5-oxo-[1,4]oxazepan-4-yl)-benzoesäure, (1*R*)-1-(5-Chlor-1*H*-benzimidazol-2-yl)-2-methoxy-ethylamin, TBTU und NMM in DMF mit anschließender Reinigung durch präparative
HPLC.
Ausbeute: 83 %
C₂₃H₂₅ClN₄O₄ (456.92)
Massenspektrum: (M+H)⁺ = 457/459 (Chlorisotope)
Rₜ-Wert: 2.23 min

### Beispiel 91

*N*-[(1*S*)-1-(5-Chlor-1*H*-benzimidazol-2-yl)-ethyl]-4-(4,4-dimethyl-2-oxo-imidazolidin-1-yl)-3-methyl-benzamid

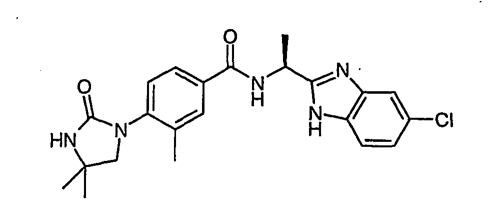

### (a) 4-(3[1,1-Dimethyl-2-hydroxy-ethyl]-ureido-3-methyl-benzoesäure-methylester

5.70 g (29.8 mmol) 4-Isocyanato-3-methyl-benzoesäure-methylester werden in 100 ml THF gelöst vorgelegt und eine Lösung von 2.86 ml (30.0 mmol) 2-Amino-2-methyl-propan-1-ol in 25 ml THF zugetropft. Die Mischung wird für 2 Stunden bei Raumtemperatur gerührt und dann i. Vak. eingeengt. Der Rückstand wird ohne weitere Reinigung weiter umgesetzt.
Ausbeute: 8.40 g (quant.)
C₁₄H₂₀N₂O₄ (280.32)
Massenspektrum: (M-H)⁻=279
R_{f}-Wert: 0.20 (Kieselgel; Dichlormethan/Ethanol =19:1)

### (b) 4-(4,4-Dimethyl-2-oxo-imidazolidin-l-yl)-3-methyl-benzoesäure-methylester

7.00 g (25.0 mmol) 4-(3-[1,1-Dimethyl-2-hydroxy-ethyl]-ureido)-3-methyl-benzoesäure-methylester werden gelöst in 400 ml THF bei 0°C mit 6.73 g (60.0 mmol) Kalium-*tert*.-butylat versetzt. Nach Rühren für 15 Minuten bei 0°C wird eine Lösung von 5.72 g (30.0 mmol) p-Toluolsulfonsäure in 50 ml THF zugetropft. Nach weiteren 10 Minuten Rühren bei 0°C werden 300 ml Wasser zugefügt, mit 1-molarer Salzsäure neutralisiert und i. Vak. das THF entfernt. Der Rückstand wird mit Dichlormethan extrahiert. Die vereinigten organischen Phasen werden über Natriumsulfat getrocknet und i. Vak. eingeengt. Anschließend erfolgt Reinigung des Rückstands durch Chromatographie an Kieselgel (Eluens-Gradient: Dichlormethan/Ethanol = 100:0 -> 97:3).
Ausbeute: 2.50 g (38 %)
C₁₄H₁₈N₂O₃ (262.30)
Massenspektrum: (M+H)⁺ = 263
R_{f}-Wert: 0.30 (Kieselgel; Dichlormethan/Ethanol =19:1)

### (c) 4-(4,4-Dimethyl-2-oxo-imidazolidin-1-yl)-3-methyl-benzoesäure

2.70 g (10.3 mmol) 4-(4,4-Dimethyl-2-oxo-imidazolidin-1-yl)-3-methyl-benzoesäure-methylester werden in 75 ml Methanol suspendiert und mit einer Lösung von 1.68 g (30.0 mmol) Kaliumhydroxid in 10 ml Wasser versetzt. Es wird 3 Stunden bei Raumtemperatur gerührt und anschließend i. Vak. eingeengt. Der wässrige Rückstand wird mit Wasser verdünnt, mit 1-molarer Salzsäure angesäuert und der anfallende Niederschlag abgesaugt, mit Wasser gewaschen und getrocknet.
Ausbeute: 2.30 g (90%)
C₁₃H₁₆N₂O₃ (248.28)
Massenspektrum: (M+H)⁺ = 249
R_{f}-Wert: 0.50 (Kieselgel; Dichlormethan/Methanol =9:1)

### (d) N-[(1S)-1-(5-Chlor-1H-benzimidazol-2-yl)-ethyl]-3-methyl-4-([1,3]oxazepan-2-on-3-yl)-benzamid

Hergestellt analog Beispiel 1f aus 4-(4,4-Dimethyl-2-oxo-imidazolidin-1-yl)-3-methyl-benzoesäure, (1*S*)-1-(5-Chlor-1*H*-benzimidazol-2-yl)-ethylamin, TBTU und DIPEA in THF und Reinigung des Rückstands durch Chromatographie an Kieselgel.
Ausbeute: 59 %
C₂₂H₂₄ClN₅O₂ (425.91)
Massenspektrum: (M+H)⁺ = 426/428 (Chlorisotope)
R_{f}-Wert: 0.40 (Kieselgel; Dichlormethan/Methanol = 9:1)

Analog wurde folgende Verbindung hergestellt:

| **Nr.** | **Strukturformel** | **Ausbeute** | **Massenpeak(s)** | **R_{f}**-Wert |
|---|---|---|---|---|
| | **Name** | | | |
| **92** | 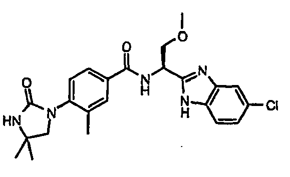 | Σ: 29% | (M+H)⁺ = 456/458 (Chlorisotope) | 0.45 (Kieselgel, CH₂Cl₂ /C₂H₅OH 9:1) |
| | *N-*[(1*R*)-1-(5-Chlor-1*H*-benzimidazol-2-yl)-2-methoxy-ethyl]-4-(4,4-dimethyl-2-oxo-imidazolidin-1-yl)-3-methyl-benzamid | | | |

### Beispiel 93

3-Chlor-*N*-[(1*S*)-1-(5-chlor-1*H*-benzimidazol-2-yl)-ethyl]-4-(4-methyl-2-oxo-oxazolidin-3-yl)-benzamid

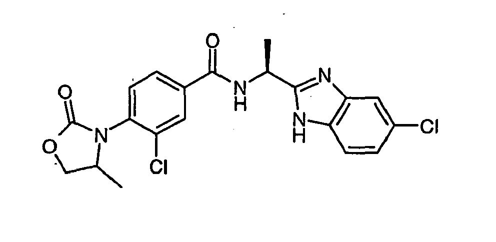

### (a) 3-Chlor-4-(2-hydroxy-1-methyl-ethylamino)-benzonitril

4.00g (25.7 mmol) 3-Chlor-4-fluor-benzonitril werden in 20 ml DMSO mit 8.00 g (106.5 mmol) 2-Amino-1-propanol unter Rühren für 2 Stunden auf 60°C erhitzt. Danach wird das Reaktionsgemisch in Wasser eingegossen und mit Ethylacetat extrahiert. Die vereinigten organischen Phasen werden mit Wasser und ges. Natriumchlorid-Lösung gewaschen, über Natriumsulfat getrocknet und i. Vak. eingeengt.
Ausbeute: 5.20 g (96 %)
C₁₀H₁₁ClN₂O (210.66)
Massenspektrum: (M+H)⁺ = 211/213 (Chlorisotope)
R_{f}-Wert: 0.27 (Kieselgel; Dichlormethan/Methanol = 19:1)

### (b) 3-Chlor-4-(2-hydroxy-1-methyl-ethylamino)-benzoesäure

5.20 g (24.7 mmol) 3-Chlor-4-(2-hydroxy-1-methyl-ethylamino)-benzonitril werden in 50 ml konz. Salzsäure unter Rühren für 6 Stunden zum Rückfluß erhitzt. Anschließend wird i. Vak. eingeengt, mit konz. Ammoniak-Lösung basisch gestellt und mit Ethylacetat ausgeschüttelt. Nach Ansäuern der wäßrigen Phase mit Essigsäure wird mit Ethylacetat extrahiert, die vereinigten organischen Phasen mit Wasser und ges. Natriumchlorid-Lösung gewaschen, über Natriumsulfat getrocknet und i. Vak. eingeengt.
Ausbeute: 5.00 g (88 %)
C₁₀H₁₂ClNO₃ (229.66)
Massenspektrum: (M+H)⁺ = 230/232 (Chlorisotope)
R_{f}-Wert: 0.44 (Kieselgel; Dichlormethan/Methanol = 9:1)

### (c) 3-Chlor-4-(2-hydrox-1-methyl-ethylamino)-benzoesäure-ethylester

5.00 g (21.8 mmol) 3-Chlor-4-(2-hydroxy-1-methyl-ethylamino)-benzoesäure werden in 100 ml ges. ethanolischer Salzsäure für 16 Stunden bei Raumtemperatur gerührt und anschließend i. Vak. eingeengt. Der Rückstand wird mit Wasser und konz. Ammoniak-Lösung versetzt und mit Ethylacetat extrahiert. Die vereinigten organischen Phasen werden mit Wasser und ges. Natriumchlorid-Lösung gewaschen, über Natriumsulfat getrocknet und i. Vak. eingeengt. Der Rückstand wird durch Chromatographie an Kieselgel gereinigt (Eluens: Dichlormethan / Methanol = 50:1).
Ausbeute: 3.40 g (61 %)
C₁₂H₁₆ClNO₃ (257.71)
Massenspektrum: (M+H)⁺ = 258/260 (Chlorisotope)
R_{f}-Wert: 0.34 (Kieselgel; Dichlormethan/Methanol = 19:1)

### (d) 3-Chlor-4-(4-methyl-2-oxo-oxazolidin-3-yl)-benzoesäure-ethylester

0.50 g (1.94 mmol) 3-Chlor-4-(2-hydroxy-1-methyl-ethylamino)-benzoesäureethylester werden zusammen mit 0.22 g (2.20 mmol) Triethylamin in 30 ml THF vorgelegt und unter Rühren bei Raumtemperatur 1.10 ml (2.08 mmol) 20%-ige Phosgen-Lösung in Toluol zugefügt. Die Mischung wird für 1 Stunde bei Raumtemperatur gerührt, dann 1 ml Wasser zugefügt und noch 10 Minuten gerührt. Anschließend wird die Mischung i. Vak. eingeengt, mit Wasser versetzt und mit Ethylacetat extrahiert. Die vereinigten organischen Phasen werden mit Wasser und ges. Natriumchlorid-Lösung gewaschen, über Natriumsulfat getrocknet und i. Vak. eingeengt.
Ausbeute: 0.54 g (98 %)
C₁₃H₁₄ClNO₄ (283.71)
Massenspektrum: (M+H)⁺ = 284/286 (Chlorisotope)
R_{f}-Wert: 0.71 (Kieselgel; Dichlormethan/Methanol = 19:1)

### (e) 3-Chlor-4-(4-methyl-2-oxo-oxazolidin-3-yl)-benzoesäure

0.90 g (3.17 mmol) 3-Chlor-4-(4-methyl-2-oxo-oxazolidin-3-yl)-benzoesäureethylester werden in 50 ml Methanol mit 10 ml 1-molarer wäßriger Lithiumhydroxid-Lösung bei Raumtemperatur für 1 Stunde gerührt. Danach wird die Mischung i. Vak. auf 20 ml eingeengt, mit konz. Salzsäure angesäuert und mit Ethylacetat extrahiert. Die vereinigten organischen Phasen werden mit ges. Natriumchlorid-Lösung gewaschen, über Natriumsulfat getrocknet und i. Vak. eingeengt. Der Rückstand wird aus wenig Diethylether kristallisiert und abgesaugt.
Ausbeute: 0.45 g (56 %)
C₁₁H₁₀ClNO₄ (255.65)
Massenspektrum: (M+H)⁺ = 256/258 (Chlorisotope)
R_{f}-Wert: 0.26 (Kieselgel; Dichlormethan/Methanol = 9:1)

### (f) 3-Chlor-N-[(1S)-1-(5-chlor-1H-benzimidazol-2-yl)-ethyl]-4-(4-methyl-2-oxo-oxazolidin-3-vl)-benzamid

Hergestellt analog Beispiel 1f aus 3-Chlor-(4-methyl-2-oxo-oxazolidin-3-yl)-benzoesäure, (1*S*)-1-(5-Chlor-1*H*-benzimidazol-2-yl)-ethylamin, TBTU und DIPEA in THF und Reinigung des Rückstands durch Chromatographie an Aluminiumoxid.
Ausbeute: 45 %
C₂₀H₁₈Cl₂N₄O₃ (433.29)
Massenspektrum: (M+H)⁺ = 433/435/437 (Chlorisotope)
R_{f}-Wert: 0.65 (Kieselgel; Dichlormethan/Methanol = 9:1)

Analog wurden folgende Verbindungen hergestellt:

| **Nr.** | **Strukturformel** | **Ausbeute** | **Massenpeak(s)** | **R_{f}-Wert bzw. Rₜ** |
|---|---|---|---|---|
| | **Name** | | | |
| **94** | 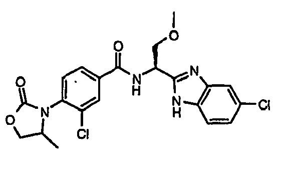 | Σ: 18% | (M+H)⁺ = 463/465/467 (Chlorisotope) | 0.60 (Kieselgel, CH₂Cl₂/CH₃OH 19:1) |
| | 3-Chlor-*N*-[(1*R*)-1-(5-chlor-1*H*-benzimidazol-2-yl)-2-methoxy-ethyl]-4-(4-methyl-2-oxo-oxazolidin-3-yl)-benzamid | | | |
| **102** | 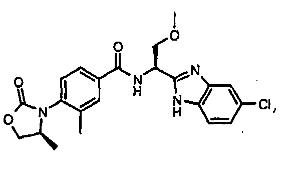 | Σ: 18% | (M+H)⁺ = 443/445 (Chlorisotope) | 0.20 (Kieselgel, CH₂Cl₂ /CH₃OH 19:1) |
| | *N*-[(1*R*)-1-(5-Chlor-1*H*-benzimidazol-2-yl)-2-methoxy-ethyl]-3-methyl-4-((4*S*)-4-methyl-2-oxo-oxazolidin-3-yl)-benzamid | | | |
| **103** | 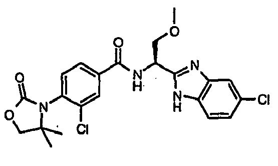 | Σ: 9.4% | (M+H)⁺ = 477/479/481 (Chlorisotope) | 0.30 (Kieselgel, CH₂Cl₂/CH₃OH 19:1) |
| | 3-Chlor-*N-*[(1*R*)-1-(5-chlor-1*H*-benzimidazol-2-yl)-2-methoxy-ethyl]-4-(4,4-dimethyl-2-oxo-oxazolidin-3-yl)-benzamid | | | |
| **104** | 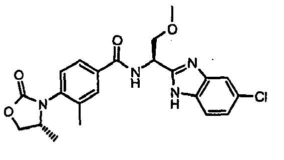 | Σ: 13% | (M+H)⁺ = 443/445 (Chlorisotope) | 0.80 (Kieseigel, CH₂Cl₂/CH₃OH 19:1) |
| | *N*-[(1*R*)-1-(5-Chlor-1*H*-benzimidazol-2-yl)-2-methoxy-ethyl]-3-methyl-4-((4*R*)-4-methyl-2-oxo-oxazolidin-3-yl)-benzamid | | | |
| **105** | 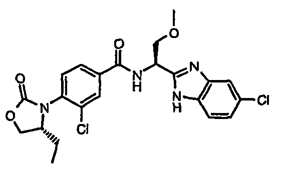 | Σ: 18% | (M+H)⁺ = 477/479/481 (Chlorisotope) | 0.65 (Kieselgel, CH₂Cl₂/CH₃OH 19:1) |
| | 3-Chlor-*N*-[(1*R*)-1-(5-chlor-1*H*-benzimidazol-2-yl)-2-methoxy-ethyl]-4-((4*R*)-4-ethyl-2-oxo-oxazolidin-3-yl)-benzamid | | | |

### Beispiel 95

*N*-[1-(5-Chlor-1*H*-benzimidazol-2-yl)-1-phenyl-methyl]-3-methyl-4-(morpholin-3-on-4-yl)-benzamid

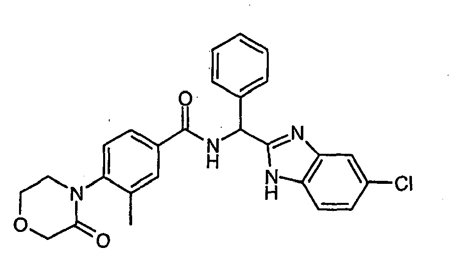

### (a) N'-(2-Amino-4-chlor-phenyl-N-Boc-phenyl-glycinamid und N'-(2-Amino-5-chlor-phenyl)-N-Boc-phenyl-glycinamid

Hergestellt analog Beispiel 47 a aus *N*-Boc-phenyl-glycin, 4-Chlor-1,2-phenylendiamin und DCC in THF.
Ausbeute: quant. Gemisch beider Regioisomere
C₁₉H₂₂ClN₃O₃ (375.85)
Massenspektrum: (M+H)⁺ = 376/378 (Chlorisotope)
R_{f}-Wert: 0.41 (Kieselgel; Dichlormethan/Ethanol = 19:1)

### (b) N-Acetyl-1-(5-chlor-1H-benzimidazol-2-yl)-1-phenyl-methylamin

3.65 g (9.71 mmol) des bei 95a erhaltenen Gemisches werden in 8 ml Essigsäure gelöst und für 6 Stunden unter Rühren zum Rückfluß erhitzt. Die Reaktionsmischung wird i. Vak. eingeengt und der Rückstand durch Chromatographie an Kieselgel gereinigt (Eluens-Gradient: Dichlormethan/Ethanol = 100.0 -> 94:6).
Ausbeute: 1.34 g (46 %)
C₁₆H₁₄ClN₃O (299.76)
Massenspektrum: (M+H)⁺ = 300/302 (Chlorisotope)
R_{f}-Wert: 0.19 (Kieselgel; Dichlormethan/Ethanol = 19:1)

### (c) 1-(5-Chlor-1H-benzimidazol-2-yl)-1-phenyl-methylamin

1.34 g (4.47 mmol) *N*-Acetyl-1-(5-chlor-1*H*-benzimidazol-2-yl)-1-phenyl-methylamin werden in 9 ml Ethanol mit 18 ml konz. Salzsäure versetzt und 2 Tage auf 50°C erhitzt. Die Reaktionsmischung wird i. Vak. eingeengt und 2 mal in Ethanol aufgenommen und wieder eingeengt.
Ausbeute: 1.26 g (96 %)
C₁₄H₁₂ClN₃ * HCl (294.18/257.72)
Massenspektrum: (M+H)⁺ = 258/260 (Chlorisotope)

### (d) N-(5-Chlor-1H-benzimidazol-2-yl)-1-phenyl-methyl]-3-methyl-4-(morpholin-3-on-4-yl)-benzamid

Hergestellt analog Beispiel 1f aus 3-Methyl-4-(morpholin-3-on-4-yl)-benzoesäure, 1-(5-Chlor-1*H*-benzimidazol-2-yl)-1-phenyl-methylamin, TBTU und DIPEA in THF.
Ausbeute: 89 %
C₂₆H₂₃ClN₄O₃ (474.94)
Massenspektrum: (M+H)⁺ = 475/477 (Chlorisotope)
R_{f}-Wert: 0.68 (Kieselgel; Dichlormethan/Ethanol = 9:1)

Analog wurde folgende Verbindung hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **108** | 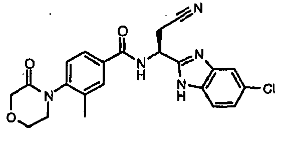 | Σ: 12% | (M+H)⁺= 437/439 (Chlorisotope) | 0.51 (Kieseigel; CH₂Cl₂/Ethanol = 9:1) |
| | *N*-[1-(1*S*)-(5-Chlor-1*H* benzimidazol-2-yl)-2-cyano-ethyl]-3-methyl-4-(morpholin-3-on-4-yl)-benzamid | | | |
| **140** | 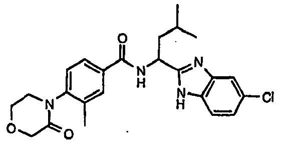 | Σ: 2.9% | (M+H)⁺ = 455/457 (Chlorisotope) | 0.60 (Kieselgel, CH₂Cl₂/C₂H₅OH 9:1) |
| | *N*-[1-(5-Chlor-1*H*-benzimidazol-2-yl)-3-methyl-butyl]-3-methyl-4-(morpholin-3-on-4-yl)-benzamid | | | |

### Beispiel 96

*N*-[(1*S*)-1-(5-Chlor-1*H*-benzimidazol-2-yl)-propyl)-3-methyl-4-(morpholin-3-on-4-yl)-benzamid

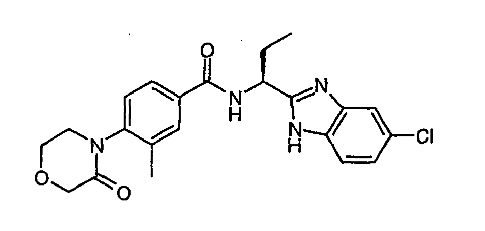

### (a) N'-(2-Amino-4-chlor-phenyl)-(2S)-2-(Boc-amino)-buttersäureamid und N'-(2-Amino-5-chlor-phenyl)-(2S)-2-(Boc-amino)-buttersäureamid

Hergestellt analog Beispiel 47a aus (2*S*)-2-(Boc-amino)-buttersäure, 4-Chlor-1,2-phenylendiamin und DCC in THF.
Ausbeute: 89 % Gemisch beider Regioisomere
C₁₅H₂₂ClN₃O₃ (327.81)
Massenspektrum: (M+H)⁺ =328/330 (Chlorisotope)
R_{f}-Wert: 0.63 (Kieselgel; Dichlormethan/Ethanol = 19:1)

### (b) (1S)-N-Boc-1-(5-chlor-1H-benzimidazol-2-1-propylamin

Hergestellt analog Beispiel 47b aus dem bei 95a erhaltenen Gemisch und Essigsäure und Reinigung des Rückstands durch Chromatographie an Kieselgel.
Ausbeute: 94 %
C₁₅H₂₀ClN₃O₂ (309.79)
Massenspektrum: (M+H)⁺ =310/312 (Chlorisotope)
R_{f}-Wert: 0.63 (Kieselgel; Dichlormethan/Ethanol = 19:1)

### (c) (1S)-1-(5-Chlor-1H-benzimidazol-2-yl-1-phenyl-methylamin

Hergestellt analog Beispiel 75c aus (1*S*)-*N*-Boc-1-(5-chlor-1*H*-benzimidazol-2-yl)-propylamin und Trifluoressigsäure in Dichlormethan.
Ausbeute: quant.
C₁₀H₁₂ClN₃ * 2 CF₃COOH (437.72 / 209.68)
Massenspektrum: (M+H)⁺ =210/212 (Chlorisotope)

### (d) N-[(1S)-1-(5-Chlor-1H-benzimidazol-2-yl)-propyl]-3-methyl-4-(morpholin-3-on-4-yl)-benzamid

Hergestellt analog Beispiel 1f aus 3-Methyl-4-(morpholin-3-on-4-yl)-benzoesäure, (1*S*)-1-(5-Chlor-1*H*-benzimidazol-2-yl)-propylamin, TBTU und DIPEA in THF.
Ausbeute: 17 %
C₂₂H₂₃ClN₄O₃ (426.90)
Massenspektrum: (M+H)⁺ =427/429 (Chlorisotope)
R_{f}-Wert: 0.45 (Kieselgel; Dichlormethan/Ethanol = 9:1)

Analog wurden folgende Verbindungen hergestellt:

| **Nr.** | **Strukturformel** | **Ausbeute** | **Massenpeak(s)** | **R_{f}**-Wert bzw. R**ₜ** |
|---|---|---|---|---|
| | **Name** | | | |
| **107** | 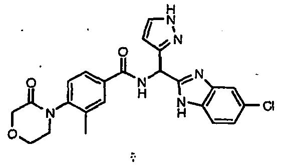 | Σ: 4.0% | (M+H)⁺ = 465/467 (Chlorisotope) | 0.15 (Kieselgel, CH₂Cl₂ /CH₃OH 9:1) |
| | *N*-[1-(5-Chlor-1*H* benzimidazol-2-yl)-1-(1*H*-pyrazol-3-yl)-methyl]-3-methyl-4-(morpholin-3-on-4-yl)-benzamid | | | |
| **141** | 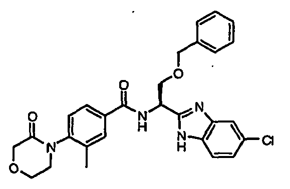 | Σ: 62% | (M-H)⁺= 519/521 (Chlorisotope) | 0.54 (Kieselgel, CH₂Cl₂ /C₂H₂OH 9:1) |
| | *N*-[(1*R*)-1-(5-Chlor-1*H*-benzimidazol-2-yl)-2-benzyloxy-ethyl]-3-methyl-4-(morpholin-3-on-4-yl)-benzamid | | | |
| | 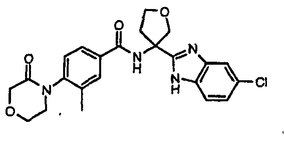 | Σ: 54% | (M-H)⁻ = 453/455 (Chlorisotope) | 0.55 (Kieselgel, CH₂Cl₂ /C₂H₅OH 9:1) |
| | *N*-[3-(5-Chlor-1*H*-benzimidazol-2-yl)-tetrahydrofuran-3-yl]-3-methyl-4-(morpholin-3-on-4-yl)-benzamid | | | |

### Beispiel 106

*N*-[(1*R*)-1-(5-Chlor-1*H*-benzimidazol-2-yl)-2-methoxy-ethyl]-3-fluor-4-(morpholin-3-on-4-yl)-benzamid

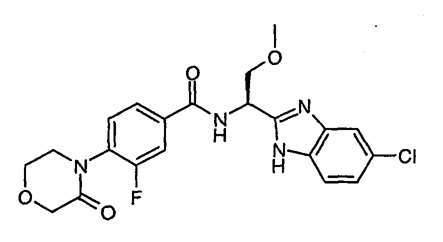

### (a) 4-[(2-Chlor-ethoxy)-acetyl-amino]-3-fluor-benzoesäure-ethylester

Hergestellt analog Beispiel 46a aus (2-Chlor-ethoxy)-acetylchlorid und 4-Amino-3-fluor-benzoesäure-ethylester mit TEA in THF.
Ausbeute: 44 %
C₁₃H₁₅ClFNO₄ (303.71)
Massenspektrum: (M+H)⁺ = 304/306 (Chlorisotope)
R_{f}-Wert: 0.29 (Kieselgel; Dichlormethan)

### (b) 4-(2-Carboxymethoxy-ethylamino)-3-fluor-benzoesäure

580 mg (1.91 mmol) 4-[(2-Chlor-ethoxy)-acetyl-amino]-3-fluor-benzoesäureethylester werden in 6 ml Dioxan mit 3.82 ml (7.64 mmol) 2-molarer Kalilauge und 2 ml Wasser versetzt. Anschließend wird für 2 h auf 70°C erhitzt, dann mit Wasser verdünnt und mit 6-molarer Salzsäure angesäuert. Nach Zugabe von Dichlormethan wird der entstandene Niederschlag abgesaugt und im Trockenschrank bei 50°C getrocknet.
Ausbeute: 390 mg (79 %)
C₁₁H₁₂FNO₅ (257.22)
Massenspektrum: (M+H)⁺ =258
R_{f}-Wert: 0.66 (reversed phase RP-8; Methanol/5%-ige NaCl-Lösung = 6:4)

### (c) 3-Fluor-4-(morpholin-3-on-4-yl)-benzoesäure-chlorid

Hergestellt analog Beispiel 41 c aus 4-(2-Carboxymethoxy-ethylamino)-3-fluorbenzoesäure und Thionylchorid in Dichlormethan mit DMF.
Ausbeute: quant.
C₁₁H₉ClFNO₃ (257.65)

### (d) N-[(1R)-1-(5-Chlor-1H-benzimidazol-2-yl)-2-methoxy-ethyl]-3-fluor-4-(morpholin-3-on-4-yl)-benzamid

Hergestellt analog Beispiel 41 d aus 3-Fluor-4-(morpholin-3-on-4-yl)-benzoesäure-chlorid und (1*R*)-1-(5-Chlor-1*H*-benzimidazol-2-yl)-2-methoxyethylamin mit TEA in THF.
Ausbeute: 47 %
C₂₁H₂₀ClFN₄O₄ (446.86)
Massenspektrum: (M+H)⁺ = 447/449 (Chlorisotope)
R_{f}-Wert: 0.40 (Kieselgel; Dichlormethan/Ethanol = 9:1)

### Beispiel 109

*N*-[1-(5-Chlor-1*H*-benzimidazol-2-yl)-1-(pyridin-3-yl)-methyl]-3-methyl-4-(morpholin-3-on-4-yl)-benzamid

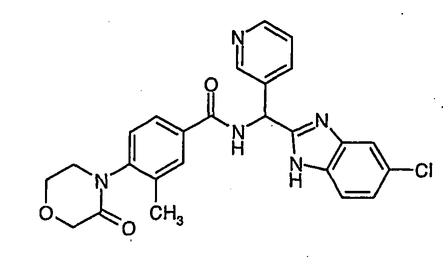

### (a) N'-(2-Amino-4-chlor-phenyl)-2-(Boc-amino)-2-(pyridin-3-yl)-essigsäureamid und N'-(2-Amino-5-chlor-phenyl)-2-Boc-amino)-2-(pyridin-3-yl)-essigsäureamid

1.00 g (3.96 mmol) *N*-Boc-Amino-2-(pyridin-3-yl)-essigsäure werden zusammen mit 0.59 g (4.16 mmol) 4-Chlor-1,2-phenylendiamin bei 0°C in 20 ml THF vorgelegt und 2.92 ml (4.96 mmol) 50%-ige PPA-Lösung in Ethylacetat sowie 1.24 ml (8.92 mmol) TEA zugefügt. Nach Rühren für 30 min bei 0°C wird für 5 h bei Raumtemperatur gerührt und anschließend i. Vak. vollständig eingeengt. Der Rückstand wird durch Chromatographie an Kieselgel gereinigt (Eluens-Gradient: Dichlormethan / Ethanol = 10:0 -> 9:1).
Ausbeute: 1.32 g (88 %) Gemisch beider Regioisomeren
C₁₈H₂₁ClN₄O₃ (376.84)
Massenspektrum: (M+H)⁺ = 377/379 (Chlorisotope)
R_{f}-Wert: 0.40 (Kieselgel; Dichlormethan/Ethanol = 9:1)

### (b) N-Boc-1-(5-Chlor-1H-benzimidazol-2-yl)-1-(pyridin-3-yl)-methylamin

Hergestellt analog Beispiel 47b aus dem bei Beispiel 111 a erhaltenen Produkt und Essigsäure.
Ausbeute: 81 %
C₁₈H₁₉ClN₄O (358.82)
Massenspektrum: (M+H)⁺ = 359/361 (Chlorisotope)
R_{f}-Wert: 0.51 (Kieselgel; Dichlormethan/Ethanol = 9:1)

### (c) 1-(5-Chlor-1H-benzimidazol-2-yl)-1-(pyridin-3-yl)-methylamin

Hergestellt analog Beispiel 1g aus *N*-Boc-1-(5-Chlor-1*H*-benzimidazol-2-yl)-1-(pyridin-3-yl)-methylamin und Trifluoressigsäure in Dichlormethan.
Ausbeute: 66 %
C₁₃H₁₁ClN₄ (258.71)
Massenspektrum: (M+H)⁺ = 259/261 (Chlorisotope)
R_{f}-Wert: 0.62 (Kieselgel; Dichlormethan/Ethanol = 9:1)

### (d) N-[1-(5-Chlor-1H-benzimidazol-2-yl)-1-(pyridin-3-yl)-methyl]-3-methyl-4-(morpholin-3-on-4-yl)-benzamid

Hergestellt analog Beispiel 1f aus 3-Methyl-4-(morpholin-3-on-4-yl)-benzoesäure und 1-(5-Chlor-1*H-*benzimidazol-2-yl)-1-(pyridin-3-yl)-methylamin mit TBTU und DIPEA in THF.
Ausbeute: 84 %
C₂₅H₂₂ClN₅O₉ (475.93)
Massenspektrum: (M+H)⁺ = 476/478 (Chlorisotope)
R_{f}-Wert: 0.31 (Kieselgel; Dichlormethan/Ethanol = 9:1)

### Beispiel 110

*N*-[1-(5-Chlor-1*H-*benzimidazol-2-yl)-1-(1-methyl-pyrazol-3-yl)-methyl]-3-methyl-4-(morpholin-3-on-4-yl)-benzamid

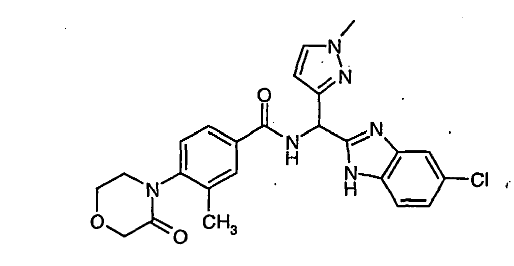

### (a) Ethoxycarbonyl-methoxyimino-(1-methyl-pyrazol-3-yl)-essigsäure-methylester

5.00 g (20.7 mmol) Ethoxycarbonyl-methoxyimino-(pyrazol-3-yl)-essigsäure werden zusammen mit 5.73 g (41.5 mmol) Kaliumcarbonat bei Raumtemperatur in 20 ml DMF vorgelegt, bis zur Beendigung der Gasentwicklung gerührt, anschließend 2.58 ml (41.5 mmol) Methyliodid zugefügt und für 2 h bei 50°C gerührt. Nach Einengen des Reaktionsgemisches i. Vak. wird der Rückstand mit Wasser und Ethylacetat versetzt, die organische Phase mit Wasser gewaschen, über Magnesiumsulfat getrocknet und i. Vak. vollständig eingeengt. Der Rückstand wird durch Chromatographie an Kieselgel gereinigt (Eluens-Gradient: Petrolether / Ethylacetat = 80:20 -> 65:35).
Ausbeute: 2.61 g (26 %) im Gemisch mit Regioisomeren
C₁₁H₁₅N₃O₅ (269.25)
Massenspektrum: (M+H)⁺ = 270
R_{f}-Wert: 0.25 (Kieselgel; Petrolether/Ethylacetat = 1:1)

### (b) 2-Amino-2-(1-methyl-pyrazol-3-yl)-essigsäure-methylester

2.61 g (9.69 mmol) Ethoxycarbonyl-methoxyimino-(1-methyl-pyrazol-3-yl)-essigsäure-methylester werden in 60 ml Ethanol mit 1.1 g 5%-iger Palladiumkohle für 16 h bei 50°C unter 3.4 bar Druck in einer Wasserstoff-Atmosphäre hydriert. Anschließend wird abgesaugt und das Filtrat i. Vak. vollständig eingeengt.
Ausbeute: 1.90 g (quant.), leicht verunreinigt
C₇H₁₁N₃O₂ (169.18)
Massenspektrum: (M+H)⁺ = 170
R_{f}-Wert: 0.30 (Kieselgel; Dichlormethan/Ethanol = 9:1)

### (c) N-Boc-2-Amino-2-(1-methyl-pyrazol-3-yl)-essigsäure-methylester

Hergestellt analog Beispiel 1d aus 2-Amino-2-(1-methyl-pyrazol-3-yl)-essigsäure-methylester und Pyrokohlensäure-di-*tert*-butylester mit TEA in Dichlormethan.
Ausbeute: 81 %
C₁₂H₁₉N₃O₄ (269.30)
Massenspektrum: (M+H)⁺ = 270

### (d) N-Boc-2-Amino-2-(1-methyl-pyrazol-3-yl)-essigsäure

1.16 g (4.31 mmol) *N-*Boc-2-Amino-2-(1-methyl-pyrazol-3-yl)-essigsäure-methylester werden in 16 ml THF mit 10 ml Wasser versetzt und 10 ml 1-molarer Lithiumhydroxid-Lösung zugefügt. Nach Rühren bei Raumtemperatur für 2 h wird i. Vak. eingeengt, der Rückstand mit Wasser versetzt, filtriert und das Filtrat mit Kaliumhydrogensulfat-Lösung auf pH 5 gebracht. Nach vollständigem Einengen i. Vak. wird der Rückstand mit Dichlormethan und wenig Ethanol behandelt, abgesaugt und das Filtrat i. Vak. vollständig eingeengt.
Ausbeute: 0.92 g (84 %)
C₁₁H₁₇N₃O₄ (255.27)
Massenspektrum: (M+H)⁺ = 256
R_{f}-Wert: 0.15 (Kieselgel; Dichlormethan/Ethanol = 8:2)

### (e) N'-(2-Amino-4-chlor-phenyl)-2-(Boc-amino)-2-(1-methyl-pyrazol-3-yl)-essigsäureamid und N'-(2-Amino-5-chlor-phenyl)-2-(Boc-amino)-2-(1-methyl-pyrazol-3-yl)-essigsäureamid

Hergestellt analog Beispiel 111 a aus *N-*Boc-2-Amino-2-(1-methyl-pyrazol-3-yl)-essigsäure und 4-Chlor-1,2-phenylendiamin mit PPA in Ethylacetat und NMM in Dichlormethan.
Ausbeute: 55 % Gemisch beider Regioisomeren
C₁₇H₂₂ClN₅O₃ (379.84)
R_{f}-Wert: 0.61 (Kieselgel; Dichlormethan/Ethanol = 9:1)

### (f) N-Boc-1-(5-Chlor-1H-benzimidazol-2-yl)-1-(1-methyl-pyrazol-3-yl)-methylamin

Hergestellt analog Beispiel 47b aus dem bei Beispiel 112e erhaltenen Produkt und Essigsäure.
Ausbeute: 81 %
C₁₇H₂₀ClN₅O₂ (361.83)
Massenspektrum: (M+H)⁺ = 362/364 (Chlorisotope)
R_{f}-Wert: 0.60 (Kieselgel; Dichlormethan/Ethanol = 9:1)

### (g) 1-(5-Chlor-1H-benzimidazol-2-yl)-1-(1-methyl-pyrazol-3-yl)-methylamin

Hergestellt analog Beispiel 1g aus *N-*Boc-1-(5-Chlor-1*H-*benzimidazol-2-yl)-1-(1-methyl-pyrazol-3-yl)-methylamin und Trifluoressigsäure in Dichlormethan. Ausbeute: 77 %
C₁₂H₁₂ClN₅ (261.71)
Massenspektrum: (M-NH₃+H)⁺ = 245/247 (Chlorisotope)
R_{f}-Wert: 0.30 (Kieselgel; Dichlormethan/Ethanol/konz. Ammoniak-Lösung = 9:1:0.1)

### (h) N-[1-(5-Chlor-1H-benzimidazol-2-yl)-1-(1-methyl-pyrazol-3-yl)-methyl]-3-methyl-4-(morpholin-3-on-4-yl)-benzamid

Hergestellt analog Beispiel 1f aus 3-Methyl-4-(morpholin-3-on-4-yl)-benzoesäure und 1-(5-Chlor-1*H-*benzimidazol-2-yl)-1-(1-methyl-pyrazol-3-yl)-methylamin mit TBTU und DIPEA in THF.
Ausbeute: 38 %
C₂₄H₂₃ClN₆O₃ (478.93)
Massenspektrum: (M+H)⁺ = 479/481 (Chlorisotope)
Rₜ-Wert: 0.50 (Kieselgel; Dichlormethan/Ethanol = 9:1)

### Beispiel 111

3-Chlor-*N-*[(1*R*)-1-(5-chlor-1*H-*benzimidazol-2-yl)-2-methoxy-ethyl]-4-(5-methyl-morpholin-3-on-4-yl)-benzamid

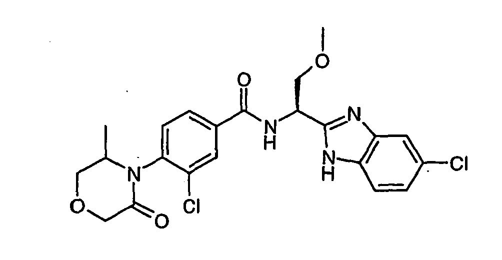

### (a) 4-[2-(tert.-Butoxycarbonyl-methoxy)-1-methyl-ethylamino]-3-chlorbenzoesäure-ethylester

1.12 g (4.35 mmol) 3-Chlor-4-(2-hydroxy-1-methyl-ethylamino)-benzoesäureethylester werden in 10 ml DMF mit 0.21 g (4.78 mmol) 55%-iger Natriumhydrid-Dispersion versetzt und für 5 min bei Raumtemperatur gerührt. Anschließend werden 0.67 ml Bromessigsäure-*tert*.-butylester zugegeben und weitere 16 h bei Raumtemperatur gerührt. Dann wird das Reaktionsgemisch in Wasser eingegossen und mit Ethylacetat extrahiert. Die vereinigten organischen Phasen werden mit Wasser und ges. Natriumchlorid-Lösung gewaschen, über Natriumsulfat getrocknet und i. Vak. vollständig eingeengt. Der Rückstand wird durch Chromatographie an Kieselgel gereinigt (Eluens-Gradient: Petrolether/Ethylacetat = 95:5 -> 80:20)
Ausbeute: 230 mg (14 %)
C₁₈H₂₆ClNO₅ (371.86)
Massenspektrum: (M+H)⁺ = 372/374 (Chlorisotope)
R_{f}-Wert: 0.65 (Kieselgel; Petrolether/Ethylacetat = 7:3)

### (b) 3-Chlor-4-[2-(hydroxycarbonyl-methoxy)-1-methyl-ethylamino]-benzoesäure-ethylester

Hergestellt analog Beispiel 1g aus 4-[2-(*tert*.-Butoxycarbonyl-methoxy)-1-methyl-ethylamino]-3-chlor-benzoesäure-ethylester und Trifluoressigsäure in Dichlormethan.
Ausbeute: 87 %
C₁₄H₁₈ClNO₅ (315.75)
Massenspektrum: (M+H)⁺ = 316/318
R_{f}-Wert: 0.40 (Kieselgel; Dichlormethan/Ethanol = 9:1)

### (c) 3-Chlor-4-(5-methyl-morpholin-3-on-4-yl)-benzoesäure-ethylester

Hergestellt analog Beispiel 41 c aus 3-Chlor-4-[2-(hydroxycarbonyl-methoxy)-1-methyl-ethylamino]-benzoesäure-ethylester und Thionylchlorid mit DMF in Dichlormethan.
Ausbeute: 69 % (verunreinigt)
C₁₄H₁₆ClNO₄ (297.73)
Massenspektrum: (M+H)⁺ = 298/300 (Chlorisotope)
R_{f}-Wert: 0.40 (Kieselgel; Dichlormethan/Ethanol = 19:1)

### (d) 3-Chlor-4-(5-methyl-morpholin-3-on-4-yl)-benzoesäure

Hergestellt analog Beispiel 31 b aus 3-Chlor-4-(5-methyl-morpholin-3-on-4-yl)-benzoesäure-ethylester mit Lithiumhydroxid in THF und Wasser.
Ausbeute: 91 %
C₁₂H₁₂ClNO₄ (269.68)
R_{f}-Wert: 0.30 (Kieselgel; Dichlormethan/Ethanol = 9:1)

### (e) 3-Chlor-N-[(1R)-1-(5-Chlor-1H-benzimidazol-2-yl)-2-methoxy-ethyl]-4-(5-methyl-morpholin-3-on-4-yl)-benzamid

Hergestellt analog Beispiel 1f aus 3-Chlor-4-(5-methyl-morpholin-3-on-4-yl)-benzoesäure und (1*R*)-1-(5-Chlor-1*H-*benzimidazol-2-yl)-2-methoxy-ethylamin mit TBTU und TEA in DMF.
Ausbeute: 28 %
C₂₂H₂₂Cl₂N₄O₄ (477.34)
Massenspektrum: (M-H)⁻ = 475/477/479 (Chlorisotope)
R_{f}-Wert: 0.50 (Kieselgel; Dichlormethan/Ethanol = 9:1)

### Beispiel112

3-Chlor-*N-*[(1*S*)-1-(5-chlor-1*H-*benzimidazol-2-yl)-ethyl]-4-(3-dimethylamino-pyrrolidin-1-yl)-benzamid

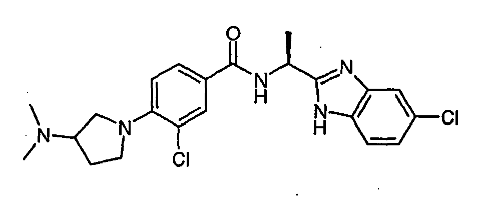

### (a) 3-Chlor-4-(3-dimethylamino-pyrrolidin-1-yl)-benzonitril

0.75 g (4.82 mmol) 3-Chlor-4-fluor-benzonitril werden zusammen mit 0.65 ml (0.58 g, 5.06 mmol) 3-Dimethylamino-pyrrolidin in 12 ml DMF bei Raumtemperatur unter Rühren und Argon-Atmosphäre mit 231 mg (5.30 mmol) 55%-iger Natriumhydrid-Dispersion versetzt. Nach Rühren bei Raumtemperatur für 3.5 h wird das Reaktionsgemisch in Wasser eingegossen und nach starker Durchmischung mit Ethylacetat extrahiert. Die vereinigten organischen Phasen werden mit ges. Natriumchlorid-Lösung gewaschen, über Magnesiumsulfat getrocknet und i. Vak. vollständig eingeengt.
Ausbeute: 1.11 g (92 %)
C₁₃H₁₆ClN₃ (249.74)
Massenspektrum: (M+H)⁺ = 250/252 (Chlorisotope)
R_{f}-Wert: 0.42 (Kieselgel; Petrolether/Ethylacetat = 1:1)

### (b) 3-Chlor-4-(3-dimethylamino-pyrrolidin-1-yl)-benzoesäure

Hergestellt analog Beispiel 13b aus 3-Chlor-4-(3-dimethylamino-pyrrolidin-1-yl)-benzonitril mit 10-molarer Natronlauge und Ethanol.
Ausbeute: 27 %
C₁₃H₁₇ClN₂O₂ (268.74)
Massenspektrum: (M+H)⁺ = 269/271 (Chlorisotope)

### (c) 3-Chlor-N-[(1S)-1-(5-chlor-1H-benzimidazol-2-yl)-ethyl]-4-3-dimethylamino-pyrrolidin-1-yl)-benzamid

Hergestellt analog Beispiel 1f aus dem 3-Chlor-4-(3-dimethylamino-pyrrolidin-1-yl)-benzoesäure und (1*S*)-1-(5-Chlor-1*H-*benzimidazol-2-yl)-ethylamin mit TBTU und TEA in DMF.
Ausbeute: 74 %, leicht verunreinigt
CnH₂₅Cl2N₅O (446.37)
Massenspektrum: (M+H)⁺ = 446/448/450 (Chlorisotope)
R_{f}-Wert: 0.65 (Kieselgel; Dichlormethan/Methanol = 8:2 + 0.5% konz. Ammoniak-Lösung)

### Beispiel 113

*N*-[(1*S*)-1-(5-Chlor-1*H-*benzimidazol-2-yl)-ethyl]-4-(pyrazolidin-3-on-1-yl)-3-trifluormethyl-benzamid

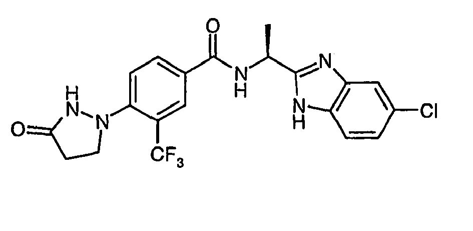

### (a) 4-(Pyrazolidin-3-on-1-yl)-3-trifluormethyl-benzonitril

1.00 g (5.29 mmol) 4-Fluor-3-trifluormethyl-benzonitril werden zusammen mit 1.35 g (12.0 mmol) Kalium-*tert*.-butylat ein 4 ml DMSO bei Raumtemperatur unter Argon-Atmosphäre für 35 min gerührt und dann 1.00 g (8.16 mmol) Pyrazolidin-3-on-hydrochlorid in 3 ml DMSO zugesetzt. Nach Rühren bei Raumtemperatur für 68 h wird das Reaktionsgemisch in halbges. Natriumchlorid-Lösung eingegossen und mit Ethylacetat extrahiert. Die vereinigten organischen Phasen werden über Magnesiumsulfat getrocknet und i. Vak. vollständig eingeengt.
Ausbeute: 0.58 g (43 %)
C₁₁H₈F₃N₃O (255.20)
Massenspektrum: (M+H)⁺ = 256
R_{f}-Wert: 0.15 (Kieselgel; Dichlormethan + 0.5% konz. Ammoniak-Lösung)

### (b) 4-(Pyrazolidin-3-on-1-yl)-3-trifluormethyl-benzoesäure

Hergestellt analog Beispiel 13b aus 3-Chlor-4-(3-dimethylamino-pyrrolidin-1-yl)-benzonitril mit 10-molarer Natronlauge und Ethanol.
Ausbeute: 56 %
C₁₁H₉F₃N₂O₃ (274.20)
R_{f}-Wert: 0.60 (Kieselgel; Dichlormethan/Ethanol = 8:2 + 0.5% Essigsäure)

### (c) N-[(1S)-1-(5-Chlor-1H-benzimidazol-2-yl)-ethyl]-4-(pyrazolidin-3-on-1-yl)-3-trifluormethyl-benzamid

Hergestellt analog Beispiel 1f aus dem 4-(pyrazolidin-3-on-1-yl)-3-trifluormethyl-benzoesäure und (1*S*)-1-(5-Chlor-1*H-*benzimidazol-2-yl)-ethylamin mit TBTU und TEA in DMF.
Ausbeute: 12 %, verunreinigt
C₂₀H₁₇ClF₃N₅O₂ * 2 CF₃COOH (679.88/451.83)
Massenspektrum: (M+H)⁺ = 452/454 (Chlorisotope)
R_{f}-Wert: 0.58 (Kieselgel; Dichlormethan/Methanol = 8:2 + 0.5% konz. Ammoniak-Lösung)

### Beispiel 127

*N-*[(1*S*)-1-(7-Chlor-imidazo[1,2a]pyridin-2-yl)-3-methyl-butyl]-3-methyl-4-(morpholin-3-on-4-yl)-benzamid

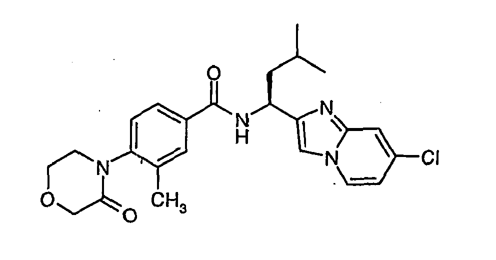

### (a) (1S)-N-Boc-1-(7-Chlor-imidazol[1,2a]pyridin-2-yl)-3-methyl-butylamin

1.68 g (6.37 mmol) [1-(2-Chlor-acetyl)-3-methyl-butyl]-carbaminsäure-*tert*.-butylester werden in 15 ml Methanol bei Raumtemperatur unter Rühren mit 819 mg (6.37 mmol) 2-Amino-4-chlor-pyridin versetzt und die Mischung für 3 Tage zum Rückfluß erhitzt. Nach Einengen i. Vak. wird der Rückstand mit 5%-iger Natriumhydrogencarbonat-Lösung versetzt und für 20 h bei Raumtemperatur gerührt. Danach wird mit Dichlormethan extrahiert, die vereinigten organischen Phasen über Natriumsulfat getrocknet und i. Vak. vollständig eingeengt. Der Rücstand wird durch Chromatographie an Kieselgel gereinigt (Eluens-Gradient:
Dichlormethan/Ethanol = 100:0 -> 94:6).
Ausbeute: 180 mg (8 %)
C₁₇H₂₄ClN₃O₂ (337.85)
Massenspektrum: (M+H)⁺ = 338/340 (Chlorisotope)
R_{f}-Wert: 0.61 (Kieselgel; Dichlormethan/Ethanol = 9:1)

### (b) (1S)-1-(7-Chlor-imidazo[1,2a]pyridin-2-yl)-3-methyl-butylamin

Hergestellt analog Beispiel 1g aus (1*S*)-*N-*Boc-1-7-Chlor-imidazo[1,2a]pyridin-2-yl)-3-methyl-butylamin mit Trifluoressigsäure in Dichlormethan.
Ausbeute: quant.
C₁₂H₁₆ClN₃ * 2 CF₃CO₂H (465.78/237.73)
Massenspektrum: (M+H)⁺ = 238/240 (Chlorisotope)

### (c) N-[(1S)-1-(5-Chlor-imidazo[1,2a]pyridin-2-yl)-3-methyl-butyl]-3-methyl-4-(morpholin-3-on-4-yl)-benzamid

Hergestellt analog Beispiel 1f aus dem 3-Methyl-4-(morpholin-3-on-4-yl)-benzoesäure und (1*S*-1-(5-Chlor-imidazo[1,2a]pyridin-2-yl)-3-methyl-butylamin mit TBTU und DIPEA in THF.
Ausbeute: quant.
C₂₄H₂₇ClN₄O₃ (454.95)
Massenspektrum: (M+H)⁺ = 4551457 (Chlorisotope)
R_{f}-Wert: 0.54 (Kieselgel; Dichlormethan/Ethanol = 9:1)

Die nachfolgenden Beispiele beschreiben die Herstellung pharmazeutischer Anwendungsformen, die als Wirkstoff eine beliebige Verbindung der allgemeinen Formel I enthalten:

### Beispiel I

### Trockenampulle mit 75 mg Wirkstoff pro 10 ml

### Zusammensetzung:

Wirkstoff 75.0 mg
Mannitol 50.0 mg
Wasser für Injektionszwecke ad 10.0 ml

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

### Trockenampulle mit 35 mg Wirkstoff pro 2 ml

### NOT FURNISHED UPON FILING

### Beispiel IV

### Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.

Durchmesser der Tabletten: 12 mm.

### Beisaiel V

### Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mag |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel VI

### Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 0 abgefüllt.

### Beispiel VII

### Suppositorien mit 100 mg Wirkstoff

| | |
|---|---|
| 1 Zäpfchen enthält: | |
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Substituierte Carbonsäureamide der allgergeinen Formel 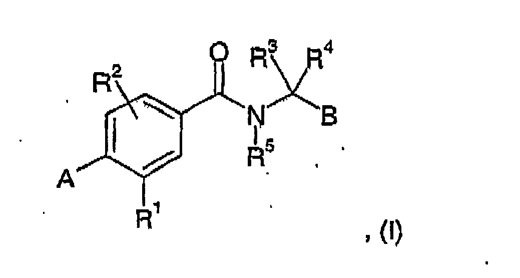 in der
A eine Gruppe der allgerneinen Formel 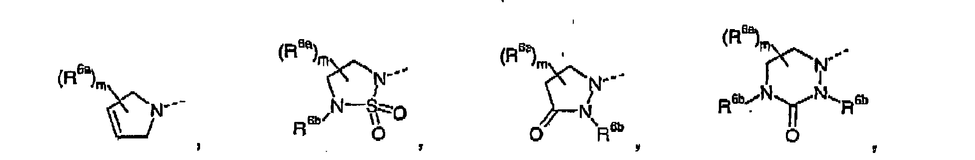 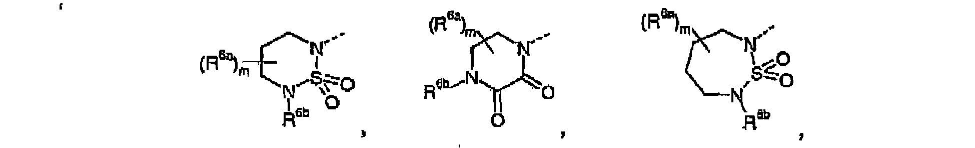 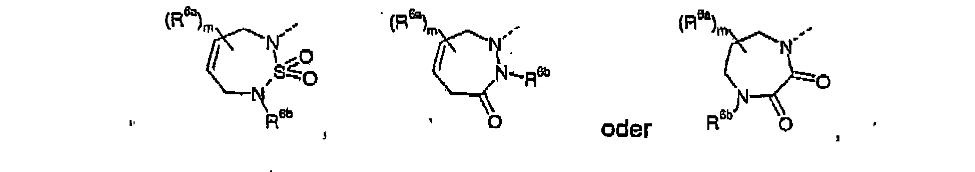 in der
m die Zahl 1 oder 2,
R^{6a} unabhängig voneinander ein Wasserstoff-, Fluor-, Chlor oder Bromatom oder eine C₁₋₃-Alkyl-, Hydroxy-, Amino-, C₁₋₃-Alklamino-, Di-(C₁₋₃-alkyl)-amino-, Aminocarbonyl-, C₁₋₃-alkylamincarbonyl-, Di-(C₁₋₃-alkyl)-aminocarbonyl-oder C₁₋₃-Alkylcarbonylaminogruppe darstellt und
R^{6b} unabhängig voneinander ein Wasserstoffatom, eine C₁₋₄-Alkyl-, C₁₋₄-Alkylcarbonyl-, C₁₋₄-Alkyloxycarbonyl- oder C₁₋₃-Alkylsulfonylgruppe sein können, mit der Maßgabe, dass
bei den voranstehend genannten substituierten 5- bis 7-gliedrigen Gruppen A die gegebenenfalls als Substituenten eingeführten Heteroatome, nicht durch genau ein Kohlenstoffatom von einem anderen Heteroatom getrennt
→ sind, oder
eine Gruppe der allgerneinen Formel 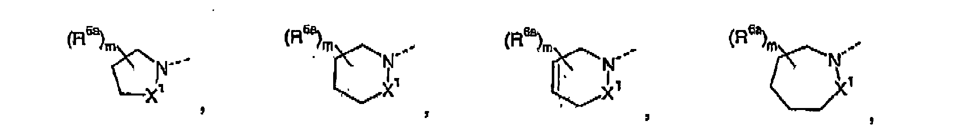 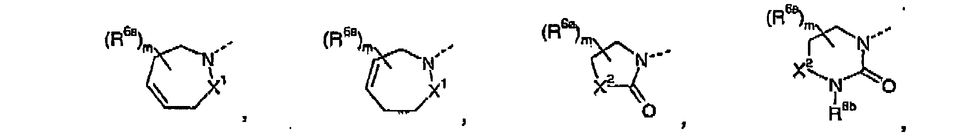 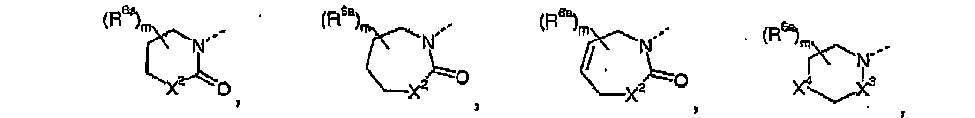 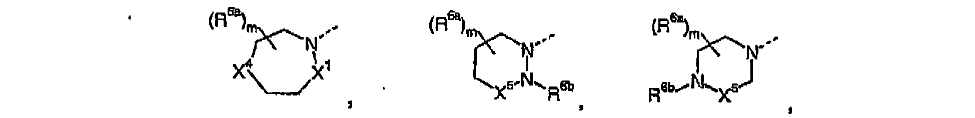 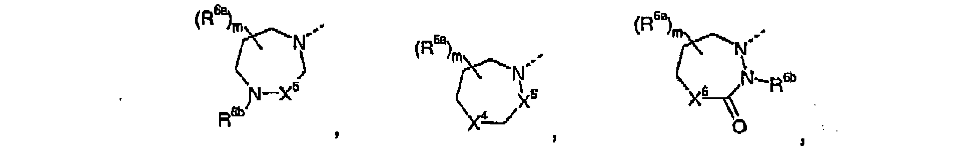 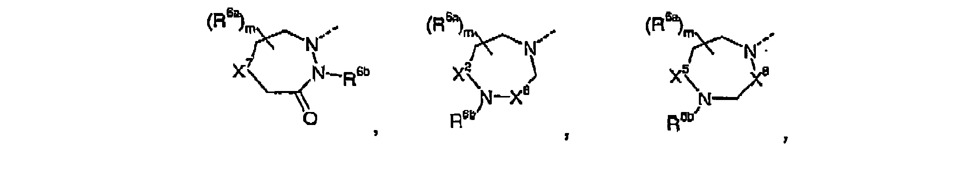 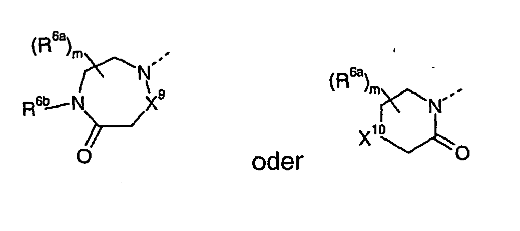 in der
m die Zahl 1 oder 2,
X¹ ein Sauerstoffatom oder eine Methylen-, -NR^{6b}-, Carbonyl- oder Sulfonylgruppe,
X² ein Sauerstoffatom oder eine -NR^{6b}-Gruppe,
X³ eine Methylen-, Carbonyl- oder Sulfonylgruppe,
X⁴ ein Sauerstoff- oder Schwefelatom, eine -NR^{6b}- oder Carbonylgruppe, X⁵ eine Carbonyl- oder Sulfonylgruppe,
X⁶ ein Sauerstoffatom, eine -NR^{6b}- oder Methylengruppe,
X⁷ ein Sauerstoff- oder Schwefelatom oder eine -NR^{6b}-Gruppe,
X⁸ eine Methylen- oder Carbonylgruppe,
X⁹ eine -NR^{6b}- oder Carbonylgruppe,
X¹⁰ eine Sulfinyl- oder Sulfonylgruppe und
R^{6a} unabhängig voneinander ein Wasserstoff-, Fluor-, Chlor- oder Bromatom oder eine C₁-₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl- oder C₁₋₃-Alkylcarbonylaminogruppe darstellt und
R^{6b} unabhängig voneinander ein Wasserstoffatom, eine C₁₋₄-Alkyl-, C₁₋₄-Alkylcarbonyl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen Gruppen A die gegebenenfalls als Substituenten, eingeführten Heteroatome nicht durch genau ein Kohlenstoffatom von einem anderen Heteroatom getrennt sind, bedeuten,
R¹ ein Wasserstoff-, Fluor-, Chlor- oder Bromatom, eine C₁₋₃-Alkylgruppe, in der die Wasserstoffatome ganz oder teilweise durch Fluoratome ersetzt sein können, eine C₂₋₃-Alkenyl-, C₂₋₃-Alkinyl-, Nitro-, Amino-, C₁₋₃-Alkoxy-, eine Mono-, Di- oder Trifluormethoxygruppe,
R² ein Wasserstoff-, Fluor-, Chlor- oder Bromatom oder eine C₁₋₃-Alkylgruppe,
R³ ein Wasserstoffatom, eine C₂₋₃-Alkenyl- oder C₂₋₃-Alkinylgruppe oder eine geradkettige oder verzweigte C₁₋₆-Alkylgruppe, in der die Wasserstoffatome ganz oder teilweise durch Fluoratome ersetzt sein können, und die gegebenenfalls durch eine Nitril-, Hydroxy-, eine C₁₋₅-Alkyloxygruppe, in der die Wasserstoffatome ganz oder teilweise durch Fluoratome ersetzt sein können, eine Allyloxy-, Propargyloxy-, Benzyloxy-, C₁₋₅-Alkylcarbonyloxy-, C₁₋₅-Alkyloxycarbonyloxy-, Carboxy-C₁₋₃-alkyloxy-, C₁₋₅-Alkyloxycarbonyl-C₁₋₃-alkyloxy-, C₁₋₈-Alkyloxycarbonylamino-, Mercapto-, C₁₋₃-Alkylsulfanyl-, C₁₋₃-Alkylsulfinyl-, C₁₋₃-Alkylsulfonyl-, C₁₋₃-Alkylcarbonylamino-C₁₋₃-alkylsulfanyl-, C₁₋₃-Alkylcarbonylamino-C₁₋₃-alkylsulfinyl-, C₁₋₃-Alkylcarbonylamino-C₁₋₃-alkylsulfonyl-, Carboxy-, C₁₋₃-Alkyloxycarbonyl-, Allyloxycarbonyl-, Propargyloxycarbonyl-, Benzyloxycarbonyl-, Aminocarbonyl-, C₁₋₃-Alkylaminocarbonyl-, Di-(C₁₋₃-alkyl)-aminocarbonyl-, C₃₋₆-Cycloalkyleniminocarbonyl-, Aminosulfonyl-, C₁₋₃-Alkylaminosulfonyl-, Di-(C₁₋₃-alkyl)-aminosulfonyl-, C₃₋₆-Cycloalkyleniminosulfonyl-, Amino-, C₁₋₃-Alkylamino-, Di-(C₁₋₃-alkyl)-amino-, C₁₋₅-Alkylcarbonylamino-, C₁₋₃-Alkylsulfonylamino- *N-*(C₁₋₃-Alkylsulfonyl)-. C₁₋₃-alkylamino-, C₃₋₆-Cycloalkylcarbonylamino-, Aminocarbonylamino-, C₁₋₃-Alkylaminocarbonylamino-, Di-(C₁₋₃-alkyl)-aminocarbonylamino-, eine 4- bis 7-gliedrige Cycloalkyleniminocarbonylamino-, Benzyloxycarbonylamino-, Phenylcarbonylamino-oder Guanidinogruppe substituiert ist,
eine Carboxy-, Aminocarbonyl-, C₁₋₄-Alkylaminocarbonyl-, C₃₋₆-Cycloalkylamino-carbonyl-, Di-(C₁₋₃-alkyl)-aminocarbonyl-, C₁₋₄-Alkoxycarbonyl-, C₄₋₆-Cycloalkyleniminocarbonylgruppe,
eine Phenyl- oder Heteroaryl-, Phenylcarbonyl-C₁₋₃-alkyl-, Phenyl-C₁₋₃-alkyl-oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, Amino-, C₁₋₃-Alkylamino-, Di-(C₁₋₃-alkyl)-amino-, Hydroxy-, C₁₋₄-Alkyloxy-, Mono-, Di- oder Trifluormethoxy-, Benzyloxy-, Carboxy-C₁₋₃-alkyloxy-, C₁₋₃-Alkyloxycarbonyl-C₁₋₃-alkyloxy-, Aminocarbonyl-C₁₋₃-alkyloxy-, C₁₋₃-Alkylaminocarbonyl-C₁₋₃-alkyloxy-, Di-(C₁₋₃-alkyl)-aminocarbonyl-C₁₋₃-alkyloxy-, eine 4- bis 7-gliedrige Cycloalkyleniminocarbonyl-C₁₋₃-alkoxy-, Carboxy-, C₁₋₃-Alkyloxycarbonyl- oder C₁₋₃-Alkyloxy-carbonylaminogruppe substituiert ist,
eine 3- bis 7-gliedrige Cycloalkyl-, Cycloalkylenimino-, Cycloalkyl-C₁₋₃-alkyl-oder Cycloalkylenimino-C₁₋₃-alkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann und in der zusätzlich eine der einer -NH-, -N(C₁₋₃-Alkylcarbonyl)- oder -N(C₁₋₃-Alkyl)-Gruppe benachbarte Methylengruppe jeweils durch eine Carbonyl- oder Sulfonylgruppe ersetzt sein kann, mit der Maßgabe, dass eine wie oben definierte Cycloalkyleniminogruppe, in der zwei Stickstoffatome durch genau eine -CH₂-Gruppe voneinander getrennt sind, ausgeschlossen ist,
R⁴ ein Wasserstoffatom oder eine C₁₋₃-Alkylgruppe oder
R³ und R⁴ zusammen mit dem Kohlenstoffatom, an dem sie gebunden sind, eine C₃₋₇-Cycloalkylgruppe, wobei
eine der Methylengruppen der C₃₋₇-Cycloalkylgruppe durch eine Imino-, C₁₋₃-Alkylimino-, Acylamino- oder Sulfonyliminogruppe ersetzt sein kann,
R⁵ ein Wasserstoffatome oder eine C₁₋₃-Alkylgruppe,
B eine Gruppe der Formel 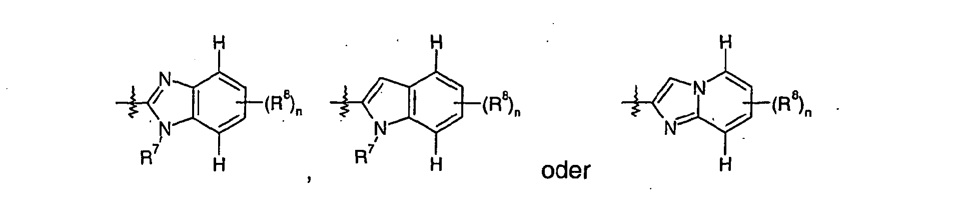 in der
n die Zahl 1 oder 2,
R⁷ ein Wasserstoffatom oder eine C₁₋₃-Alkyl-, Hydroxy-, C₁₋₅-Alkyloxycarbonyl-, Carboxy-C₁₋₃-alkyl-, C₁₋₃-Alkyloxycarbonyl-C₁₋₃-alkyl-, Amino- oder C₁₋₃-Alkylaminogruppe und
R⁸ unabhängig voneinander ein Wasserstoff-, Fluor-, Chlor-, Brain- oder lodatom, eine C₁₋₃-Alkylgruppe, in der die Wasserstoffatome ganz oder teilweise durch Fluoratome ersetzt sein können, eine C₂₋₃-Alkenyl- oder C₂₋₃-Alkinyl-, eine Hydroxy-, C₁₋₃-Alkoxy-, Trifluormethoxy-, Amino-, Nitro-oder Nitrilgruppe darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe ein Sauerstoff- oder Schwefelatom oder
eine- gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine 4- bis 7-gliedrige Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnte Definitionen enthaltenden Methyl- oder Ethylgruppen ganz oder teilweise durch Fluoratome ersetzt sein können,
deren Tautomere, deren Enantiomere, deren Diastereomere, deren Gemische und deren Salze.

2. Substituierte Carbonsäureamide der allgemeinen Formel I gemäß Anspruch 1, in der
A eine Gruppe der allegerneinen Formel 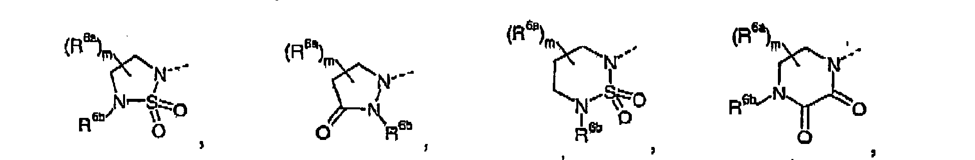 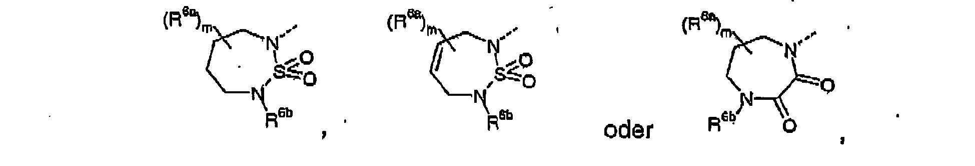 → in der m die Zahl 1 oder 2,
R^{6a} unabhängig voneinander ein Wasserstoff- oder Fluoratom, eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl- oder
C₁₋₃-alkylaminocarbonylaminogruppe darstellt und
R^{6b} unabhängig voneinander ein Wasserstoffatom, eine C₁₋₄-Alkyl-, C₁₋₄-Alkylcarbonyl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen Gruppen A die gegebenenfalls als Substituenten eingeführten Heteroatom nicht
durch genau ein Kohlenstoffatom von einem anderen Heteroatom getrennt
→ sind, oder eine Gruppe der allgemeinen Formel 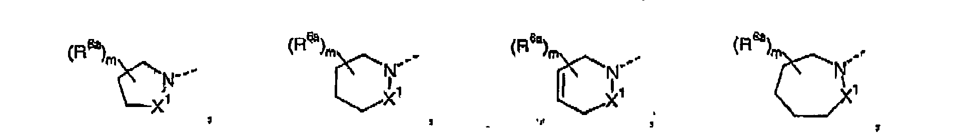 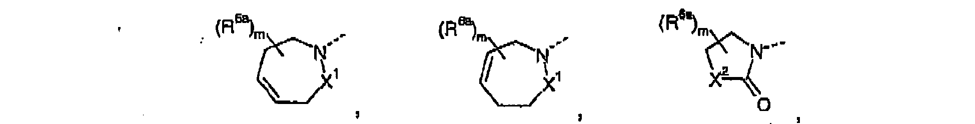 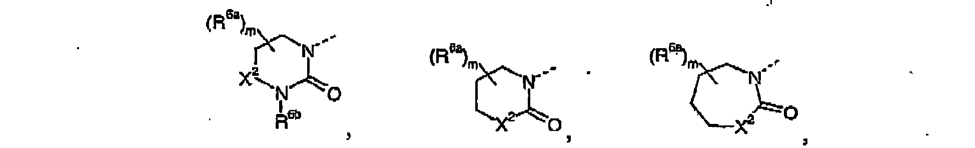 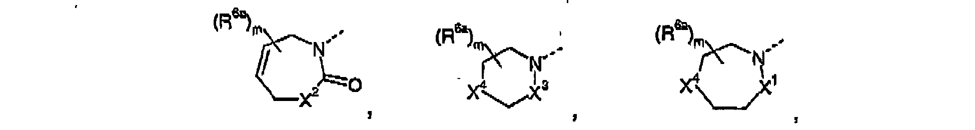 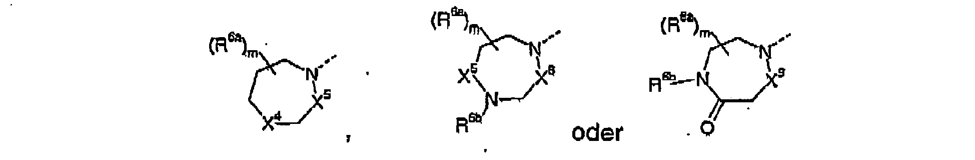 in der
m die Zahl 1 oder 2,
X¹ eine Methylen-, -NR^{6b}-, Carbonyl- oder Sulfonylgruppe, X² ein Sauerstoffatom oder eine -NR^{8b}-Gruppe,
X³ eine Methylen-, Carbonyl- oder Sulfonylgruppe,
X⁴ ein Sauerstoff- oder Schwefelatom, eine -NR^{6b}- oder Carbonylgruppe,
X⁵ eine Carbonyl- oder Sulfonylgruppe,
X⁸ eine Carbonylgruppe,
X⁹ eine Carbonylgruppe,
R^{6a} unabhängig voneinander ein Wasserstoff- oder Fluoratom, eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl-oder C₁₋₃-Alkylcarbonylaminogruppe darstellt und
R^{6b} unabhängig voneinander ein Wasserstoffatom, eine C₁₋₄-Alkyl-, C₁₋₄-Alkylcarbonyl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen cyclischen Gruppen A die als Substituenten eingeführten Heteroatome nicht durch genau ein Kohlenstoffatom von einem anderen Heteroatom getrennt sind, bedeuten,
R¹ ein Wasserstoff-, Fluor-, Chlor- oder Bromatom, eine C₁₋₃-Alkylgruppe, in der die Wasserstoffatome ganz oder teilweise durch Fluoratome ersetzt sein können, eine C₂₋₃-Alkenyl-, C₂₋₃-Alkinyl-, Nitro-, Amino-, C₁₋₃-Alkoxy-, eine Mono-, Di- oder Trifluormethoxygruppe,
R² ein Wasserstoff-, Fluor-, Chlor- oder Bromatom oder eine C₁₋₃-Alkylgruppe,
R³ eine C₂₋₃-Alkenyl- oder C₂₋₃-Alkinylgruppe oder eine geradkettige oder verzweigte C₁₋₆-Alkylgruppe, in der die Wasserstoffatome ganz oder teilweise durch Fluoratome ersetzt sein können, und die gegebenenfalls durch eine Nitril-, Hydroxy-, eine C₁₋₅-Alkyloxygruppe, in der die Wasserstoffatome ganz oder teilweise durch Fluoratome ersetzt sein können, eine Alkyloxy-, Propargyloxy-, Benzyloxy-, C₁₋₅-Alkylcarbonyloxy-, C₁₋₅-Alkyloxycarbonyloxy-, Carboxy-C₁₋₃-alkyloxy-, C₁₋₃-Alkyloxycarbonyl-C₁₋₃-alkyloxy-, C₁₋₈-Alkyloxycarbonylamino-, Mercapto-, C₁₋₃-Alkylsulfanyl-, C₁₋₃-Alkylsulfinyl-, C₁₋₃-Alkylsulfonyl-, C₁₋₃-Alkylcarbonylamino-C₁₋₃-alkylsulfanyl-, C₁₋₃-Alkylcarbonylamino-C₁₋₃-alkylsulfinyl-, C₁₋₃-Alkylcarbonylamino-C₁₋₃-alkylsulfonyl-, Carboxy-, C₁₋₃-Alkyloxycarbonyl-, Allyloxycarbonyl-, Propargyloxycarbonyl-, Benzyloxycarbonyl-, Aminocarbonyl-, C₁₋₃-Alkylaminocarbonyl-, Di-(C₁₋₃-alkyl)-aminocarbonyl-, C₃₋₆-Cycloalkyleniminocarbonyl-, Aminosulfonyl-, C₁₋₃-Alkylaminosulfonyl-, Di-(C₁₋₃-alkyl)-aminosulfonyl-, C₃₋₆-Cycloalkyleniminosulfonyl-, Amino-, C₁₋₃-Alkylamino-, Di-(C₁₋₃-alkyl)-amino-, C₁₋₅-Alkylcarbonylamino-, C₁₋₃-Alkylsulfonylamino-, *N-*(C₁₋₃-Alkylsulfonyl)-C₁₋₃-alkylamino-, C₃₋₆-Cycloalkylcarbonylamino-, Aminocarbonylamino-, C₁₋₃-Alkylaminocarbonylamino-, Di-(C₁₋₃-alkyl)-aminocarbonylamino-, eine 4- bis 7-gliedrige Cycloalkyleniminocarbonylamino-, Benzyloxycarbonylamino-, Phenylcarbonylamino- oder Guanidinogruppe substituiert ist,
eine Carboxy-, Aminocarbonyl-, C₁₋₄-Alkylaminocarbonyl-, C₃₋₆-Cycloalkylamino-carbonyl-, Di-(C₁₋₃-alkyl)-aminocarbonyl-, C₁₋₄-Alkoxycarbonyl-, C₄₋₆-Cycloalkyleniminocarbonylgruppe,
eine Phenyl- oder Heteroaryl-, Phenylcarbonyl-C₁₋₃-alkyl-, Phenyl-C₁₋₃-alkyl-oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, Amino-, C₁₋₃-Alkylamino-, Di-(C₁₋₃-alkyl)-amino-, Hydroxy-, C₁₋₄-Alkyloxy-, Mono-, Di- oder Trifluormethoxy-, Benzyloxy-, Carboxy-C₁₋₃-alkyloxy-, C₁₋₃-Alkyloxycarbonyl-C₁₋₃-alkyloxy-, Aminocarbonyl-C₁₋₃-alkyloxy-, C₁₋₃-Alkylaminocarbonyl-C₁₋₃-alkyloxy-, Di-(C₁₋₃-alkyl)-aminocarbonyl-C₁₋₃-alkyloxy-, eine 4- bis 7-gliedrige Cycloalkyleniminocarbonyl-C₁₋₃-alkoxy-, Carboxy-, C₁₋₃-Alkyloxycarbonyl- oder C₁₋₃-Alkyloxy-carbonylaminogruppe substituiert ist,
eine 3- bis 7-gligdri.ge Cycloalkyl-, Cycloalkylenimino-, Cycloalkyl-C₁₋₃-alkyl-oder Cycloalkylenimino-C₁₋₃-alkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann und in der zusätzlich eine der einer -NH-, -N(C₁₋₃-Alkylcarbonyl)- oder -N(C₁₋₃-Alkyl)-Gruppe benachbarte Methylengruppe jeweils durch eine Carbonyl- oder Sulfonylgruppe ersetzt sein kann, mit der Maßgabe, dass eine wie oben definierte Cycloalkyleniminogruppe, in der zwei Stickstoffatome durch genau eine -CH₂-Gruppe voneinander getrennt sind,
ausgeschlossen ist,
R⁴ ein Wasserstoffatom oder eine C₁₋₃-Alkylgruppe,
R⁵ ein Wasserstoffatom oder eine C₁₋₃-Alkylgruppe,
B eine Gruppe der Formel 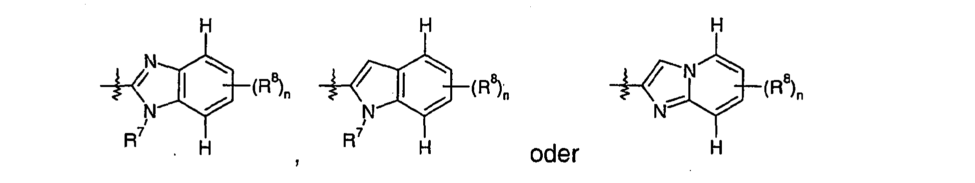 in der
n die Zahl 1 oder 2,
R⁷ ein Wasserstoffatom, eine C₁₋₃-Alkyl-, Hydroxy-, C₁₋₅-Alkyloxycarbonyl-, Carboxy-C₁₋₃-alkyl-, C₁₋₃-Alkyloxycarbonyl-C₁₋₃-alkyl-, Amino- oder C₁₋₃-Alkylaminogruppe und
R⁸ unabhängig voneinander ein Wasserstoff-, Fluor-, Chlor-, Brom- oder lodatom, eine C₁₋₃-Alkylgruppe, in der die Wasserstoffatome ganz oder teilweise durch Fluoratome ersetzt sein können, eine C₂₋₃-Alkenyl- oder C₂₋₃-Alkinyl-, eine Hydroxy-, C₁₋₃-Alkoxy-, Trifluormethoxy-, Amino Nitro-,
oder Nitrilgruppe darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Phenyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Phenyl-, Amino-C₂₋₃-alkyl-, C₁₋₃-Alkylamino-C₂₋₃-alkyl-, Di-(C₁₋₃-alkyl)-amino-C₂₋₃-alkyl-, eine 4- bis 7-gliedrige Cycloalkylenimino-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte iminogruppe und zwei oder drei Stickstoffatome enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann
und die Bindung über ein Stickstoffatom oder über ein Kohlenstoffatom des heterocyclischen Teils oder eines ankondensierten Phenylrings erfolgt,
wobei die in den voranstehend erwähnten Definitionen enthaltenen Alkyl- und Alkoxygruppen, die mehr als zwei Kohlenstoffatome aufweisen, soweit nichts anderes erwähnte wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

3. Substituierte Carbonsäureamide der allgemeinen Formel I gemäß Anspruch 1, in der
A eine Gruppe der allgemeinen Formel 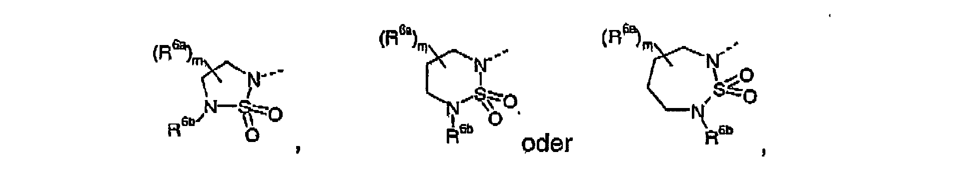 → in der m die Zahl 1 oder 2,
R^{6a} unabhängig voneinander ein Wasserstoff- oder Fluoratom, eine C₁₋₃₋Alk_{Y}l-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl- oder C₁₋₃-Alkylcarbonylaminogruppe darstellt und
R^{6b} unabhängig voneinander ein Wasserstoffatom, eine C₁₋₄-Alkyl-, C₁₋₄-AlkylCarbonyl-, C₁₋₄-Alkoxycarbonyl- oder C₁₋₃-Alkylsulfonylgruppe sein können, mit der Maßgabe, dass
bei den voranstehend genannten substituierten 5- bis 7-gliedrigen Gruppen
A die gegebenenfalls als Substituenten eingeführten Heteroatome nicht durch genau ein Kohlenstoffatom von einem anderen Heteroatom getrennt
→ sind, oder
eine Gruppe der allgemeinen Formel 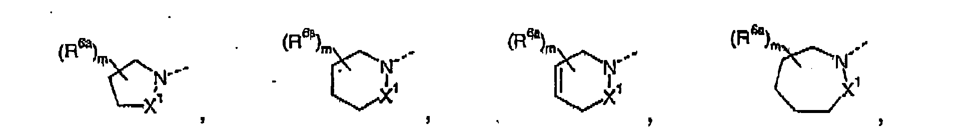 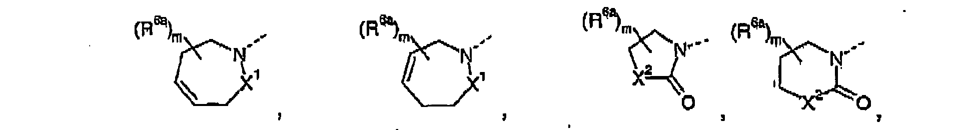 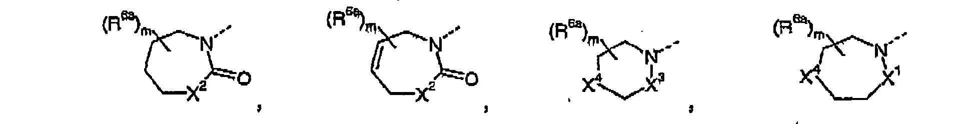 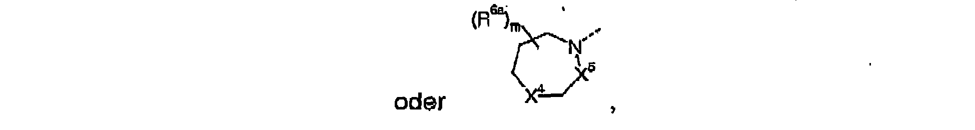 in der
m die Zahl 1 oder 2,
X¹ eine Methylen-, -NR^{6b}-, Carbonyl- oder Sulfonylgruppe, X² ein Sauerstoffatom oder eine -NR^{6b}-Gruppe,
X³ eine Methylen-, Carbonyl- oder Sulfonylgruppe,
X⁴ ein Sauerstoff- oder Schwefelatom oder eine -NR^{6b}-Gruppe, X⁵ eine Carbonyl- oder Sulfonylgruppe,
R^{6a} unabhängig voneinander ein ^{.}Wasserstoff- oder Fluoratom, eine C₁₋₃-Alkyl-, Hydroxy-, Amino-, C₁₋₃-Alkylamino-, Di-(C₁₋₃-alkyl)-amino-, Aminocarbonyl-, C₁₋₃-Alkylaminocarbonyl-, Di-(C₁₋₃-alkyl)-aminocarbonyl-oder C₁₋₃-Alkylcarbonylaminogruppe darstellt und
R^{6b} unabhängig voneinander ein Wasserstoffatom, eine C₁₋₄-Alkyl-, C₁₋₄-Alkylcarbonyl-, G₁₋₄-Alkoxycarbonyl- oder C₁₋₃-Alkylsulfonylgruppe sein können, mit der Maßgabe, dass
bei den voranstehend genannten substituierten 5- bis 7-gliedrigen cyclischen Gruppen A die als Substituenten eingeführten Heteroatome nicht durch genau ein Kohlenstoffatom von einem anderen Heteroatom getrennt sind, bedeuten,
R¹ ein Fluor-, Chlor- oder Bromatom, eine C₁₋₃-Alkylgruppe, in der die Wasserstoffatome ganz oder teilweise durch Fluoratome ersetzt sein können, eine Nitro-, C₁₋₃-Alkoxy-, eine Mono-, Di- oder Trifluormethoxygruppe,
R² ein Wasserstoffatom,
R³ eine geradkettige oder verzweigte C₁₋₆-Alkylgruppe, in der die Wasserstoffatome ganz oder teilweise durch Fluoratome ersetzt sein können, und die gegebenenfalls durch eine Nitril-, Hydroxy-, Benzyloxy-, eine C₁₋₅-Alkyloxygruppe, in der die Wasserstoffatome ganz oder teilweise durch Fluoratome ersetzt sein können, eine Allyloxy-, C₁₋₅-Alkylcarbonyloxy-, C₁₋₅-Alkyloxycarbonyloxy-, Carboxy-C₁₋₃-alkyloxy-, C₁₋₅-Alkyloxycarbonyl-C₁₋₃-alkyloxy-, C₁₋₈-Alkyloxycarbonylamino-, C₁₋₃-Alkylsulfanyl-, C₁₋₃-Alkylsulfonyl-, Carboxy-, C₁₋₃-Alkyloxycarbonyl-, C₁₋₃-Alkylaminocarbonyl-, Di-(C₁₋₃-alkyl)-aminocarbonyl-, C₃₋₆-Cycloalkyleniminocarbonyl-, Aminocarbonylamino-, C₁₋₃-Alkylaminocarbonylamino- oder Di-(C₁₋₃-alkyl)-aminocarbonylaminogruppe substituiert ist,
eine Aminocarbonyl-, C₁₋₄-Alkylaminocarbonyl-, C₃₋₆-Cycloalkylaminocarbonyl-oder Di-(C₁₋₃-alkyl)-aminocarbonylgruppe,
eine Phenyl- oder Heteroaryl-, Phenyl-C₁₋₃-alkyl- oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, Amino-, C₁₋₃-Alkylamino-, Di-(C₁₋₃-alkyl)-amino-, Hydroxy-, C₁₋₄-Alkyloxy-, Mono-, Di-oder Trifluormethoxy-, Carboxy-, oder C₁₋₃-Alkyloxycarbonylgruppe substituiert ist,
eine 3- bis 7-gliedrige Cycloalkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann,
R⁴ ein Wasserstoffatom,
R⁵ ein Wasserstoffatom,
B eine Gruppe der Formel 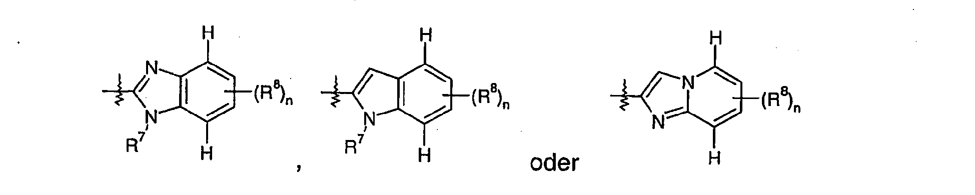 in der
n die Zahl 1,
R⁷ ein Wasserstoffatom und
R⁸ ein Wasserstoff-, Fluor-, Chlor-, Brom- oder Iodatom, eine Methyl-,
C₂₋₃-Alkinyl-, oder Methoxygruppe, in denen die Wasserstoffatome ganz
oder teilweise durch Fluoratome ersetzt sein können, darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe substituierte Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

4. Substituierte Carbonsäureamide der allgemeinen Formel I gemäß Anspruch 3, in der
A, R¹, R², R⁴, R⁵ und B wie in Anspruch 3 definiert sind und
R³ eine geradkettige oder verzweigte C₁₋₆-Alkylgruppe, in der die Wasserstoffatome ganz oder teilweise durch Fluoratome ersetzt sein können, und die gegebenenfalls durch eine Nitril-, Hydroxy-, Benzyloxy-, eine C₁₋₅-Alkyloxygruppe, in der die Wasserstoffatome ganz oder teilweise durch Fluoratome ersetzt sein können, eine Allyloxy-, C₁₋₅-Alkylcarbonyloxy-, C₁₋₅-Alkyloxycarbonyloxy-, Carboxy-C₁₋₃-alkyloxy-, C₁₋₅-Alkyloxycarbonyl-C₁₋₃-alkyloxy-, C₁₋₈-Alkyloxycarbonylamino-, C₁₋₃-Alkylsulfanyl-, C₁₋₃-Alkylsulfonyl-, Carboxy-, C₁₋₃-Alkyloxycarbonyl-, C₁₋₃-Alkylaminocarbonyl-, Di-(C₁₋₃-alkyl)-aminocarbonyl-, C₃₋₆-Cycloalkyleniminocarbonyl-, Aminocarbonylamino-, C₁₋₃-Alkylaminocarbonylamino- oder Di-(C₁₋₃-alkyl)-aminocarbonylaminogruppe substituiert ist,
eine Aminocarbonyl-, C₁₋₄-Alkylaminocarbonyl-, C₃₋₆-Cycloalkylaminocarbonyl-oder Di-(C₁₋₃-alkyl)-aminocarbönylgruppe darstellt,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

5. Substituierte Carbonsäureamide der allgemeinen Formel I gemäß Anspruch 3, in der
A, R¹, R², R⁴, R⁵ wie in Anspruch 3 definiert sind und
R³ eine Phenyl- oder Heteroaryl-, Phenyl-C₁₋₃-alkyl- oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, Amino-, C₁₋₃-Alkylamino-, Di-(C₁₋₃-alkyl)-amino-, Hydroxy-, C₁₋₄-Alkyloxy-, Mono-, Di-oder Trifluormethoxy-, Carboxy-, oder C₁₋₃-Alkyloxycarbonylgruppe substituiert ist,
eine 3- bis 7-gliedrige Cycloalkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann, darstellt,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom, erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können, und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

6. Substituierte Carbonsäureamide der allgemeinen Formel I gemäß Anspruch 1, in der
A eine Gruppe der allgemeinen Formel 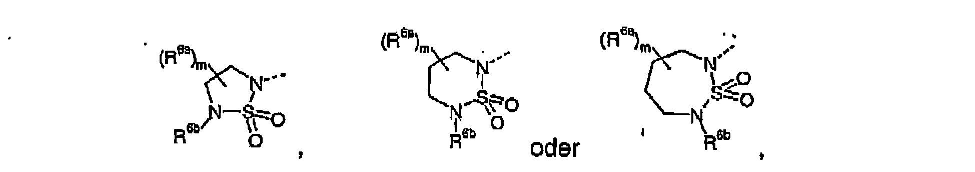 in der m die Zahl 1 oder 2,
R^{6a} unabhängig voneinander ein Wasserstoff- oder Fluoratom oder eine C₁₋₃-Alkylgruppe darstellt und
R^{6b} ein Wasserstoffatom oder eine C₁₋₃-Alkylgruppe sein kann, mit der
Maßgabe, dass
bei den voranstehend genannten substituierten 5- bis 7-gliedfigen Gruppen A die gegebenenfalls als Substituenten eingeführen Heteroatome nicht durch genau ein Kohlenstoffatom von einem anderen Heteroatom getrennt sind, oder
eine Gruppe der allgemeinen Formel 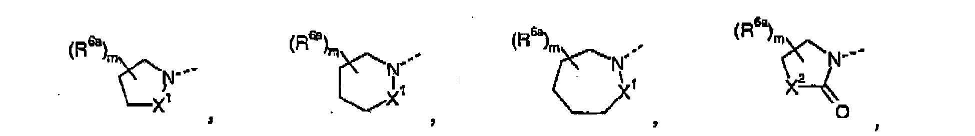 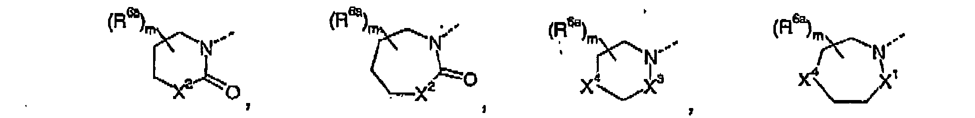 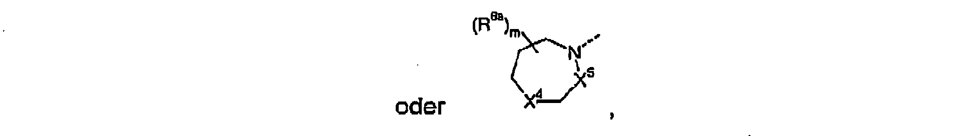 in der
m die Zahl 1 oder 2,
X¹ eine Methylen-, -NR^{6b}-, Carbonyl- oder Sulfonylgruppe, X² ein Sauerstoffatom oder eine -NR^{6b}-Gruppe,
X³ eine Methylen-, Carbonyl, oder Sulfonylgruppe,
X⁴ ein Sauerstoff oder Schwefelatom oder eine -NR^{6b}-Gruppe,
X⁵ eine Carbonyl- oder Sulfonylgruppe,
R^{6a} unabhängig voneinander ein Wasserstoff- oder Fluoratom oder eine C₁₋₃-Alkylgruppe darstellt und
R^{6b} unabhängig voneinander ein Wasserstoffatom oder eine C₁₋₃-Alkylgruppe sein können, mit der Maßgabe, dass
bei den voranstehend genannten substituierten 5- bis 7-gliedrigen cyclischen Gruppen A die als Substituenten eingeführten Heteroatome nicht durch genau ein Kohlenstoffatom von einem anderen Heteroatom getrennt sind, bedeuten,
R¹ ein Chlor- oder Bromatom, eine Methyl- oder Methoxygruppe, in denen die Wasserstoffatome ganz oder teilweise durch Fluoratome ersetzt sein können, oder eine Nitrogruppe,
R² ein Wasserstoffatom,
R³ eine geradkettige oder verzweigte C₁₋₄-Alkylgruppe, in der die Wasserstoffatome ganz oder teilweise durch Fluoratome ersetzt sein können, und die gegebenenfalls durch eine Hydroxy-, eine C₁₋₄-Alkyloxygruppe, in der die Wasserstoffatome ganz oder teilweise durch Fluoratome ersetzt sein können, eine C₁₋₃-Alkylsulfanyl-, C₁₋₃-Alkylsulfonyl-, Carboxy- oder C₁₋₃-Alkyloxycarbonylgruppe substituiert ist,
eine Phenyl- oder Heteroaryl-, Phenyl-C₁₋₃-alkyl- oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, C₁₋₄-Alkyloxy-, Mono-, Di- oder Trifluormethoxy-, Carboxy-, oder C₁₋₃-Alkyloxycarbonylgruppe substituiert ist,
R⁴ ein Wasserstoffatom,
R⁵ ein Wasserstoffatom und B eine Gruppe der Formel 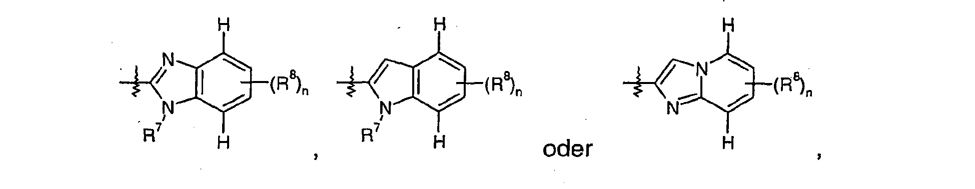 in der
n die Zahl 1,
R⁷ ein Wasserstoffatom und
R⁸ ein Chlor- oder Bromatom oder die Ethinylgruppe darstellen,
wobei, soweit nichts anderes erwähnt wurde, unter dem voranstehend in den Definitionen erwähnten Ausdruck "Heteroaryigruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe subsituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können, und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

7. Substituierte Carbonsäureamide der allgemeinen Formel I gemäß Anspruch 6, in der
A, R¹, R², R⁴, R⁵ und B wie in Anspruch 6 definiert sind und
R³ eine geradkettige oder verzweigte C₁₋₄-Alkylgruppe, in der die Wasserstoffatome ganz oder teilweise durch Fluoratome ersetzt sein können, und die gegebenenfalls durch eine Hydroxy-, eine C₁₋₄-Alkyloxygruppe, in der die Wasserstoffatome ganz oder teilweise durch Fluoratome ersetzt sein können, eine C₁₋₃-Alkylsulfanyl-, C₁₋₃-Alkylsulfonyl-, Carboxy- oder C₁₋₃-Alkyloxycarbonylgruppe substituiert ist,
wobei die in den voranstehend erwähnten Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

8. Substituierte Carbonsäureamide der allgemeinen Formel I gemäß Anspruch 6, in der
A, R¹, R², R⁴, R⁵ und B wie in Anspruch 6 definiert sind und
R³ eine Phenyl- oder Heteroaryl-, Phenyl-C₁₋₃-alkyl- oder Heteroaryl-C₁₋₃-alkylgruppe, die im Phenyl- oder Heteroarylteil gegebenenfalls ein- oder mehrfach durch Fluor-, Chlor- oder Bromatome, C₁₋₃-Alkyl-, C₁₋₄-Alkyloxy-, Mono-, Di- oder Trifluormethoxy-, Carboxy-, oder C₁₋₃-Alkyloxycarbonylgruppe substituiert ist,
eine 3- bis 7-gliedrige Cycloalkylgruppe, in der im cyclischen Teil eine Methylengruppe durch eine gegebenenfalls durch eine C₁₋₃-Alkyl- oder C₁₋₃-Alkylcarbonylgruppe substituierte -NH-Gruppe oder ein Sauerstoffatom ersetzt sein kann,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können, und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

9. Substituierte Carbonsäureamide der allgemeinen Formel I gemäß Anspruch 1, in der
A eine Gruppe der Formel 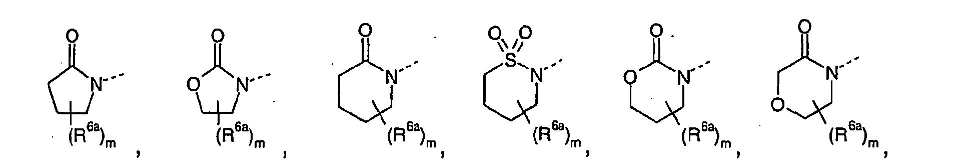 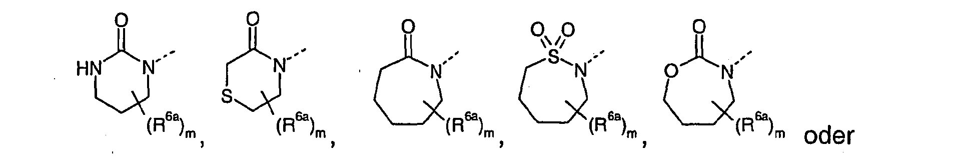 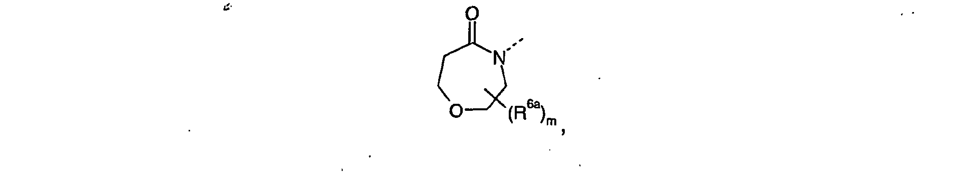 in der
m die Zahl 1 oder 2,
R^{6a} unabhängig voneinander ein Wasserstoff- oder Fluoratom oder eine C₁₋₃-Alkylgruppe darstellt, mit der Maßgabe, dass
bei den voranstehend genannten substituierten 5- bis 7-gliedrigen cyclischen Gruppen A die als Substituenten eingeführten Fluoratome nicht durch genau ein Kohlenstoffatom von einem anderen Heteroatom getrennt sind, bedeuten,
R¹ ein Chlor- oder Bromatom, eine Methyl- oder Methoxygruppe, in denen die Wasserstoffatome ganz oder teilweise durch Fluoratome ersetzt sein können, oder eine Nitrogruppe,
R² ein Wasserstoffatom,
R³ eine geradkettige oder verzweigte C₁₋₄-Alkylgruppe, in der die Wasserstoffatome ganz oder teilweise durch Fluoratome ersetzt sein können, und die gegebenenfalls durch eine Hydroxy-, eine C₁₋₄-Alkyloxygruppe, in der die Wasserstoffatome ganz oder teilweise durch Fluoratome ersetzt sein können, eine C₁₋₃-Alkylsulfanyl-, C₁₋₃-Alkylsulfonyl-, Carboxy- oder
C₁₋₃-Alkyloxycarbonylgruppe substituiert ist,
eine Furanyl-, Thiophenyl-, Pyrrolyl-, Pyrazolyl-, Imidazolyl-, Oxazolyl-, Isoxazolyl-, Thiazolyl-, Pyridinyl-, Pyrimidinyl-, Pyrazinyl-, Pyridinyl-C₁₋₂-alkyl-oder Imidazolyl-C₁₋₂-alkylgruppe, die gegebenenfalls im Heteroaryl-Teil mit einer oder zwei C₁₋₃-Alkylgruppen, C₁₋₃-Alkyloxygruppen, Carboxy- oder C₁₋₃-Alkyloxycarbonylgruppen substituiert sein können, bedeutet und
R⁴ ein Wasserstoffatom,
R⁵ ein Wasserstoffatom und
B eine Gruppe der Formel 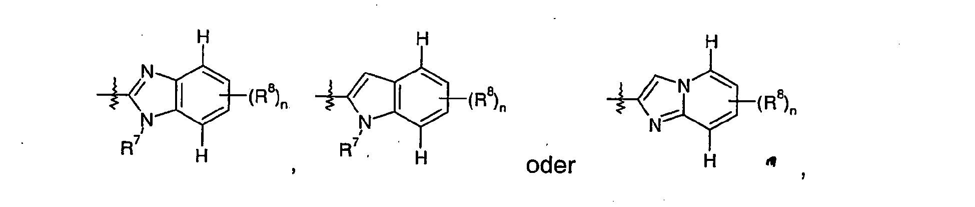 in der
n die Zahl 1,
R⁷ ein Wasserstoffatom und
R⁸ ein Chlor- oder Bromatom oder eine Ethinylgruppe darstellen,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können, und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

10. Substituierte Carbonsäureamide der allgemeinen Formel I gemäß Anspruch 9, in der
A, R¹, R², R⁴, R⁵ und B wie in Anspruch 9 definiert sind und
R³ eine geradkettige oder verzweigte C₁₋₄-Alkylgruppe, in der die Wasserstoffatome ganz oder teilweise durch Fluoratome ersetzt sein können, und die gegebenenfalls durch eine Hydroxy-, eine C₁₋₄-Alkyloxygruppe, in der die Wasserstoffatome ganz oder teilweise durch Fluoratome ersetzt sein können, eines C₁₋₃-Alkylsulfanyl-, C₁₋₃-Alkylsulfonyl-, Carboxy- oder C₁₋₃-Alkyloxycarbonylgruppe substituiert ist,
wobei die in den voranstehend erwähnter Definitionen enthaltenen Alkyl- und Alkoxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

11. Substituierte Carbonsäureamide der allgemeinen Formel I gemäß Anspruch 9, in der
A, R¹, R², R⁴, R⁵ und B wie in Anspruch 9 definiert sind und
eine Furanyl-, Thiophenyl-, Pyrrolyl-, Pyrazolyl-, Imidazolyl-, Oxazolyl-, Isoxazolyl-, Thiazolyl-, Pyridinyl-, Pyrimidinyl-, Pyrazinyl-, Pyridinyl-C₁₋₂-alkyl-oder Imidazolyl-C₁₋₂-alkylgruppe, die gegebenenfalls im Heteroaryl-Teil mit einer oder zwei C₁₋₃-Alkylgruppen, bei denen die Wasserstoffatome ganz oder teilweise durch Fluoratome ersetzt sein können, C₁₋₃-Alkyloxygruppen, bei denen die Wasserstoffatome ganz oder teilweise durch Fluoratome ersetzt sein können, Carboxy- oder C₁₋₃-Alkyloxycarbonylgruppen substituiert sein können, bedeutet und
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome und
die 5-gliedrige Heteroarylgruppe eine gegebenenfalls durch eine C₁₋₃-Alkylgruppe Iminogruppe, ein Sauerstoff- oder Schwefelatom oder
eine gegebenenfalls durch eine C₁₋₃-Alkylgruppe substituierte Iminogruppe oder ein Sauerstoff- oder Schwefelatom und zusätzlich ein Stickstoffatom oder
eine gegebenenfalls durch eine C₁₋₃-Alkylgruppe substituierte Iminogruppe und zwei oder drei Stickstoffatome enthält,
und die Bindung über ein Stickstoffatom oder über ein Kohlenstoffatom erfolgt,
wobei die in den voranstehend erwähnten Definitionen enthaltenen Alkylgruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen ganz oder teilweise durch Fluoratome ersetzt sein können,
deren Tautomere, deren Enantiomere, deren Diastereomere, deren Gemische und deren Salze.

12. Substituierte Carbonsäureamide der allgemeinen Formel I gemäß einem der Ansprüche 1 bis 7 oder 8, in denen die Gruppe X¹ eine Methylengruppe darstellt.

13. Substituierte Carbonsäureamide der allgemeinen Formel I gemäß einem der Ansprüche 1 bis 8, in denen die Gruppe X¹ eine Carbonylgruppe darstellt.

14. Substituierte Carbonsäureamide der allgemeinen Formel I gemäß einem der Ansprüche 1 bis 8, 12 oder 13, in denen die Gruppe X³ eine Methylengruppe darstellt.

15. Substituierte Carbonsäureamide der allgemeinen Formel I gemäß einem der Ansprüche 1 bis 8, 12 oder 13 in denen die Gruppe X³ eine Carbonylgruppe darstellt.

16. Substituierte Carbonsäureamide der allgemeinen Formel I gemäß einem der Ansprüche 1 bis 8, oder 12 bis 15, in denen die Gruppe X⁴ ein Sauerstoffatom darstellt.

17. Substituierte Carbonsäureamide der allgemeinen Formel I gemäß einem der Ansprüche 1 bis 16, in denen der Rest B die Gruppe 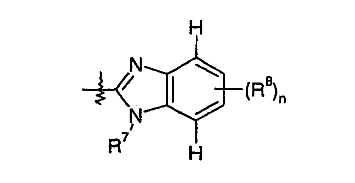 bedeutet.

18. Substituierte Carbonsäureamide der allgemeinen Formel I gemäß einem der Ansprüche 1 bis 16, in denen der Rest B die Gruppe 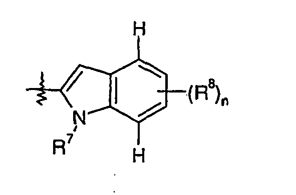 bedeutet.

19. Substituierte Carbonsäureamide der allgemeinen Formel I gemäß einem der Ansprüche 1 bis 16, in denen der Rest B die Gruppe 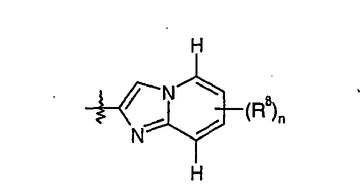 bedeutet.

20. Substituierte Carbonsäureamide der allgemeinen Formel I gemäß einem der Ansprüche 1 bis 19, in denen der Rest R⁸ ein Chloratom darstellt.

21. Substituierte Carbonsäureamide der allgemeinen Formel I gemäß einem der Ansprüche 1 bis 19, in denen der Rest R⁸ ein Bromatom darstellt.

22. Substituierte Carbonsäureamide der allgemeinen Formel I gemäß einem der Ansprüche 1 bis 19, in denen der Rest R⁸ eine Ethinylgruppe darstellt.

23. Substituierte Carbonsäureamide der allgemeinen Formel I gemäß einem der Ansprüche 1 bis 22, die der allgemeinen Formel Ia 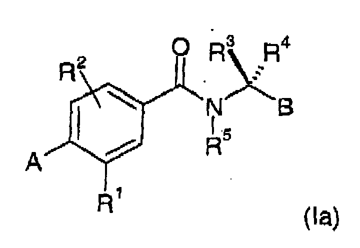 entsprechen.

24. Folgende Verbindungen der allgemeinen Formel I gemäß Anspruch 1:
(1) 4-(Azepan-2-on-1-yl)-*N*-[(1*R*)-1-(6-chlor-1*H*-benzimidazol-2-yl)-2- methoxy-ethyl]-3-methyl-benzamid,
(2) 4-(Azepan-2-on-1-yl)-*N*-[(1*R*)-1-(5-chlor-1*H*-benzimidazol-2-yl)-2- hydroxy-ethyl]-3-methyl-benzamid,
(3) *N*-[(1*S*)-1-(5-Chlor-1*H*-berizimidazol-2-yl)-3-methylsulfanyl-propyl]-3- methyl-4-(pyrrolidin-2-on-1-yl)-benzamid,
(4) *N*-[(1*R*)-1-(5-Chlor-1*H*-benzimidazol-2-yl)-2-methoxy-ethyl]-4-(morpholin- 3-on-4-yl)-3-trifluormethyl-benzamid,
(5) *N*-[(1*R*)-1-(5-Chlor-1*H*-benzimidazol-2-yl)-2-methoxy-ethyl]-3-methyl-4- (morpholin-3-on-4-yl)-benzamid,
(6) *N*-[(1*S*)-1-(5-Chlor-1*H*-benzimidazol-2-yl)-3-methylsulfanyl-propyl]-3- methyl-4-(morpholin-3-on-4-yl)-benzamid,
(7) *N*-[(1*R*)-1-(5-Chlor-1*H*-benzimidazol-2-yl)-2-hydroxy-ethyl]-3-methyl-4- (morpholin-3-on-4-yl)-benzamid,
(8) 4-(Azepan-2-on-1-yl)-3-chlor-*N*-[(1*R*)-1-(5-chlor-1*H*-berizimidazol-2-yl)-2- methoxy-ethyl]-benzamid,
(9) 4-(Azepan-2-on-1-yl)-*N*-[(1*R*)-1-(5-chlor-1*H*-benzimidazol-2-yl)-2- methoxy-ethyl]-3-trifluormethyl-benzamid,
(10) *N*-[(1*S*)-1-(5-Chlor-1*H*-benzimidazol-2-yl)-ethyl]-3-methyl-4- ([1,3]oxazepan-2-on-3-yl)-benzamid
(11) *N*-[(1*S*)-1-(5-Chlor-1*H*-benzimidazol-2-yl)-3-methylsulfanyl-propyl]-4- (morpholin-3-on-4-yl)-3-nitro-benzamid,
(12) *N*-[(1*R*)-1-(5-Chlor-1*H*-benzimidazol-2-yl)-2-methoxy-ethyl]-3-methyl-4- ([1,3]oxazepan-2-on-3-yl)-benzamid,
(13) 3-Chlor-*N*-[(1*R*)-1-(5-chlor-1*H*-benzimidazol-2-yl)-2-methoxy-ethyl]-4- ([1,4]oxazepan-5-on-4-yl)-benzamid,
(14) 3-Chlor-*N*-[(1*R*)-1-(5-chlor-1*H*-benzimidazol-2-yl)-2-methoxy-ethyl]-4- (piperidin-2-on-1-yl)-benzamid,
(15) *N*-[(1*R*)-1-(5-Brom-1*H*-benzimidazol-2-yl)-2-hydroxy-ethyl]-3-methyl-4- (morpholin-3-on-4-yl)-benzamid,
(16) *N*-[(1*R*,2*S*)-1-(5-Chlor-1*H*-benzimidazol-2-yl)-2-methoxy-propyl]-3- methyl-4-(morpholin-3-on-4-yl)-benzamid,
(17) *N*-[(15)-1-(5-chlor-1*H*-benzimidazol-2-yl)-ethyl]-3-methyl-4- (thiomorpholin-3-on-4-yl)-benzamid,
(18) *N*-[(1*R*)-1-(5-Chlor-1*H*-benzimidazol-2-yl)-2-methoxy-ethyl]-3-methyl-4- (piperidin-2-on-1-yl)-benzamid,
(19) *N*-[(1*R*)-1-(5-Chlor-1*H*-benzimidazol-2-yl)-2-methoxy-ethyl]-3-methyl-4- ([1,3]-oxazinan-2-on-3-yl)-benzamid,
(20) *N*-[(1*R*)-1-(5-Chlor-1*H*-benzimidazol-2-yl)-2-methoxy-ethyl]-4-(piperidin-2- on-1-yl)-3-trifluormethyl-benzamid,
(21) *N*-[(1*R*)-1-(5-Chlor-1*H*-benzimidazol-2-yl)-2-methoxy-ethyl]-4-(1,1-dioxo- [1,2]thiazinan-2-yl)-3-methyl-benzamid,
(22) *N*-[(1*S*)-1-(5-Chlor-1*H*-benzimidazol-2-yl)-butyl]-3-methyl-4-(morpholin-3- on-4-yl)-benzamid,
(23) *N*-[(1*R*)-1-(5-Chlor-1*H*-benzimidazol-2-yl)-2-methoxy-ethyl]-4- ([1,3]oxazepan-2-on-3-yl)-3-trifluormethyl-benzamid,
(24) *N*-[(1*R*)-1-(5-Chlor-1*H*-benzimidazol-2-yl)-2-methoxy-ethyl]-4-(1,1-dioxo- [1,2,6]thiadiazinan-2-yl)-3-methyl-benzamid,
(25) *N*-[(1*R*)-1-(5-Brom-1*H*-benzimidazol-2-yl)-2-methoxy-ethyl]-3-methyl-4- (morpholin-3-on-4-yl)-benzamid,
(26) *N*-[(1*S*)-1-(5-Chlor-1*H*-benzimidazol-2-yl)-3-(1*H*-tetrazol-5-yl)-propyl]-3- methyl-4-(morpholin-3-on-4-yl)-benzamid,
(27) *N*-[(1*S*)-1-(5-Chlor-1*H*-benzimidazol-2-yl)-3-methoxy-propyl]-3-methyl-4- (morpholin-3-on-4-yl)-benzamid,
(28) *N*-[(1*R*)-1-(5-Chlor-1*H*-benzimidazol-2-yl)-2-methylsulfanyl-ethyl]-3- methyl-4-(morpholin-3-on-4-yl)-benzamid,
(29) *N*-[(1*R*)-1-(5-Chlor-1*H*-benzimidazol-2-yl)-2-methoxy-ethyl]-3-methyl-4- (thiomorpholin-3-on-4-yl)-benzamid,
(30) 3-Chlor-*N*-[(1*R*)-1-(5-chlor-1*H*-benzimidazol-2-yl)-2-methoxy-ethyl]-4- (morpholin-3-on-4-yl)-benzamid,
(31) 3-Chlor-*N*-[(1*R*)-1-(5-chlor-1*H*-benzimidazol-2-yl)-2-methoxy-ethyl]-4- (1,1-dioxo[1,2]thiazinan-2-yl)-benzamid,
(32) *N*-[(1*R*)-1-(5-Chlor-1*H*-benzimidazol-2-yl)-2-methoxy-ethyl]-4-(1,1-dioxo- [1,2]thiazepan-2-yl)-3-methyl-benzamid,
(33) 3-Brom-*N*-(1*S*)-1-(5-chlor-1*H*-benzimidazol-2-yl)-ethyl]-4-(piperidin-2-on- 1-yl)-benzamid,
(34) 3-Brom-*N*-[(1*R*)-1-(5-chlor-1*H*-benzimidazol-2-yl)-2-methoxy-ethyl]-4- (piperidin-2-on-1-yl)-benzamid und
(35) *N*-[1-(5-Chlor-1*H*-benzimidazol-2-yl)-1-(furan-2-yl)-methyl]-3-methyl-4- (piperidin-2-on-1-yl)-benzamid,
deren Tautomere, deren Enantiomere, deren Diastereomeren, deren Gemische und deren Salze besonders bevorzugt sind.

25. Physiologisch verträgliche Salze der Verbindungen gemäß den Ansprüchen 1 bis 24.

26. Arzneimittel, enthaltend eine Verbindung nach mindestens einem der Ansprüche 1 bis 24 oder ein physiologisch verträgliches Salz gemäß Anspruch 25 neben gegebenenfalls einem oder mehreren inerten Trägerstoffen und/oder Verdünnungsmitteln.

27. Verwendung einer Verbindung nach mindestens einem der Ansprüche 1 bis 24 oder eines physiologisch verträglichen Salzes gemäß Anspruch 25 zur Herstellung eines Arzneimittels mit einem inhibitorischen Effekt auf Faktor Xa und/oder einem inhibitorischen Effekt auf verwandte Serinproteasen.

28. Verfahren zur Herstellung eines Arzneimittels gemäß Anspruch 26, **dadurch gekennzeichnet, dass** auf nichtchemischem Wege eine Verbindung nach mindestens einem der Ansprüche 1 bis 24 oder ein physiologisch verträgliches Salz gemäß Anspruch 25 in einen oder mehrere inerte Trägerstoffe und/oder Verdünnungsmittel eingearbeitet wird.

## Claims

1. Substituted carboxylic acid amides of general formula 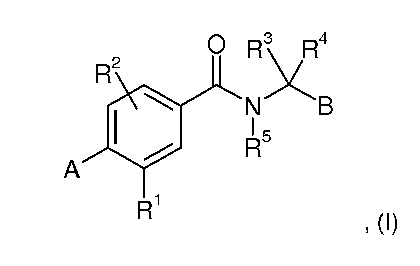 wherein
A denotes a group of general formula 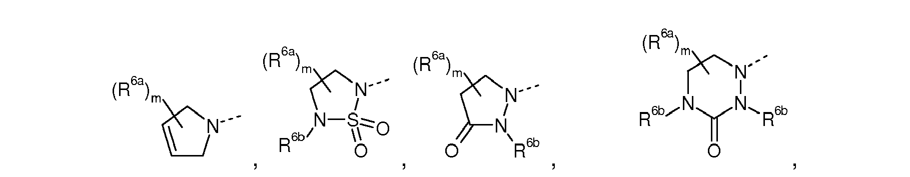 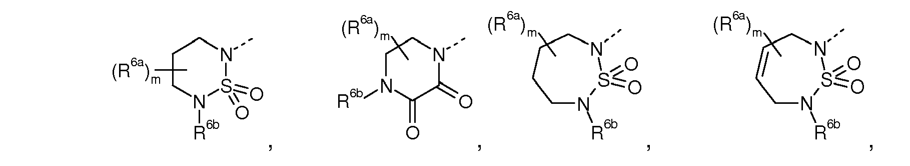 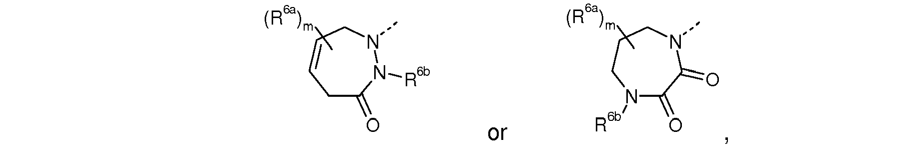 wherein
m denotes the number 1 or 2,
R^{6a} independently of one another denotes a hydrogen, fluorine, chlorine or bromine atom or a C₁₋₃-alkyl, hydroxy, amino, C₁₋₃-alkylamino, di-(C₁₋₃-alkyl)-amino, aminocarbonyl, C₁₋₃-alkylaminocarbonyl, di-(C₁₋₃-alkyl)-aminocarbonyl or C₁₋₃-alkylcarbonylamino group and
R^{6b} independently of one another may be a hydrogen atom, a C₁₋₄-alkyl, C₁₋₄-alkylcarbonyl, C₁₋₄-alkyloxycarbonyl or C₁₋₃-alkylsulphonyl group, with the proviso that
in the above-mentioned substituted 5- to 7-membered groups A the heteroatoms optionally introduced as substituents are not separated from another heteroatom by precisely one carbon atom, or
a group of general formula 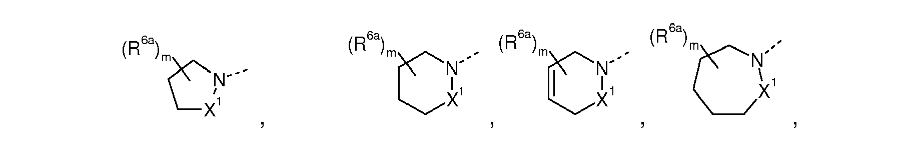 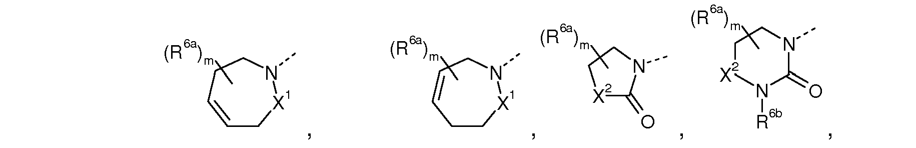 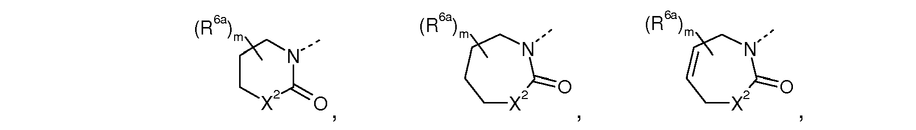 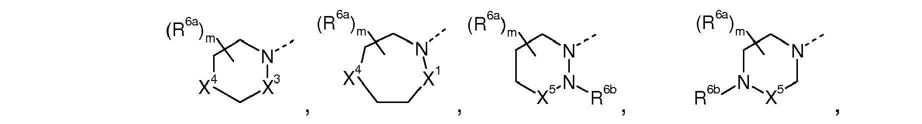 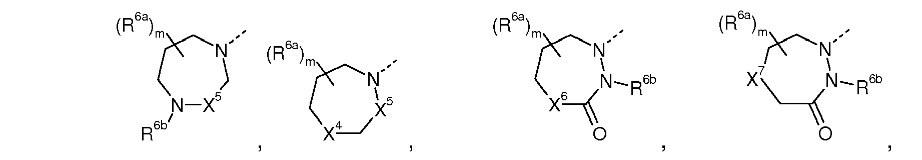 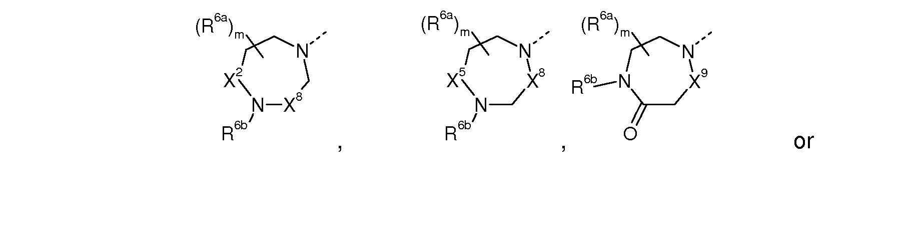 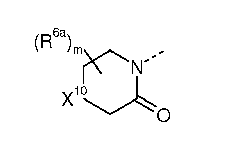 wherein
m denotes the number 1 or 2,
X¹ denotes an oxygen atom or a methylene, -NR^{6b}-, carbonyl or sulphonyl group, X² denotes an oxygen atom or a -NR^{6b}- group,
X³ denotes a methylene, carbonyl or sulphonyl group,
X⁴ denotes an oxygen or sulphur atom, a -NR^{6b}- or carbonyl group,
X⁵ denotes a carbonyl or sulphonyl group,
X⁶ denotes an oxygen atom, a -NR^{6b}- or methylene group,
X⁷ denotes an oxygen or sulphur atom or a -NR^{6b}- group,
X⁸ denotes a methylene or carbonyl group,
X⁹ denotes a -NR^{6b}- or carbonyl group,
X¹⁰ denotes a sulphinyl or sulphonyl group and
R^{6a} independently of one another denote a hydrogen, fluorine, chlorine or bromine atom or a C₁₋₃-alkyl, hydroxy, amino, C₁₋₃-alkylamino, di-(C₁₋₃-alkyl)-amino, aminocarbonyl, C₁₋₃-alkylaminocarbonyl, di-(C₁₋₃-alkyl)-aminocarbonyl or C₁₋₃-alkylcarbonylamino group and
R^{6b} independently of one another may be a hydrogen atom, a C₁₋₄-alkyl, C₁₋₄-alkylcarbonyl, C₁₋₄-alkoxycarbonyl or C₁₋₃-alkylsulphonyl group, with the proviso that
in the above-mentioned substituted 5- to 7-membered groups A the heteroatoms optionally introduced as substituents are not separated from another heteroatom by precisely one carbon atom,
R¹ denotes a hydrogen, fluorine, chlorine or bromine atom, a C₁₋₃-alkyl group wherein the hydrogen atoms may be wholly or partly replaced by fluorine atoms, a C₂₋₃-alkenyl, C₂₋₃-alkynyl, nitro, amino, C₁₋₃-alkoxy, a mono-, di- or trifluoromethoxy group,
R² denotes a hydrogen, fluorine, chlorine or bromine atom or a C₁₋₃-alkyl group,
R³ denotes a hydrogen atom, a C₂₋₃-alkenyl or C₂₋₃-alkynyl group or a straight-chain or branched C₁₋₆-alkyl group wherein the hydrogen atoms may be wholly or partly replaced by fluorine atoms, and which is optionally substituted by a nitrile, hydroxy, a C₁₋₅-alkyloxy group wherein the hydrogen atoms may be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₃-alkyloxy, C₁₋₅-alkyloxycarbonyl-C₁₋₃-alkyloxy, C₁₋₈-alkyloxycarbonylamino, mercapto, C₁₋₃-alkylsulphanyl, C₁₋₃-alkylsulphinyl, C₁₋₃-alkylsulphonyl, C₁₋₃-alkylcarbonylamino-C₁₋₃-alkylsulphanyl, C₁₋₃-alkylcarbonylamino-C₁₋₃-alkylsulphinyl, C₁₋₃-alkylcarbonylamino-C₁₋₃-alkylsulphonyl, carboxy, C₁₋₃-alkyloxycarbonyl, allyloxycarbonyl, propargyloxycarbonyl, benzyloxycarbonyl, aminocarbonyl, C₁₋₃-alkylaminocarbonyl, di-(C₁₋₃-alkyl)-aminocarbonyl, C₃₋₆-cycloalkyleneiminocarbonyl, aminosulphonyl, C₁₋₃-alkylaminosulphonyl, di-(C₁₋₃-alkyl)-aminosulphonyl, C₃₋₆-cycloalkyleneiminosulphonyl, amino, C₁₋₃-alkylamino, di-(C₁₋₃-alkyl)-amino, C₁₋₅-alkylcarbonylamino, C₁₋₃-alkylsulphonylamino, *N*-(C₁₋₃-alkylsulphonyl)-C₁₋₃-alkylamino, C₃₋₆-cycloalkylcarbonylamino, aminocarbonylamino, C₁₋₃-alkylaminocarbonylamino, di-(C₁₋₃-alkyl)-aminocarbonylamino, a 4- to 7-membered cycloalkyleneiminocarbonylamino, benzyloxycarbonylamino, phenylcarbonylamino or guanidino group,
a carboxy, aminocarbonyl, C₁₋₄-alkylaminocarbonyl, C₃₋₆-cycloalkylaminocarbonyl, di-(C₁₋₃-alkyl)-aminocarbonyl, C₁₋₄-alkoxycarbonyl, C₄₋₆-cycloalkyleneiminocarbonyl group,
a phenyl or heteroaryl, phenylcarbonyl-C₁₋₃-alkyl, phenyl-C₁₋₃-alkyl or heteroaryl-C₁₋₃-alkyl group which is optionally mono- or polysubstituted in the phenyl or heteroaryl moiety by fluorine, chlorine or bromine atoms, C₁₋₃-alkyl, amino, C₁₋₃-alkylamino, di-(C₁₋₃-alkyl)-amino, hydroxy, C₁₋₄-alkyloxy, mono-, di- or trifluoromethoxy, benzyloxy, carboxy-C₁₋₃-alkyloxy, C₁₋₃-alkyloxycarbonyl-C₁₋₃-alkyloxy, aminocarbonyl-C₁₋₃-alkyloxy, C₁₋₃-alkylaminocarbonyl-C₁₋₃-alkyloxy, di-(C₁₋₃-alkyl)-aminocarbonyl-C₁₋₃-alkyloxy, a 4- to 7-membered cycloalkyleneiminocarbonyl-C₁₋₃-alkoxy, carboxy, C₁₋₃-alkyloxycarbonyl or C₁₋₃-alkyloxycarbonylamino group,
a 3- to 7-membered cycloalkyl, cycloalkyleneimino, cycloalkyl-C₁₋₃-alkyl or cycloalkyleneimino-C₁₋₃-alkyl group wherein in the cyclic moiety a methylene group may be replaced by an -NH- group optionally substituted by a C₁₋₃-alkyl or C₁₋₃-alkylcarbonyl group or by an oxygen atom and wherein additionally a methylene group adjacent to the -NH-, -N(C₁₋₃-alkylcarbonyl)- or -N(C₁₋₃-alkyl)- group may be replaced in each case by a carbonyl or sulphonyl group, with the proviso that a cycloalkyleneimino group as hereinbefore defined wherein two nitrogen atoms are separated from one another by precisely one -CH₂- group is excluded,
R⁴ denotes a hydrogen atom or a C₁₋₃-alkyl group or
R³ and R⁴ together with the carbon atom to which they are bound denote a C₃₋₇-cycloalkyl group, while
one of the methylene groups of the C₃₋₇-cycloalkyl group may be replaced by an imino, C₁₋₃-alkylimino, acylimino or sulphonylimino group,
R⁵ denotes a hydrogen atom or a C₁₋₃-alkyl group,
B denotes a group of formula 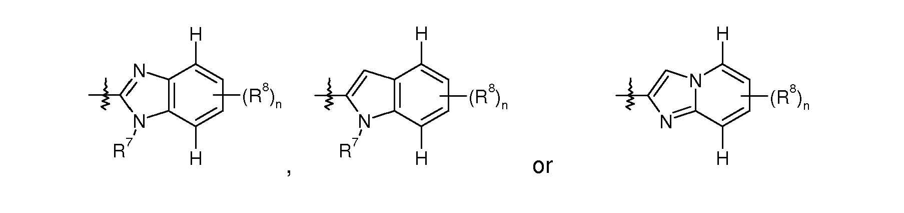 wherein
n denotes the number 1 or 2,
R⁷ denotes a hydrogen atom or a C₁₋₃-alkyl, hydroxy, C₁₋₅-alkyloxycarbonyl, carboxy-C₁₋₃-alkyl, C₁₋₃-alkyloxycarbonyl-C₁₋₃-alkyl, amino or C₁₋₃-alkylamino group and
R⁸ independently of one another denote a hydrogen, fluorine, chlorine, bromine or iodine atom, a C₁₋₃-alkyl group wherein the hydrogen atoms may be wholly or partly replaced by fluorine atoms, a C₂₋₃-alkenyl or C₂₋₃-alkynyl, a hydroxy, C₁₋₃-alkoxy, trifluoromethoxy, amino, nitro or nitrile group,
while, unless otherwise stated, by the term "heteroaryl group" mentioned hereinbefore in the definitions is meant a monocyclic 5- or 6-membered heteroaryl group, while
the 6-membered heteroaryl group contains one, two or three nitrogen atoms and
the 5-membered heteroaryl group contains an imino group optionally substituted by a C₁₋₃-alkyl, phenyl or phenyl-C₁₋₃-alkyl group, an oxygen or sulphur atom or
an imino group optionally substituted by a C₁₋₃-alkyl, phenyl, amino-C₂₋₃-alkyl, C₁₋₃-alkylamino-C₂₋₃-alkyl, di-(C₁₋₃-alkyl)-aminO-C₂-₃-alkyl, a 4- to 7-membered cycloalkyleneimino-C₁₋₃-alkyl or phenyl-C₁₋₃-alkyl group or an oxygen or sulphur atom and additionally a nitrogen atom or
an imino group optionally substituted by a C₁₋₃-alkyl or phenyl-C₁₋₃-alkyl group and two or three nitrogen atoms,
and moreover a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms
and the bond is effected via a nitrogen atom or a carbon atom of the heterocyclic moiety or a fused-on phenyl ring,
while the alkyl and alkoxy groups contained in the above-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

2. Substituted carboxylic acid amides of general formula I according to claim 1, wherein
A denotes a group of general formula 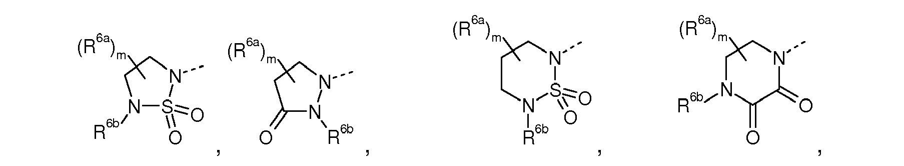 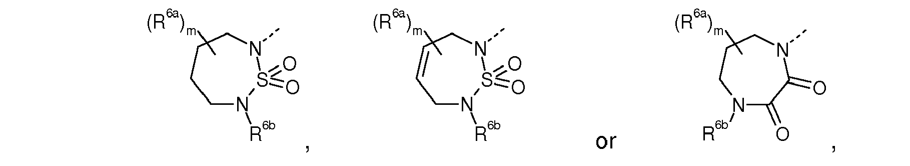 wherein m denotes the number 1 or 2,
R^{6a} independently of one another denote a hydrogen or fluorine atom, a C₁₋₃-alkyl, hydroxy, amino, C₁₋₃alkylamino, di-(C₁₋₃-alkyl)-amino, aminocarbonyl, C₁₋₃-alkylaminocarbonyl, di-(C₁₋₃-alkyl)-aminocarbonyl or C₁₋₃-alkylcarbonylamino group and
R^{6b} independently of one another may be a hydrogen atom, a C₁₋₃-alkyl, C₁₋₄-alkylcarbonyl, C₁₋₄-alkoxycarbonyl or C₁₋₃-alkylsulphonyl group, with the proviso that
in the above-mentioned substituted 5- to 7-membered groups A the heteroatoms optionally introduced as substituents are not separated from another heteroatom by precisely one carbon atom, or
a group of general formula 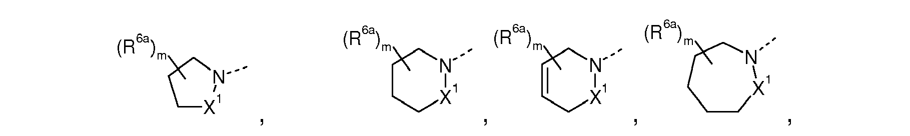 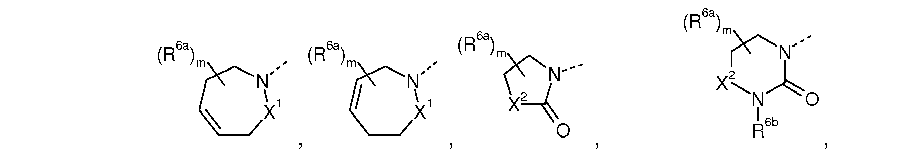 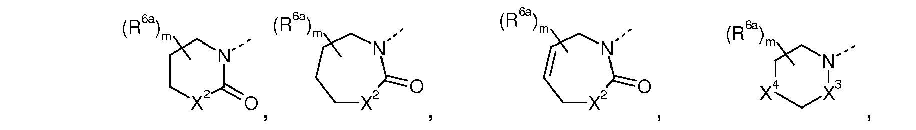 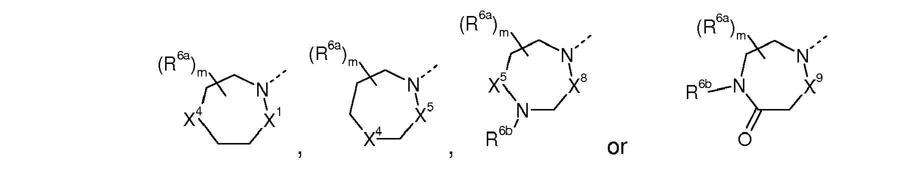 wherein
m denotes the number 1 or 2,
X¹ denotes a methylene, -NR^{6b}-, carbonyl or sulphonyl group,
X² denotes an oxygen atom or a -NR^{6b}- group,
X³ denotes a methylene, carbonyl or sulphonyl group,
X⁴ denotes an oxygen or sulphur atom, a -NR^{6b}- or carbonyl group,
X⁵ denotes a carbonyl or sulphonyl group,
X⁸ denotes a carbonyl group,
X⁹ denotes a carbonyl group,
R^{6a} independently of one another denote a hydrogen or fluorine atom, a C₁₋₃-alkyl, hydroxy, amino, C₁₋₃-alkylamino, di-(C₁₋₃-alkyl)-amino, aminocarbonyl, C₁₋₃-alkylaminocarbonyl, di-(C₁₋₃-alkyl)-aminocarbonyl or C₁₋₃-alkylcarbonylamino group and
R^{6b} independently of one another may be a hydrogen atom, a C₁₋₃-alkyl, C₁₋₃-alkylcarbonyl, C₁₋₃-alkoxycarbonyl or C₁₋₃-alkylsulphonyl group, with the proviso that
in the above-mentioned substituted 5- to 7-membered cyclic groups A the heteroatoms introduced as substituents are not separated from another heteroatom by precisely one carbon atom,
R¹ denotes a hydrogen, fluorine, chlorine or bromine atom, a C₁₋₃-alkyl group wherein the hydrogen atoms may be wholly or partly replaced by fluorine atoms, a C₂₋₃-alkenyl, C₂₋₃-alkynyl, nitro, amino, C₁₋₃-alkoxy, a mono-, di- or trifluoromethoxy group,
R² denotes a hydrogen, fluorine, chlorine or bromine atom or a C₁₋₃-alkyl group,
R³ denotes a C₂₋₃-alkenyl or C₂₋₃-alkynyl group or a straight-chain or branched C₁₋₆-alkyl group wherein the hydrogen atoms may be wholly or partly replaced by fluorine atoms, and which is optionally substituted by a nitrile, hydroxy, a C₁₋₅-alkyloxy group wherein the hydrogen atoms may be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₃-alkyloxy, C₁₋₅-alkyloxycarbonyl-C₁₋₃-alkyloxy, C₁₋₈-alkyloxycarbonylamino, mercapto, C₁₋₃-alkylsulphanyl, C₁₋₃-alkylsulphinyl, C₁₋₃-alkylsulphonyl, C₁₋₃-alkylcarbonylamino-Cₗ-₃-alkylsulphanyl, C₁₋₃-alkylcarbonylamino-C₁₋₃-alkylsulphinyl, C₁₋₃-alkylcarbonylamino-C₁₋₃-alkylsulphonyl, carboxy, C₁₋₃-alkyloxycarbonyl, allyloxycarbonyl, propargyloxycarbonyl, benzyloxycarbonyl, aminocarbonyl, C₁₋₃-alkylaminocarbonyl, di-(C₁₋₃-alkyl)-aminocarbonyl, C₃₋₆-cycloalkyleneiminocarbonyl, aminosulphonyl, C₁₋₃-alkylaminosulphonyl, di-(C₁₋₃-alkyl)-aminosulphonyl, C₃₋₆-cycloalkyleneiminosulphonyl, amino, C₁₋₃-alkylamino, di-(C₁₋₃-alkyl)-amino, C₁₋₃-alkylcarbonylamino, C₁₋₃-alkylsulphonylamino, *N*-(C₁₋₃-alkylsulphonyl)-C₁₋₃-alkylamino, C₃₋₆-cycloalkylcarbonylamino, aminocarbonylamino, C₁₋₃-alkylaminocarbonylamino, di-(C₁₋₃-alkyl)-aminocarbonylamino, a 4- to 7-membered cycloalkyleneiminocarbonylamino, benzyloxycarbonylamino, phenylcarbonylamino or guanidino group,
a carboxy, aminocarbonyl, C₁₋₄-alkylaminocarbonyl, C₃₋₆-cycloalkylaminocarbonyl, di-(C₁₋₃-alkyl)-aminocarbonyl, C₁₋₄-alkoxycarbonyl, C₄₋₆-cycloalkyleneiminocarbonyl group,
a phenyl or heteroaryl, phenylcarbonyl-C₁₋₃-alkyl, phenyl-C₁₋₃-alkyl or heteroaryl-C₁₋₃-alkyl group which is optionally mono- or polysubstituted in the phenyl or heteroaryl moiety by fluorine, chlorine or bromine atoms, C₁₋₃-alkyl, amino, C₁₋₃-alkylamino, di-(C₁₋₃-alkyl)-amino, hydroxy, C₁₋₄-alkyloxy, mono-, di- or trifluoromethoxy, benzyloxy, carboxy-C₁₋₃-alkyloxy, C₁₋₃-alkyloxycarbonyl-C₁₋₃-alkyloxy, aminocarbonyl-C₁₋₃-alkyloxy, C₁₋₃-alkylaminocarbonyl-C₁₋₃-alkyloxy, di-(C₁₋₃-alkyl)-aminocarbonyl-C₁₋₃-alkyloxy, a 4- to 7-membered cycloalkyleneiminocarbonyl-C₁₋₃-alkoxy, carboxy, C₁₋₃-alkyloxycarbonyl or C₁₋₃-alkyloxycarbonylamino group,
a 3- to 7-membered cycloalkyl, cycloalkyleneimino, cycloalkyl-C₁₋₃-alkyl or cycloalkyleneimino-C₁₋₃-alkyl group wherein in the cyclic moiety a methylene group may be replaced by a -NH- group optionally substituted by a C₁₋₃-alkyl or C₁₋₃-alkylcarbonyl group or by an oxygen atom and wherein additionally a methylene group adjacent to the -NH-, -N(C₁₋₃-alkylcarbonyl)- or -N(C₁₋₃-alkyl)- group may be replaced in each case by a carbonyl or sulphonyl group, with the proviso that a cycloalkyleneimino group as hereinbefore defined wherein two nitrogen atoms are separated from one another by precisely one -CH₂- group is excluded,
R⁴ denotes a hydrogen atom or a C₁₋₃-alkyl group,
R⁵ denotes a hydrogen atom or a C₁₋₃-alkyl group,
B denotes a group of formula 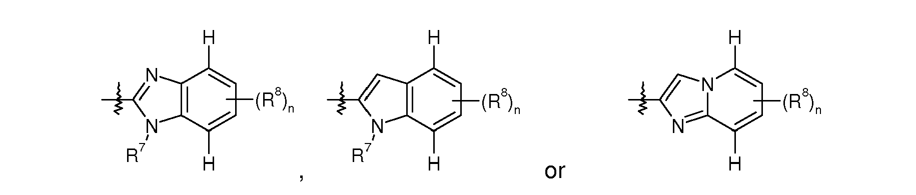 wherein
n denotes the number 1 or 2,
R⁷ denotes a hydrogen atom, a C₁₋₃-alkyl, hydroxy, C₁₋₃-alkyloxycarbonyl, carboxy-C₁₋₃-alkyl, C₁₋₃-alkyloxycarbonyl-C₁₋₃-alkyl, amino or C₁₋₃-alkylamino group and
R⁸ independently of one another denote a hydrogen, fluorine, chlorine, bromine or iodine atom, a C₁₋₃-alkyl group wherein the hydrogen atoms may be wholly or partly replaced by fluorine atoms, a C₂₋₃-alkenyl or C₂₋₃-alkynyl, a hydroxy, C₁₋₃-alkoxy, trifluoromethoxy, amino, nitro or nitrile group,
while, unless otherwise stated, by the term "heteroaryl group" mentioned hereinbefore in the definitions is meant a monocyclic 5- or 6-membered heteroaryl group, while
the 6-membered heteroaryl group contains one, two or three nitrogen atoms and the 5-membered heteroaryl group contains an imino group optionally substituted by a C₁₋₃-alkyl, phenyl or phenyl-C₁₋₃-alkyl group, an oxygen or sulphur atom or an imino group optionally substituted by a C₁₋₃-alkyl, phenyl, amino-C₂₋₃-alkyl, C₁₋₃-alkylaminO-C₂₋₃-alkyl, di-(C₁₋₃-alkyl)-amino-C₂₋₃-alkyl, a 4- to 7-membered cycloalkyleneimino-C₁₋₃-alkyl or phenyl-C₁₋₃-alkyl group or an oxygen or sulphur atom and additionally a nitrogen atom or
an imino group optionally substituted by a C₁₋₃-alkyl or phenyl-C₁₋₃-alkyl group and two or three nitrogen atoms,
and moreover a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms
and the bond is effected via a nitrogen atom or via a carbon atom of the heterocyclic moiety or a fused-on phenyl ring,
while the alkyl and alkoxy groups contained in the above-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

3. Substituted carboxylic acid amides of general formula I according to claim 1, wherein
A denotes a group of general formula 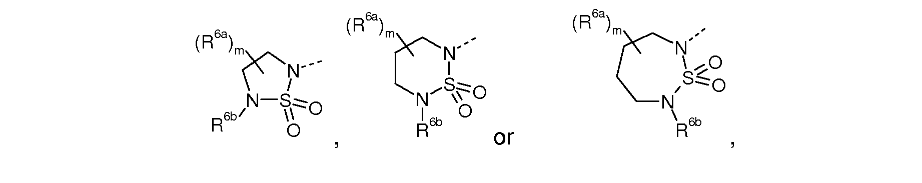 wherein m denotes the number 1 or 2,
R^{6a} independently of one another denote a hydrogen or fluorine atom, a C₁₋₃-alkyl, hydroxy, amino, C₁₋₃-alkylamino, di-(C₁₋₃-alkyl)-amino, aminocarbonyl, C₁₋₃-alkylaminocarbonyl, di-(C₁₋₃-alkyl)-aminocarbonyl or C₁₋₃-alkylcarbonylamino group and
R^{6b} independently of one another may be a hydrogen atom, a C₁₋₄-alkyl, C₁₋₄-alkylcarbonyl, C₁₋₄-alkoxycarbonyl or C₁₋₄-alkylsulphonyl group, with the proviso that
in the above-mentioned substituted 5- to 7-membered groups A the heteroatoms optionally introduced as substituents are not separated from another heteroatom by precisely one carbon atom, or
a group of general formula 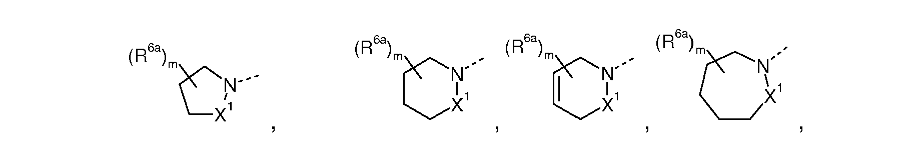 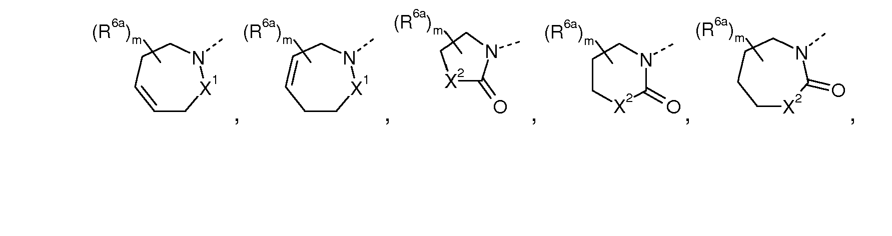 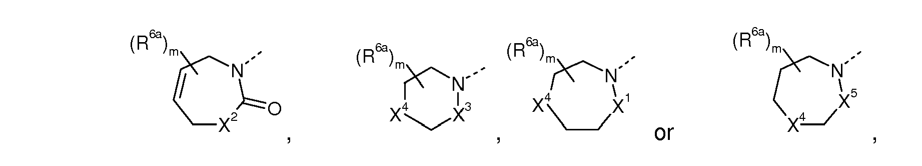 wherein
m denotes the number 1 or 2,
X¹ denotes a methylene, -NR^{6b}-, carbonyl or sulphonyl group,
X² denotes an oxygen atom or a -NR^{6b}- group,
X³ denotes a methylene, carbonyl or sulphonyl group,
X⁴ denotes an oxygen or sulphur atom or a -NR^{6b}- group,
X⁵ denotes a carbonyl or sulphonyl group,
R^{6a} independently of one another denote a hydrogen or fluorine atom, a C₁₋₃-alkyl, hydroxy, amino, C₁₋₃-alkylamino, di-(C₁₋₃-alkyl)-amino, aminocarbonyl, C₁₋₃-alkylaminocarbonyl, di-(C₁₋₃-alkyl)-aminocarbonyl or C₁₋₃-alkylcarbonylamino group and
R^{6b} independently of one another may be a hydrogen atom, a C₁₋₄-alkyl, C₁₋₄-alkylcarbonyl, C₁₋₄-alkoxycarbonyl or C₁₋₃-alkylsulphonyl group, with the proviso that
in the above-mentioned substituted 5- to 7-membered cyclic groups A the heteroatoms introduced as substituents are not separated from another heteroatom by precisely one carbon atom,
R¹ denotes a fluorine, chlorine or bromine atom, a C₁₋₃-alkyl group wherein the hydrogen atoms may be wholly or partly replaced by fluorine atoms, a nitro, C₁₋₃-alkoxy, a mono-, di- or trifluoromethoxy group,
R² denotes a hydrogen atom,
R³ denotes a straight-chain or branched C₁₋₆-alkyl group wherein the hydrogen atoms may be wholly or partly replaced by fluorine atoms, and which is optionally substituted by a nitrile, hydroxy, benzyloxy, a C₁₋₅-alkyloxy group wherein the hydrogen atoms may be wholly or partly replaced by fluorine atoms, an allyloxy, C₁₋₅-alkylcarbonyloxy, C₁₋₅-alkyloxycarbonyloxy, carboxy-C₁₋₃-alkyloxy, C₁₋₅-alkyloxycarbonyl-C₁₋₃-alkyloxy, C₁₋₈-alkyloxycarbonylamino, C₁₋₃-alkylsulphanyl, C₁₋₃-alkylsulphonyl, carboxy, C₁₋₃-alkyloxycarbonyl, C₁₋₃-alkylaminocarbonyl, di-(C₁₋₃-alkyl)-aminocarbonyl, C₃₋₆-cycloalkyleneiminocarbonyl, aminocarbonylamino, C₁₋₃-alkylaminocarbonylamino or di-(C₁₋₃-alkyl)-aminocarbonylamino group,
an aminocarbonyl, C₁₋₄-alkylaminocarbonyl, C₃₋₆-cycloalkylaminocarbonyl or di-(C₁₋₃-alkyl)-aminocarbonyl group,
a phenyl or heteroaryl, phenyl-C₁₋₃-alkyl or heteroaryl-C₁₋₃-alkyl group which is optionally mono- or polysubstituted in the phenyl or heteroaryl moiety by fluorine, chlorine or bromine atoms, C₁₋₃-alkyl, amino, C₁₋₃-alkylamino, di-(C₁₋₃-alkyl)-amino, hydroxy, C₁₋₄-alkyloxy, mono-, di- or trifluoromethoxy, carboxy, or C₁₋₃-alkyloxycarbonyl group,
a 3- to 7-membered cycloalkyl group wherein in the cyclic moiety a methylene group may be replaced by a -NH- group optionally substituted by a C₁₋₃-alkyl or C₁₋₃-alkylcarbonyl group, or an oxygen atom,
R⁴ denotes a hydrogen atom,
R⁵ denotes a hydrogen atom,
B denotes a group of formula 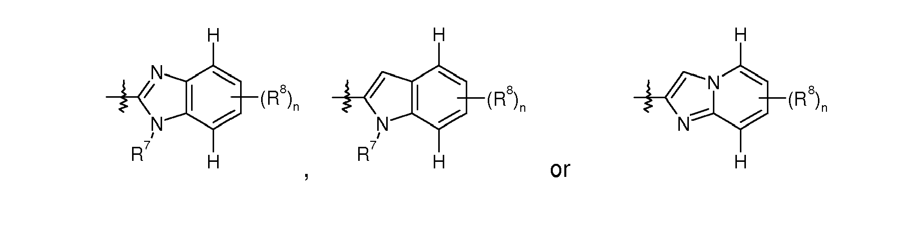 wherein
n denotes the number 1,
R⁷ denotes a hydrogen atom and
R⁸ denotes a hydrogen, fluorine, chlorine, bromine or iodine atom, a methyl, C₂₋₃-alkynyl, or methoxy group, wherein the hydrogen atoms may be wholly or partly replaced by fluorine atoms,
while, unless otherwise stated, by the term "heteroaryl group" mentioned hereinbefore in the definitions is meant a monocyclic 5- or 6-membered heteroaryl group, while
the 6-membered heteroaryl group contains one, two or three nitrogen atoms and the 5-membered heteroaryl group contains an imino group optionally substituted by a C₁₋₃-alkyl group, an oxygen or sulphur atom or
an imino group optionally substituted by a C₁₋₃-alkyl group or an oxygen or sulphur atom and additionally a nitrogen atom or
an imino group optionally substituted by a C₁₋₃-alkyl group and two or three nitrogen atoms,
and the bond is effected via a nitrogen atom or via a carbon atom,
while the alkyl and alkoxy groups contained in the above-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

4. Substituted carboxylic acid amides of general formula I according to claim 3, wherein
A, R¹, R², R⁴, R⁵ and B are defined as in claim 3 and
R³ denotes a straight-chain or branched C₁₋₆-alkyl group wherein the hydrogen atoms may be wholly or partly replaced by fluorine atoms, and which is optionally substituted by a nitrile, hydroxy, benzyloxy, a C₁₋₅-alkyloxy group wherein the hydrogen atoms may be wholly or partly replaced by fluorine atoms, an allyloxy, C₁₋₅-alkylcarbonyloxy, C₁₋₅-alkyloxycarbonyloxy, carboxy-C₁₋₃-alkyloxy, C₁₋₅-alkyloxycarbonyl-C₁₋₃-alkyloxy, C₁₋₈-alkyloxycarbonylamino, C₁₋₃-alkylsulphanyl, C₁₋₃-alkylsulphonyl, carboxy, C₁₋₃-alkyloxycarbonyl, C₁₋₃-alkylaminocarbonyl, di-(C₁₋₃-alkyl)-aminocarbonyl, C₃₋₆-cycloalkyleneiminocarbonyl, aminocarbonylamino, C₁₋₃-alkylaminocarbonylamino or di-(C₁₋₃-alkyl)-aminocarbonylamino group,
an aminocarbonyl, C₁₋₄-alkylaminocarbonyl, C₃₋₆-cycloalkylaminocarbonyl or di-(C₁₋₃-alkyl)-aminocarbonyl group,
while the alkyl and alkoxy groups contained in the above-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

5. Substituted carboxylic acid amides of general formula I according to claim 3, wherein
A, R¹, R², R⁴, R⁵ are defined as in claim 3 and
R³ denotes a phenyl or heteroaryl, phenyl-C₁₋₃-alkyl or heteroaryl-C₁₋₃-alkyl group which is optionally mono- or polysubstituted in the phenyl or heteroaryl moiety by fluorine, chlorine or bromine atoms, C₁₋₃-alkyl, amino, C₁₋₃-alkylamino, di-(C₁₋₃-alkyl)-amino, hydroxy, C₁₋₄-alkyloxy, mono-, di- or trifluoromethoxy, carboxy, or C₁₋₃-alkyloxycarbonyl group,
a 3- to 7-membered cycloalkyl group wherein in the cyclic moiety a methylene group may be replaced by a -NH- group optionally substituted by a C₁₋₃-alkyl or C₁₋₃-alkylcarbonyl group, or by an oxygen atom,
while, unless otherwise stated, by the term "heteroaryl group" mentioned hereinbefore in the definitions is meant a monocyclic 5- or 6-membered heteroaryl group, wherein
the 6-membered heteroaryl group contains one, two or three nitrogen atoms and
the 5-membered heteroaryl group contains an imino group optionally substituted by a C₁₋₃-alkyl group, or an oxygen or sulphur atom or
an imino group optionally substituted by a C₁₋₃-alkyl group or an oxygen or sulphur atom and additionally a nitrogen atom or
an imino group optionally substituted by a C₁₋₃-alkyl group and two or three nitrogen atoms,
and the bond is effected via a nitrogen atom or via a carbon atom,
while the alkyl groups contained in the foregoing definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

6. Substituted carboxylic acid amides of general formula I according to claim 1, wherein
A denotes a group of general formula 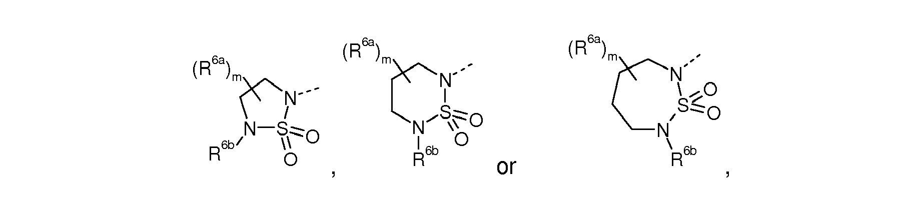 wherein m denotes the number 1 or 2,
R^{6a} independently of one another denote a hydrogen or fluorine atom or a C₁₋₃-alkyl group and
R^{6b} may be a hydrogen atom or a C₁₋₃-alkyl group, with the proviso that
in the above-mentioned substituted 5- to 7-membered groups A the heteroatoms optionally introduced as substituents are not separated from another heteroatom by precisely one carbon atom, or
a group of general formula 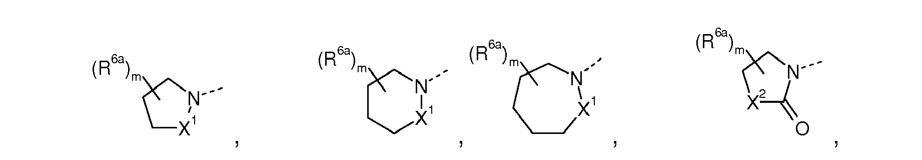 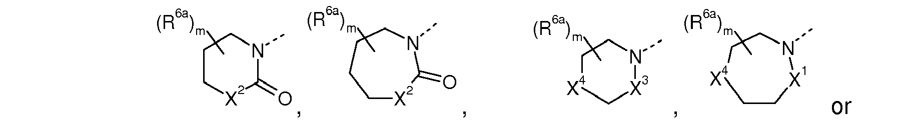 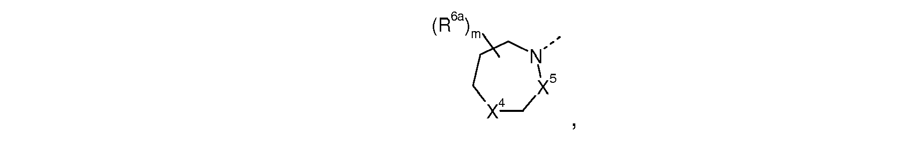 wherein
m denotes the number 1 or 2,
X¹ denotes a methylene, -NR^{6b}-, carbonyl or sulphonyl group,
X² denotes an oxygen atom or a -NR^{6b}- group,
X³ denotes a methylene, carbonyl or sulphonyl group,
X⁴ denotes an oxygen or sulphur atom or a -NR^{6b}- group,
X⁵ denotes a carbonyl or sulphonyl group,
R^{6a} independently of one another denote a hydrogen or fluorine atom or a C₁₋₃-alkyl group and
R^{6b} independently of one another may be a hydrogen atom or a C₁₋₃-alkyl group, with the proviso that
in the above-mentioned substituted 5- to 7-membered cyclic groups A the heteroatoms introduced as substituents are not separated from another heteroatom by precisely one carbon atom,
R¹ denotes a chlorine or bromine atom, a methyl or methoxy group, wherein the hydrogen atoms may be wholly or partly replaced by fluorine atoms, or a nitro group,
R² denotes a hydrogen atom,
R³ denotes a straight-chain or branched C₁₋₄-alkyl group wherein the hydrogen atoms may be wholly or partly replaced by fluorine atoms, and which is optionally substituted by a hydroxy, a C₁₋₄-alkyloxy group wherein the hydrogen atoms may be wholly or partly replaced by fluorine atoms, a C₁₋₃-alkylsulphanyl, C₁₋₃-alkylsulphonyl, carboxy or C₁₋₃-alkyloxycarbonyl group,
a phenyl or heteroaryl, phenyl-C₁₋₃-alkyl or heteroaryl-C₁₋₃-alkyl group which is optionally mono- or polysubstituted in the phenyl or heteroaryl moiety by fluorine, chlorine or bromine atoms, C₁₋₃-alkyl, C₁₋₄-alkyloxy, mono-, di- or trifluoromethoxy, carboxy, or C₁₋₃-alkyloxycarbonyl group,
R⁴ denotes a hydrogen atom,
R⁵ denotes a hydrogen atom and
B denotes a group of formula 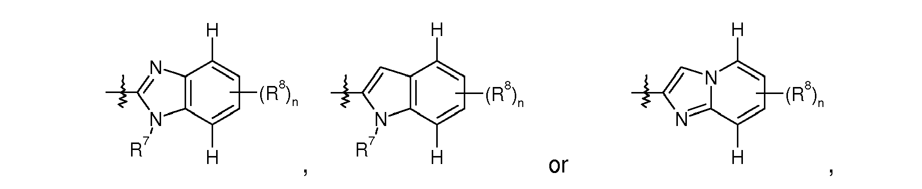 wherein
n denotes the number 1,
R⁷ denotes a hydrogen atom and
R⁸ denotes a chlorine or bromine atom or the ethynyl group, while, unless otherwise stated, by the term "heteroaryl group" mentioned hereinbefore in the definitions is meant a monocyclic 5- or 6-membered heteroaryl group, while
the 6-membered heteroaryl group contains one, two or three nitrogen atoms and the 5-membered heteroaryl group contains an imino group optionally substituted by a C₁₋₃-alkyl group, or an oxygen or sulphur atom or
an imino group optionally substituted by a C₁₋₃-alkyl group or an oxygen or sulphur atom and additionally a nitrogen atom or
an imino group optionally substituted by a C₁₋₃-alkyl group and two or three nitrogen atoms,
and the bond is effected via a nitrogen atom or via a carbon atom,
while the alkyl groups contained in the foregoing definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

7. Substituted carboxylic acid amides of general formula I according to claim 6, wherein
A, R¹, R², R⁴, R⁵ and B are defined as in claim 6 and
R³ denotes a straight-chain or branched C₁₋₄-alkyl group wherein the hydrogen atoms may be wholly or partly replaced by fluorine atoms, and which is optionally substituted by a hydroxy, a C₁₋₄-alkyloxy group wherein the hydrogen atoms may be wholly or partly replaced by fluorine atoms, a C₁₋₃-alkylsulphanyl, C₁₋₃-alkylsulphonyl, carboxy or C₁₋₃-alkyloxycarbonyl group,
while the alkyl and alkoxy groups contained in the above-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

8. Substituted carboxylic acid amides of general formula I according to claim 6, wherein
A, R¹, R², R⁴, R⁵ and B are defined as in claim 6 and
R³ denotes a phenyl or heteroaryl, phenyl-C₁₋₃-alkyl or heteroaryl-C₁₋₃-alkyl group which is optionally mono- or polysubstituted in the phenyl or heteroaryl moiety by fluorine, chlorine or bromine atoms, C₁₋₃-alkyl, C₁₋₄-alkyloxy, mono-, di- or trifluoromethoxy, carboxy, or C₁₋₃-alkyloxycarbonyl group,
a 3- to 7-membered cycloalkyl group wherein in the cyclic moiety a methylene group may be replaced by a -NH- group optionally substituted by a C₁₋₃-alkyl or C₁₋₃-alkylcarbonyl group, or by an oxygen atom,
while, unless otherwise stated, by the term "heteroaryl group" mentioned hereinbefore in the definitions is meant a monocyclic 5- or 6-membered heteroaryl group, while
the 6-membered heteroaryl group contains one, two or three nitrogen atoms and
the 5-membered heteroaryl group contains an imino group optionally substituted by a C₁₋₃-alkyl group, an oxygen or sulphur atom or
an imino group optionally substituted by a C₁₋₃-alkyl group or an oxygen or sulphur atom and additionally a nitrogen atom or
an imino group optionally substituted by a C₁₋₃-alkyl group and two or three nitrogen atoms,
and the bond is effected via a nitrogen atom or via a carbon atom,
while the alkyl groups contained in the foregoing definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

9. Substituted carboxylic acid amides of general formula I according to claim 1, wherein
A denotes a group of formula 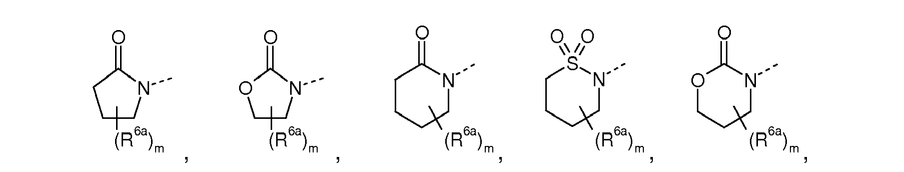 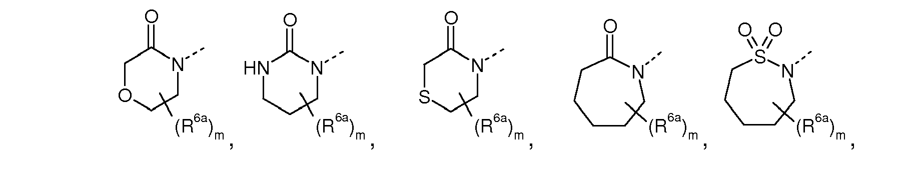 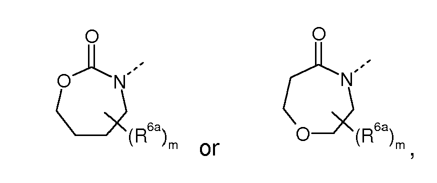 wherein
m denotes the number 1 or 2,
R^{6a} independently of one another denote a hydrogen or fluorine atom or a C₁₋₃-alkyl group, with the proviso that
in the above-mentioned substituted 5- to 7-membered cyclic groups A the fluorine atoms introduced as substituents are not separated from another heteroatom by precisely one carbon atom,
R¹ denotes a chlorine or bromine atom, a methyl or methoxy group, wherein the hydrogen atoms may be wholly or partly replaced by fluorine atoms, or a nitro group,
R² denotes a hydrogen atom,
R³ denotes a straight-chain or branched C₁₋₄-alkyl group wherein the hydrogen atoms may be wholly or partly replaced by fluorine atoms, and which is optionally substituted by a hydroxy, a C₁₋₄-alkyloxy group wherein the hydrogen atoms may be wholly or partly replaced by fluorine atoms, a C₁₋₃-alkylsulphanyl, C₁₋₃-alkylsulphonyl, carboxy or C₁₋₃-alkyloxycarbonyl group,
a furanyl, thiophenyl, pyrrolyl, pyrazolyl, imidazolyl, oxazolyl, isoxazolyl, thiazolyl, pyridinyl, pyrimidinyl, pyrazinyl, pyridinyl-C₁₋₂-alkyl or imidazolyl-C₁₋₂-alkyl group which may optionally be substituted in the heteroaryl moiety by one or two C₁₋₃-alkyl groups, C₁₋₃-alkyloxy groups, carboxy or C₁₋₃-alkyloxycarbonyl groups, and
R⁴ denotes a hydrogen atom,
R⁵ denotes a hydrogen atom and
B denotes a group of formula 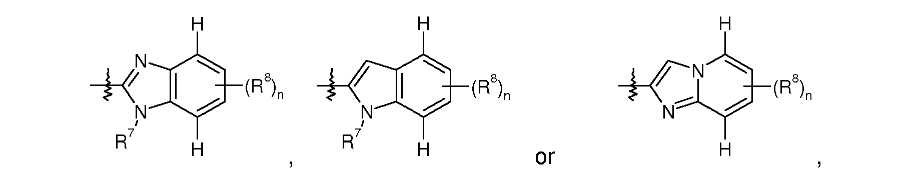 wherein
n denotes the number 1,
R⁷ denotes a hydrogen atom and
R⁸ denotes a chlorine or bromine atom or an ethynyl group,
while, unless otherwise stated, by the term "heteroaryl group" mentioned hereinbefore in the definitions is meant a monocyclic 5- or 6-membered heteroaryl group, while
the 6-membered heteroaryl group contains one, two or three nitrogen atoms and
the 5-membered heteroaryl group contains an imino group optionally substituted by a C₁₋₃-alkyl group, an oxygen or sulphur atom or
an imino group optionally substituted by a C₁₋₃-alkyl group or an oxygen or sulphur atom and additionally a nitrogen atom or
an imino group optionally substituted by a C₁₋₃-alkyl group and two or three nitrogen atoms,
and the bond is effected via a nitrogen atom or via a carbon atom,
while the alkyl groups contained in the foregoing definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

10. Substituted carboxylic acid amides of general formula I according to claim 9, wherein
A, R¹, R², R⁴, R⁵ and B are defined as in claim 9 and
R³ denotes a straight-chain or branched C₁₋₄-alkyl group wherein the hydrogen atoms may be wholly or partly replaced by fluorine atoms, and which is optionally substituted by a hydroxy, a C₁₋₄-alkyloxy group wherein the hydrogen atoms may be wholly or partly replaced by fluorine atoms, a C₁₋₃-alkylsulphanyl, C₁₋₃-alkylsulphonyl, carboxy or C₁₋₃-alkyloxycarbonyl group,
while the alkyl and alkoxy groups contained in the above-mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

11. Substituted carboxylic acid amides of general formula I according to claim 9, wherein
A, R¹, R², R⁴, R⁵ and B are defined as in claim 9 and
denote a furanyl, thiophenyl, pyrrolyl, pyrazolyl, imidazolyl, oxazolyl, isoxazolyl, thiazolyl, pyridinyl, pyrimidinyl, pyrazinyl, pyridinyl-C₁₋₂-alkyl or imidazolyl-C₁₋₂-alkyl group which may optionally be substituted in the heteroaryl moiety by one or two C₁₋₃-alkyl groups, wherein the hydrogen atoms may be wholly or partly replaced by fluorine atoms, C₁₋₃-alkyloxy groups, wherein the hydrogen atoms may be wholly or partly replaced by fluorine atoms, carboxy or C₁₋₃-alkyloxycarbonyl groups, and
unless otherwise stated, by the term "heteroaryl group" mentioned hereinbefore in the definitions is meant a monocyclic 5- or 6-membered heteroaryl group, while
the 6-membered heteroaryl group contains one, two or three nitrogen atoms and the 5-membered heteroaryl group contains an imino group optionally substituted by a C₁₋₃-alkyl group, an oxygen or sulphur atom or
an imino group optionally substituted by a C₁₋₃-alkyl group or an oxygen or sulphur atom and additionally a nitrogen atom or
an imino group optionally substituted by a C₁₋₃-alkyl group and two or three nitrogen atoms,
and the bond is effected via a nitrogen atom or via a carbon atom,
while the alkyl groups contained in the foregoing definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions may be wholly or partly replaced by fluorine atoms,
the tautomers, the enantiomers, the diastereomers, the mixtures thereof and the salts thereof.

12. Substituted carboxylic acid amides of general formula I according to one of claims 1 to 7 or 8, wherein the group X¹ denotes a methylene group.

13. Substituted carboxylic acid amides of general formula I according to one of claims 1 to 8, wherein the group X¹ denotes a carbonyl group.

14. Substituted carboxylic acid amides of general formula I according to one of claims 1 to 8, 12 or 13, wherein the group X³ denotes a methylene group.

15. Substituted carboxylic acid amides of general formula I according to one of claims 1 to 8, 12 or 13 wherein the group X³ denotes a carbonyl group.

16. Substituted carboxylic acid amides of general formula I according to one of claims 1 to 8, or 12 to 15, wherein the group X⁴ denotes an oxygen atom.

17. Substituted carboxylic acid amides of general formula I according to one of claims 1 to 16, wherein the group B denotes the group

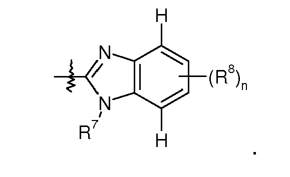

18. Substituted carboxylic acid amides of general formula I according to one of claims 1 to 16, wherein the group B denotes the group

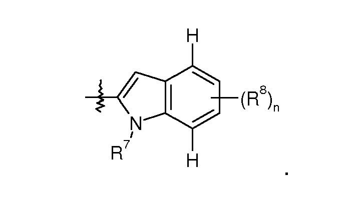

19. Substituted carboxylic acid amides of general formula I according to one of claims 1 to 16, wherein the group B denotes the group

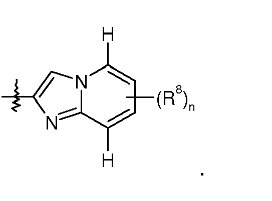

20. Substituted carboxylic acid amides of general formula I according to one of claims 1 to 19, wherein the group R⁸ denotes a chlorine atom.

21. Substituted carboxylic acid amides of general formula I according to one of claims 1 to 19, wherein the group R⁸ denotes a bromine atom.

22. Substituted carboxylic acid amides of general formula I according to one of claims 1 to 19, wherein the group R⁸ denotes an ethynyl group.

23. Substituted carboxylic acid amides of general formula I according to one of claims 1 to 22, which correspond to general formula la

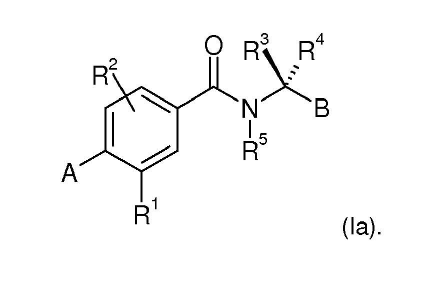

24. The following compounds of general formula I according to claim 1:
(1) 4-(azepan-2-on-1-yl)-*N*-[(1*R*)-1-(5-chloro-1*H*-benzimidazol-2-yl)-2-methoxy- ethyl]-3-methyl-benzam ide,
(2) 4-(azepan-2-on-1-yl)-*N*-[(1*R*)-1-(5-chloro-1*H*-benzimidazol-2-yl)-2-hydroxy- ethyl]-3-methyl-benzam ide,
(3) *N*-[(1*S*)-1-(5-chloro-1*H*-benzimidazol-2-yl)-3-methylsulphanyl-propyl]-3-
(4) methyl-4-(pyrrolidin-2-on-1-yl)-benzamide, *N*-[(1*R*)-1-(5-chloro-1*H*-benzimidazol-2-yl)-2-methoxy-ethyl]-4-(morpholin-3- on-4-yl)-3-trifluoromethyl-benzamide,
(5) *N*-[(1*R*)-1-(5-chloro-1*H*-benzimidazol-2-yl)-2-methoxy-ethyl]-3-methyl-4- (morpholin-3-on-4-yl)-benzamide,
(6) *N*-[(1*S*)-1-(5-chloro-1*H*-benzimidazol-2-yl)-3-methylsulphanyl-propyl]-3- methyl-4-(morpholin-3-on-4-yl)-benzamide,
(7) *N*-[(1*R*)-1-(5-chloro-1*H*-benzimidazol-2-yl)-2-hydroxy-ethyl]-3-methyl-4- (morpholin-3-on-4-yl)-benzamide,
(8) 4-(azepan-2-on-1-yl)-3-chloro-*N*-[(1*R*)-1-(5-chloro-1*H*-benzimidazol-2-yl)-2- methoxy-ethyl]-benzamide,
(9) 4-(azepan-2-on-1-yl)-*N*-[(1*R*)-1-(5-chloro-1*H*-benzimidazol-2-yl)-2-methoxy- ethyl]-3-trifluoromethyl-benzamide,
(10) *N*-[(1*S*)-1-(5-chloro-1*H*-benzimidazol-2-yl)-ethyl]-3-methyl-4-([1,3]oxazepan- 2-on-3-yl)-benzamide
(11) *N*-[(1*S*)-1-(5-chloro-1*H*-benzimidazol-2-yl)-3-methylsulphanyl-propyl]-4- (morpholin-3-on-4-yl)-3-nitro-benzamide,
(12) *N*-[(1*R*)-1-(5-chloro-1*H*-benzimidazol-2-yl)-2-methoxy-ethyl]-3-methyl-4- ([1,3]oxazepan-2-on-3-yl)-benzamide,
(13) 3-chloro-*N*-[(1*R*)-1-(5-chloro-1*H*-benzimidazol-2-yl)-2-methoxy-ethyl]-4- ([1,4]oxazepan-5-on-4-yl)-benzamide,
(14) 3-chloro-*N*-[(1*R*)-1-(5-chloro-1*H*-benzimidazol-2-yl)-2-methoxy-ethyl]-4- (piperidin-2-on-1-yl)-benzamide,
(15) *N*-[(1*R*)-1-(5-bromo-1*H*-benzimidazol-2-yl)-2-hydroxy-ethyl]-3-methyl-4- (morpholin-3-on-4-yl)-benzamide,
(16) *N*-[(1*R*,2*S*)-1-(5-chloro-1*H*-benzimidazol-2-yl)-2-methoxy-propyl]-3-methyl-4- (morpholin-3-on-4-yl)-benzamide,
(17) *N*-[(1*S*)-1-(5-chloro-1*H*-benzimidazol-2-yl)-ethyl]-3-methyl-4-(thiomorpholin-3- on-4-yl)-benzamide,
(18) *N*-[(1*R*)-1-(5-chloro-1*H*-benzimidazol-2-yl)-2-methoxy-ethyl]-3-methyl-4- (piperidin-2-on-1-yl)-benzamide,
(19) *N*-[(1*R*)-1-(5-chloro-1*H*-benzimidazol-2-yl)-2-methoxy-ethyl]-3-methyl-4-([1,3]- oxazinan-2-on-3-yl)-benzamide,
(20) *N*-[(1*R*)-1-(5-chloro-1*H*-benzimidazol-2-yl)-2-methoxy-ethyl]-4-(piperidin-2-on- 1-yl)-3-trifluoromethyl-benzamide,
(21) *N*-[(1*R*)-1-(5-chloro-1*H*-benzimidazol-2-yl)-2-methoxy-ethyl]-4-(1,1-dioxo- [1,2]thiazinan-2-yl)-3-methyl-benzamide,
(22) *N*-[(1*S*)-1-(5-chloro-1*H*-benzimidazol-2-yl)-butyl]-3-methyl-4-(morpholin-3-on- 4-yl)-benzamide,
(23) *N*-[(1*R*)-1-(5-chloro-1*H*-benzimidazol-2-yl)-2-methoxy-ethyl]-4- ([1,3]oxazepan-2-on-3-yl)-3-trifluoromethyl-benzamide,
(24) *N*-[(1*R*)-1-(5-chloro-1-*H*-benzimidazol-2-yl)-2-methoxy-ethyl]-4-(1,1-dioxo- [1,2,6]thiadiazinan-2-yl)-3-methyl-benzamide,
(25) *N*-[(1*R*)-1-(5-bromo-1*H*-benzimidazol-2-yl)-2-methoxy-ethyl]-3-methyl-4- (morpholin-3-on-4-yl)-benzamide,
(26) *N*-[(1*S*)-1-(5-chloro-1*H*-benzimidazol-2-yl)-3-(1*H*-tetrazol-5-yl)-propyl]-3- methyl-4-(morpholin-3-on-4-yl)-benzamide,
(27) *N*-[(1*S*)-1-(5-chloro-1*H*-benzimidazol-2-yl)-3-methoxy-propyl]-3-methyl-4- (morpholin-3-on-4-yl)-benzamide,
(28) *N*-[(1*R*)-1-(5-chloro-1*H*-benzimidazol-2-yl)-2-methylsulphanyl-ethyl]-3-methyl- 4-(morpholin-3-on-4-yl)-benzamide,
(29) *N*-[(1*R*)-1-(5-chloro-1*H*-benzimidazol-2-yl)-2-methoxy-ethyl]-3-methyl-4- (thiomorpholin-3-on-4-yl)-benzamide,
(30) 3-chloro-*N*-[(1*R*)-1-(5-chloro-1*H*-benzimidazol-2-yl)-2-methoxy-ethyl]-4- (morpholin-3-on-4-yl)-benzamide,
(31) 3-chloro-*N*-[(1*R*)-1-(5-chloro-1*H*-benzimidazol-2-yl)-2-methoxy-ethyl]-4-(1,1- dioxo-[1,2]thiazinan-2-yl)-benzamide,
(32) *N*-[(1*R*)-1-(5-chloro-1*H*-benzimidazol-2-yl)-2-methoxy-ethyl]-4-(1,1-dioxo- [1,2]thiazepan-2-yl)-3-methyl-benzamide,
(33) 3-bromo-*N*-[(1*S*)-1-(5-chloro-1*H*-benzimidazol-2-yl)-ethyl]-4-(piperidin-2-on-1- yl)-benzamide,
(34) 3-bromo-*N*-[(1*R*)-1-(5-chloro-1*H*-benzimidazol-2-yl)-2-methoxy-ethyl]-4- (piperidin-2-on-1-yl)-benzamide and
(35) *N*-[1-(5-chloro-1*H*-benzimidazol-2-yl)-1-(furan-2-yl)-methyl]-3-methyl-4- (piperidin-2-on-1-yl)-benzamide,
the tautomers, the enantiomers, the diastereomers, the mixtures thereof and the salts thereof are particularly preferred.

25. Physiologically acceptable salts of the compounds according to claims 1 to 24.

26. Medicament containing a compound according to at least one of claims 1 to 24 or a physiologically acceptable salt according to claim 25, optionally together with one or more inert carriers and/or diluents.

27. Use of a compound according to at least one of claims 1 to 24 or a physiologically acceptable salt according to claim 25, for preparing a medicament with an inhibitory effect on factor Xa and/or an inhibitory effect on related serine proteases.

28. Process for preparing a medicament according to claim 26, **characterised in that** a compound according to at least one of claims 1 to 24 or a physiologically acceptable salt according to claim 25 is incorporated in one or more inert carriers and/or diluents by a non-chemical method.

## Revendications

1. Amides d'acide carboxylique substitués de formule générale 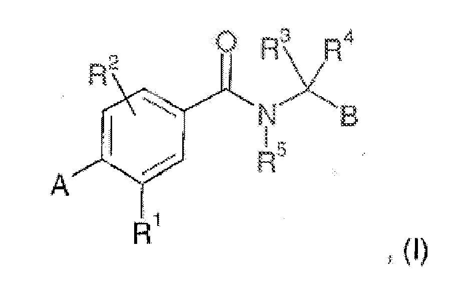 dans laquelle
A est un groupe de formule générale 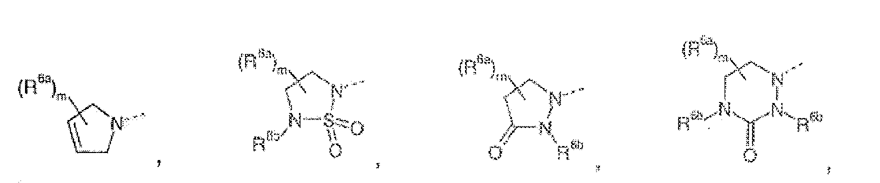 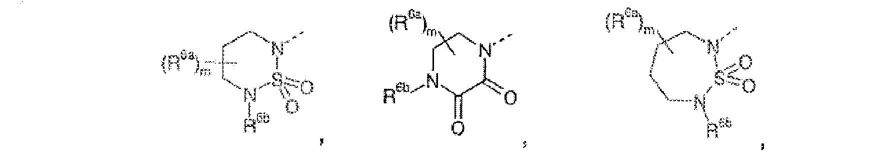 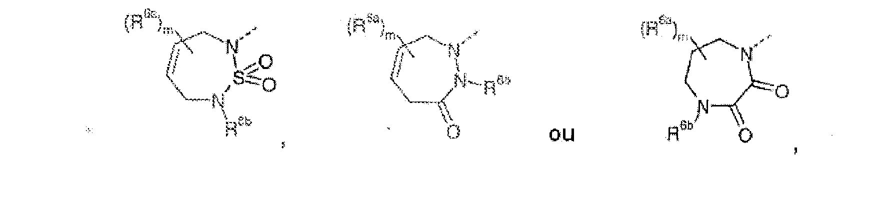 dans laquelle
m représente le nombre 1 ou 2,
les R^{6a}, indépendamment les uns des autres, représentent un atome d'hydrogène, de fluor, de chlore ou de brome ou un groupe alkyle en C₁ à C₃, hydroxy, amino, alkylamino en C₁ à C₃, di-(alkyle en C₁ à C₃)-amino, aminocarbonyle, alkylaminocarbonyle en C₁ à C₃, di-(alkyle en C₁ à C₃)-aminocarbonyle ou alkylcarbonylamino en C₁ à C₃, et
les R^{6b}, indépendamment les uns des autres, peuvent être un atome d'hydrogène, un groupe alkyle en C₁ à C₄, alkylcarbonyle en C₁ à C₄, alkyloxycarbonyle en C₁ à C₄ ou alkylsulfonyle en C₁ à C₃, à condition que, dans les groupes A de 5 à 7 chaînons substitués précédemment cités, les hétéroatomes servant éventuellement de substituants ne soient pas séparés d'un autre hétéroatome par exactement un atome de carbone, ou
un groupe de formule générale 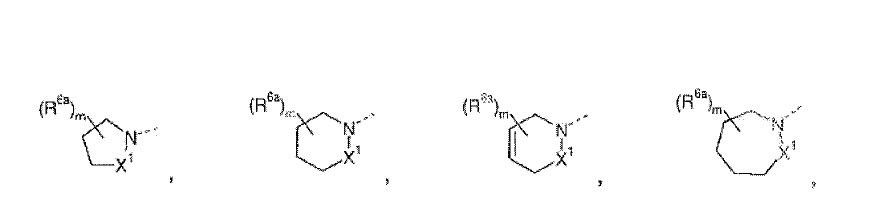 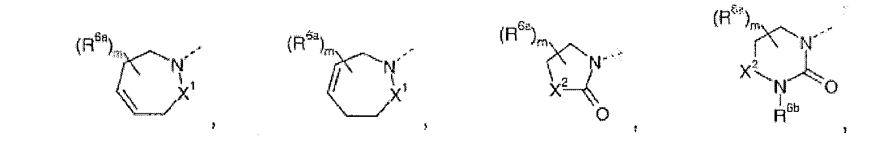 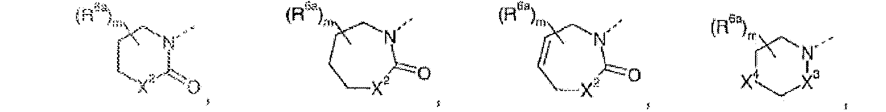 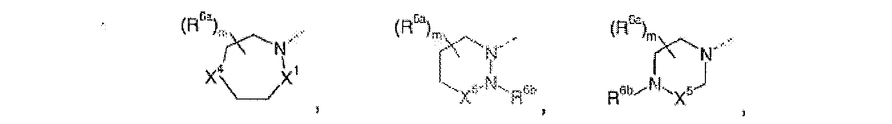 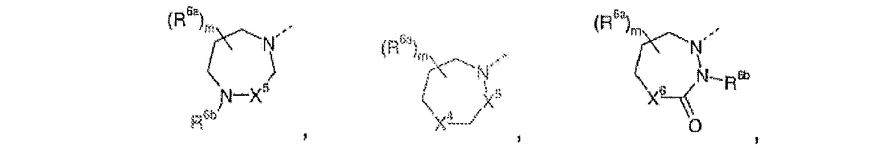 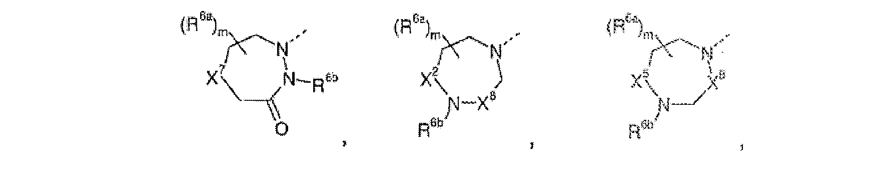 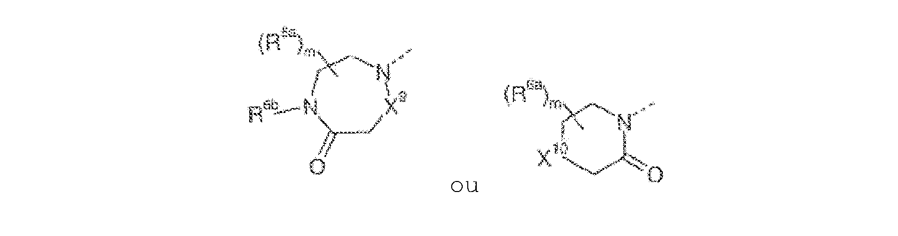 dans laquelle m représente le nombre 1 ou 2, X¹ représente un atome d'oxygène ou un groupe méthylène, -NR^{6b}-, carbonyle ou sulfonyle,
X² représente un atome d'oxygène ou un groupe - NR^{6b}-,
X³ est un groupe méthylène, carbonyle ou sulfonyle, X⁴ est un atome d'oxygène ou de soufre, un groupe NR^{6b} ou carbonyle,
X⁵ représente un groupe carbonyle ou sulfonyle,
X⁶ représente un atome d'oxygène, un groupe -NR^{6b}- ou méthylène,
X⁷ représente un atome d'oxygène ou de soufre ou un groupe -NR^{6b}-,
X⁸ représente un groupe méthylène ou carbonyle,
X⁹ représente un groupe -NR^{6b}- ou carbonyle,
X¹⁰ représente un groupe sulfinyle ou sulfonyle et les R^{6a} représentent, indépendamment les uns des autres, un atome d'hydrogène, de fluor, de chlore ou de brome ou un groupe alkyle en C₁ à C₃, hydroxy, amino, alkylamino en C₁ à C₃, di-(alkyle en C₁ à C₃)-amino, aminocarbonyle, alkylaminocarbonyle en C₁ à C₃, di-(alkyle en C₁ à C₃)-aminocarbonyle ou alkylcarbonylamino en C₁ à C₃, et
les R^{6b}, indépendamment les uns des autres, peuvent être un atome d'hydrogène, un groupe alkyle en C₁ à C₄, alkylcarbonyle en C₁ à C₄, alcoxycarbonyle en C₁ à C₄ ou alkylsulfonyle en C₁ à C₃, à condition que, dans les groupes A de 5 à 7 chaînons substitués précédemment cités, les hétéroatomes servant éventuellement de substituants ne soient pas séparés d'un autre hétéroatome par exactement un atome de carbone,
R¹ représente un atome d'hydrogène, de fluor, de chlore ou de brome, un groupe alkyle en C₁ à C₃ dans lequel les atomes d'hydrogène peuvent être remplacés totalement ou partiellement par des atomes de fluor, un groupe alcényle en C₂ à C₃, alcinyle en C₂ à C₃, nitro, amino, alcoxy en C₁ à C₃, un groupe mono-, di- ou trifluorométhoxy,
R² représente un atome d'hydrogène, de fluor, de chlore ou de brome ou un groupe alkyle en C₁ à C₃,
R³ représente un atome d'hydrogène, un groupe alcényle en C₂ à C₃ ou alcinyle en C₂ à C₃ ou un groupe alkyle en C₁ à C₆ à chaîne linéaire ou ramifiée dans lequel les atomes d'hydrogène peuvent être remplacés totalement ou partiellement par des atomes de fluor et qui est substitué éventuellement par un groupe nitrile, hydroxy, un groupe alkyloxy en C₁ à C₆, dans lequel les atomes d'hydrogène peuvent être remplacés totalement ou partiellement par des atomes de fluor, un groupe allyloxy, propargyloxy, benzyloxy, alkylcarbonyloxy- en C₁ à C₅, alkyloxycarbonyloxy en C₁ à C₅, carboxy-alkyloxy en C₁ à C₃, alkyloxycarbonle en C₁ à C₅, alkyloxy en C₁ à C₃, alkyloxycarbonylamino en C₁ à C₈, mercapto, alkylsulfanyle en C₁ à C₃, alkylsulfinyle en C₁ à C₃, alkylsulfonyle en C₁ à C₃, alkylcarbonylamino en C₁ à C₃-alkylsulfanyle en C₁ à C₃, alkylcarbonylamino en C₁ à C₃-alkylsulfinyle en C₁ à C₃, alkylcarbonylamino en C₁ à C₃, alkylsulfonyle en C₁ à C₃, carboxy, alkyloxycarbonyle en C₁ à C₃, allyloxycarbonyle, propargyloxycarbonyle, benzyloxycarbonyle, aminocarbonyle, alkylaminocarbonyle en C₁ à C₃, di-(alkyle en C₁ à C₃)-aminocarbonyle, cycloalkylène-iminocarbonyle en C₃ à C₆, aminosulfonyle, alkylaminosulfonyle en C₁ à C₃, di-(alkyle en C₁ à C₃)-aminosulfonyle, cycloalkylène-iminosulfonyle en C₃ à C₆, amino, alkylamino en C₁ à C₃, di-(alkyle en C₁ à C₃) - amino, alkylcarbonylamino en C₁ à C₅, alkylsulfonylamino en C₁ à C₃, N-(C-alkylsulfonyle en C₁ à C₃)-alkylamino en C₁ à C₃, cycloalkylcarbonylamino en C₃ à C₆, aminocarbonylamino, alkylaminocarbonylamino en C₁ à C₃, di-(alkyle en C₁ à C₃)-aminocarbonylamino, un groupe cycloalkylène-iminocarbonylamino de 4 à 7 chaînons, benzyloxycarbonylamino, phénylcarbonylamino ou guanidino,
un groupe carboxy, aminocarbonyle, alkylaminocarbonyle en C₁ à C₄, cycloalkylamino en C₃ à C₆, carbonyle, di-(alkyle en C₁ à C₃)-aminocarbonyle, alcoxycarbonyle en C₁ à C₄, cycloalkylène-iminocarbonyle en C₄ à C₆,
un groupe phényle ou hétéroaryle, phénylcarbonyle-alkyle en C₁ à C₃, phényl-alkyle en C₁ à C₃ ou hétéroaryl-alkyle en C₁ à C₃ qui est substitué dans la partie phényle ou hétéroaryle, éventuellement une ou plusieurs fois par des atomes de fluor, de chlore ou de brome, un groupe alkyle en C₁ à C₃, amino, alkylamino en C₁ à C₃, di-(alkyle en C₁ à C₃)amino, hydroxy, alkyloxy en C₁ à C₄, mono-, di- ou trifluorométhoxy, benzyloxy, carboxy, alkyloxy en C₁ à C₃, alkyloxycarbonyle en C₁ à C₃-alkyloxy en C₁ à C₃, aminocarbonyle-alkyloxy en C₁ à C₃, alkylaminocarbonyle en C₁ à C₃-alkyloxy en C₁ à C₃, di-(alkyle en C₁ à C₃)-aminocarbonyl-alkyloxy en C₁ à C₃, un groupe cycloalkylène-iminocarbonyle de 4 à 7 chaînons-alcoxy en C₁ à C₃, carboxy, alkyloxycarbonyle en C₁ à C₃ ou alkyloxy en C₁ à C₃-carbonylamino,
un groupe cycloalkyle de 3 à 7 chaînons, cycloalkylène-imino, cycloalkyl-alkyle en C₁ à C₃ ou cycloalkylène-imino-alkyle en C₁ à C₃, dans lequel dans la partie cyclique, un groupe méthylène peut être remplacé par un groupe -NH- éventuellement substitué par un groupe alkyle en C₁ à C₃ ou alkylcarbonyle en C₁ à C₃, ou un atome d'oxygène et dans lequel en outre, un groupe méthylène voisin d'un groupe -NH-, - N(alkylcarbonyle en C₁ à C₃) ou -N(alkyle en C₁ à C₃) peut être remplacé respectivement par un groupe carbonyle ou sulfonyle, à condition qu'un groupe cycloalkylène-imino tel que défini ci-dessus dans lequel deux atomes d'azote sont séparés l'un de l'autre par exactement un groupe -CH₂- soit exclus,
R⁴ est un atome d'hydrogène ou un groupe alkyle en C₁ à C₅, ou
R³ et R⁴ conjointement avec l'atome de carbone auquel ils sont liés représentent un groupe cycloalkyle en C₃ à C₇, dans lequel
l'un des groupes méthylène du groupe cycloalkyle en C₃ à C₇ peut être remplacé par un groupe imino, alkylimino en C₁ à C₃, acylimino ou sulfonylimino,
R⁵ représente un atome d'azote ou un groupe alkyle en C₁ à C₃,
B représente un groupe de formule 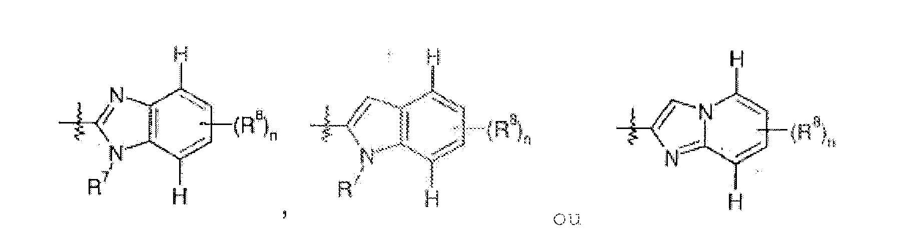 dans laquelle n représente le nombre 1 ou 2,
R⁷ représente un atome d'hydrogène ou un groupe alkyle en C₁ à C₃, hydroxy, alkyloxycarbonyle en C₁ à C₅, carboxy-alkyle en C₁ à C₃, alkyloxycarbonyle en C₁ à C₃-alkyle en C₁ à C₃, amino ou alkylamino en C₁ à C₃ et
les R⁸ indépendamment les uns des autres représentent un atome d'hydrogène, de fluor, de chlore, de brome ou d'iode, un groupe alkyle en C₁ à C₃ dans lequel les atomes d'hydrogènes peuvent être remplacés totalement ou partiellement par des atomes de fluor, un groupe alcényle en C₂ à C₃ ou alcinyle en C₂ à C₃, un groupe hydroxy, alcoxy en C₁ a C₃, trifluorométhoxy, amino, nitrol ou nitrile,
où sauf indication contraire, l'expression « groupe hétéroaryle » mentionné dans les définitions précédentes désigne un groupe hétéroaryle monocyclique de 5 ou 6 chaînons,
le groupe hétéroaryle à 6 chaînons comportant deux ou trois atomes d'azote, et
le groupe hétéroaryle à 5 chaînons comporte un groupe imino éventuellement substitué par un groupe alkyle en C₁ à C₃, phényle ou phényl-alkyle en C₁ à C₃, un atome d'oxygène ou de soufre, ou
un groupe imino substitué éventuellement par un groupe alkyle en C₁ à C₃, phényle, amino-alkyle en C₂ à C₃, alkylamino en C₁ à C₃-alkyle en C₂ à C₃, di-(alkyle en C₁ à C₃)-amino-alkyle en C₂ à C₃, un groupe cycloalkylène-mino-alkyle en C₁ à C₃ou phényl-alkyle en C₁ à C₃ de 4 à 7 chaînons ou un atome d'oxygène ou de soufre et éventuellement un atome d'azote, ou
un groupe imino éventuellement substitué par un groupe alkyle en C₁ à C₃ ou phényl-alkyle en C₁ à C₃ et deux ou trois atomes d'azote,
et en outre un cycle phényle substitué éventuellement par un atome de fluor, de chlore ou de brome, un groupe alkyle en C₁ à C₃, hydroxy, alkyloxy en C₁ à C₃, amino, a lkylamino en C₁ à C₃, di-(alkyle en C₁ à C₃) -amino ou cycloalkylène-imino en C₃ à C₆ peut être condensé aux groupes hétéroaryle monocyclique mentionnés précédemment par deux atomes de carbone voisins,
et la liaison est réalisée par un atome d'azote ou par un atome de carbone de la partie hétérocyclique ou d'un cycle phényle condensé,
où les groupes alkyle et alcoxy contenus dans les définitions précédentes qui présentent plus de deux atomes de carbone, sauf indication contraire, peuvent être à chaîne linéaire ou ramifiée et les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels.

2. Amides d'acide carboxylique substitués de formule générale 1 selon la revendication 1, dans laquelle
A représente un groupe de formule générale 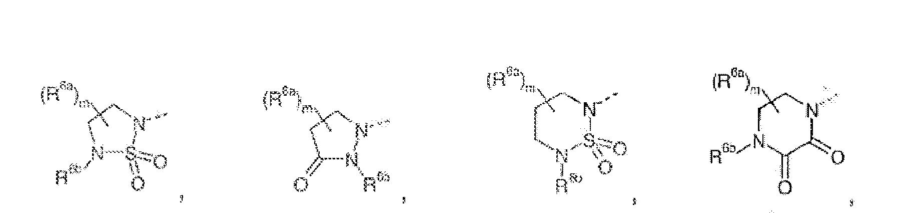 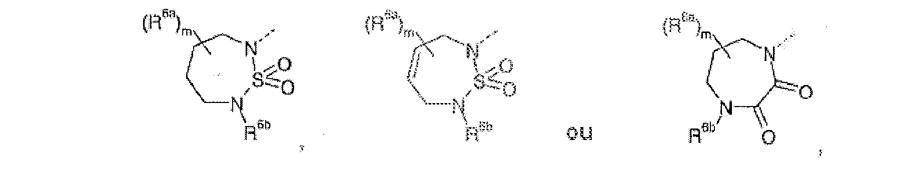 dans laquelle
m représente le nombre 1 ou 2,
les R^{6a}, Indépendamment les uns des autres, représentent un atome d'hydrogène ou de fluor, un groupe alkyle en C₁ à C₃, hydroxy, amino, alkylamino en C₁ à C₃, di-(alkyle en C₁ à C₃)-amino, aminocarbonyle, alkylaminocarbonyle en C₁ à C₃, di- (alkyle en C₁ à C₃) - aminocarbonyle ou alkylcarbonylaminc en C₁ à C₃, et
les R^{6b}, indépendamment les uns des autres, peuvent être un atome d'hydrogène, un groupe alkyle en C₁ à C₄, alkylcarbonyle en C₁ à C₄, alkyloxycarbonyle en C₁ à C₄ ou alkylsulfonyle en C₁ à C₃, à condition que, dans les groupes A de 5 à 7 chaînons substitués précédemment cités, les hétéroatomes servant éventuellement de substituants ne soient pas séparés d'un autre hétéroatome par exactement un atome de carbone, ou
un groupe de formule générale 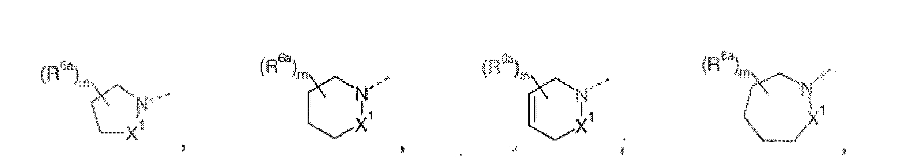 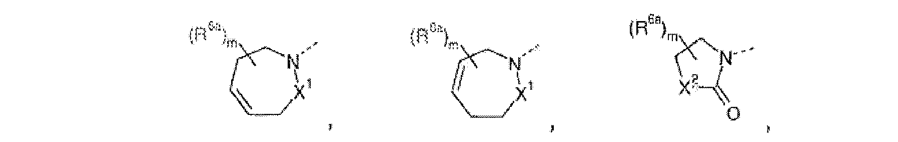 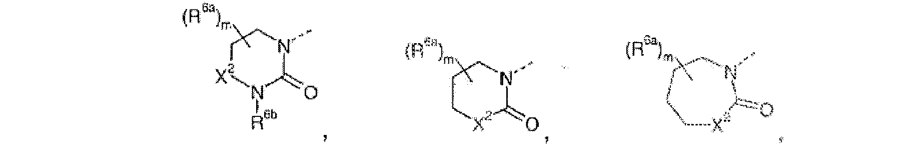 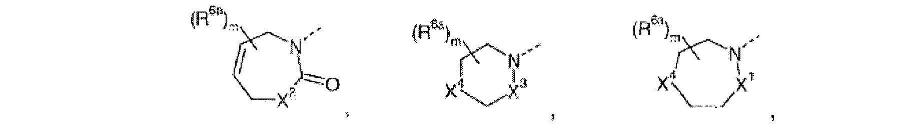 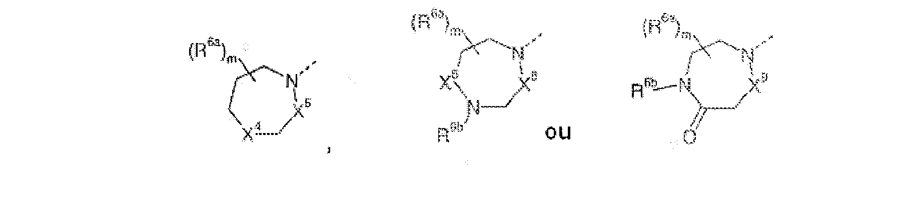 dans laquelle m représente le nombre 1 ou 2, X¹ représente un groupe méthylène, -NR^{6b}-, carbonyle ou sulfonyle,
X² représente un atome d'oxygène ou un groupe - NR^{6b}-,
X³ représente un groupe méthylène, carbonyle ou sulfonyle,
X⁴ représente un atome d'oxygène ou de soufre, un groupe NR^{6b} ou carbonyle,
X⁵ représente un groupe carbonyle ou sulfonyle,
X⁸ représente un groupe carbonyle,
X⁹ représente un groupe carbonyle,
les R^{6a} représentent, indépendamment les uns des autres, un atome d'hydrogène ou de fluor, un groupe alkyle en C₁ à C₃, hydroxy, amino, alkylamino en C₁ à C₃, di-(alkyle en C₁ à C₃) -amino, aminocarbonyle, alkylaminocarbonyle en C₁ à C₃, di-(alkyle en C₁ à C₃) - aminocarbonyle ou alkylcarbonylamino en C₁ à C₃, et
les R^{6b}, indépendamment les uns des autres, peuvent être un atome d'hydrogène, un groupe alkyle en C₁ à C₄, alkylcarbonyle en C₁ à C₄, alcoxycarbonyle en C₁ à C₄ ou alkylsulfonyle en C₁ à C₃, à condition que, dans les groupes A cycliques de 5 à 7 chaînons substitués précédemment cités, les hétéroatomes servant éventuellement de substituants ne soient pas séparés d'un autre hétéroatome par exactement un atome de carbone,
R¹ représente un atome d'hydrogène, de fluor, de chlore ou de brome, un groupe alkyle en C₁ à C₃ dans lequel les atomes d'hydrogène peuvent être remplacés totalement ou partiellement par des atomes de fluor, un groupe alcényle en C₂ à C₃, alcinyle en C₂ à C₃, nitro, amino, alcoxy en C₁ à C₃, un groupe mono-, di- ou trifluorométhoxy,
R² représente un atome d'hydrogène, de fluor, de chlore ou de brome ou un groupe alkyle en C₁ à C₃,
R³ représente un groupe alcényle en C₂ à C₃ ou alcinyle en C₂ à C₃ ou un groupe alkyle en C₁ à C₆ à chaîne linéaire ou ramifiée dans lequel les atomes d'hydrogène peuvent être remplacés totalement ou partiellement par des atomes de fluor et qui est substitué éventuellement par un groupe nitrile, hydroxy, alkyloxy en C₁ à C₈, dans lequel les atomes d'hydrogène peuvent être remplacés totalement ou partiellement par des atomes de fluor, un groupe allyloxy, propargyloxy, benzyloxy, alkylcarbonyloxy- en C₁ a C₅, alkyloxycarbonyloxy en C₁ à C₅, carboxy-alkyloxy en C₁ à C₃, alkyloxycarbonyle en C₁ à C₅-alkyloxy en C₁ à C₃, alkyloxycarbonylamino en C₁ à C₈, mercapto, alkylsulfanyle en C₁ à C₃, alkylsulfinyle en C₁ à C₃, alkylsulfonyle en C₁ à C₃, alkylcarbonylamino en C₁ à C₃-alkylsulfanyle en C₁ à C₃, alkylcarbonylamino en C₁ à C₃-alkylsulfinyle en C₁ à C₃, alkylcarbonylamino en C₁ à C₃-alkylsulfonyle en C₁ à C₃, carboxy, alkyloxycarbonyle en C₁ à C₃, allyloxycarbonyle, propargyloxycarbonyle, benzyloxycarbonyle, aminocarbonyle, alkylaminocarbonyle en C₁ à C₃, di-(alkyle en C₁ à C₃)-aminocarbonyle, cycloalkylène-iminocarbonyle en C₃ à C₆, aminosulfonyle, alkylaminosulfonyle en C₁ à C₃, di-(alkyle en C₁ à C₃) - aminosulfonyle, cycloalkylène-iminosulfonyle en C₃ à C₆, amino, alkylamino en C₁ à C₃, di-(alkyle en C₁ à C₃) - amino, alkylcarbonylamino en C₁ à C₅, alkylsulfonylamino en C₁ à C₃, N-(alkylsulfonyle en C1 à C₃)-alkylamino en C₁ à C₃, cycloalkylcarbonylamino en C₃ à C₆, aminocarbonylamino, alkylaminocarbonylamino en C₁ à C₃, di-(alkyle en C₁ à C₃)-aminocarbonylamino, un groupe cycloalkylène-iminocarbonylamino de 4 à 7 chaînons, benzyloxycarbonylamino, phénylcarbonylamino ou guanidino,
un groupe carboxy, aminocarbonyle, alkylaminocarbonyle en C₁ à C₄, cycloalkylamino en C₃ à C₆, carbonyle, di-(alkyle en C₁ à C₃)-aminocarbonyle, alcoxycarbonyle en C₁ à C₄, cycloalkylène-iminocarbonyle en C₄ à C₆,
un groupe phényle ou hétéroaryle, phénylcarbonyle-alkyle en C₁ à C₃, phényl-alkyle en C₁ à C₃ ou hétéroaryl-alkyle en C₁ à C₃ qui est substitué dans la partie phényle ou hétéroaryle, éventuellement une ou plusieurs fois par des atomes de fluor, de chlore ou de brome, un groupe alkyle en C₁ à C₃, amino, alkylamino en C₁ à C₃, di-(alkyle en C₁ à C₃) amino, hydroxy, alkyloxy en C₁ à C₄, mono-, di- ou trifluorométhoxy, benzyloxy, carboxy, alkyloxy en C₁ à C₃, alkyloxycarbonyle en C₁ à C₃-alkyloxy en C₁ à C₃, aminocarbonyle-alkyloxy en C₁ à C₃, alkylaminocarbonyle en C₁ à C₃-alkyloxy en C₁ à C₃, di-(alkyle en C₁ à C₃) - aminocarbonyl-alkyloxy en C₁ à C₃, un groupe cycloalkylène-iminocarbonyle de 4 à 7 chaînons-alcoxy en C₁ à C₃, carboxy, alkyloxycarbonyle en C₁ à C₃ ou alkyloxy en C₁ à C₃-carbonylamino,
un groupe cycloalkyle de 3 à 7 chaînons, cycloalkylène-imino, cycloalkyl-alkyle en C₁ à C₃ ou cycloalkylène-imino-alkyle en C₁ à C₃, dans lequel dans la partie cyclique, un groupe méthylène peut être remplacé par un groupe -NH- éventuellement substitué par un groupe alkyle en C₁ à C₃ ou alkylcarbonyle en C₁ à C₃, ou un atome d'oxygène et dans lequel en outre, un groupe méthylène voisin d'un groupe -NH-, N (alkylcarbonyle en C₁ à C₃) ou -N (alkyle en C₁ à C₃) peut être remplacé respectivement par un groupe carbonyle ou sulfonyle, a condition qu'un groupe cycloalkylène-imino tel que défini ci-dessus dans lequel deux atomes d'azote sont séparés l'un de l'autre par exactement un groupe -CH₂- soit exclus,
R⁴ représente un atome d'hydrogène ou un groupe alkyle en C₁ à C₃, ou
R⁵ représente un atome d' hydrogène ou un groupe alkyle en C₁ à C₃,
B représente un groupe de formule 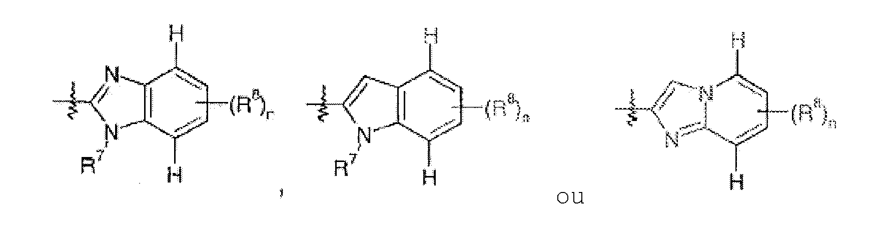 dans laquelle
n représente le nombre 1 ou 2,
R⁷ représente un atome d'hydrogène, un groupe alkyle en C₁ à C₃, hydroxy, alkyloxycarbonyle en C₁ à C₅, carboxy-alkyle en C₁ à C₃, alkyloxycarbonyle en C₁ à C₃- alkyle en C₁ à C₃, amino ou alkylamino en C₁ à C₃ et
les R⁸ indépendamment les uns des autres représentent un atome d'hydrogène, de fluor, de chlore, de brome ou d'iode, un groupe alkyle en C₁ à C₃ dans lequel les atomes d'hydrogènes peuvent être remplacés totalement ou partiellement par des atomes de fluor, un groupe alcényle en C₂ à C₃ ou alcinyle en C₂ à C₃, un groupe hydroxy, alkoxy en C₁ à C₃, trifluorométhoxy, amino, nitro ou nitrile,
où sauf indication contraire, l'expression « groupe hétéroaryle » mentionnée dans les définitions précédentes désigne un groupe hétéroaryle monocyclique de 5 ou 6 chaînons,
le groupe hétéroaryle à 6 chaînons comportant un, deux ou trois atomes d'azote, et
le groupe hétéroaryle à 5 chaînons comporte un groupe imino éventuellement substitué par un groupe alkyle en C₁ à C₃, phényle ou phényl-alkyle en C₁ à C₃, un atome d'oxygène ou de soufre, ou
un groupe imino substitué éventuellement par un groupe alkyle en C₁ à C₃, phényle, amino-alkyle en C₂ à C₃-alkylamino-alkyle, en C₁ à C₃-alkyle en C₂ à C₃, di-(alkyle en C₁ à C₃)-amino-alkyle en C₂ à C₃, un groupe cycloalkylène-mino-alkyle en C₁ à C₃ ou phényl-alkyle en C₁ à C₃ de 4 à 7 chaînons ou un atome d'oxygène ou de soufre et éventuellement un atome d'azote, ou
un groupe imino éventuellement substitué par un groupe alkyle en C₁ à C₃ ou phényl-alkyle en C₁ à C₃ et deux ou trois atomes d'azote,
et en outre un cycle phényle substitué éventuellement par un atome de fluor, de chlore ou de brome, un groupe alkyle en C₁ à C₃, hydroxy, alkyloxy en C₁ à C₃, amino, alkylamino en C₁ à C₃, di-(alkyle en C₁ à C₃)-amino ou cycloalkylène-imino en C₃ à C₆ peut être condensé aux groupes hétèroaryle monocycliques mentionnés précédemment par deux atomes de carbone voisins,
et à liaison est réalisée par un atome d'azote ou par un atome de carbone de la partie hétérocyclique ou d'un cycle phényle condensé,
où les groupes alkyle et alkoxy contenus dans les définitions précédentes qui présentent plus de deux atomes de carbone, sauf indication contraire, peuvent être à chaîne linéaire ou ramifiée et, les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels,

3. Amides d'acide carboxylique substitués de formule générale I selon la revendication 1, dans laquelle
A représente un groupe de formule générale 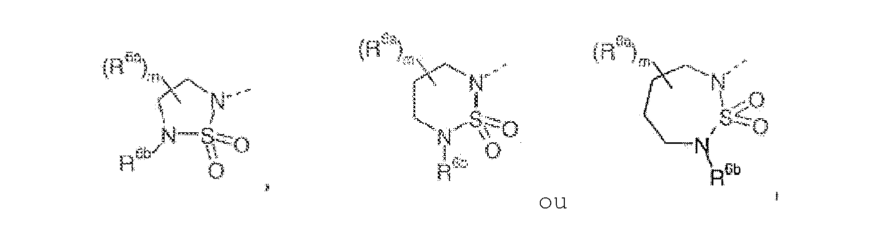 dans laquelle
m représente le nombre 1 ou 2,
les R^{6a}, indépendamment les uns des autres, représentent un atome d'hydrogène ou de fluor, un groupe alkyle en C₁ à C₃, hydroxy, amino, alkylamino en C₁ à C₃, di-(alkyle en C₁ à C₃)-aminio, aminocarbonyle, alkylamrnocarbonyle en C₁ à C₃, di-(alkyle en C₁ à C₃)-aminocarbonyle ou alkylcarbonylamino en C₁ à C₃, et
les R^{6b}, indépendamment les uns des autres, peuvent être un atome d'hydrogène, un groupe alkyle en C₁ à C₄, alkylcarbonyle en C₁ à C₄, alkyloxycarbonyle en C₁ à C₄ ou alkylsulfonyle en C₁ à C₃, à condition que, dans les groupes A de 5 à 7 chaînons substitués précédemment cités, les hétéroatomes servant éventuellement de substituants ne soient pas séparés des autres hétéroatomes par exactement un atome de carbone, ou
un groupe de formule générale 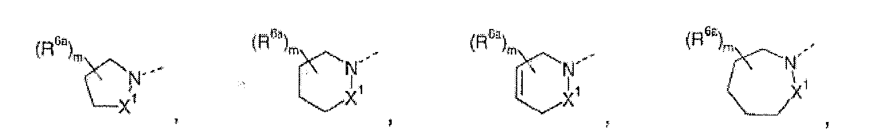 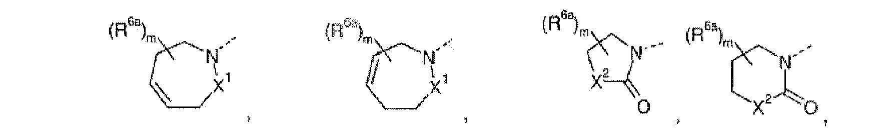 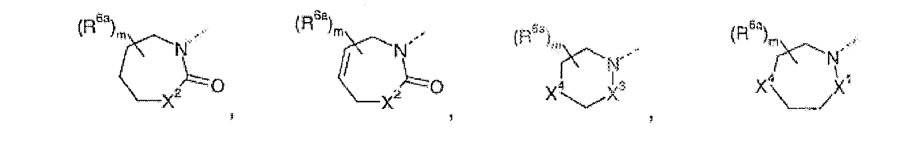 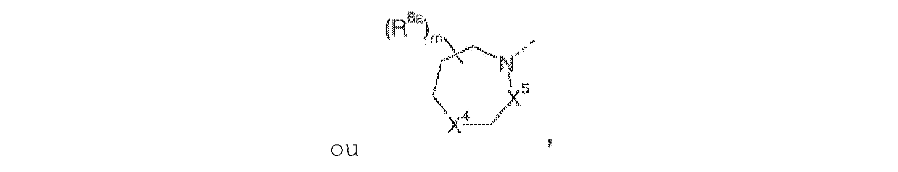 dans laquelle m représente le nombre 1 ou 2, X¹ représente un groupe méthylène, -NR^{6b}-, carbonyle ou sulfonyle,
X² représente un atome d'oxygène ou un groupe - NR^{6b}-,
X³ représente un groupe méthylène, carbonyle ou sulfonyle,
X⁴ représente un atome d'oxygène ou de soufre, un groupe NR^{6b},
X⁵ représente un groupe carbonyle ou sulfonyle, les R^{6a}, indépendamment les uns des autres, représentent un atome d'hydrogène ou de fluor, un groupe alkyle en C₁ à C₃, hydroxy, amino, alkylamino en C₁ à C₃, di-(alkyle en C₁ à C₃) -amino, aminocarbonyle, alkylaminocarbonyle en C₁ à C₃, di-(alkyle en C₁ à C₃) - aminocarbonyle ou alkylcarbonylamino en C₁ à C₃, et
les R^{6b}, indépendamment les uns des autres, peuvent être un atome d'hydrogène, un groupe alkyle en C₁ à C₄ alkylcarbonyle en C₁ à C₄, alkyloxycarbonyle en C₁ à C₄ ou alkylsulfonyle en C₁ à C₃, à condition que, dans les groupes A de 5 à 7 chaînons substitués précédemment cités, les hétéroatomes servant éventuellement de substituants ne soient pas séparés des autres hétéroatomes par exactement un atome de carbone, ou
R¹ représente un atome de fluor, de chlore ou de brome, un groupe alkyle en C₁ à C₃ dans lequel les atomes d'hydrogène peuvent être remplacés totalement ou partiellement par des atomes de fluor, un groupe nitro, alkoxy en C₁ à C₃, un groupe mono-, di- ou trifluorométhoxy,
R² représente un atome d'hydrogène,
R³ représente un groupe alkyle en C₁ à C₆ à chaîne linéaire ou ramifiée dans lequel les atomes d'hydrogène peuvent être remplacés totalement ou partiellement par des atomes de fluor et qui est substitué éventuellement par un groupe nitrile, hydroxy, benzyloxy, un groupe alkyloxy en C₁ à C₅, dans lequel les atomes d'hydrogène peuvent être remplacés totalement ou partiellement par des atomes de fluor, un groupe allyloxy, alkylcarbonyloxy- en C₁ à C₅, alkyloxycarbonyloxy en C₁ à C₅, carbonxy-alkyloxy en C₁ à C₃, alkyloxycarbonyle en C₁ à C₅-alkyloxy en C₁ à C₃, alkyloxycarbonylamino en C₁ à C₈, alkylsulfanyle en C₁ à C₃, alkylsulfonyle en C₁ à C₃, carboxy, alkyloxycarbonyle en C₁ à C₃, alkylaminocarbonyle en C1 à C3, di-(alkyle en C₁ à C₃)-aminocarbonyle, cycloalkylène-iminocarbonyle en C₃ à C₆, aminocarbonylamino, alkylaminocarbonylamino en C₁ à C₃ ou di-(alkyle en C₁ à C₃)-aminocarbonylamino,
un groupe aminocarbonyle, alkylaminocarbonyle en C₁ à C₄, cycloalkylaminocarbonyle en C₃ à C₆ ou un groupe di-(alkyle en C₁ à C₃)-aminocarbonyle,
un groupe phényle ou hétéroaryle, phényl-alkyle en C₁ à C₃, ou hétéroaryl-alkyle en C₁ à C₃ qui est substitué dans la partie phényle ou hétéroaryle, éventuellement une ou plusieurs fois par des atomes de fluor, de chlore ou de brome, un groupe alkyle en C₁ à C₃, amino, alkylamino en C₁ à C₃, di-(alkyle en C₁ à C₃)amino, hydroxy, alkyloxy en C₁ à C₄, mono-, di- ou trifluorométhoxy, carboxy ou alkyloxycarbonyle en C₁ à C₃,
un groupe cycloalkyl de 3 à 7 chaînons, dans lequel dans la partie cyclique, un groupe méthylène peut être remplacé éventuellement par un groupe -NH- éventuellement substitué par un groupe alkyle en C₁ à C₃ ou alkylcarbonyle en C₁ à C₃, ou un atome d'oxygène,
R⁴ représente un atome d' hydrogène,
R⁵ représente un atome d'hydrogène,
B représente un groupe de formule 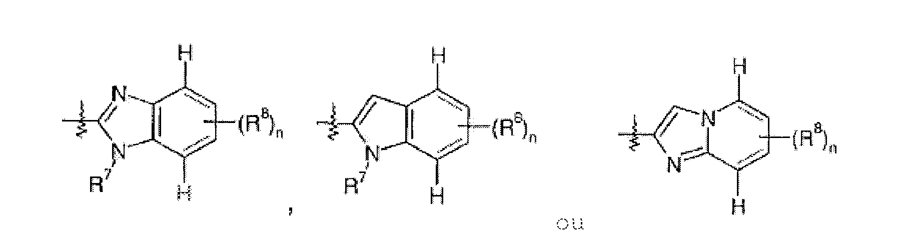 dans laquelle
n représente le nombre 1,
R⁷ représente un atome d'hydrogène, et
les R⁸ représentent un atome d'hydrogène, de fluor, de chlore, de brome ou d'iode, un groupe méthyle, alcinyle en C₂ à C₃, ou un groupe méthoxy dans lesquels les atomes d'hydrogène peuvent être remplacés totalement ou partiellement par des atomes de fluor,
où sauf indication contraire, l'expression « groupe hétéroaryle » mentionnée dans les définitions précédentes désigne un groupe hétéroaryle monocyclique de 5 ou 6 chaînons,
le groupe hétéroaryle à 6 chaînons comportant deux ou trois atomes d'azote, et
le groupe hétéroaryle à 5 chaînons comporte un groupe imino éventuellement substitué par un groupe alkyle en C₁ à C₃, un atome d'oxygène ou de soufre, ou
un groupe imino substitué éventuellement par un groupe alkyle en C₁ à C₃, ou un atome d'oxygène ou de soufre et en outre, un atome d'azote, ou
un groupe imino éventuellement substitué par un groupe alkyle en C₁ à C₃ ou deux ou trois atomes d'azote,
et la liaison est réalisée par un atome d'azote ou par un atome de carbone,
où les groupes alkyle et alkoxy contenus dans les définitions précédemment citées qui présentent plus de deux atomes de carbone, sauf indication contraire, peuvent être à chaîne linéaire ou ramifiée et les groupes alkyle dans les radicaux dialkylés précédemment cités., par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels.

4. Amides d'acide carboxylique substitués de formule générale I selon la revendication 3, dans laquelle
A, R¹, R², R⁴, R⁵ et B sont tels que définis dans la revendication 3, et
R³ représente un groupe alkyle en C₁ à C₆ à chaîne linéaire ou ramifiée dans lequel les atomes d'hydrogène peuvent être remplacés totalement ou partiellement par des atomes de fluor et qui est substitué éventuellement par un groupe nitrile, hydroxy, benzyloxy, un groupe alkyloxy en C₁ à C₅, dans lequel les atomes d'hydrogène peuvent être remplacés totalement ou partiellement par des atomes de fluor, un groupe allyloxy, alkylcarbonyloxy- en C₁ à C₅, alkyloxycarbonyloxy en C₁ à C₅, carbonxy-alkyloxy en C₁ à C₃, alkyloxycarbonyle en C₁ à C₅-alkyloxy en C₁ à C₃, alkyloxycarbonylamino sen C₁ à C₈, alkylsulfanyle en C₁ à C₃, alkylsulfonyle en C₁ à C₃, carboxy, alkyloxycarbonyle en C₁ à C₁ alkylaminocarbonyle en C₁ à C₃, di-(alkyle en C₁ à C₃) - aminocarbonyle, cycloalkylène-iminocarbonyle en C₃ à C₆, aminocarbonylamino, alkylaminocarbonylamino en C₁ à C₃ ou di-(alkyle en C₁ à C₃)-aminocarbonylamino,
un groupe aminocarbonyle, alkylaminocarbonyle en C₁ à C₄, cycloalkylaminocarbonyle en C₃ à C₆ ou un groupe di-(alkyle en C₁ à C₃)-aminocarbonyle,
un groupe alkyle et alkoxy contenus dans les définitions précédemment citées, sauf indication contraire, peuvent être à chaîne linéaire ou ramifiée et les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels.

5. Amides d'acide carboxylique substitués de formule générale I selon la revendication 3, dans laquelle
A, R¹, R², R⁴ R⁵ et B sont tels que définis dans la revendication 3, et
R³ représente un groupe phényle ou hétéroaryle, phényl-alkyle en C₁ à C₃ ou hétéro-aryl-alkyle en C₁ à C₃ qui est mono- ou poly-substitué dans la partie phényle ou hétéroaryle éventuellement par un atome de fluor, de chlore ou de brome, un groupe alkyle en C₁ à C₃, amino, alkylamino en C₁ à C₃, di-(alkyle en C₁ à C₃) -amino, hydroxy, alkyloxy en C₁ à C₁, mono-, di- ou trifluorométhoxy, carboxy ou alkyloxycarbonyle en C₁ à C₃,
un groupe cycloalkyle de 3 à 7 chaînons dans lequel, dans la partie cyclique, un groupe méthylène peut être substitué par un groupe --NH éventuellement substitué par un groupe alkyle en C₁ à C₃ ou alkylcarbonyle en C₁ à C₃ ou un atome d'oxygène,
où, sauf indication contraire, l'expression « groupe hétéroaryle » mentionnée dans les définitions précédentes désigne un groupe hétéroaryle monocyclique de 5 ou 6 chaînons,
le groupe hétéroaryle à 6 chaînons comportant deux ou trois atomes d'azote, et
le groupe hétéroaryle à 5 chaînons comporte un groupe imino éventuellement substitué par un groupe alkyle en C₁ à C₃, un atome d'oxygène ou de soufre, ou
un groupe imino substitué éventuellement par un groupe alkyle en C₁ à C₃, ou un atome d'oxygène ou de soufre et en outre, un atome d'azote, ou
un groupe imino éventuellement substitué par un groupe alkyle en C₁ à C₃ ou deux ou trois atomes d'azote,
et la liaison est réalisée par un atome d'azote ou par un atome de carbone,
où les groupes alkyle contenus dans les définitions précédemment citées qui présentent plus de deux atomes de carbone, sauf indication contraire, peuvent être à chaîne linéaire ou ramifiée et les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels.

6. Amides d'acide carboxylique substitués de formule générale I selon la revendication 1, dans laquelle
A représente un groupe de formule générale 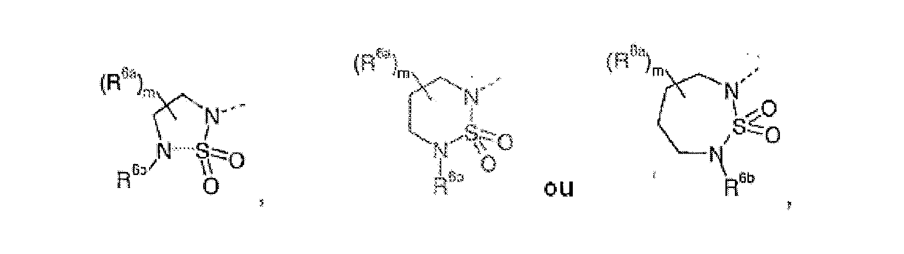 dans laquelle
m représente le nombre 1 ou 2,
les R^{6a}, indépendamment les uns des autres, représentent un atome d'hydrogène ou de fluor, un groupe alkyle en C₁ à C₃, et
les R^{6b} peuvent être un atome d'hydrogène ou un groupe alkyle en C₁ à C₃, à condition que, dans les groupes A de 5 à 7 chaînons substitués précédemment cités, les hétéroatomes servant éventuellement de substituants ne soient pas séparés des autres hétéroatomes par exactement un atome de carbone, ou
un groupe de formule générale 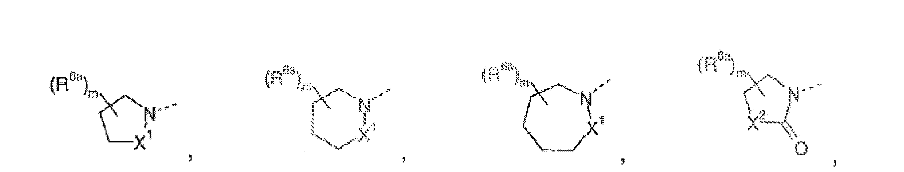 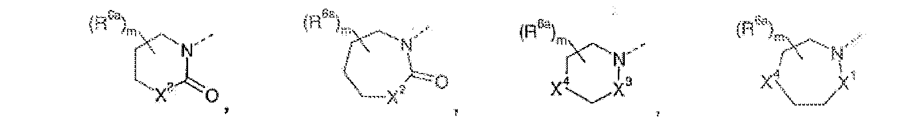 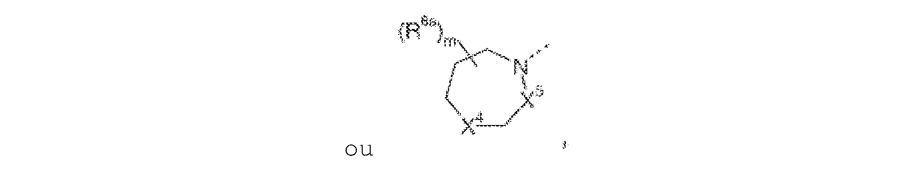 dans laquelle
m représente le nombre 1 ou 2,
X¹ représente un groupe méthylène, -NR^{6b}-, carbonyle ou sulfonyle,
X² représente un atome d'oxygène ou un groupe - NR^{6b}-,
X³ représente un groupe méthylène, carbonyle ou sulfonyle,
X⁴ représente un atome d'oxygène ou de soufre, un groupe NR^{6b},
X⁵ représente un groupe carbonyle ou sulfonyle,
les R^{6a}, indépendamment les uns des autres, représentent un atome d'hydrogène ou de fluor ou un groupe alkyle en C₁ à C₃,
les R^{6b}, indépendamment les uns des autres, peuvent être un atome d' hydrogène ou un groupe alkyle en C₁ à C₃, à condition que, dans les groupes A de 5 à 7 chaînons substitués précédemment cités, les hétéroatomes servant éventuellement de substituants ne soient pas séparés d'un autre hétéroatome par exactement un atome de carbone, ou
R¹ représente un atome de chlore ou de brome, un groupe méthyle ou méthoxy dans lesquels les atomes d' hydrogène peuvent être remplacés totalement ou partiellement par des atomes de fluor ou un groupe nitro,
R² représente un atome d'hydrogène,
R³ représente un groupe alkyle en C₁ à C₄ à chaîne linéaire ou ramifiée dans lequel les atomes d'hydrogène peuvent être remplacés totalement ou partiellement par des atomes de fluor et qui est substitué éventuellement par un groupe hydroxy, un groupe alkyloxy en C₁ à C₄, dans lequel les atomes d'hydrogène peuvent être remplacés totalement ou partiellement par des atomes de fluor, un groupe alkylsulfanyle en C₁ à C₃, alkylsulfonyle en C₁ à C₃, carboxy, ou alkyloxycarbonyle en C₁ à C₃,
un groupe phényle ou hétéroaryle, phényl-alkyle en C₁ à C₃, ou hétéroaryl-alkyle en C₁ à C₃ qui est substitué dans la partie phényle ou hétéroaryle, éventuellement une ou plusieurs fois par des atomes de fluor, de chlore ou de brome, un groupe alkyle en C₁ à C₃, alkyloxy en C₁ à C₄, mono-, di- ou trifluorométhoxy, carboxy ou alkyloxycarbonyle en C₁ à C₃,
R⁴ représente un atome d'hydrogène,
R⁵ représente un atome, d'hydrogène, et
B représente un groupe de formule 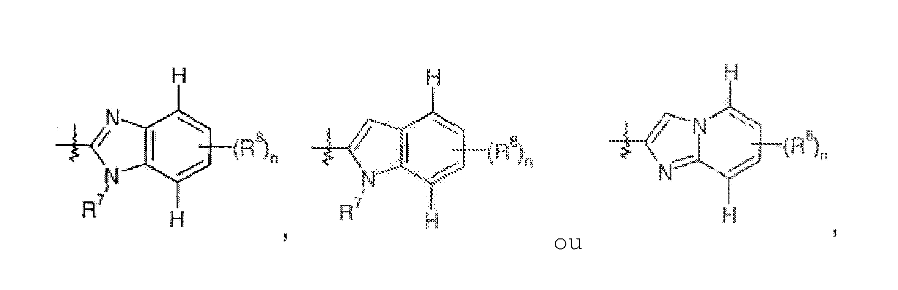 dans laquelle
n représente le nombre 1,
R⁷ représente un atome d'hydrogène, et
les R⁸ représentent un atome de chlore ou de brome ou le groupe éthinyle
où sauf indication contraire, l'expression « groupe hétéroaryle » mentionnée dans les définitions précédentes désigne un groupe hétéroaryle monocyclique de 5 ou 6 chaînons,
le groupe hétéroaryle à 6 chaînons comportant un, deux ou trois atomes d'azote, et
le groupe hétéroaryle à 5 chaînons comporte un groupe imino éventuellement substitué par un groupe alkyle en C₁ à C₃, un atome d'oxygène ou de soufre, ou
un groupe imino substitué éventuellement par un groupe alkyle en C₁ à C₃, ou un atome d'oxygène ou de soufre et en outre, un atome d'azote, ou
un groupe imino éventuellement substitué par un groupe alkyle en C₁ à C₃ et deux ou trois atomes d'azote,
et la liaison est réalisée par un atome d'azote ou par un atome de carbone,
où les groupes alkyle contenus dans les définitions précédemment citées qui présentent plus de deux atomes de carbone, sauf indication contraire, peuvent être à chaîne linéaire ou ramifiée et les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels.

7. Amides d'acide carboxylique substitués de formule générale I selon la revendication 6, dans laquelle
A, R¹ R², R⁴ R⁵ et B sont tels que définis dans la revendication 6, et
R³ représente un groupe alkyle en C₁ à C₄ à chaîne linéaire ou ramifiée dans lequel les atomes d'hydrogène peuvent être remplacés totalement ou partiellement par des atomes de fluor et qui est substitué éventuellement par un groupe hydroxy, un groupe alkyloxy en C₁ à C₄, dans lequel les atomes d'hydrogène peuvent être remplacés totalement ou partiellement par des atomes de fluor, un groupe alkylsulfanyle en C₁ à C₃, alkylsulfonyle en C₁ à C₃, carboxy ou alkyloxycarbonyle en C₁ à C₃,
les groupes alkyle et alcoxy contenus dans les définitions précédemment citées qui présentent plus de deux atomes de carbone, sauf indication contraire, peuvent être à chaîne linéaire ou ramifiée et les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels

8. Amides d'acide carboxylique substitués de formule générale I selon la revendication 6, dans laquelle
A, R¹, R², R⁴, R⁵ et B sont tels que définis dans la revendication 6, et
R³ représente un groupe phényle ou hétéroaryle, phényl-alkyle en C₁ à C₃ ou hétéro-aryl-alkyle en C₁ à C₃ qui est mono- ou poly-substitué dans la partie phényle ou hétéroaryle éventuellement par un atome de fluor, de chlore ou de brome, un groupe alkyle en C₁ à C₃, alkyloxy en C1 à C4, mono-, di- ou trifluorométhoxy, carboxy ou alkyloxycarbonyle en C₁ à C₃,
un groupe cycloalkyl de 3 à 7 chaînons dans lequel, dans la partie cyclique, un groupe méthylène peut être substitué par un groupe -NH éventuellement substitué par un groupe alkyle en C₁ à C₃ ou alkylcarbonyle en C₁ à C₃ ou un atome d'oxygène,
où, sauf indication contraire, l'expression « groupe hétéroaryle » mentionnée dans les définitions précédentes désigne un groupe hétéroaryle monocyclique de 5 ou 6 chaînons,
le groupe hétéroaryle à 6 chaînons comportant deux ou trois atomes d'azote, et
le groupe hétéroaryle à 5 chaînons comporte un groupe imino éventuellement substitué par un groupe alkyle en C₁ à C₃, un atome d'oxygène ou de soufre, ou
un groupe imino substitué éventuellement par un groupe alkyle en C₁ à C₃, ou un atome d'oxygène ou de soufre et en outre, un atome d'azote, ou
un groupe imino éventuellement substitué par un groupe alkyle en C₁ à C₃ ou deux ou trois atomes d'azote,
et la liaison est réalisée par un atome d'azote ou par un atome de carbone,
où les groupes alkyle contenus dans les définitions précédemment citées qui présentent plus de deux atomes de carbone, sauf indication contraire, peuvent être à chaîne linéaire ou ramifiée et les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement pair des atomes de fluor,
leurs tautomères, leurs énantiomères, leurs diastéréomères, leurs mélanges et leurs sels.

9. Amides d'acide carboxylique substitués de formule générale I selon la revendication 1, dans laquelle
A représente un groupe de formule générale 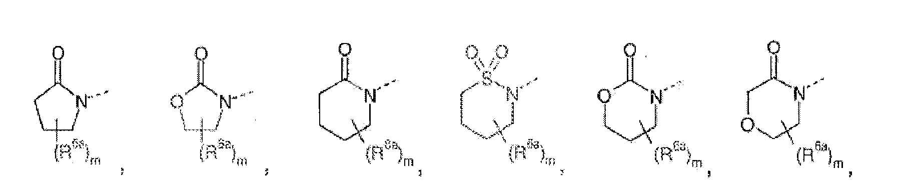 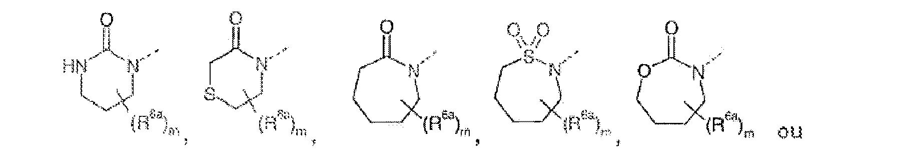 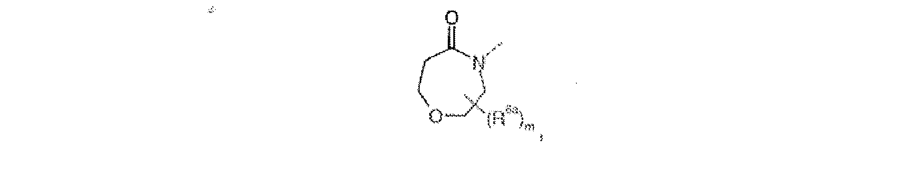 dans laquelle
m représente le nombre 1 ou 2,
es, indépendamment les uns des autres, représentent un atome d'hydrogène ou de fluor, un groupe alkyle en C₁ à C₃, à condition que, dans les groupes A de 5 à 7 chaînons substitués précédemment cités, les atomes de fluor servant éventuellement de substituants ne soient pas séparés d'un autre hétéroatome par exactement un atome de carbone,
R¹ représente un atome de chlore ou de brome, un groupe méthyle ou méthoxy dans lesquels les atomes d'hydrogène peuvent être remplacés totalement ou partiellement par des atomes de fluor ou un groupe nitro,
R² représente un atome d'hydrogène,
R₃ représente un groupe alkyle en C₁ à C₄ à chaîne linéaire ou ramifiée dans lequel les atomes d'hydrogène peuvent être remplacés totalement ou partiellement par des atomes de fluor et qui est substitué éventuellement par un groupe hydroxy, un groupe alkyloxy en C₁ à C₄, dans lequel les atomes d' hydrogène peuvent être remplacés totalement ou partiellement par des atomes de fluor, un groupe alkylsulfanyle en C₁ à C₃, alkylsulfonyle en C₁ à C₃, carboxy, ou alkyloxycarbonyle en C₁ à C₃,
un groupe furanyle, thiophényle, pyrrolyle, imidazolyle, oxazolyle, isoxazolyle, thiazolyle, pyridinyle, pyrimidinyle, pyrazinyle, pyridinyl-alkyle en C₁ à C₂ ou imidazolyl-alkyle en C₁ à C₂ qui est éventuellement substitué dans la partie hétèroaryle, par un ou deux groupes alkyle en C₁ à C₃, alkyloxy en C₁ à C₃, carboxy ou alkyloxycarbonyle en C₁ à C₃, et
R⁴ représente un atome d'hydrogène,
R⁵ représente un atome d'hydrogène, et
B représente un groupe de formule 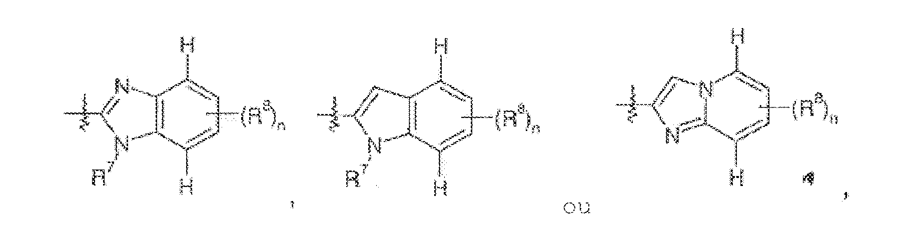 dans laquelle
n représente le nombre 1,
R⁷ représente un atome d'hydrogène, et
les R⁸ représentent un atome de chlore ou de brome ou un groupe éthinyle
où sauf indication contraire, l'expression « groupe hétéroaryle » mentionnée dans les définitions précédentes désigne un groupe hétéroaryle monocyclique de 5 ou 6 chaînons,
le groupe hétéroaryle à 6 chaînons comportant un, deux ou trois atomes d'azote, et
le groupe hétéroaryle à 5 chaînons comporte un groupe imino éventuellement substitué par un groupe alkyle en C₁ à C₃, un atome d'oxygéne ou de soufre, ou
un groupe imino substitué éventuellement par un groupe alkyl en C₁ à C₃, ou un atome d'oxygène ou de soufre et en outre, un atome d'azote, ou
un groupe imino éventuellement substitué par un groupe alkyle en C₁ à C₃ et deux ou trois atomes d'azote,
et la liaison est réalisée par un atome d'azote ou par uns atome de carbone,
où les groupes alkyle contenus dans les définitions précédemment citées qui présentent plus de deux atomes de carbone, sauf indication contraire, peuvent être à chaîne linéaire ou ramifiée et les groupes alkyle dans les radicaux dialkylés précédemment citées, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels.

10. Amides d'acide carboxylique substitués de formule générale 1 selon la revendication 9, dans laquelle
A, R¹, R², R⁴, R⁵ et B sont tels que définis dans la revendication 9, et
R³ représente un groupe alkyle en C₁ à C₄ à chaîne linéaire ou ramifiée dans lequel les atomes d'hydrogène peuvent être remplacés totalement ou partiellement par des atomes de fluor et qui est substitué éventuellement par un groupe hydroxy, un groupe alkyloxy en C₁ à C₄, dans lequel les atomes d'hydrogène peuvent être remplacés totalement ou partiellement par des atomes de fluor, un groupe alkylsulfanyle en C₁ à C₃, alkylsulfonyle en C₁ C₃, carboxy ou alkyloxycarbonyle en C₁ à C₃,
les groupes alkyle et alkoxy contenus dans les définitions précédemment citées qui présentent plus de deux atomes de carbone, sauf indication contraire, peuvent être à chaîne linéaire ou ramifiée et les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels.

11. Amides d'acide carboxylique substitués de formule générale I selon la revendication 9, dans laquelle
A, R¹, R², R⁴, R⁵ et B sont tels que définis dans la revendication 9, et
un groupe furanyle, thiophényle, pyrrolyle, pyrazolyle, imidazolyle, oxazolyle, vsoxazolyle, thiazolyle, pyridinyle, pyrimidinyle, pyrazinyle, pyridinyl-alkyle en C₁ a C₂ ou imidazolyl-alkyle en C₁ à C₂ qui est éventuellement substitué dans la partie hétéroaryle, par un ou deux groupes alkyl en C₁ à C₃, dans lequel les atomes d'hydrogène peuvent être remplacés totalement ou partiellement par des atomes de fluor, alkyloxy en C₁ à C₃, dans lesquels les atomes d'hydrogène peuvent être remplacés totalement ou partiellement par des atomes de fluor, carboxy ou alkyloxycarbonyle en C₁ à C₃, et
où, sauf indication contraire, l'expression « groupe hétéroaryle » mentionnée dans les définitions précédentes désigne un groupe hétéroaryle monocyclique de 5 ou 6 chaînons,
le groupe hétéroaryle à 6 chaînons comportant un, deux ou trois atomes d'azote, et
le groupe hétéroaryle à 5 chaînons comporte un groupe imino éventuellement substitué par un groupe alkyle en C₁ à C₃, un atome d'oxygène ou de soufre, ou
un groupe imino substitué éventuellement par un groupe alkyle en C₁ à C₃, ou un atome d'oxygène ou de soufre et en outre, un atome d'azote, ou
un groupe imino éventuellement substitué par un groupe alkyle en C₁ à C₃ ou deux ou trois atomes d'azote,
et la liaison est réalisée par un atome d'azote ou par un atome de carbone,
où les groupes alkyle contenus dans les définitions précédemment citées qui présentent plus de deux atomes de carbone, saut indication contraire, peuvent être à chaîne linéaire ou ramifiée et les groupes alkyle dans les radicaux dialkylés précédemment cités, par exemple les groupes dialkylamino peuvent être identiques ou différents,
et où les atomes d'hydrogène des groupes méthyle ou éthyle mentionnés dans les définitions précédentes peuvent être remplacés totalement ou partiellement par des atomes de fluor,
leurs tautomères, leurs énantiomères, leurs diastéréomères, leurs mélanges et leurs sels,

12. Amides d'acide carboxylique substitués de formule générale I selon l'une des revendications 1 à 7 ou 8, dans lesquels le groupe X¹ représente un groupe méthylène.

13. Amides d'acide carboxylique substitués de formule générale I selon l'une des revendications 1 à 8, dans lesquels le groupe X¹ représente un groupe carbonyle.

14. Amides d'acide carboxylique substitués de formule générale I selon l'une des revendications 1 à 8, 12 ou 13, dans lesquels le groupe X³ représente un groupe méthylène,

15. Amides d'acide carboxylique substitués de formule générale I selon l'une des revendications 1 à 8, 12 ou 13, dans lesquels le groupe X³ représente un groupe carbonyle

16. Amides d'acide carboxylique substitués de formule générale I selon l'une des revendications 1 à 8 ou 12 à 15, dans lesquels le groupe X⁴ représente un atome d'oxygène.

17. Amides d'acide carboxylique substitués de formule générale I selon l'une des revendications 1 à 16, dans lesquels le radical B représente le groupe

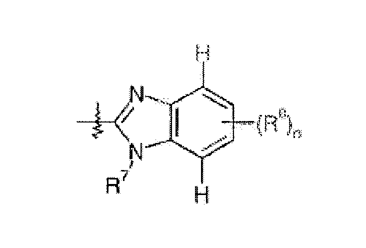

18. Amides d'acide carboxylique substitués de formule générale I selon l'une des revendications 1 à 16, dans lesquels le radical B représente le groupe

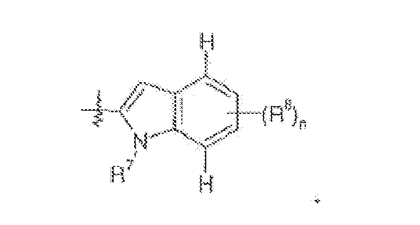

19. Amides d'acide carboxylique substitués de formule générale I selon l'une des revendications 1 à 16, dans lesquels le radical B représente le groupe

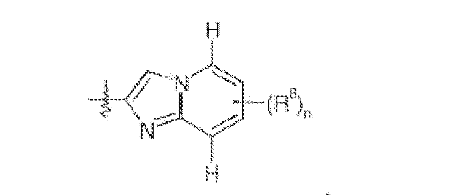

20. Amides d'acide carboxylique substitués de formule générale I selon l'une des revendications 1 à 19, dans lesquels le radical R⁸ représente un atome de chlore.

21. Amides d'acide carboxylique substitués de formule générale I selon l'une des revendications 1 à 19, dans lesquels le radical R⁸ représente un atome de brome.

22. Amides d'acide carboxylique substitués de formule générale I selon l'une des revendications 1 à 19, dans lesquels le radical R⁸ représente un groupe éthinyle.

23. Amides d'acide carboxylique substitués de formule générale I selon l'une des revendications 1 à 22, qui correspondent à la formule générale la

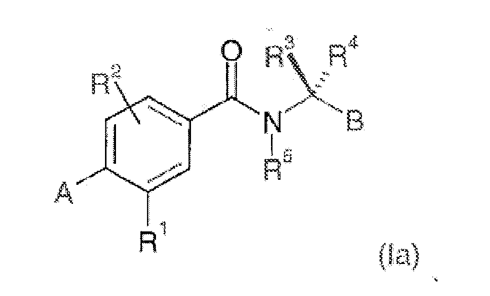

24. Composés suivants de formule générale I selon la revendication 1:
(1) 4-(azépan-2-on-1-yl)-N-[(1R)-1-(5-chloro-1H-benzimidazol-2-yl)-2-méthoxy-éthyl]-3-méthyl-benzamide,
(2) 4-(azèpan-2-on-l-yl)-N-[(1R)-1-(5-chloro-1H-benzimidazol-2-yl)-2-hydroxy-éthyl]-3-méthyl-benzamide,
(3) N-[(1S)-1-(5-chloro-1H-benzimidazol-2-yl)-3-méthylsulranyl-propyl]-3-méthyl-4-(pyrrolidin-2-on-1-yl)-benzamide
(4) N-[(1R)-1-(5-chloro-1H-benzimidazol-2-yl)-2-méthoxy-éthyl]-4-(morpholin-3-on-4-yl)-3-trifluorométhyl-benzamide
(5) N-[(1R)-1-(5-chloro-1H-benzimidazol-2-yl)-2-méthoxy-éthyl]-3-méthyl-4-(morpholin-3-on-4-yl)-benzamide,
(6) N-[(1S)-1-(5-chloro-1H-benzimidazol-2-yl)-3-méthylsulfanyl-propyl]-3-méthyl-4-(pyrrolidin-3-on-4,
(7) N-[(1R)-1-(5-chloro-1H-benzimidazol-2-yl)-2-hydroxy-éthyl]-3-méthyl-4-(morpholin-3-on-4-yl)-benzamide
(8) 4-(azépan-2-on-1-yl)-3-chloro-N-[(1R)-1-(5-chloro-1H-benzimidazol-2-yl)-2-méthoxy-éthyl]-benzamide,
(9) 4-(azépan-2-on-1-yl)-N-[(1R)-1-(5-chloro-1H-benzimidazol-2-yl)-2-méthoxy-éthyl]-3-trifluorométhyl-benzamide,
(10) N-[(1S)-1-(5-chloro-1H-benzimidazol-2-yl)-éthyl]-3-méthyl-4-([1,3]oxazépan-2-on-3-yl)-benzamide,
(11) N-[(1S)-1-(5-chloro-1H-benzimidazol-2-yl)-3-méthylsulfanyl-propyl]-4-(morpholin-3-on-4-yl)-3-nitro-benzamide,
(12) N-[(1R)-1-(5-chloro-1H-benzimidazol-2-yl)-2-méthoxy-éthyl]-3-méthyl-4-([1,3]oxazépan-2-on-3-yl)-benzamide,
(13) 3-chloro-N-[(1R)-1-(5-chloro-1H-benzimidazol-2-yl)-2-méthoxy-éthyl]-4-([1,4]oxazépan-5-on-4-yl)-benzamide,
(14) 3-chloro-N-[(1R)-1-(5-chloro-1H-benzimidazol-2-yl)-2-méthoxy-éthyl]-4-(pipéridin-2-on-1-yl)-benzamide,
(15) N-[(1R)-1-(5-bromo-1H-benzimidazol-2-yl)-2-hydroxy-éthyl]-3-méthyl-4-(morpholin-3-on-4-yl)-benzamide,
(16) N-[(1R, 2S)-1-(5-chloro-1H-benzimidazol-2-yl)-2-méthoxy-propyl]-3-méthyl-4-(morpholin-3-on-4-yl)-benzamide,
(17) N-[(1S)-1-(5-chloro-1H-benzimidazol-2-yl)-éthyl]-3-méthyl-4-(thiomorpholin-3-on-4-yl)-benzamide,
(18) N-[(1R)-1-(5-chloro-1H-benzimidazol-2-yl)-2-méthoxy-éthyl]-3-méthyl-4-(pipéridin-2-on-1-yl)-benzamide,
(19) N-[(1R)-1-(5-chloro-1H-benzimidazol-2-yl)-2-méthoxy-éthyl]-3-méthyl-4-([1,3]-oxazinan-2-on-3-yl)-benzamide,
(20) N-[(1R)-1-(5-chloro-1H-benzimidazol-2-yl)-2-méthoxy-éthyl]-4-(pipéridin-2-on-1-yl)-3-trifluorométhyl-benzamide,
(21) N-[(1R)-1-(5-chloro-1H-benzimidazol-2-yl)-2-méthoxy-éthyl]-4-(1,1-dioxo-[1,2]thiazinan-2-yl)-3-méthyl-benzamide,
(22) N-[(1S)-1-(5-chloro-1H-benzimidazol-2-yl)-butyl]-3-méthyl-4-(morpholin-3-on-4-yl)-benzamide,
(23) N-[(1R)-1-(5-chloro-1H-benzimidazol-2-yl)-2-méthoxy-éthyl]-4-([1,3]oxazépan-2-on-3-yl)-3-trifluorométhyl-benzamide,
(24) N-[(1R)-1-(5-chloro-1H-benzimidazol-2-yl)-2-méthoxy-éthyl]-4-(1,1-dioxo-[1,2,6]thiadiazinan-2-yl)-3-méthyl-benzamide,
(25) N-[(1R)-1-(5-bromo-1H-benzimidazol-2-yl)-2-méthoxy-éthyl]-3-méthyl-4-(morpholin-3-on-4-yl)-benzamide,
(26) N-[(1S)-1-(5-chloro-1H-benzimidazol-2-yl)-3-(1H-tétrazol-5-yl)-propyl]-3-méthyl-4-(morpholin-3-on-4-yl)-benzamide,
(27) N-[(1S)-1-(5-chloro-1H-benzimidazol-2-yl)-3-méthoxy-propyl]-3-méthyl-4-(morpholin-3-on-4-yl)-benzamide,
(28) N-[(1R)-1-(5-chloro-1H-benzimidazol-2-yl)-2-méthylsulfanyl-éthyl]-3-méthyl-4-(morpholin-3-on-4-yl)-benzamide,
(29) N-[(1R)-1-(5-chloro-1H-benzimidazol-2-yl)-2-méthoxy-éthyl]-3-méthyl-4-(thiomorpholin-3-on-4-yl)-benzamide,
(30) 3-chloro-N-[(1R)-1-(5-chloro-1H-benzimidazol-2-yl)-2-méthoxy-éthyl]-4-(morpholin-3-on-4-yl)-benzamide,
(31) 3-chloro-N-[(1R)-1-(5-chloro-1H-benzimidazol-2-yl)-2-méthoxy-éthyl]-4-(1,1-dioxo-[1,2]thiazinan-2-yl)-benzamide,
(32) N-[(1R)-1-(5-chloro-1H-benzimidazol-2-yl)-2-méthoxy-éthyl]-4-(1,1-dioxo-[1,2]thiazépan-2-yl)-3-méthyl-benzamide,
(33) 3-bromo-N-[(1S)-1-(5-chloro-1H-benzimidazol-2-yl)-éthyl]-4-(pipéridin-2-on-1-yl)-benzamide,
(34) 3-bromo-N-[(1R)-1-(5-chloro-1H-benzimidazol-2-yl)-2-méthoxy-éthyl]-4-(pipéridin-2-on-1-yl)-benzamide et
(35) N-[1-(5-chloro-1H-benzimidazol-2-yl)-1-(furan-2-yl)-méthyl]-3-méthyl-4-(pipéridin-2-on-1-yl)-benzamide,
leurs tautomères, leurs énantiomères, leurs diastéréomères, leurs mélanges et leurs sels sont particulièrement préférés.

25. Sels physiologiquement acceptables des composés selon les revendications 1 à 24.

26. Médicament contenant un composé selon au moins l'une des revendications 1 à 24 ou sel physiologiquement acceptable selon la revendication 25, et éventuellement un ou plusieurs véhicules et/ou diluants.

27. Utilisation d'un composé selon au moins l'une des revendications 1 à 24, ou d'un sel physiologiquement acceptable selon la revendication 25 pour la production d'un médicament présentant un effet inhibiteur sur le facteur Xa et/ou un effet inhibiteur sur des sérine-protéases utilisées.

28. Procédé de production d'un médicament selon la revendication 26, **caractérisé en ce que**, de façon non chimique, un composé selon au moins l'une des revendications 1 à 24 ou un sel physiologiquement acceptable selon la revendication 25 est intégré dans un ou plusieurs véhicules et/ou diluants.
